# EUROPEAN PATENT APPLICATION

(11) **EP 4 455 133 A1**
(43) Date of publication of application: **30.10.2024**
(21) Application number: 22910146.4
(22) Date of filing: 22.12.2022
(51) Int. Cl.: C07D 401/14, A61K 31/4035, A61K 31/444, A61K 31/4427, A61K 31/4545, A61K 31/4523, A61K 31/506, A61K 31/515, A61K 31/4188, A61K 31/4196, A61K 31/343, A61K 31/517, A61P 35/00, A61P 31/00, A61P 31/18, A61P 31/14, A61P 31/20, A61P 37/00

(54) **MULTI-PROTEIN DEGRADATION AGENT HAVING IMIDE SKELETON**

(30) Priority: 24.12.2021 CN 202111599314
(71) Applicant: Suzhou Kintor Pharmaceuticals, Inc., Jiangsu 215123 (CN)
(72) Inventor: YANG, Zhaohui, Suzhou, Jiangsu 215123 (CN); LIU, Songying, Suzhou, Jiangsu 215123 (CN); PAN, Jie, Suzhou, Jiangsu 215123 (CN); CHEN, Dong, Suzhou, Jiangsu 215123 (CN); XU, Ruo, Suzhou, Jiangsu 215123 (CN); MA, Liandong, Suzhou, Jiangsu 215123 (CN); TONG, Youzhi, Suzhou, Jiangsu 215123 (CN)
(74) Representative: HGF
(86) International application number: PCT/CN2022/141113
(87) International publication number: WO 2023/116835

(57) **Abstract**

Provided is a multi-protein degradation agent having an imide skeleton, which can degrade multiple proteins comprising c-Myc, GSPT1, CK1α and IKZF (1/2/3) proteins, and therefore can be used for treating diseases or disorders related thereto, such as cancer.

## Description

### Cross-Reference to Related Applications

The present application claims the benefit of Chinese Patent Application No. 2021115993144 filed on December 24, 2021. The application number 2021115993144 is hereby incorporated by reference in its entirety.

### Technical Field

The present invention relates to the field of medicine. Specifically, the present invention relates to a multi-protein degradation agent having imide skeleton.

### Background Art

Protein synthesis and degradation are strictly regulated in time and space. Aberrant protein function or protein imbalance is hallmark of many diseases. The ubiquitin-proteasome system (UPS) is an important system for maintaining protein balance. Targeted protein degradation (TPD) which include protein proteolysis-targeting chimeras (PROTACs) and molecular glue, uses intracellular UPS to degrade disease-related proteins to treat diseases. Compared to Protac compounds, molecular glue compounds have several advantages like smaller molecular weight, more drug-like and no hook effect (Dong, Guoqiang, et al. Journal of Medicinal Chemistry 64.15 (2021): 10606-10620) . So it has attracted more attention.

The MYC family includes c-Myc, N-Myc and L-Myc, which belongs basic helix-loop-helix (bHLH) leucine zipper transcription factors family. Among them, c-Myc can regulate various biological functions, such as cell proliferation, apoptosis, cell cycle progression, cell metabolism and embryonic development, and plays a very important role in the occurrence, development and evolution of diseases. A large number of studies have shown that c-Myc is closely related to various tumor types, including lymphoma, breast cancer, prostate cancer, colon cancer, cervical cancer, multiple myeloma, myeloid leukemia, melanoma, osteosarcoma, malignant glioma, osteosarcoma, small cell lung cancer and medulloblastoma. c-Myc can promote occurrence and growth of tumors in many aspects (C Yu, et al. Scientific Reports 6.1 (2016): 1-11).

GSPT1 (G1 to S phase transition 1) mediates the recognition of stop codons and promotes the release of transcribed peptide chains from ribosomes. In addition to playing a role in the transcription termination process, GSPT1 is also closely associated with a variety of important cellular activities, such as cell cycle regulation, cytoskeleton formation and apoptosis. GSPT1 is considered to be an oncogenic factor for various tumors, including breast cancer, liver cancer, gastric cancer and prostate cancer (Cui, Jian, et al. International Journal of Oncology, 2020, 56(4): 867-878).

Casein kinase 1α (CK1α) can phosphorylate p53 protein. As a tumor suppressor protein, p53 protein participates in various signal transductions within cells and plays an important role in processes such as cell cycle regulation and apoptosis. Phosphorylated p53 can bind to murine double minute 2 (MDM2) and then be degraded by ubiquitination (Huart, Anne-Sophie, et al. Journal of Biological Chemistry 284.47 (2009): 32384-32394). Therefore, blocking CK1α activity can stabilize p53, thereby exerting its tumor suppressor activity.

The zinc finger transcription factors IKZF1/2/3 (Aiolos, Helios and Ikaros) belong to the Ikaros zinc-finger (IKZF) family, and are essential for the survival of lymphoid cells. For example, Aiolos can bind to the enhancer of B-cell lymphoma-2 (Bcl-2), thereby upregulating the effect of Bcl-2 protein on cell survival. Moreover, abnormally activated Helios and Ikaros can upregulate the expression of Bcl-XL and drive the occurrence and development of various hematological tumors.

In summary, it has become a new therapeutic strategy to develop a novel compound that can use the ubiquitin-proteasome system to degrade important proteins in the survival process of tumor cells and inhibit tumor growth in multi-manners.

### Summary of the invention

The object of the present invention is to provide a multi-protein degradation agent having imide skeleton.

Therefore, in one aspect, the present invention provides a compound of formula (I₀) or a pharmaceutically acceptable salt, solvate, hydrate, stereoisomer, tautomer, or prodrug thereof: 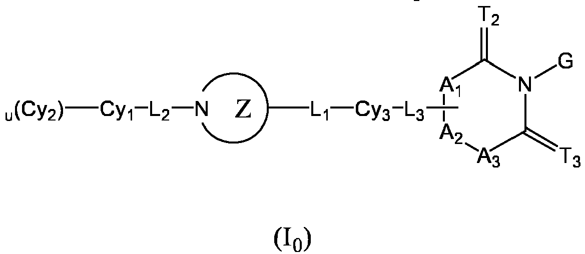 wherein,
G is selected from: H or C₁₋₆ alkyl, wherein the C₁₋₆ alkyl is optionally substituted with a group selected from the following: hydroxyl, -OPO(OR^{G})₂, - OCOR^{G}, carboxyl, -OS(O)₁₋₂R^{G}, and sulfo, wherein each R^{G} is independently selected from: H, or C₁₋₄ alkyl;
T₂-T₃ are independently selected from: O or S;
A₁-A₂ are independently selected from: C(R^{A})₂ or NR^{A'}, wherein each R^{A} is independently selected from: H, halogen, C₁₋₁₅ alkyl optionally substituted with halogen, C₃₋₁₅ cycloalkyl, -CONR'R", -COOR', -OR ', or -NR'R", and R^{A'} is selected from: H, C₁₋₁₅ alkyl optionally substituted with halogen, C₃₋₁₅ cycloalkyl, or -COR';
A₃ is selected from: a bond, or C(R^{A})₂, wherein each R^{A} is independently selected from: H, halogen, C₁₋₁₅ alkyl optionally substituted with halogen, C₃₋₁₅ cycloalkyl, -CONR'R", -COOR', -OR', or -NR'R";
R' and R" are independently selected from: H, C₁₋₁₅ alkyl optionally substituted with halogen, or C₃₋₆ cycloalkyl;
L₃ is selected from: a bond, or a group represented by the structure of the following general formula:

   -(CHR¹)ₖ-;

   -(CHR¹)ₘ-O-(CHR¹)ₙ-;

   -(CHR¹)ₘ-N(R¹)-(CHR¹)ₙ-;

   -(CHR¹)ₘ-CO-(CHR¹)ₙ-;

   -(CHR¹)ₘ-CO-N(R¹)-(CHR¹)ₙ-;

   -(CHR¹)ₘ-N(R¹)-CO-(CHR¹)ₙ-;

   -(CHR¹)ₘ-CO-O-(CHR¹)ₙ-;

   -(CHR¹)ₘ-O-CO-(CHR¹)ₙ-;

   -(CHR¹)ₘ-SO₂-N(R¹)-(CHR¹)ₙ-;

   -(CHR¹)ₘ-N(R¹)-SO₂-(CHR¹)ₙ-;
or substituted or unsubstituted triazolylidene;
wherein k is selected from 0, 1, 2, 3, 4, 5, 6, 7, 8, 9, 10, 11, and 12, m and n are independently selected from: 0, 1, 2, 3, 4, 5, and 6, each R¹ is independently selected from: H, or C₁₋₆ alkyl optionally substituted with halogen;
Cy₃ is selected from optionally substituted aryl, optionally substituted heteroaryl, optionally substituted heterocyclyl, optionally substituted biaryl or optionally substituted biheteroaryl;
L₁ represents a linking group;
ring Z represents an optionally substituted heterocycloalkyl containing at least one N atom as a heteroatom, and is linked to L₂ through the N;
L₂ is selected from: a bond, or a group represented by the structure of the following general formula:

   -(CHR¹)ₖ-;

   -(CHR¹)ₘ-O-(CHR¹)ₙ-;

   -(CHR¹)ₘ-N(R¹)-(CHR¹)ₙ-;

   -(CHR¹)ₘ-CO-(CHR¹)ₙ-;

   -(CHR¹)ₘ-CO-N(R¹)-(CHR¹)ₙ-;

   -(CHR¹)ₘ-N(R¹)-CO-(CHR¹)ₙ-;
wherein k is selected from 0, 1, 2, 3, 4, 5, 6, 7, 8, 9, 10, 11, and 12, m and n are independently selected from: 0, 1, 2, 3, 4, 5, and 6, each R¹ is independently selected from: H, or C₁₋₆ alkyl optionally substituted with halogen;
Cy₁ is selected from: optionally substituted aryl, optionally substituted heteroaryl, optionally substituted heterocyclyl, optionally substituted biaryl or optionally substituted biheteroaryl;
Cy₂ is selected from H, optionally substituted aryl, optionally substituted heteroaryl, optionally substituted heterocyclyl, optionally substituted biaryl or optionally substituted biheteroaryl;
u is selected from 0, 1, 2, 3, 4, and 5.

Preferably, the present invention provides a compound of formula (I) or a pharmaceutically acceptable salt, solvate, hydrate, stereoisomer, tautomer, or prodrug thereof 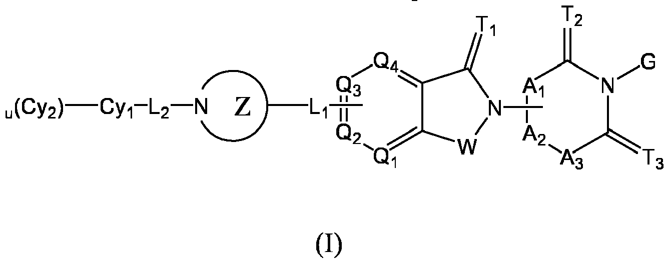 wherein,
W is selected from: C(R^{W})₂, C=O, C=S, NH or -N-C₁₋₆ alkyl;
R^{W} is selected from: H, halogen, cyano, nitro, C₁₋₁₅ alkyl optionally substituted with halogen, C₃₋₁₅ cycloalkyl, -CONR'R", -COOR', -OR', or -NR'R";
G is selected from: H or C₁₋₆ alkyl, wherein the C₁₋₆ alkyl is optionally substituted with a group selected from the following: hydroxyl, -OPO(OR^{G})₂, - OCOR^{G}, carboxyl, -OS(O)₁₋₂R^{G}, and sulfo, wherein each R^{G} is independently selected from: H, or C₁₋₄ alkyl;
T₁-T₃ are independently selected from: O or S;
A₁-A₃ are independently selected from: C(R^{A})₂, wherein each R^{A} is independently selected from: H, halogen, C₁₋₁₅ alkyl optionally substituted with halogen, C₃₋₁₅ cycloalkyl, -CONR'R", -COOR', -OR', or -NR'R";
Q₁-Q₄ are independently selected from: CR^{Q} or N;
R^{Q} is selected from: H, halogen, cyano, nitro, C₁₋₁₅ alkyl optionally substituted with halogen, C₃₋₁₅ cycloalkyl, -CONR'R", -COOR', -OR', or -NR'R";
R' and R" are independently selected from: H, C₁₋₁₅ alkyl optionally substituted with halogen, or C₃₋₆ cycloalkyl;
L₁ represents a linking group;
ring Z represents an optionally substituted heterocycloalkyl containing at least one N atom as a heteroatom, and is linked to L₂ through the N;
L₂ is selected from: a bond, or a group represented by the structure of the following general formula:

   -(CHR¹)ₖ-;

   -(CHR¹)ₘ-O-(CHR¹)ₙ-;

   -(CHR¹)ₘ-N(R¹)-(CHR¹)ₙ-;

   -(CHR¹)ₘ-CO-(CHR¹)ₙ-;

   -(CHR¹)ₘ-CO-N(R¹)-(CHR¹)ₙ-;

   -(CHR¹)ₘ-N(R¹)-CO-(CHR¹)ₙ-;
wherein k is selected from 0, 1, 2, 3, 4, 5, 6, 7, 8, 9, 10, 11, and 12, m and n are independently selected from: 0, 1, 2, 3, 4, 5, and 6, each R¹ is independently selected from: H, or C₁₋₆ alkyl optionally substituted with halogen;
Cy₁ is selected from: optionally substituted aryl, optionally substituted heteroaryl, optionally substituted heterocyclyl, optionally substituted biaryl or optionally substituted biheteroaryl;
Cy₂ is selected from H, optionally substituted aryl, optionally substituted heteroaryl, optionally substituted heterocyclyl, optionally substituted biaryl or optionally substituted biheteroaryl;
u is selected from 0, 1, 2, 3, 4, and 5.

Another aspect of the present invention provides a pharmaceutical composition comprising an effective amount of a compound of the present invention and a pharmaceutically acceptable carrier and/or excipient.

Another aspect of the present invention provides a combination drug comprising an effective amount of a compound of the present invention and at least one additional bioactive agent.

Another aspect of the present invention provides a therapeutic composition comprising an effective amount of at least two different compounds of the present invention.

Another aspect of the present invention provides the use of the compound of the present invention in the preparation of a protein degradation agent.

Another aspect of the present invention provides the use of the compound of the present invention in the preparation of a drug for preventing or treating a disease or a disorder associated with protein degradation.

Another aspect of the present invention provides a method of treating a disease or a disorder in a subject, comprising the steps of administering the compound of the present invention to the subject in need thereof.

Another aspect of the present invention provides the use of the compound of the present invention in the preparation of a proteolysis-targeting chimera (Protac) or an antibody-drug conjugate (ADC).

Another aspect of the present invention provides a method for preparing the compound of the present invention.

Beneficial effects of the present invention are as follows:

The compound of the present invention not only has excellent ability to inhibit tumor cell proliferation (IC₅₀ for HL-60 is at nmol level), but also can degrade many proteins including c-Myc, GSPT1, CK1α and IKZF (1/2/3), and has better degradation ability than the clinical compound CC-90009. In addition, the compound of the present invention has excellent bioavailability and can be administered orally.

### Brief Description of the Drawings

Fig. 1 shows the degradation of myc and GSPT1 proteins in HL60 cells by compounds CC-90009, A62 and A80;
Fig. 2 shows the degradation of myc and GSPT1 proteins in HL60 cells by compounds A146, A169 and A145;
Fig. 3 shows the degradation of myc and GSPT1 proteins in HL60 cells by compounds A131, A134 and A162.

### Detailed Description of Embodiments

### Definitions

Unless defined otherwise, the terms used herein have the same meanings as commonly understood by those of ordinary skill in the art to which the present invention pertains. The terms used in the specification are merely used to describe specific embodiments, and are not intended to limit the present invention.

Herein, " 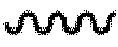 " indicates a connection point for connecting moieties.

Herein, "*" indicates that the carbon atom may have chirality, that is, the carbon atom may be in R configuration or S configuration.

Herein, A is selected from MH and B is linked to A, which means that B replaces H in MH to form a B-M bond; For example, "Q₁ is selected from CH and L₁ is linked to Q₁" means that L₁ and CH in Q₁ form L₁-C bond. And so on.

The term "alkyl" refers to a linear or branched fully saturated hydrocarbon group, preferably C1-15, more preferably C1-12, C1-6 or C1-4 alkyl. Examples of alkyl include methyl, ethyl, n-propyl, isopropyl, n-butyl, sec-butyl, isobutyl, tert-butyl, pentyl (such as n-pentyl), hexyl (such as n-hexyl), heptyl (such as n-heptyl), octyl (such as n-octyl), nonyl (such as n-nonyl), decyl (such as n-decyl), and the like. Alkyl may be optionally substituted.

The term "cycloalkyl" refers to a C3-20 monocyclic or polycyclic alkyl, preferably a C3-15 monocyclic or polycyclic alkyl, and most preferably a C3-10 or C3-6 monocyclic or polycyclic alkyl. Examples of cycloalkyl include cyclopropyl, cyclobutyl, cyclopentyl, cyclohexyl, cycloheptyl, and the like. Cycloalkyl may be optionally substituted.

The term "aryl" refers to an optionally substituted C6-18 aromatic group having a single ring (such as phenyl) or a fused ring (such as naphthyl, anthracenyl, phenanthrenyl, etc.), preferably C6-14 , more preferably C6-10 aromatic groups. Aryl may be optionally substituted.

The term "heteroaryl" refers to a 5- to 18-membered monocyclic or fused aromatic group, preferably a 5- to 10-membered, more preferably a 5- to 7-membered, 5- to 6-membered aromatic group, which may be optionally substituted and contains at least one (e.g. 1, 2, 3, 4 or 5) heteroatom (e.g. N, O, S or P). Examples of heteroaryl include furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl (such as 2-pyridyl, 3-pyridyl or 4-pyridyl), pyrimidinyl (such as 2-pyrimidinyl, 4-pyrimidinyl or 5-pyrimidinyl), pyridazinyl, pyrazinyl, triazinyl, indolyl, isoindolyl, benzoimidazolyl, benzopyrazolyl, benzofuranyl, benzothienyl, benzoxazolyl, benzothiazolyl, carbazolyl (including carbazol-9-yl), azacarbazolyl (including azacarbazol-9-yl), quinolinyl, isoquinolinyl, indolizinyl, azaindolizinyl, dibenzofuranyl, dibenzothienyl, naphthyridinyl; In addition, the case where the ring carbon atoms in the above groups are replaced by nitrogen is also included. Heteroaryl may be optionally substituted.

The term "heterocyclyl" refers to a 3- to 18-membered cyclic group, preferably 3- to 10-membered or 3- to 7-membered or 3- to 6-membered nonaromatic cyclic group, which may contain at least one (e.g., 1, 2, 3, 4 or 5) heteroatom (e.g., N, O, S or P) and may be fully saturated or partially saturated. Examples of heterocyclyl include: azetidinyl, oxacyclobutyl, pyrrolidinyl, pyrrolinyl, pyrazolidinyl, pyrazolinyl, imidazolidinyl, imidazolinyl, oxazolidinyl, oxazolinyl, isoxazolidinyl, isoxazolinyl, thiazolidinyl, thiazolinyl, isothiazolidinyl, isothiazolinyl, dihydrofuranyl, dihydrothienyl, tetrahydrofuranyl, tetrahydrothienyl, dioxacyclopentyl (such as 1,3-dioxacyclopentyl), dithiacyclopentyl, oxathiacyclopentyl, oxanyl (such as 1,3-dioxanyl, 1,4-dioxanyl), thianyl (such as 1,3-dithianyl, 1,4-dithianyl), dihydropyranyl, piperidinyl, morpholinyl, thiomorpholinyl, piperazinyl, N-methylpiperazinyl, dithianyl, hexahydropyridazinyl, hexahydropyrimidinyl, homopiperidinyl, homopiperazinyl, tetrahydroquinolyl (such as 1,2,3,4-tetrahydroquinolyl, including 1,2,3,4-tetrahydroquinolyl-1-yl), indolinyl, pyridonyl, 2-pyrrolidonyl, 1,4-benzodioxanyl, 1,3-benzodioxolyl, ethyleneureido, phthalimidyl, succinimidyl, etc. Heterocyclyl may be optionally substituted.

The term "biaryl" refers to two or more aryl groups linked by a single bond. Biaryl may be optionally substituted.

The term "biheteroaryl" refers to a heteroaryl linked by a single bond to at least one heteroaryl or aryl or a combination thereof. Biheteroaryl may be optionally substituted.

The terms "aralkyl" and "heteroarylalkyl" refer to a group which contains aryl-alkyl- or heteroaryl-alkyl-, respectively, and is linked to other groups through the alkyl.

The term "heterocycloalkyl" refers to a 3- to 18-membered monocyclic or polycyclic alkyl, preferably a 3- to 15-membered monocyclic or polycyclic alkyl, most preferably a 3- to 10-membered, 3- to 7-membered or 3- to 6-membered monocyclic or polycyclic alkyl in which at least one (e.g. 1, 2, 3, 4 or 5) ring carbon atoms in its cyclic structure is replaced by heteroatoms selected from the group consisting of N, O, S or P. Examples of heterocycloalkyl include: azetidinyl, oxacyclobutyl, pyrrolidinyl, pyrazolidinyl, imidazolidinyl, oxazolidinyl, isoxazolidinyl, thiazolidinyl, isothiazolidinyl, tetrahydrofuranyl, tetrahydrothienyl, dioxacyclopentyl (such as 1,3-dioxacyclopentyl), dithiacyclopentyl, oxathiacyclopentyl, oxanyl (such as 1,3-dioxanyl, 1,4-dioxanyl), thianyl (such as 1,3-dithianyl, 1,4-dithianyl), piperidinyl, morpholinyl, thiomorpholinyl, piperazinyl, dithianyl, hexahydropyridazinyl, hexahydropyrimidinyl, homopiperidinyl, homopiperazinyl, azabicyclo[3.2.1]octyl, azabicyclo[3.1.1]octyl, etc. Heterocycloalkyl may be optionally substituted.

The term "ylene" refers to the general name of a divalent group left after one hydrogen atom is further removed from the group. For example, "alkylene" refers to the general name of a divalent group left after one hydrogen atom is further removed from alkyl, and so on.

The term "amine group" refers to -N(R^{N'})₂, wherein R^{N'} is selected from H, alkyl, preferably H, and C1-6 alkyl. The amine group includes amino, alkylamino, dialkylamino, etc., and is preferably amino, C₁₋₆ alkylamino, and di(C₁₋₆ alkyl)amino.

The term "halogen" refers to F, Cl, Br or I.

The term "unsubstituted" means substitution only with a hydrogen atom.

The term "substituted" or "optionally substituted" refers to the independent presence of one or more substituents at any carbon (or nitrogen) position of the molecule/group, preferably 1, 2, 3, 4 or 5 substituents, most preferably 1, 2 or 3 substituents, the substituents may be: deuterium, halogen, hydroxyl, thiol, carboxyl, cyano, nitro, amino, optionally substituted alkyl, optionally substituted cycloalkyl, optionally substituted alkoxy, optionally substituted alkylthio, optionally substituted aryl, optionally substituted aryloxy, optionally substituted arylthio, optionally substituted heteroaryl, optionally substituted heterocyclyl, optionally substituted acyl, alkylamino, dialkylamino, optionally substituted amido, optionally substituted ester group, etc. Preferably, the substituents may be: deuterium, halogen (preferably, 1, 2 or 3 halogen), hydroxyl, thiol, carboxyl, cyano, nitro, C₁₋₁₀ alkyl (preferably, C₁₋₆ alkyl), haloC₁₋₆ alkyl (preferably, CH₂F, CHF₂, CF₃), C₃₋₇ cycloalkyl, C₁₋₆ alkoxy (preferably, methoxy, ethoxy), haloalkoxy (preferably, CF₃O), C₆₋₁₀ aryloxy, C₁₋₆ alkyl-S-, C₆₋₁₀ aryl-S-, C₆₋₁₀ aryl (especially phenyl and substituted phenyl, such as tolyl, trimethylphenyl and pentadeuterated phenyl), 5- to 7-membered heteroaryl, 5- to 7-membered heterocyclyl, C₁₋₆ acyl, C₁₋₆ alkylamino, di(C₁₋₆ alkyl)amino, amido substituted with one or two C₁₋₆ alkyl (including formamide substituted with one or two C₁₋₆ alkyl), C₁₋₆ ester group, etc. Preferably, the substituents may be: halogen, hydroxyl, carboxyl, cyano, nitro, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, cyclohexyl, phenyl, naphthyl, pyridyl, pyrimidinyl, -COOMe, - COOEt, -COMe, -COEt, amino, methylamino, dimethylamino, ethylamino, diethylamino, -CONH₂, -CONHMe, -CONHEt, -CON(Me)₂, -CON(Et)₂, etc.

The term treatment and other similar synonyms include relieving, alleviating or improving the symptoms of a disease or disorder, preventing other symptoms, improving or preventing the underlying metabolic causes leading to symptoms, inhibiting a disease or disorder, for example, stopping the development of a disease or disorder, relieving the disease or disorder, making improvement of the disease or disorder, relieving the symptoms caused by the disease or disorder, or stopping the symptoms of the disease or disorder. In addition, the term includes the purpose of prevention. The term also includes obtaining therapeutic and/or prophylactic effects. The therapeutic effect refers to the cure or improvement of the underlying disease being treated. Additionally, the cure or improvement of one or more physiological symptoms related to the underlying disease is also a therapeutic effect, for example, although the patient may still be affected by the underlying disease, the improvement of the patient's condition is observed. In terms of prophylactic effect, the composition can be administered to a patient who is at risk of suffering from a specific disease, or to a patient who has one or more physiological symptoms of the disease even though a diagnosis of the disease has not been made.

The term "effective" may mean, but is not limited to, the amount/dosage of the active pharmaceutical ingredient, which, when used in the context of its intended use, achieves or is sufficient for prevention of, inhibition of occurrence of, improvement, delay or treatment of symptoms of (to some extent, preferably completely relieve the symptoms) conditions, disorders or disease states in subjects who need or receive such treatment.

The term "pharmaceutically acceptable carrier and/or excipient" refers to a carrier and/or excipient that is pharmacologically and/or physiologically compatible with the subject and the active ingredient and that is well known in the art (see, for example, Remington's Pharmaceutical Sciences. Edited by Gennaro AR, 19thed. Pennsylvania: Mack Publishing Company, 1995), including but not limited to: pH adjusters, surfactants, adjuvants, ionic strength enhancers.

Unless otherwise indicated, the term "compound" refers to any specific compound disclosed herein and includes tautomers, regioisomers, geometric isomers, and, where applicable, stereoisomers, including optical isomers (enantiomers) and other stereoisomers (diastereomers), as well as pharmaceutically acceptable salts and derivatives (including prodrug forms) thereof. When used in the context, the term "compound" generally refers not only to a single compound, but may include other compounds, such as stereoisomers, regioisomers and/or optical isomers (including racemic mixtures), as well as specific enantiomers or enantiomer-enriched mixtures of the disclosed compounds. The term in the context also refers to a prodrug form of the compound, which has been modified to facilitate administration and delivery of the compound to an active site.

The term "pharmaceutically acceptable salt" is used to describe a salt form of one or more compounds described herein, which is provided to increase the solubility of the compounds in the gastric juices of the gastrointestinal tract of a patient, thereby facilitating dissolution and bioavailability of the compounds. Pharmaceutically acceptable salts include, where applicable, salts derived from pharmaceutically acceptable inorganic or organic bases and acids. Suitable salts include those derived from alkali metals (such as potassium and sodium) and alkaline earth metals (such as calcium, magnesium and ammonium salts), as well as salts of many other acids and bases well known in the pharmaceutical field.

The term "prodrug" will refer to a functional derivative of the compound that is readily converted to the desired compound in the body. Therefore, in the treatment methods of the present invention, the term "administer" will include the treatment of various diseases described with compounds that are specifically disclosed or with compounds that may not be specifically disclosed, but can be converted into specific compounds in vivo after administration to patients. General procedures for selecting and preparing suitable prodrug derivatives are described, for example, in "Design of Prodrugs", edited by H. Bundgaard, Elsevier, 1985.

The compounds of the present invention can form solvates with conventional organic solvents, or form hydrates with water, and such solvates or hydrates are also included in the scope of the present invention.

Included among the compounds of the present invention are all tautomers and mixtures thereof in any proportion.

The term "proteolysis targeting chimera (PROTAC)"is a hybrid bifunctional small molecule compound (Sakamoto KM. et al. Proc Natl Acad Sci USA, 2001, 98: 8554-8559), including a small molecule compound that can bind to a protein of interest (POI) connected via a linking group at an appropriate position thereof to a small molecule compound that can bind to an E3 ubiquitin ligase.

The term "antibody-drug conjugate (ADC)" refers to a product in which a biologically active small molecule drug is connected to a monoclonal antibody via a chemical linker, wherein the monoclonal antibody serves as a carrier to deliver the small molecule drug to the cells of interest.

In one aspect, the present invention provides a compound of formula (I₀) or a pharmaceutically acceptable salt, solvate, hydrate, stereoisomer, tautomer, or prodrug thereof 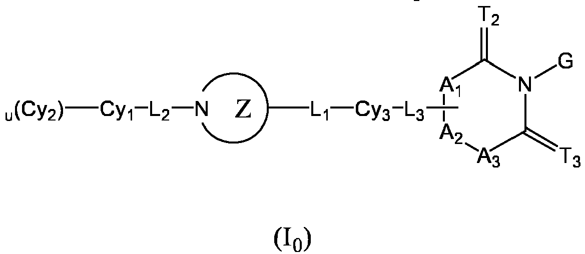 wherein,
G is selected from: H or C₁₋₆ alkyl, wherein the C₁₋₆ alkyl is optionally substituted with a group selected from the following: hydroxyl, -OPO(OR^{G})₂, - OCOR^{G}, carboxyl, -OS(O)₁₋₂R^{G}, and sulfo, wherein each R^{G} is independently selected from: H, or C₁₋₄ alkyl;
T₂-T₃ are independently selected from: O or S;
A₁-A₂ are independently selected from: C(R^{A})₂ or NR^{A'}, wherein each R^{A} is independently selected from: H, halogen, C₁₋₁₅ alkyl optionally substituted with halogen, C₃₋₁₅ cycloalkyl, -CONR'R", -COOR', -OR ', or -NR'R", and R^{A'} is selected from: H, C₁₋₁₅ alkyl optionally substituted with halogen, C₃₋₁₅ cycloalkyl, or -COR';
A₃ is selected from: a bond, or C(R^{A})₂, wherein each R^{A} is independently selected from: H, halogen, C₁₋₁₅ alkyl optionally substituted with halogen, C₃₋₁₅ cycloalkyl, -CONR'R", -COOR', -OR', or -NR'R";
R' and R" are independently selected from: H, C₁₋₁₅ alkyl optionally substituted with halogen, or C₃₋₆ cycloalkyl;
L₃ is selected from: a bond, or a group represented by the structure of the following general formula:

   -(CHR¹)ₖ-;

   -(CHR¹)ₘ-O-(CHR¹)ₙ-;

   -(CHR¹)ₘ-N(R¹)-(CHR¹)ₙ-;

   -(CHR¹)ₘ-CO-(CHR¹)ₙ-;

   -(CHR¹)ₘ-CO-N(R¹)-(CHR¹)ₙ-;

   -(CHR¹)ₘ-N(R¹)-CO-(CHR¹)ₙ-;

   -(CHR¹)ₘ-CO-O-(CHR¹)ₙ-;

   -(CHR¹)ₘ-O-CO-(CHR¹)ₙ-;

   -(CHR¹)ₘ-SO₂-N(R¹)-(CHR¹)ₙ-;

   -(CHR¹)ₘ-N(R¹)-SO₂-(CHR¹)ₙ-;
substituted or unsubstituted triazolylidene;
wherein k is selected from 0, 1, 2, 3, 4, 5, 6, 7, 8, 9, 10, 11, and 12, m and n are independently selected from: 0, 1, 2, 3, 4, 5, and 6, each R¹ is independently selected from: H, or C₁₋₆ alkyl optionally substituted with halogen;
Cy₃ is selected from optionally substituted aryl, optionally substituted heteroaryl, optionally substituted heterocyclyl, optionally substituted biaryl or optionally substituted biheteroaryl;
L₁ represents a linking group;
ring Z represents an optionally substituted heterocycloalkyl containing at least one N atom as a heteroatom, and is linked to L₂ through the N;
L₂ is selected from: a bond, or a group represented by the structure of the following general formula:

   -(CHR¹)ₖ-;

   -(CHR¹)ₘ-O-(CHR¹)ₙ-;

   -(CHR¹)ₘ-N(R¹)-(CHR¹)ₙ-;

   -(CHR¹)ₘ-CO-(CHR¹)ₙ-;

   -(CHR¹)ₘ-CO-N(R¹)-(CHR¹)ₙ-;

   -(CHR¹)ₘ-N(R¹)-CO-(CHR¹)ₙ-;
wherein k is selected from 0, 1, 2, 3, 4, 5, 6, 7, 8, 9, 10, 11, and 12, m and n are independently selected from: 0, 1, 2, 3, 4, 5, and 6, each R¹ is independently selected from: H, or C₁₋₆ alkyl optionally substituted with halogen;
Cy₁ is selected from: optionally substituted aryl, optionally substituted heteroaryl, optionally substituted heterocyclyl, optionally substituted biaryl or optionally substituted biheteroaryl;
Cy₂ is selected from H, optionally substituted aryl, optionally substituted heteroaryl, optionally substituted heterocyclyl, optionally substituted biaryl or optionally substituted biheteroaryl;
u is selected from 0, 1, 2, 3, 4, and 5.

In certain embodiments, compounds of formula (I₀) include stereoisomers thereof, for example, carbon atoms of A₁, A₂, and A₃ can be chiral (C*(R^{A})₂), and the * marked carbon atom can be in R configuration or S configuration. Therefore, in all general and structural formulas of the present invention, the carbon atoms in the groups/fragments derived from A₁, A₂, and A₃ can be chiral (C*(R^{A})₂), and the * marked carbon atom can be in R configuration or S configuration.

Therefore, in certain embodiments, the compound of formula (I₀) of the present invention is selected from the following compound of formula (I₀-a) or formula (I₀-b):

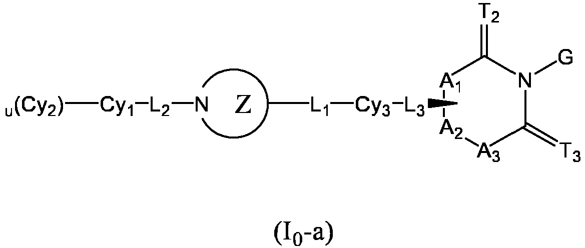

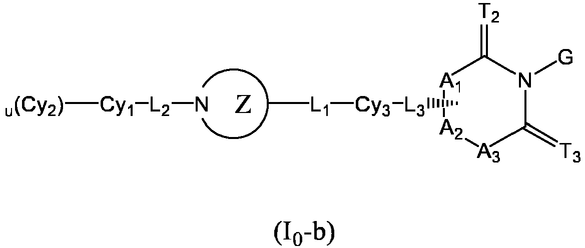

In certain embodiments, Cy₃ is selected from optionally substituted monocyclic aryl, optionally substituted monocyclic heteroaryl, optionally substituted monocyclic heterocyclyl, optionally substituted bicyclic aryl , optionally substituted bicyclic heteroaryl, optionally substituted bicyclic heterocyclyl, optionally substituted tricyclic aryl, optionally substituted tricyclic heteroaryl, and optionally substituted tricyclic heterocyclyl.
preferably, Cy₃ is selected from one of the following structures: 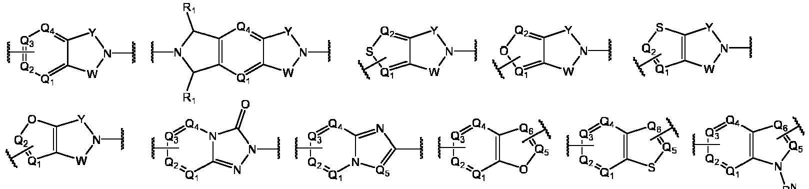 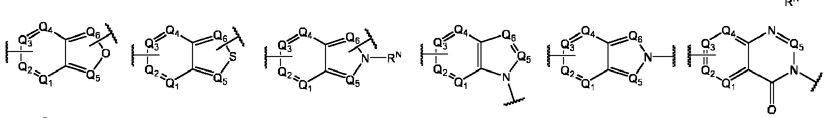 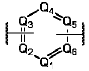
Y is selected from: C(R^{Y})₂, or C=T₁;
T₁ is selected from: O or S;
W is selected from: C(R^{W})₂, C=O, C=S, NH or -N-C₁₋₆ alkyl;
R^{Y} and R^{W} are independently selected from: H, halogen, cyano, nitro, C₁₋₁₅ alkyl optionally substituted with halogen, C₃₋₁₅ cycloalkyl, -CONR'R", -COOR', - OR', or -NR'R";
R^{N} is selected from: H, C₁₋₁₅ alkyl optionally substituted with halogen, or C₃₋₁₅ cycloalkyl;
Q₁-Q₆ are independently selected from: CR^{Q} or N;
R^{Q} is selected from: H, halogen, cyano, nitro, C₁₋₁₅ alkyl optionally substituted with halogen, C₃₋₁₅ cycloalkyl, -CONR'R", -COOR', -OR', or -NR'R"; or R^{Q} and R^{W} together form an aromatic ring optionally substituted with halogen;
R' and R" are independently selected from: H, C₁₋₁₅ alkyl optionally substituted with halogen, or C₃₋₆ cycloalkyl.
R¹ is independently selected from: H, or C₁₋₆ alkyl optionally substituted with halogen.

In one embodiment, Cy₃ is selected from 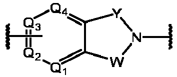 and Y is selected from C=T₁.

Therefore, in certain embodiments, the compound of formula (I₀) of the present invention is selected from the following compound of formula (I): 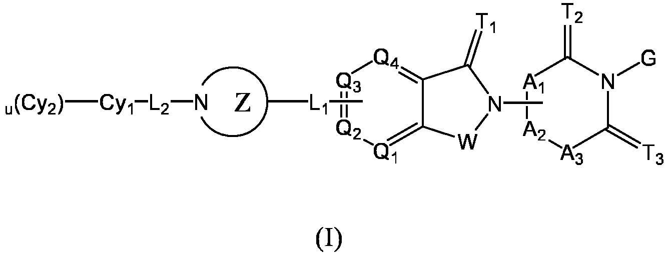 wherein,
W is selected from: C(R^{W})₂, C=O, C=S, NH or -N-C₁₋₆ alkyl;
R W is selected from: H, halogen, cyano, nitro, C₁₋₁₅ alkyl optionally substituted with halogen, C₃₋₁₅ cycloalkyl, -CONR'R", -COOR', -OR', or -NR'R";
G is selected from: H or C₁₋₆ alkyl, wherein the C₁₋₆ alkyl is optionally substituted with a group selected from the following: hydroxyl, -OPO(OR^{G})₂, - OCOR^{G}, carboxyl, -OS(O)₁₋₂R^{G}, and sulfo, wherein each R^{G} is independently selected from: H, or C₁₋₄ alkyl;
T₁-T₃ are independently selected from: O or S;
A₁-A₃ are independently selected from: C(R^{A})₂, wherein each R^{A} is independently selected from: H, halogen, C₁₋₁₅ alkyl optionally substituted with halogen, C₃₋₁₅ cycloalkyl, -CONR'R", -COOR', -OR', or -NR'R";
Q₁-Q₄ are independently selected from: CR^{Q} or N;
R^{Q} is selected from: H, halogen, cyano, nitro, C₁₋₁₅ alkyl optionally substituted with halogen, C₃₋₁₅ cycloalkyl, -CONR'R", -COOR', -OR', or -NR'R";
R' and R" are independently selected from: H, C₁₋₁₅ alkyl optionally substituted with halogen, or C₃₋₆ cycloalkyl;
L₁ represents a linking group;
ring Z represents an optionally substituted heterocycloalkyl containing at least one N atom as a heteroatom, and is linked to L₂ through the N;
L₂ is selected from: a bond, or a group represented by the structure of the following general formula:

   -(CHR¹)ₖ-;

   -(CHR¹)ₘ-O-(CHR¹)ₙ-;

   -(CHR¹)ₘ-N(R¹)-(CHR¹)ₙ-;

   -(CHR¹)ₘ-CO-(CHR¹)ₙ-;

   -(CHR¹)ₘ-CO-N(R¹)-(CHR¹)ₙ-;

   -(CHR¹)ₘ-N(R¹)-CO-(CHR¹)ₙ-;
wherein k is selected from 0, 1, 2, 3, 4, 5, 6, 7, 8, 9, 10, 11, and 12, m and n are independently selected from: 0, 1, 2, 3, 4, 5, and 6, each R¹ is independently selected from: H, or C₁₋₆ alkyl optionally substituted with halogen;
Cy₁ is selected from: optionally substituted aryl, optionally substituted heteroaryl, optionally substituted heterocyclyl, optionally substituted biaryl or optionally substituted biheteroaryl;
Cy₂ is selected from H, optionally substituted aryl, optionally substituted heteroaryl, optionally substituted heterocyclyl, optionally substituted biaryl or optionally substituted biheteroaryl;
u is selected from 0, 1, 2, 3, 4, and 5.

In certain embodiments, compounds of formula (I) include stereoisomers thereof, for example, carbon atoms of A₁, A₂, and A₃ can be chiral (C*(R^{A})₂), and the * marked carbon atom can be in R configuration or S configuration. Therefore, in all general and structural formulas of the present invention, the carbon atoms in the groups/fragments derived from A₁, A₂, and A₃ can be chiral (C*(R^{A})₂), and the * marked carbon atom can be in R configuration or S configuration.

Therefore, in certain embodiments, the compound of formula (I) of the present invention is selected from the following compound of formula (Ia) or formula (Ib):

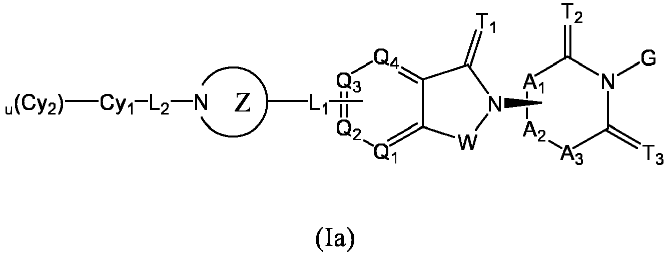

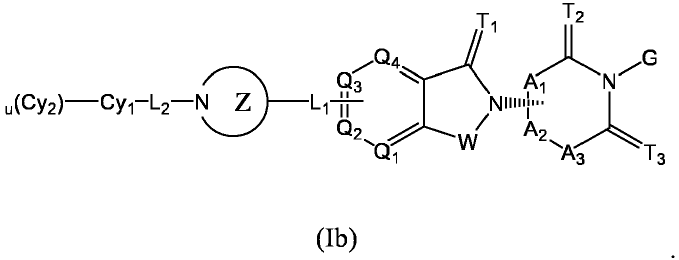

In certain embodiments, W is selected from: C(R^{W})₂, C=O, and C=S, wherein R^{W} is selected from H, halogen, cyano, C₁₋₆ alkyl optionally substituted with halogen, C₁₋₆ alkoxy optionally substituted with halogen, and C₃₋₆ cycloalkyl.
preferably, W is selected from: C(R^{W})₂ or C=O, wherein R^{W} is selected from H, halogen, cyano, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, or cyclohexyl.

More preferably, W is selected from: CH₂ or C=O.

Therefore, in certain embodiments, structure 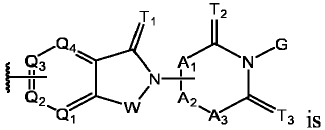 selected from one of the following structures:

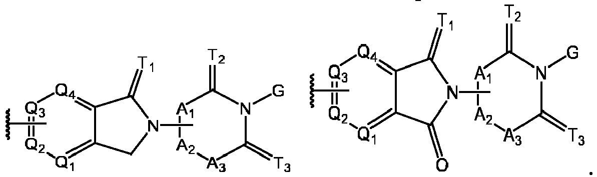

Therefore, in certain embodiments, the compound of formula (I) of the present invention is selected from the following compound of formula (II) or formula (III):

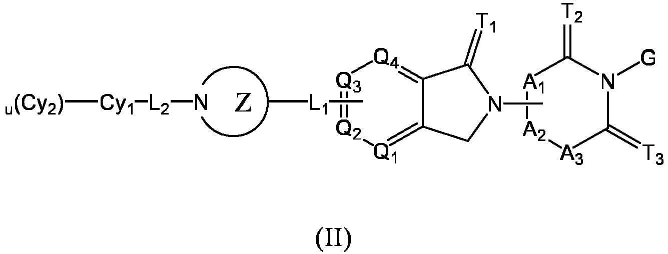

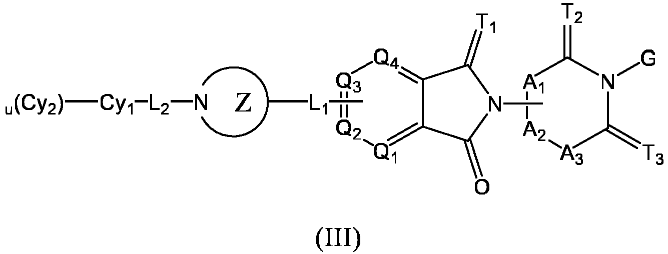

In certain embodiments, G is selected from: H.

In certain embodiments, G is selected from: C₁₋₆ alkyl, where the C₁₋₆ alkyl is substituted with a group selected from the following: -OPO(OR^{G})₂, wherein each R^{G} is independently selected from: H or C₁₋₄ alkyl.

In certain embodiments, T₁ is selected from: O.

In certain embodiments, T₂ is selected from: O.

In certain embodiments, T₃ is selected from: O.

Therefore, in certain embodiments, the structure 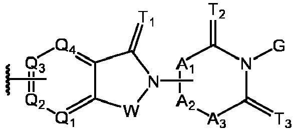 can be 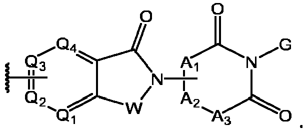 Preferably, it is selected from one of the following structures:

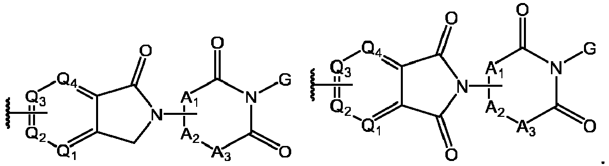

Therefore, in certain embodiments, the compound of formula (I) of the present invention is selected from the following compound of formula (II-1) or formula (III-1):

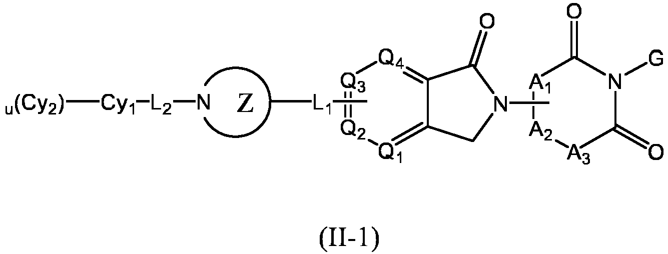

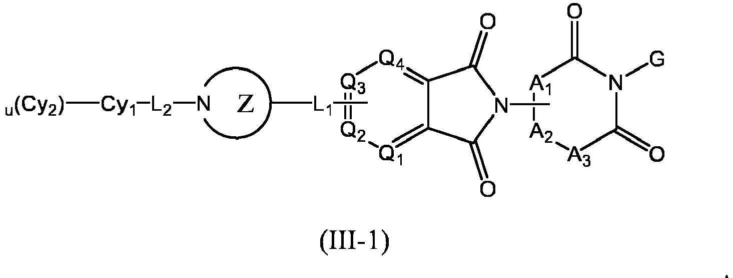

In certain embodiments, A₁-A₃ are independently selected from C(R^{A})₂, wherein each R^{A} is independently selected from: H, halogen, cyano, C₁₋₆ alkyl optionally substituted with halogen, C₁₋₆ alkoxy optionally substituted with halogen, and C₃₋₆ cycloalkyl.

Preferably, A₁-A₃ are independently selected from CHR^{A}, wherein each R^{A} is independently selected from: H, halogen, cyano, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, or cyclohexyl.

Preferably, A₁ is selected from CHR^{A} and N is linked to A₁.

Preferably, A₂ is selected from CHR^{A} and N is linked to A₂.

It should be noted that when N is linked to A₁ or A₂, the H of CHR^{A} in A₁ or A₂ is replaced by the N.

In certain embodiments, 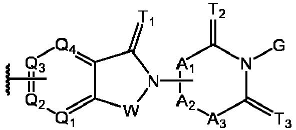 is selected from one of the following structures:

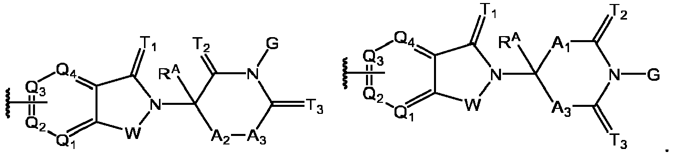

In certain embodiments, 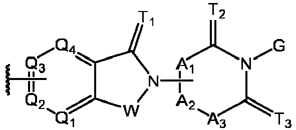 is selected from one of the following structures:

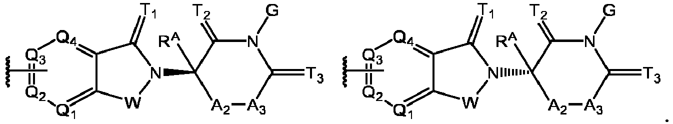

Preferably, it is selected from one of the following structures:

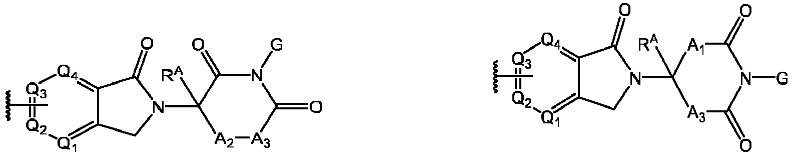

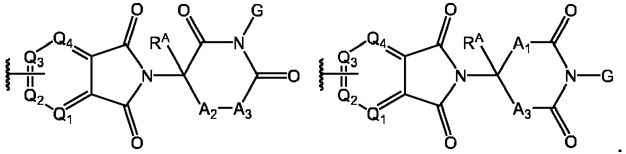

Further preferably, it is selected from one of the following structures:

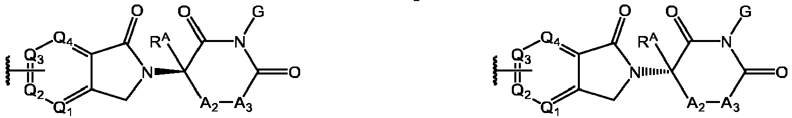

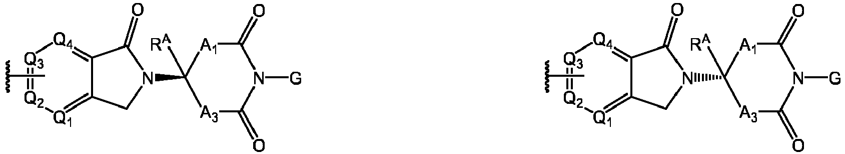

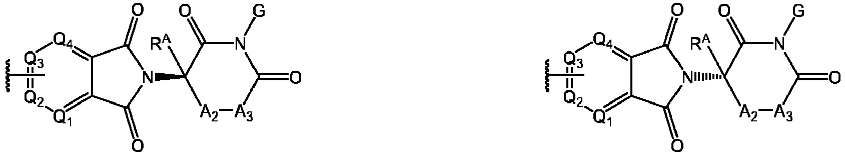

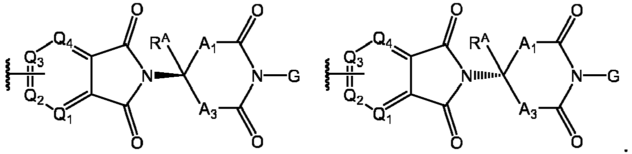

Therefore, in certain embodiments, the compound of formula (I) of the present invention is selected from the following compounds of formula (II-2), formula (II-3), formula (III-2), and formula (III-3):

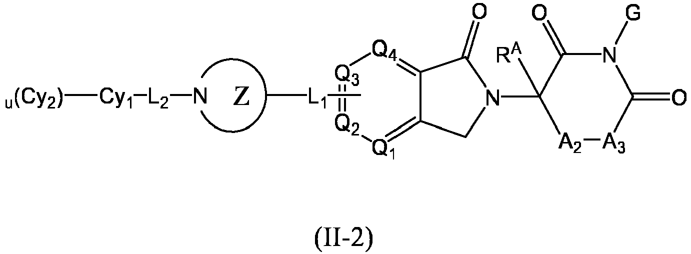

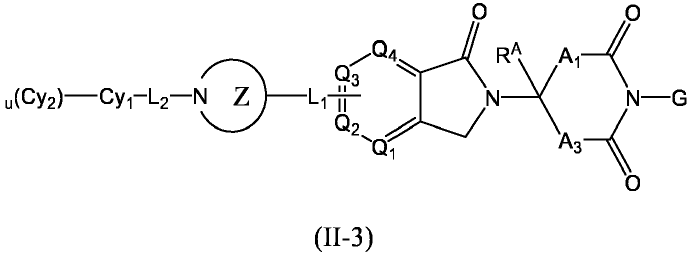

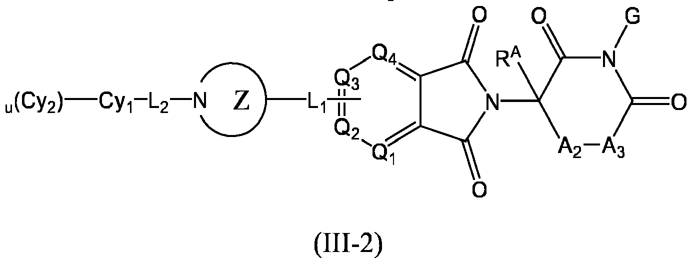

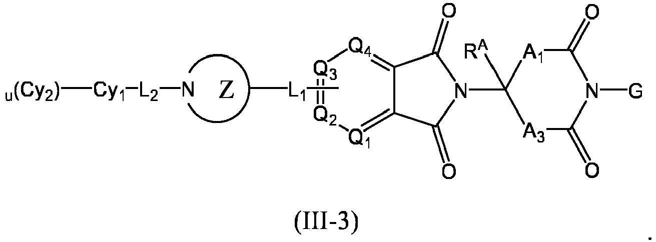

In certain embodiments, Q₁ is selected from CH and L₁ is linked to Q₁.

In certain embodiments, Q₂ is selected from CH and L₁ is linked to Q₂.

In certain embodiments, Q₃ is selected from CH and L₁ is linked to Q₃.

In certain embodiments, Q₄ is selected from CH and L₁ is linked to Q₄.

It should be noted that when L₁ is linked to Q₁, Q₂, Q₃ or Q₄, the H on Q₁, Q₂, Q₃ or Q₄ is replaced by the L₁.

Therefore, in certain embodiments, 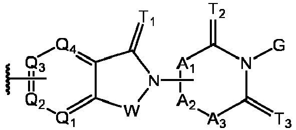 is selected from one of the following structures: 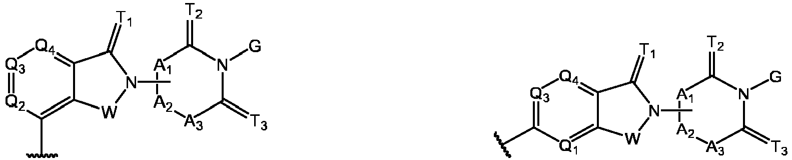 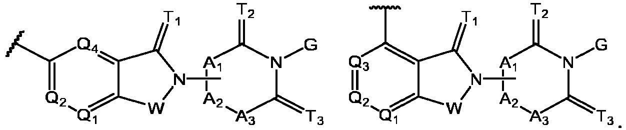
preferably, 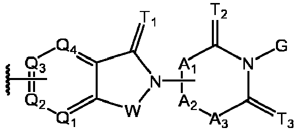 is selected from one of the following structures: 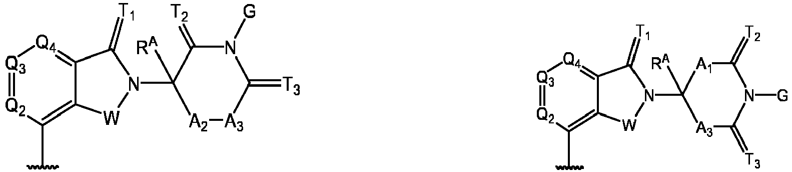 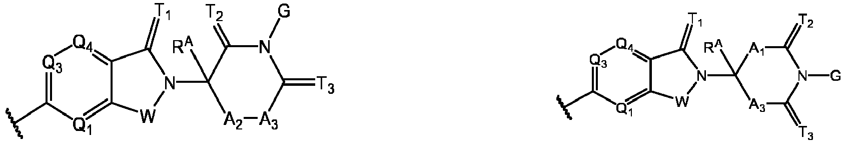 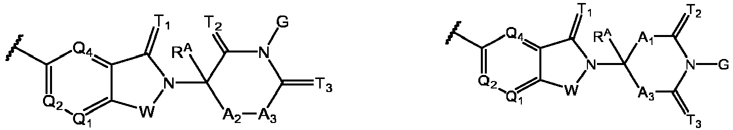 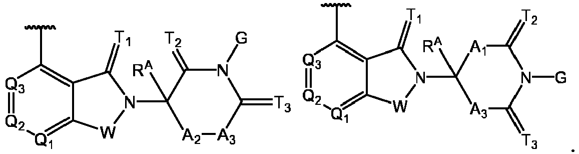
preferably, 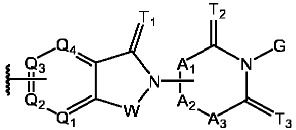 is selected from one of the following structures:

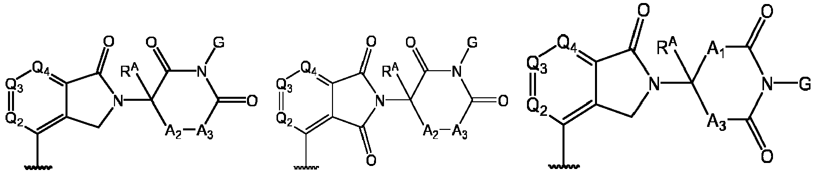

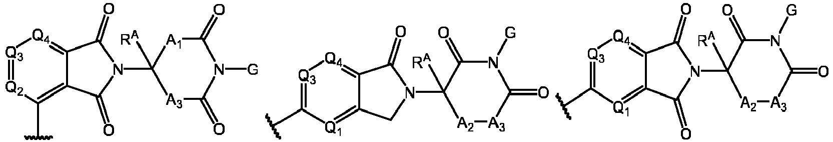

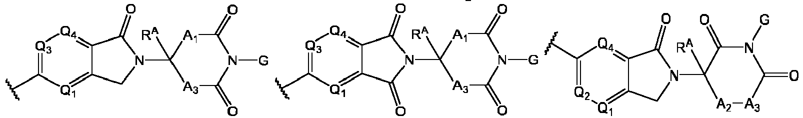

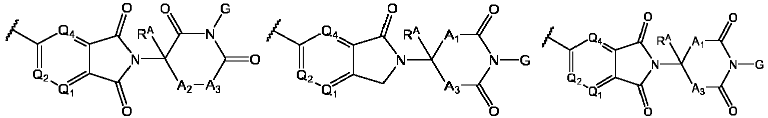

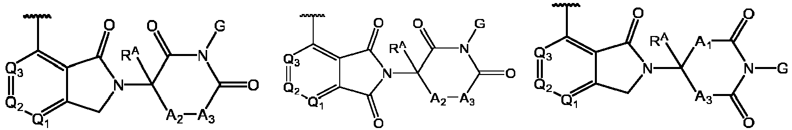

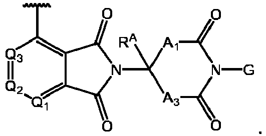

In certain embodiments, Q₁- Q₄ are each selected from: CR^{Q}.

In certain embodiments, one of Q₁-Q₄ is selected from: N, and the others are selected from: CR^{Q}.

In certain embodiments, R^{Q} is selected from: H, halogen, cyano, nitro, hydroxyl, C₁₋₆ alkyl optionally substituted with halogen, C₁₋₆ alkoxy optionally substituted with halogen, C₃₋₆ cycloalkyl, or -NR'R".
preferably, R^{Q} is selected from: H, halogen, cyano, nitro, hydroxyl, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, cyclohexyl, amino, methylamino, dimethylamino, ethylamino, or diethylamino.
more preferably, R^{Q} is selected from: H, halogen, cyano, nitro, methyl, CH₂F, CHF₂, CF₃, methoxy, ethoxy, CF₃O-, cyclopropyl, cyclobutyl, cyclopentyl, or cyclohexyl.
preferably, 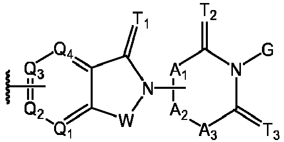 is selected from one of the following structures:

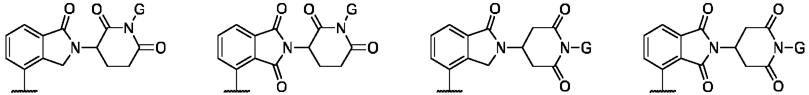

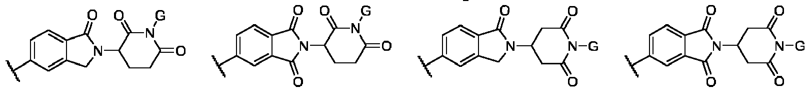

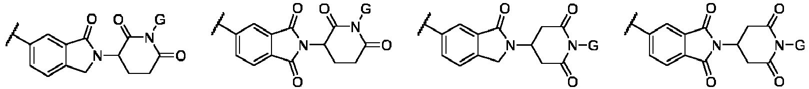

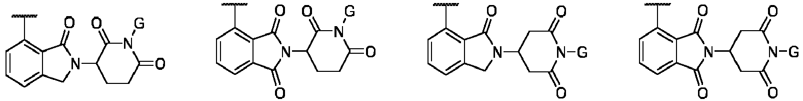

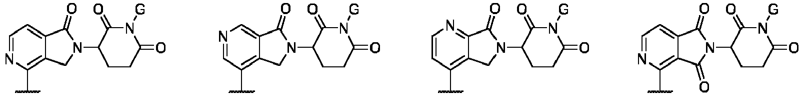

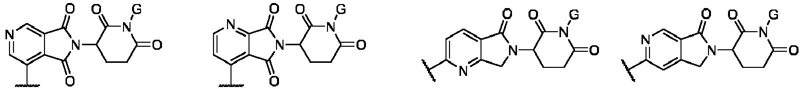

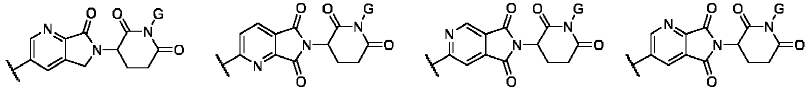

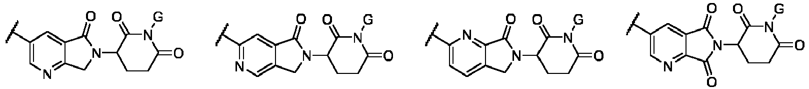

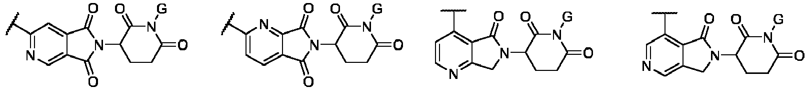

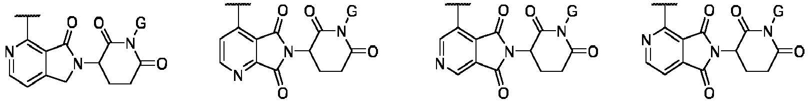

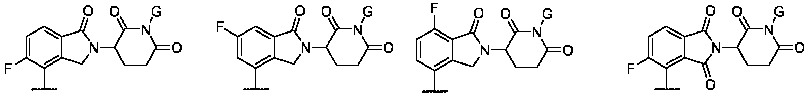

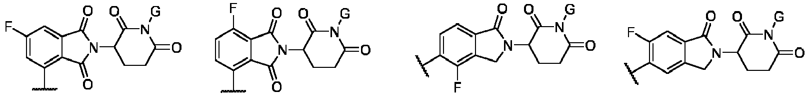

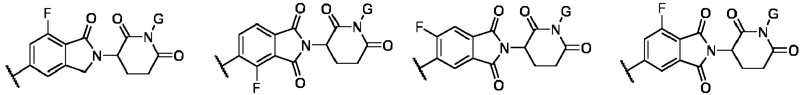

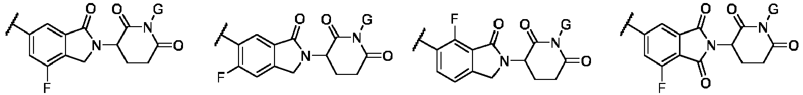

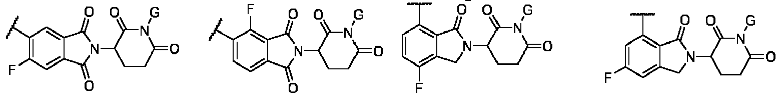

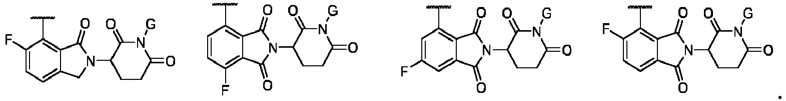

Most preferably, 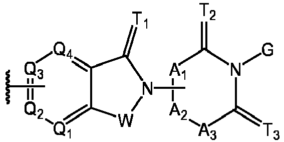 is selected from one of the following structures:

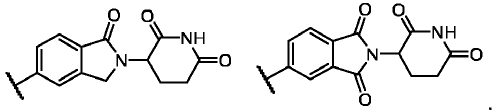

Further preferably, 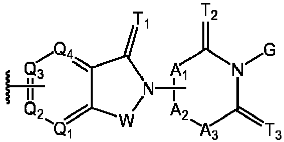 is selected from one of the following structures:

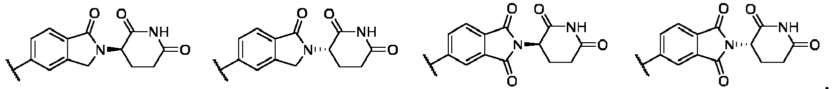

In certain embodiments, Cy₃ is selected from one of the following structures:

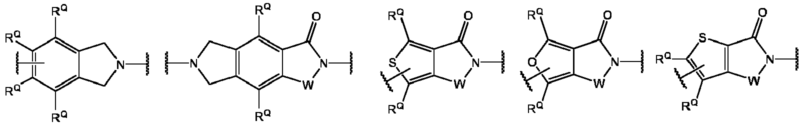

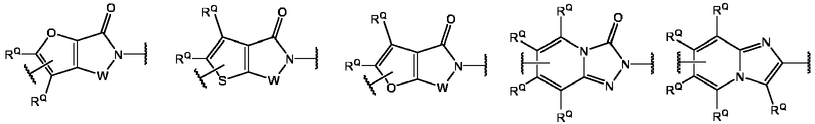

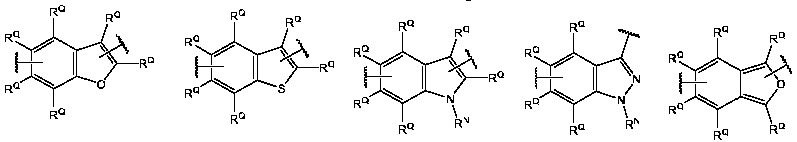

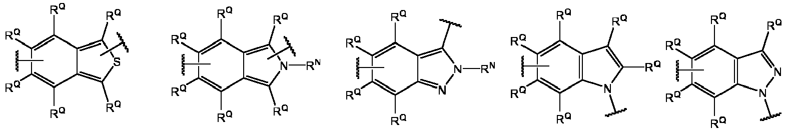

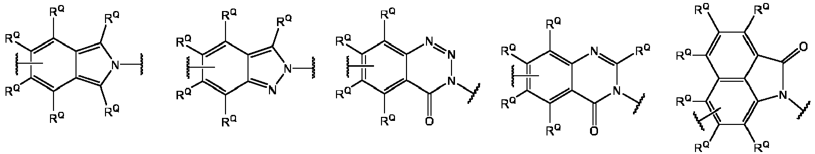

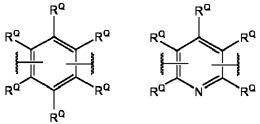

R^{Q} is selected from: H, halogen, cyano, nitro, hydroxyl, C₁₋₆ alkyl optionally substituted with halogen, C₁₋₆ alkoxy optionally substituted with halogen, C₃₋₆ cycloalkyl, or -NR'R"; R^{N} is selected from H, C₁₋₆ alkyl optionally substituted with halogen, or C₃₋₆ cycloalkyl.
preferably, R^{Q} is selected from: H, halogen, cyano, nitro, hydroxyl, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, cyclohexyl, amino, methylamino, dimethylamino, ethylamino, or diethylamino; R^{N} is selected from: H, methyl, ethyl, n-propyl, isopropyl, n-butyl, isobutyl, sec-butyl, tert-butyl, pentyl, hexyl, CH₂F, CHF₂, CF₃, cyclopropyl, cyclobutyl, cyclopentyl, or cyclohexyl.
more preferably, R^{Q} is selected from: H, halogen, cyano, nitro, methyl, CH₂F, CHF₂, CF₃, methoxy, ethoxy, CF₃O-, cyclopropyl, cyclobutyl, cyclopentyl, or cyclohexyl; R^{N} is selected from: methyl, ethyl, isopropyl, tert-butyl, CH₂F, CHF₂, CF₃, cyclopropyl, cyclobutyl, cyclopentyl, or cyclohexyl.
preferably, Cy₃ is selected from one of the following structures: 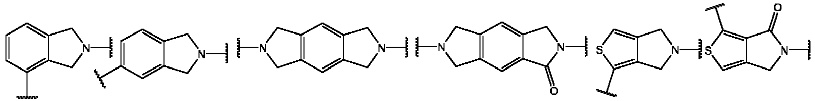 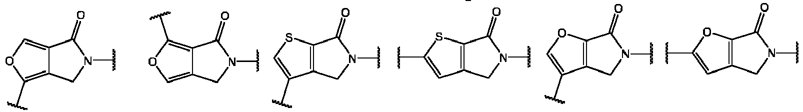 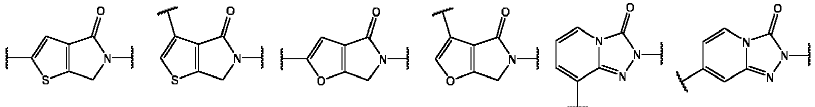 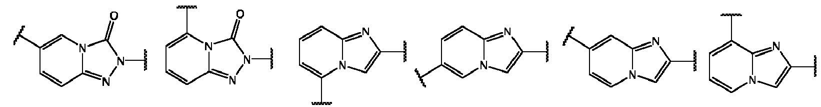 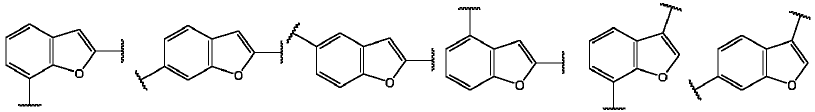 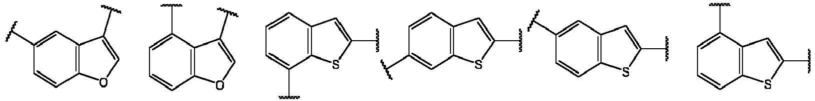 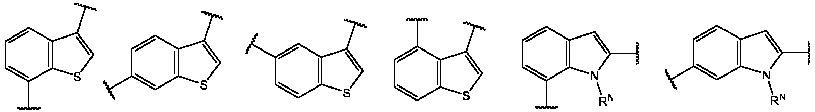 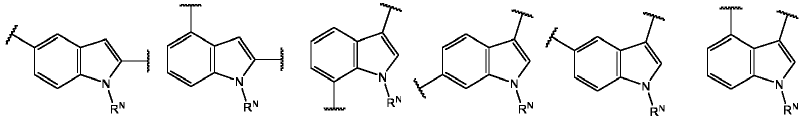 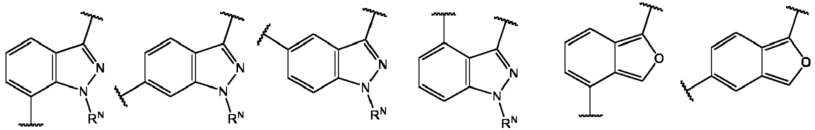 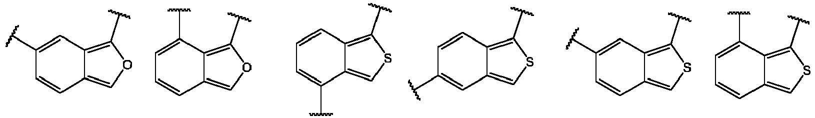 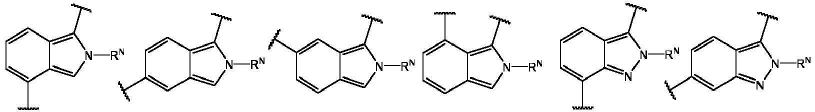 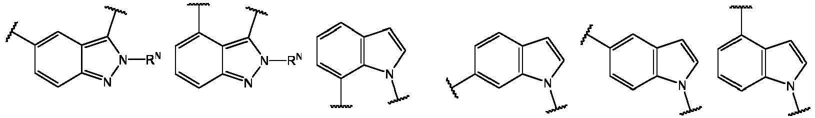 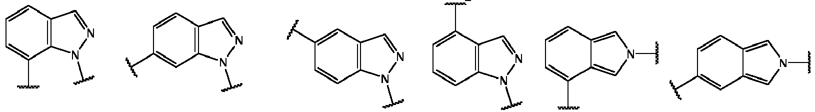 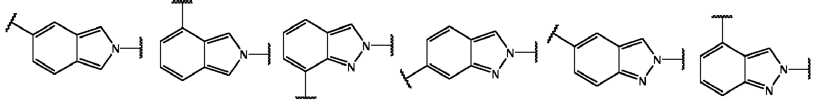 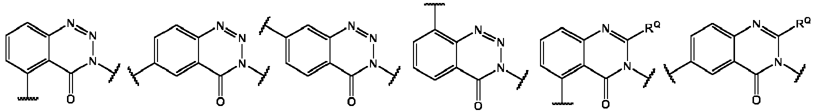 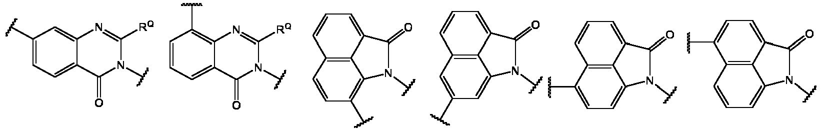 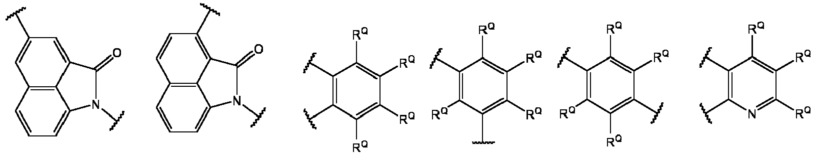 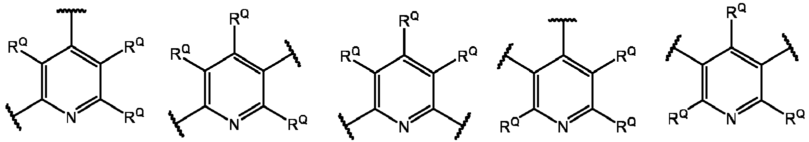
R^{Q} is selected from: H, halogen, nitro, methyl, CH₂F, CHF₂, CF₃, methoxy, ethoxy, CF₃O-, cyclopropyl, cyclobutyl, cyclopentyl, or cyclohexyl; R^{N} is selected from: methyl, ethyl, isopropyl, tert-butyl, CH₂F, CHF₂, CF₃, cyclopropyl, cyclobutyl, cyclopentyl, or cyclohexyl.

In certain embodiments, A₁-A₃ are each selected from CH₂ and L₃ is linked to A₁.

In certain embodiments, A₁ is selected from NH and L₃ is linked to A₁.

In certain embodiments, A₂ is selected from NH and L₃ is linked to A₂.

In certain embodiments, A₃ is selected from NH and L₃ is linked to A₃.

In certain embodiments, A₃ is selected from a bond; preferably, A₁-A₂ are selected from CH₂ and L₃ is linked to A₁.
preferably, 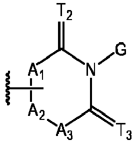 is selected from: 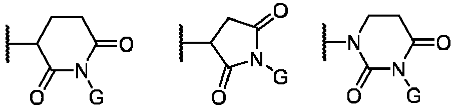
G is selected from: H or C₁₋₆ alkyl, wherein the C₁₋₆ alkyl is optionally substituted with a group selected from the following: hydroxyl, -OPO(OR^{G})₂, - OCOR^{G}, or -OS(O)₁₋₂R^{G}, wherein each R^{G} is independently selected from: H, or C₁₋₄ alkyl;
preferably, 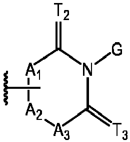 is selected from:

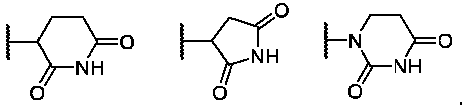

In certain embodiments, L₃ is selected from: a bond, or a group represented by the structure of the following general formula:

-(CHR¹)ₖ-;

-O-; -(CHR¹)ₘ-O-; -O-(CHR¹)ₙ-; -(CHR¹)m-O-(CHR¹)ₙ-;

-N(R')-; -(CHR¹)ₘ-N(R¹)-; -N(R¹)-(CHR¹)ₙ-; -(CHR¹)ₘ-N(R¹)-(CHR¹)ₙ-;

-CO-; -(CHR¹)ₘ-CO-; -CO-(CHR¹)ₙ-; -(CHR¹)ₘ-CO-(CHR¹)ₙ-;

-CO-N(R¹)-; -(CHR¹)ₘ-CO-N(R¹)-; -CO-N(R¹)-(CHR¹)ₙ-; -(CHR¹)ₘ-CO-N(R¹)-(CHR¹)ₙ-;

-N(R¹)-CO-; -(CHR¹)ₘ-N(R¹)-CO-; -N(R¹)-CO-(CHR¹)ₙ-; -(CHR¹)ₘ-N(R¹)-CO-(CHR¹)ₙ-;

-CO-O-; -(CHR¹)ₘ-CO-O; -CO-O-(CHR¹)ₙ-; -(CHR¹)ₘ-CO-O-(CHR¹)ₙ-;

-O-CO-; -(CHR¹)ₘ-O-CO-; -O-CO-(CHR¹)ₙ-; -(CHR¹)ₘ-O-CO-(CHR¹)ₙ-;

-SO₂-N(R¹)-; -(CHR¹)ₘ-SO₂-N(R¹)-; -SO₂-N(R¹)-(CHR¹)ₙ-; -(CHR¹)ₘ-SO₂-N(R¹)-(CHR¹)ₙ-;

-N(R¹)-SO₂-; -(CHR¹)ₘ-N(R¹)-SO₂-; -N(R¹)-SO₂-(CHR¹)ₙ-; -(CHR¹)ₘ-N(R¹)-SO₂-(CHR¹)ₙ-;

-triazolyl-

wherein k, m and n are independently selected from: 1, 2, 3, 4, 5, and 6, each R¹ is independently selected from: H, methyl, or ethyl.

In certain embodiments, L₃ is selected from: a bond, or one of the following structures:

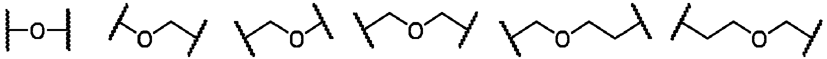

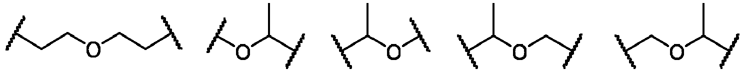

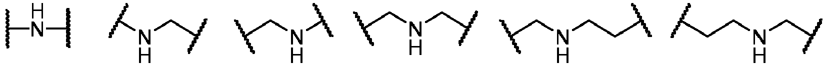

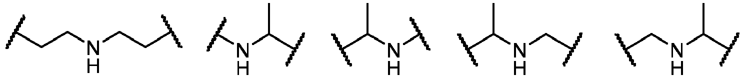

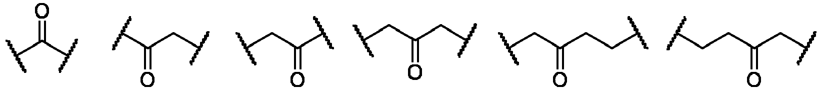

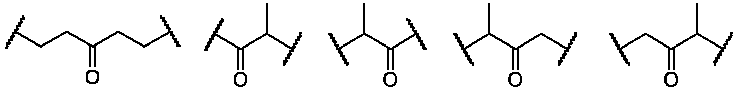

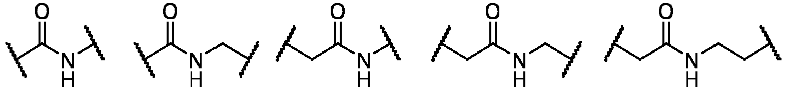

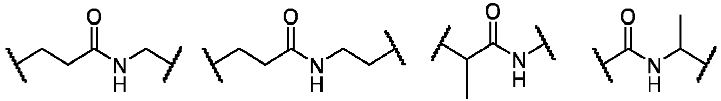

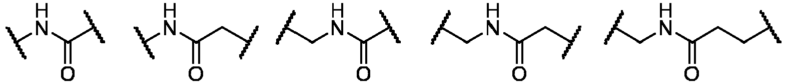

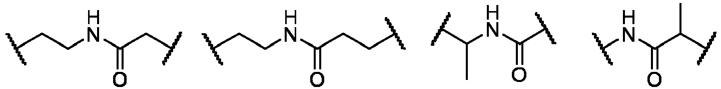

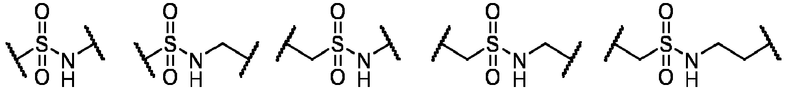

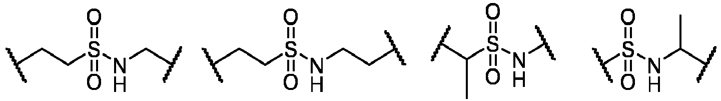

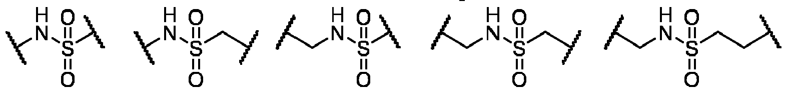

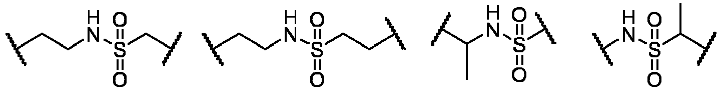

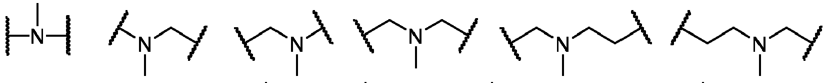

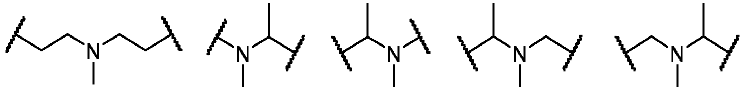

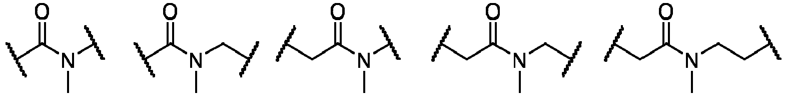

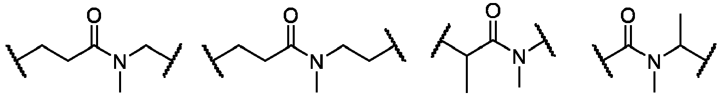

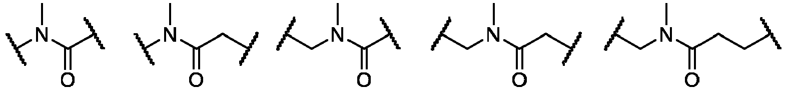

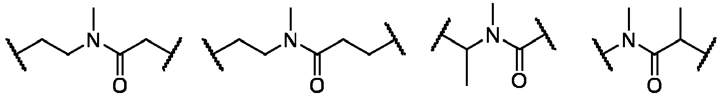

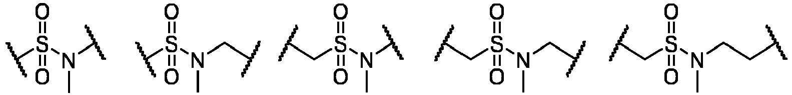

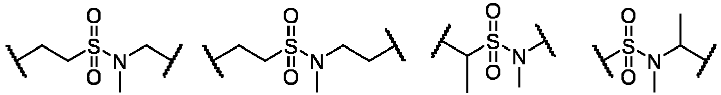

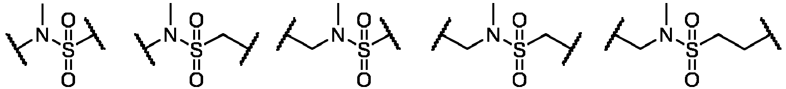

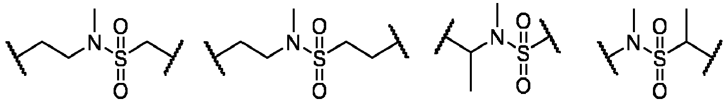

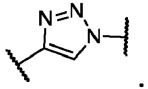

In certain embodiments, when Cy₃ is selected from optionally substituted bicyclic aryl, optionally substituted bicyclic heteroaryl, optionally substituted bicyclic heterocyclyl, optionally substituted tricyclic aryl, optionally substituted tricyclic heteroaryl, optionally substituted tricyclic heterocyclyl, L₃ is selected form a bond.

In certain embodiments, L₁ is selected from: -O-, -S-, -N(R¹)-, -CO-, -CO-O-, -O-CO-, -O-CO-O-, -CO-N (R¹)-, -N(R¹)-CO-, -N(R¹)-CO-N(R¹)-, -CO-N(R¹)-CO and optionally substituted C₁₋₁₅ alkylene-, where the head, tail or middle of the alkylene is optionally inserted by 1, 2, 3, 4, 5 or more groups selected from: -O-, - S-, -N(R¹)-, -CO-, -CO-O-, -O-CO-, -O-CO-O-, -CO-N (R¹)-, -N(R¹)-CO-, -N(R¹)-CO-N(R¹)-, -CO-N(R¹)-CO, C₂ alkenylene, C₂ alkynylene, C₃₋₆ cycloalkylene, C₆₋₁₀ arylene, 5- to 6-membered heteroarylene, 5- to 7-membered heterocyclylene or any combination thereof, wherein each R¹ is independently selected from: H, or C₁₋₆ alkyl optionally substituted with halogen.
preferably, the "optionally substituted" refers to being unsubstituted or mono- or poly-substituted with a group selected from the following: halogen, hydroxyl, thiol, cyano, nitro, amino, C₁₋₆ alkyl optionally substituted with halogen, C₁₋₆ alkoxy optionally substituted with halogen, C₁₋₆ alkylthio optionally substituted with halogen, C₃₋₆ cycloalkyl, C₁₋₆ alkylamino, di(C₁₋₆ alkyl)amino.

Preferably, L₁ is selected from a linear or branched linking group. The term "linear or branched linking group" means that the backbone of L₁ does not contain a cyclic structure.

In certain embodiments, L₁ is selected from: -O-, -S-, -N(R¹)-, -CO-, -CO-O-, -O-CO-, -O-CO-O-, -CO-N (R¹)-, -N(R¹)-CO-, -N(R¹)-CO-N(R¹)-, -CO-N(R¹)-CO and optionally substituted C₁₋₁₅ alkylene-, where the head, tail or middle of the alkylene is optionally inserted by 1, 2, 3, 4, 5 or more groups selected from: -O-, - S-, -N(R¹)-, -CO-, -CO-O-, -O-CO-, -O-CO-O-, -CO-N (R¹)-, -N(R¹)-CO-, -N(R¹)-CO-N(R¹)-, -CO-N(R¹)-CO or any combination thereof, wherein each R¹ is independently selected from: H, or C₁₋₆ alkyl optionally substituted with halogen.
preferably, the "optionally substituted" refers to being unsubstituted or mono- or poly-substituted with a group selected from the following: halogen, hydroxyl, cyano, nitro, amino, methyl, ethyl, n-propyl, isopropyl, CH₂F, CHF₂, CF₃, methoxy, ethoxy, CF₃O-, methylamino, dimethylamino, ethylamino, or diethylamino.

Preferably, L₁ is selected from a group represented by the structure of the following general formulas:

-(CHR¹)ₖ-;

-(CHR¹)ₘ-O-(CHR¹)ₙ-;

-(CHR¹)ₘ-S-(CHR¹)ₙ-;

-(CHR¹)ₘ-N(R¹)-(CHR¹)ₙ-;

-(CHR¹)ₘ-CO-(CHR¹)ₙ-;

-(CHR¹)ₘ-CO-O-(CHR¹)ₙ-;

-(CHR¹)ₘ-O-CO-(CHR¹)ₙ-;

-(CHR¹)ₘ-O-CO-O-(CHR¹)ₙ-;

-(CHR¹)ₘ-CO-N(R¹)-(CHR¹)ₙ-;

-(CHR¹)ₘ-N(R¹)-CO-(CHR¹)ₙ-;

-(CHR¹)ₘ-CO-N(R¹)-(CHR¹)ₙ-;

-(CHR¹)ₘ-N(R¹)-CO-N(R¹)-(CHR¹)ₙ-;

-(CHR¹)ₘ-CO-N(R¹)-CO-(CHR¹)ₙ-;

-(CHR¹)ₘ-O-(CHR¹)₁-O-(CHR¹)ₙ-;

-(CHR¹)ₘ-O-(CHR¹)₁-N(R¹)-(CHR¹)ₙ-;

-(CHR¹)ₘ-N(R¹)-(CHR¹)ₗ-O-(CHR¹)ₙ-;

-(CHR¹)ₘ-O-(CHR¹)ₗ-CO-(CHR¹)ₙ-;

-(CHR¹)ₘ-CO-(CHR¹)ₗ-O-(CHR¹)ₙ-;

-(CHR¹)ₘ-O-(CHR¹)ₗ-CO-N(R¹)-(CHR¹)ₙ-;

-(CHR¹)ₘ-CO-N(R¹)-(CHR¹)ₗ-O-(CHR¹)ₙ-;

-(CHR¹)ₘ-O-(CHR¹)ₗ-N(R¹)-CO-(CHR¹)ₙ-;

-(CHR¹)ₘ-N(R¹)-CO-(CHR¹)ₗ-O-(CHR¹)ₙ-;

-(CHR¹)ₘ-N(R¹)-(CHR¹)ₗ-N(R¹)-(CHR¹)ₙ-;

-(CHR¹)ₘ-N(R¹)-(CHR¹)ₗ-CO-(CHR¹)ₙ-;

-(CHR¹)ₘ-CO-(CHR¹)ₗ-N(R¹)-(CHR¹)ₙ-;

-(CHR¹)ₘ-N(R¹)-(CHR¹)ₗ-CO-N(R¹)-(CHR¹)ₙ-;

-(CHR¹)ₘ-CO-N(R¹)-(CHR¹)ₗ-N(R¹)-(CHR¹)ₙ-;

-(CHR¹)ₘ-N(R¹)-(CHR¹)ₗ-N(R¹)-CO-(CHR¹)ₙ-;

-(CHR¹)ₘ-N(R¹)-CO-(CHR¹)ₗ-N(R¹)-(CHR¹)ₙ-;

-(CHR¹)ₘ-CO-(CHR¹)ₗ-CO-N(R¹)-(CHR¹)ₙ-;

-(CHR¹)ₘ-CO-N(R¹)-(CHR¹)ₗ-CO-(CHR¹)ₙ-;

-(CHR¹)ₘ-CO-(CHR¹)ₗ-N(R¹)-CO-(CHR¹)ₙ-;

-(CHR¹)ₘ-N(R¹)-CO-(CHR¹)ₗ-CO-(CHR¹)ₙ-;

-(CHR¹)ₘ-N(R¹)-CO-(CHR¹)ₗ-CO-N(R¹)-(CHR¹)ₙ-;

-(CHR¹)ₘ-N(R¹)-CO-(CHR¹)ₗ-N(R¹)-CO-(CHR¹)ₙ-;

-(CHR¹)ₘ-CO-N(R¹)-(CHR¹)ₗ-CO-N(R¹)-(CHR¹)ₙ-,

-(CHR¹)ₘ-CO-N(R¹)-(CHR¹)ₗ-N(R¹)-CO-(CHR¹)ₙ-;

-(CHR¹)ₘ-C₆₋₁₀arylene-CO-O-(CHR¹)ₙ-;

-(CHR¹)ₘ-C₆₋₁₀arylene-O-CO-(CHR¹)ₙ-; -(CHR¹)ₘ-CO-O-C₆₋₁₀arylene-(CHR¹)ₙ-;

-(CHR¹)ₘ-O-CO-C₆₋₁₀arylene-(CHR¹)ₙ-;

-(CHR¹)ₘ-C₆₋₁₀arylene-N(R¹)-CO-(CHR¹)ₙ-;

-(CHR¹)ₘ-C₆₋₁₀arylene-CO-N(R¹)-(CHR¹)ₙ-;

-(CHR¹)ₘ-N(R¹)-CO-C₆₋₁₀arylene-(CHR¹)ₙ-;

-(CHR¹)ₘ-CO-N(R¹)-C₆₋₁₀arylene-(CHR¹)ₙ-;

wherein k is selected from: 0, 1, 2, 3, 4, 5, 6, 7, 8, 9, 10, 11, and 12, m and n are independently selected from: 0, 1, 2, 3, 4, 5, and 6, 1 is selected from 1, 2, 3, 4, 5, and 6, and each R¹ is independently selected from: H, or C₁₋₆ alkyl.

Preferably, L₁ is selected from a group represented by the structure of the following general formulas:

-(CHR¹)ₖ-;

-(CHR¹)ₘ-O-(CHR¹)ₙ-;

-(CHR¹)ₘ-S-(CHR¹)ₙ-;

-(CHR¹)ₘ-N(R¹)-(CHR¹)ₙ-;

-(CHR¹)ₘ-CO-(CHR¹)ₙ-;

-(CHR¹)ₘ-CO-O-(CHR¹)ₙ-;

-(CHR¹)ₘ-O-CO-(CHR¹)ₙ-;

-(CHR¹)ₘ-O-CO-O-(CHR¹)ₙ-;

-(CHR¹)ₘ-CO-N(R¹)-(CHR¹)ₙ-;

-(CHR¹)ₘ-N(R¹)-CO-(CHR¹)ₙ-;

-(CHR¹)ₘ-CO-N(R¹)-(CHR¹)ₙ-;

-(CHR¹)ₘ-N(R¹)-CO-N(R¹)-(CHR¹)ₙ-;

-(CHR¹)ₘ-CO-N(R¹)-CO-(CHR¹)ₙ-;

-(CHR¹)ₘ-O-(CHR¹)ₗ-O-(CHR¹)ₙ-;

-(CHR¹)ₘ-O-(CHR¹)ₗ-N(R¹)-(CHR¹)ₙ-;

-(CHR¹)ₘ-N(R¹)-(CHR¹)ₗ-O-(CHR¹)ₙ-;

-(CHR¹)ₘ-O-(CHR¹)ₗ-CO-(CHR¹)ₙ-;

-(CHR¹)ₘ-CO-(CHR¹)ₗ-O-(CHR¹)ₙ-;

-(CHR¹)ₘ-O-(CHR¹)ₗ-CO-N(R¹)-(CHR¹)ₙ-;

-(CHR¹)ₘ-CO-N(R¹)-(CHR¹)ₗ-O-(CHR¹)ₙ-;

-(CHR¹)ₘ-O-(CHR¹)ₗ-N(R¹)-CO-(CHR¹)ₙ-;

-(CHR¹)ₘ-N(R¹)-CO-(CHR¹)ₗ-O-(CHR¹)ₙ-;

-(CHR¹)ₘ-N(R¹)-(CHR¹)ₗ-N(R¹)-(CHR¹)ₙ-;

-(CHR¹)ₘ-N(R¹)-(CHR¹)ₗ-CO-(CHR¹)ₙ-;

-(CHR¹)ₘ-CO-(CHR¹)ₗ-N(R¹)-(CHR¹)ₙ-;

-(CHR¹)ₘ-N(R¹)-(CHR¹)ₗ-CO-N(R¹)-(CHR¹)ₙ-;

-(CHR¹)ₘ-CO-N(R¹)-(CHR¹)ₗ-N(R¹)-(CHR¹)ₙ-;

-(CHR¹)ₘ-N(R¹)-(CHR¹)ₗ-N(R¹)-CO-(CHR¹)ₙ-;

-(CHR¹)ₘ-N(R¹)-CO-(CHR¹)ₗ-N(R¹)-(CHR¹)ₙ-;

-(CHR¹)ₘ-CO-(CHR¹)ₗ-CO-N(R¹)-(CHR¹)ₙ-;

-(CHR¹)ₘ-CO-N(R¹)-(CHR¹)ₗ-CO-(CHR¹)ₙ-;

-(CHR¹)ₘ-CO-(CHR¹)ₗ-N(R¹)-CO-(CHR¹)ₙ-;

-(CHR¹)ₘ-N(R¹)-CO-(CHR¹)ₗ-CO-(CHR¹)ₙ-;

-(CHR¹)ₘ-N(R¹)-CO-(CHR¹)ₗ-CO-N(R¹)-(CHR¹)ₙ-;

-(CHR¹)ₘ-N(R¹)-CO-(CHR¹)ₗ-N(R¹)-CO-(CHR¹)ₙ-;

-(CHR¹)ₘ-CO-N(R¹)-(CHR¹)ₗ-CO-N(R¹)-(CHR¹)ₙ-;

-(CHR¹)ₘ-CO-N(R¹)-(CHR¹)ₗ-N(R¹)-CO-(CHR¹)ₙ-;

wherein k is selected from: 0, 1, 2, 3, 4, 5, 6, 7, 8, 9, 10, 11, and 12, m and n are independently selected from: 0, 1, 2, 3, 4, 5, and 6, 1 is selected from 1, 2, 3, 4, 5, and 6, and each R¹ is independently selected from: H, or C₁₋₆ alkyl.

Preferably, L₁ is selected from a group represented by the structure of the following general formulas:

-(CHR¹)ₖ-;

-O-; -(CHR¹)ₘ-O-; -O-(CHR¹)ₙ-; -(CHR¹)ₘ-O-(CHR¹)ₙ-;

-N(R')-; -(CHR¹)ₘ-N(R¹)-; -N(R¹)-(CHR¹)ₙ-; -(CHR¹)ₘ-N(R¹)-(CHR¹)ₙ-;

-CO-; -(CHR¹)ₘ-CO-; -CO-(CHR¹)ₙ-; -(CHR¹)ₘ-CO-(CHR¹)ₙ-;

-CO-N(R¹)-; -(CHR¹)ₘ-CO-N(R¹)-; -CO-N(R¹)-(CHR¹)ₙ-; -(CHR¹)ₘ-CO-N(R¹)-(CHR¹)ₙ-;

-N(R¹)-CO-; -(CHR¹)ₘ-N(R¹)-CO-; -N(R¹)-CO-(CHR¹)ₙ-; -(CHR¹)ₘ-N(R¹)-CO-(CHR¹)ₙ-;

-CO-O-; -(CHR¹)ₘ-CO-O; -CO-O-(CHR¹)ₙ-; -(CHR¹)ₘ-CO-O-(CHR¹)ₙ-;

-O-CO-; -(CHR¹)ₘ-O-CO-; -O-CO-(CHR¹)ₙ-; -(CHR¹)ₘ-O-CO-(CHR¹)ₙ-;

-(CHR¹)ₘ-C₆arylene-N(R¹)-CO-(CHR¹)ₙ-;

-(CHR¹)ₘ-C₆arylene-CO-N(R¹)-(CHR¹)ₙ-;

wherein k, m and n are independently selected from: 1, 2, 3, 4, 5, and 6, each R¹ is independently selected from: H, methyl, or ethyl.

Preferably, L₁ is selected from a group represented by the structure of the following general formulas:

-(CHR¹)ₖ-;

-O-; -(CHR¹)ₘ-O-; -O-(CHR¹)ₙ-; -(CHR¹)ₘ-O-(CHR¹)ₙ-;

-N(R¹)-; -(CHR¹)ₘ-N(R¹)-; -N(R¹)-(CHR¹)ₙ-; -(CHR¹)ₘ-N(R¹)-(CHR¹)ₙ-;

-CO-; -(CHR¹)ₘ-CO-; -CO-(CHR¹)ₙ-; -(CHR¹)ₘ-CO-(CHR¹)ₙ-;

-CO-N(R¹)-; -(CHR¹)ₘ-CO-N(R¹)-; -CO-N(R¹)-(CHR¹)ₙ-; -(CHR¹)ₘ-CO-N(R¹)-(CHR¹)ₙ-;

-N(R¹)-CO-; -(CHR¹)ₘ-N(R¹)-CO-; -N(R¹)-CO-(CHR¹)ₙ-; -(CHR¹)ₘ-N(R¹)-CO-(CHR¹)ₙ-;
wherein k, m and n are independently selected from: 1, 2, 3, 4, 5, and 6, each R¹ is independently selected from: H, methyl, or ethyl.
preferably, L₁ is selected from one of the following structures: 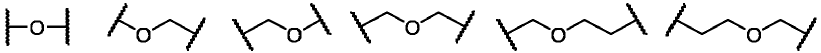 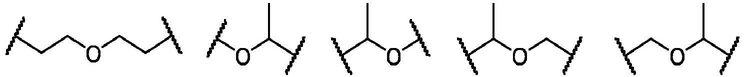 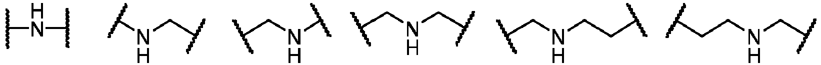 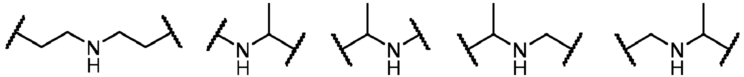 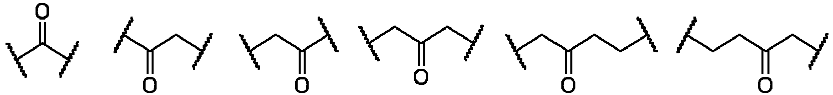 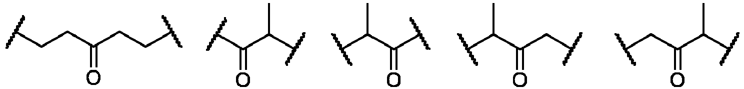 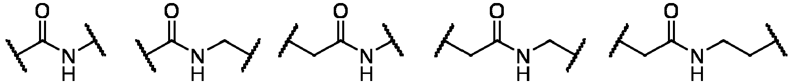 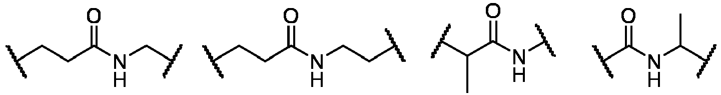 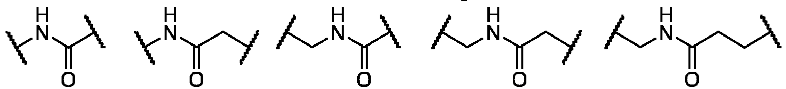 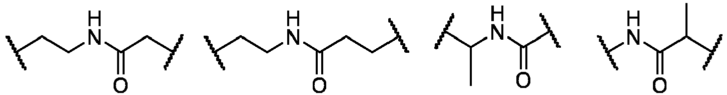 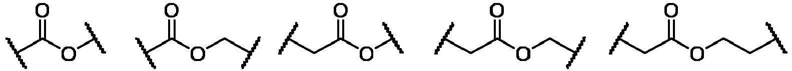 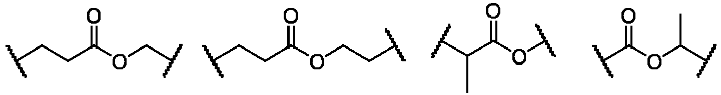 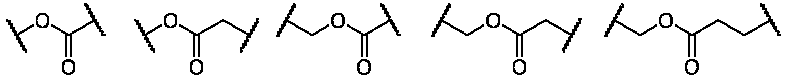 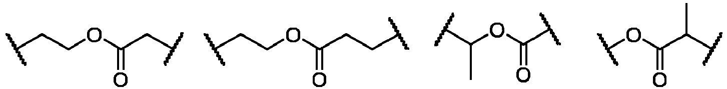 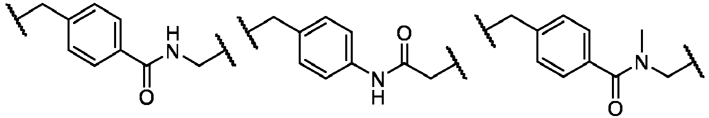 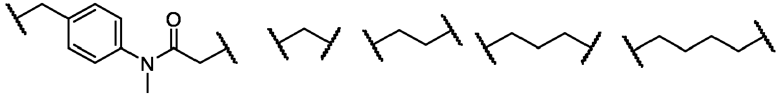
preferably, L₁ is selected from one of the following structures:

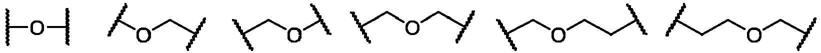

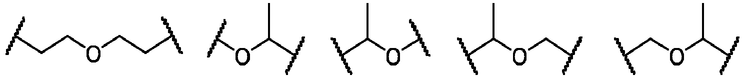

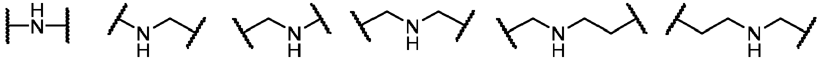

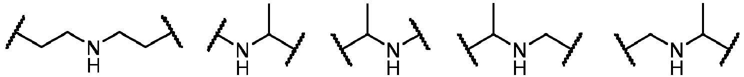

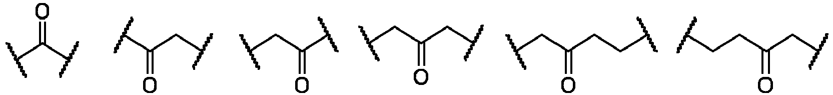

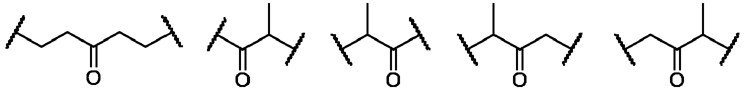

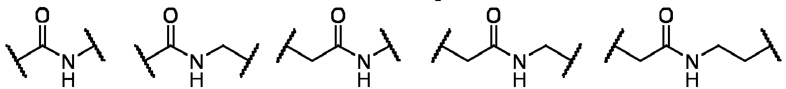

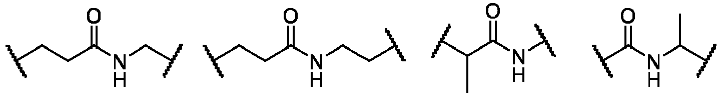

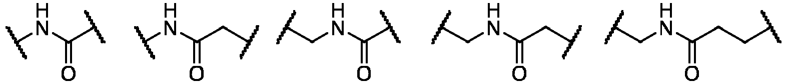

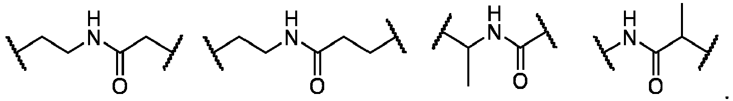

In certain embodiments, 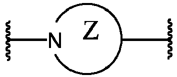 is selected from one of the following structures: 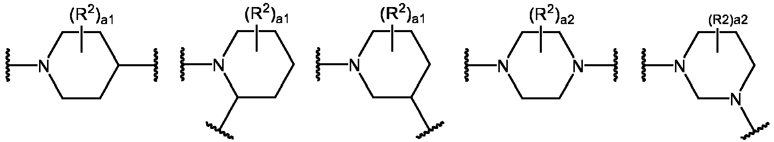 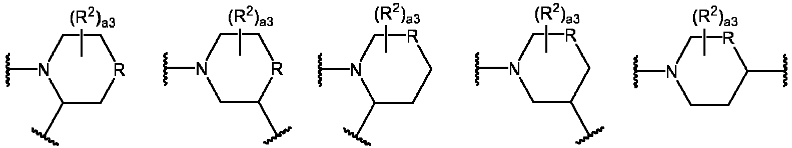 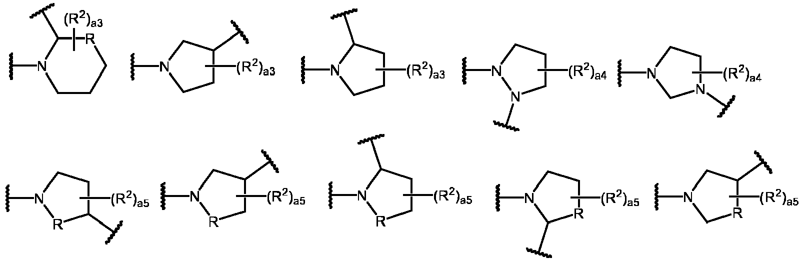 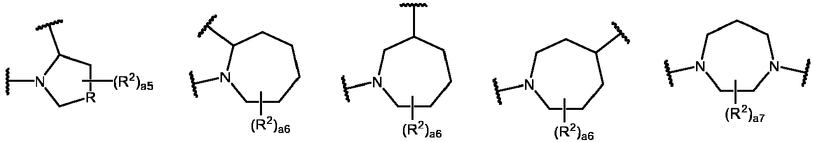 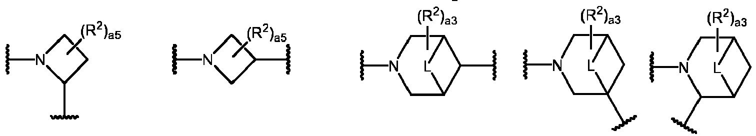 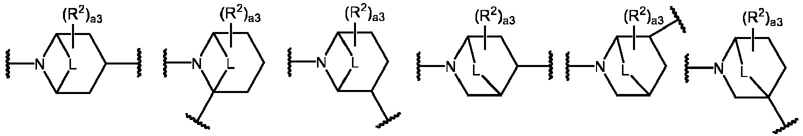 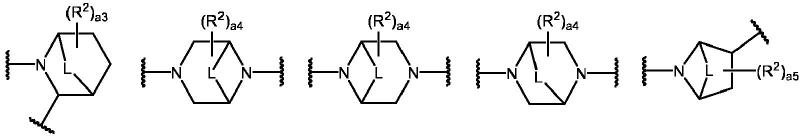 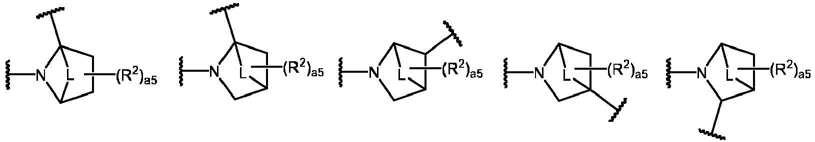 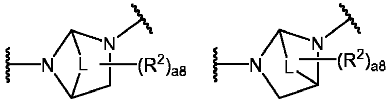
wherein each R² is independently selected from: H, halogen, cyano, nitro, C₁₋₁₅ alkyl optionally substituted with halogen, C₃₋₁₅ cycloalkyl, -COOR', -CONR' R", -OR', or -NR'R";
R' and R" are independently selected from: H, C₁₋₆ alkyl optionally substituted with halogen, or C₃₋₆ cycloalkyl;
each a1 is independently selected from: 0, 1, 2, 3, 4, 5, 6, 7, 8, and 9, each a2 is independently selected from: 0, 1, 2, 3, 4, 5, 6, 7, and 8, each a3 is independently selected from: 0, 1, 2, 3, 4, 5, 6, and 7, each a4 is independently selected from: 0, 1, 2, 3, 4, 5, and 6, each a5 is independently selected from: 0, 1, 2, 3, 4, and 5, each a6 is independently selected from: 0, 1, 2, 3, 4, 5, 6, 7, 8, 9, 10, and 11, each a7 is independently selected from: 0, 1, 2, 3, 4, 5, 6, 7, 8, 9, and 10, and each a8 is independently selected from 0, 1, 2, 3, and 4;
each R is independently selected from: O, S, or NR³, wherein R³ is selected from: H, C₁₋₆ alkyl, or -COC₁₋₆ alkyl;
each L is independently selected from: -CH₂-, -CH₂CH₂-, -C(CH₃)₂-, or - C(CH₃)₂CH₂-.
preferably, each R² is independently selected from: H, halogen, cyano, methyl, CH₂F, CHF₂, CF₃, methoxy and CF₃O.

Preferably, each R is independently selected from: O, S, NH, NCH₃, and NCOCH₃.

Preferably, each L is independently selected from: -CH₂-, or -CH₂CH₂-. more preferably, 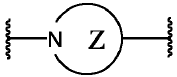 is selected from one of the following structures:

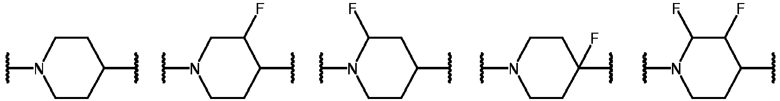

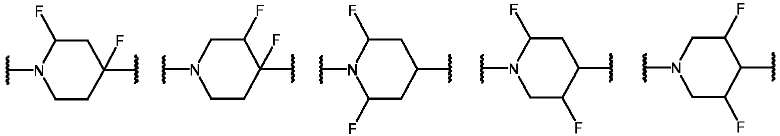

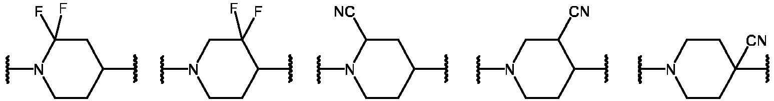

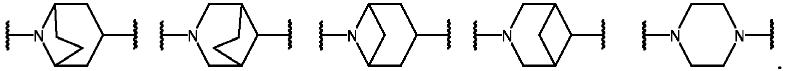

Most preferably, 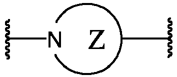 is selected from one of the following structures:

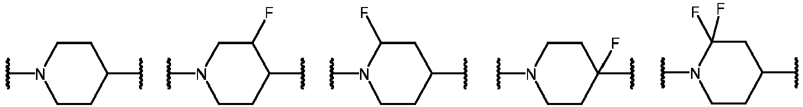

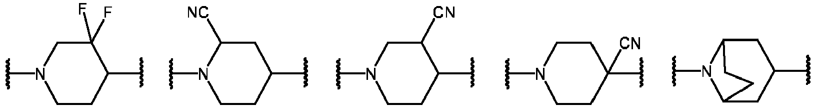

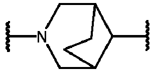

In certain embodiments, L₂ is selected from: a bond, or a group represented by the structure of the following general formula:

-(CHR¹)ₖ-;

-O-; -(CHR¹)ₘ-O-; -O-(CHR¹)ₙ-; -(CHR¹)ₘ-O-(CHR¹)ₙ-;

-N(R¹)-; -(CHR¹)ₘ-N(R¹)-; -N(R¹)-(CHR¹)ₙ₋; -(CHR¹)ₘ-N(R¹)-(CHR¹)ₙ-;

-CO-; -(CHR¹)ₘ-CO-; -CO-(CHR¹)ₙ-; -(CHR¹)ₘ-CO-(CHR¹)ₙ-;

-CO-N(R¹)-; -(CHR¹)ₘ-CO-N(R¹)-; -CO-N(R¹)-(CHR¹)ₙ-; -(CHR¹)ₘ-CO-N(R¹)-(CHR¹)ₙ-;

-N(R¹)-CO-; -(CHR¹)ₘ-N(R¹)-CO-; -N(R¹)-CO-(CHR¹)ₙ-; -(CHR¹)ₘ-N(R¹)-CO-(CHR¹)ₙ-;

wherein k, m and n are independently selected from: 1, 2, 3, 4, 5, and 6, each R¹ is independently selected from: H, methyl, or ethyl.

In certain embodiments, L₂ is selected from: a bond, or one of the following structures:

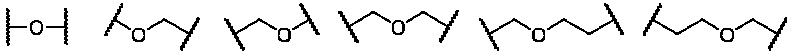

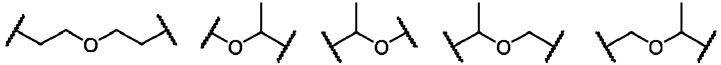

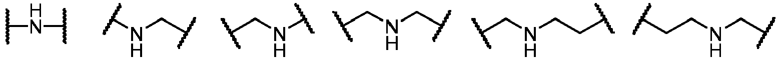

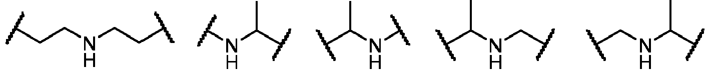

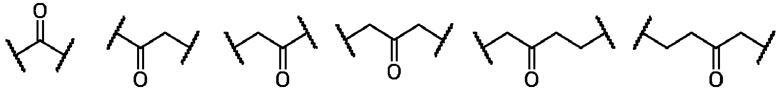

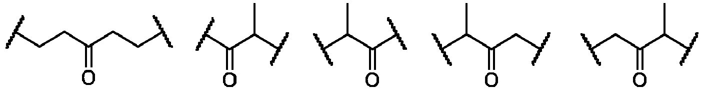

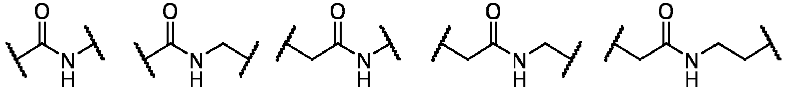

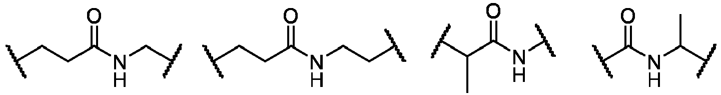

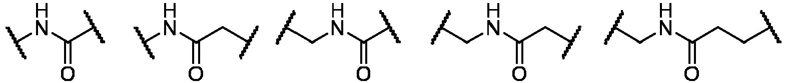

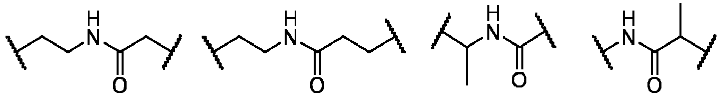

In certain embodiments, Cy₁ is selected from: optionally substituted C₆₋₁₈ aryl, optionally substituted 5- to 18-membered heteroaryl, optionally substituted 5- to 18-membered heterocyclyl, optionally substituted bi-C₆₋₁₈ aryl or optionally substituted 5- to 18-membered heteroaryl.

In certain embodiments, Cy₁ is selected from: optionally substituted C₆₋₁₄ aryl, optionally substituted 5- to 10-membered heteroaryl, optionally substituted 5- to 10-membered heterocyclyl, optionally substituted bi-C₆₋₁₄ aryl or optionally substituted 5- to 10-membered heteroaryl.

In certain embodiments, Cy₁ is selected from: optionally substituted C₆₋₁₀ aryl, optionally substituted 5- to 7-membered heteroaryl, optionally substituted 5- to 7-membered heterocyclyl, optionally substituted bi-C₆₋₁₀ aryl or optionally substituted 5- to 7-membered heteroaryl.

In certain embodiments, Cy₁ is selected from: optionally substituted C₆₋₁₀ aryl, and optionally substituted 5- to 6-membered heteroaryl.

In certain embodiments, Cy₁ is selected from optionally substituted: phenyl, naphthyl, anthracyl, phenanthrenyl, triphenylene, fluorenyl, biphenyl, furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyrimidinyl, pyridazinyl, pyrazinyl, triazinyl, indolyl, isoindolyl, benzoimidazolyl, benzopyrazolyl, benzofuranyl, benzothienyl, benzoxazolyl, benzothiazolyl, carbazolyl, azacarbazolyl, quinolinyl, isoquinolinyl, indolizinyl, azaindolizinyl, dibenzofuranyl, dibenzothienyl, naphthyridinyl, azetidinyl, oxacyclobutyl, pyrrolidinyl, pyrrolinyl, pyrazolidinyl, pyrazolinyl, imidazolidinyl, imidazolinyl, oxazolidinyl, oxazolinyl, isoxazolidinyl, isoxazolinyl, thiazolidinyl, thiazolinyl, isothiazolidinyl, isothiazolinyl, dihydrofuranyl, dihydrothienyl, tetrahydrofuranyl, tetrahydrothienyl, dioxacyclopentyl, dithiacyclopentyl, oxathiacyclopentyl, oxanyl, thianyl, dihydropyranyl, piperidinyl, morpholinyl, thiomorpholinyl, piperazinyl, N-methylpiperazinyl, dithianyl, hexahydropyridazinyl, hexahydropyrimidinyl, homopiperidinyl, homopiperazinyl, tetrahydroquinolinyl, indolinyl, pyridonyl, 2-pyrrolidonyl, 1,4-benzodioxanyl, 1,3-benzodioxolyl, ethyleneureido, phthalimidyl, or succinimidyl.
preferably, Cy₁ is selected from optionally substituted phenyl, naphthyl, anthracyl, phenanthrenyl, furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyrimidinyl, pyridazinyl, pyrazinyl, triazinyl, carbazolyl, azacarbazolyl, and tetrahydroquinolinyl.
preferably, the "optionally substituted" refers to being unsubstituted or mono- or poly-substituted with a group selected from the following: halogen, hydroxyl, thiol, carboxyl, cyano, nitro, amino, C₁₋₆ alkyl optionally substituted with halogen, C₁₋₆ alkoxy optionally substituted with halogen, C₁₋₆ alkylthio optionally substituted with halogen, C₃₋₆ cycloalkyl, C₆₋₁₄ aryl, C₆₋₁₄ arylC₁₋₂ alkyl-, 5- to 7-membered heteroaryl, 5- to 7-membered heterocyclyl, C₁₋₆ ester group, C₁₋₆ acyl, C₁₋₆ alkylamino, di(C₁₋₆ alkyl)amino, or amido substituted with 0, 1 or 2 C₁₋₆ alkyl, wherein the aryl, heteroaryl, and heterocyclyl, alone or as part of a group, can be optionally substituted with a group selected from halogen, hydroxyl, thiol, carboxyl, cyano, nitro, C₁₋₆ alkyl optionally substituted with halogen, C₁₋₆ alkoxy optionally substituted with halogen, or C₃₋₆ cycloalkyl.
preferably, the "optionally substituted" refers to being unsubstituted or mono- or poly-substituted with a group selected from the following: halogen, hydroxyl, cyano, nitro, C₁₋₆ alkyl optionally substituted with halogen, C₁₋₆ alkoxy optionally substituted with halogen, C₃₋₆ cycloalkyl, C₆₋₁₀ aryl, C₆₋₁₀ arylC₁₋₂ alkyl-, 5- to 6-membered heteroaryl, 5- to 7-membered heterocyclyl, C₁₋₆ ester group, C₁₋₆ acyl, C₁₋₆ alkylamino, di(C₁₋₆ alkyl)amino, or amido substituted with 0, 1 or 2 C₁₋₆ alkyl, wherein the aryl, heteroaryl, and heterocyclyl, alone or as part of a group, can be optionally substituted with a group selected from cyano, nitro, halogen, C₁₋₄ alkyl optionally substituted with halogen, or C₁₋₄ alkoxy optionally substituted with halogen.
preferably, the "optionally substituted" refers to being unsubstituted or mono- or poly-substituted with a group selected from the following: halogen, hydroxyl, cyano, nitro, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, cyclohexyl, phenyl, naphthyl, pyridyl, pyrimidinyl, - COOMe, -COOEt, -COMe, -COEt, amino, methylamino, dimethylamino, ethylamino, diethylamino, -CONH₂, -CONHMe, -CONHEt, -CON(Me)₂, or - CON(Et)₂.

Therefore, in certain embodiments, 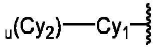 is selected from one of the following structures: 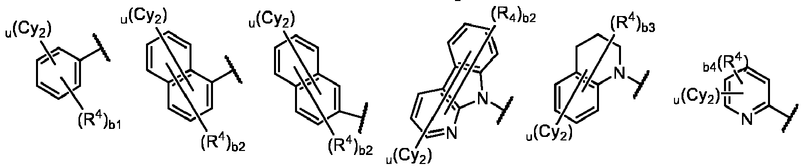 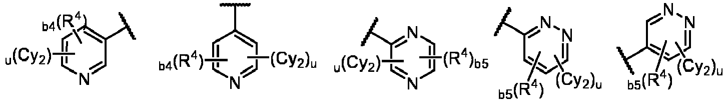 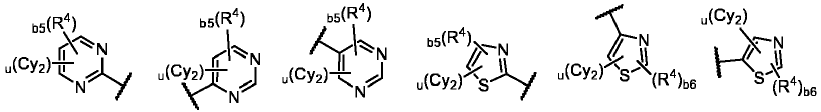 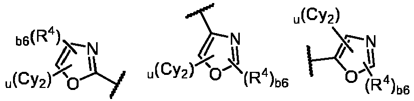
each R⁴ is independently selected from: halogen, hydroxyl, thiol, carboxyl, cyano, nitro, amino, C₁₋₆ alkyl optionally substituted with halogen, C₁₋₆ alkoxy optionally substituted with halogen, C₁₋₆ alkylthio optionally substituted with halogen, C₃₋₆ cycloalkyl, C₆₋₁₄ aryl, C₆₋₁₄ arylC₁₋₂ alkyl-, 5- to 7-membered heteroaryl, 5- to 7-membered heterocyclyl, C₁₋₆ ester group, C₁₋₆ acyl, C₁₋₆ alkylamino, di(C₁₋₆ alkyl)amino, or amido substituted with 0, 1 or 2 C₁₋₆ alkyl, wherein the aryl, heteroaryl, and heterocyclyl, alone or as part of a group, can be optionally substituted with a group selected from halogen, hydroxyl, thiol, carboxyl, cyano, nitro, C₁₋₆ alkyl optionally substituted with halogen, C₁₋₆ alkoxy optionally substituted with halogen, or C₃₋₆ cycloalkyl.
preferably, the R⁴ is independently selected from: halogen, hydroxyl, cyano, nitro, C₁₋₆ alkyl optionally substituted with halogen, C₁₋₆ alkoxy optionally substituted with halogen, C₃₋₆ cycloalkyl, C₆₋₁₀ aryl, C₆₋₁₀ arylC₁₋₂ alkyl-, 5- to 6-membered heteroaryl, 5- to 7-membered heterocyclyl, C₁₋₆ ester group, C₁₋₆ acyl, C₁₋₆ alkylamino, di(C₁₋₆ alkyl)amino, or amido substituted with 0, 1 or 2 C₁₋₆ alkyl, wherein the aryl, heteroaryl, and heterocyclyl, alone or as part of a group, can be optionally substituted with a group selected from cyano, nitro, halogen, C₁₋₄ alkyl optionally substituted with halogen, or C₁₋₄ alkoxy optionally substituted with halogen.
preferably, the R⁴ is independently selected from: halogen, hydroxyl, cyano, nitro, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, cyclohexyl, phenyl, naphthyl, pyridyl, pyrimidinyl, -COOMe, - COOEt, -COMe, -COEt, amino, methylamino, dimethylamino, ethylamino, diethylamino, -CONH₂, -CONHMe, -CONHEt, -CON(Me)₂, or -CON(Et)₂.
each b1 is independently selected from 0, 1, 2, 3, 4, and 5, each b2 is independently selected from 0, 1, 2, 3, 4, 5, 6, and 7, each b3 is independently selected from 0, 1, 2, 3, 4, 5, 6, 7, 8, 9, and 10, each b4 is independently selected from 0, 1, 2, 3, and 4, each b5 is independently selected from 0, 1, 2, and 3, and each b6 is independently selected from 0, 1, and 2.

When u is selected from 1, Cy₁ is selected from one of the following structures:

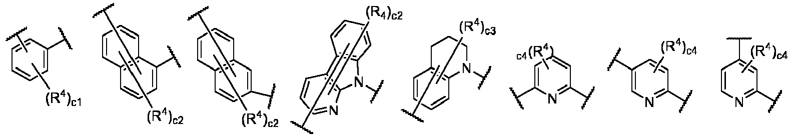

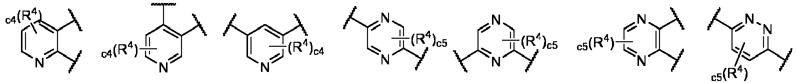

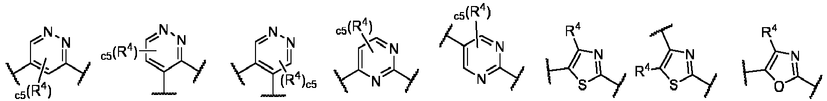

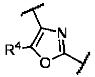

Among them, R₄ is as defined above.
each c1 is independently selected from 0, 1, 2, 3, and 4, each c2 is independently selected from 0, 1, 2, 3, 4, 5, and 6, each c3 is independently selected from 0, 1, 2, 3, 4, 5, 6, 7, 8, and 9, each c4 is independently selected from 0, 1, 2, and 3, and each c5 is independently selected from 0, 1, and 2.

In certain embodiments, Cy₂ is independently selected from: H, optionally substituted C₆₋₁₈ aryl, optionally substituted 5- to 18-membered heteroaryl, optionally substituted 5- to 18-membered heterocyclyl, optionally substituted bi-C₆₋₁₈ aryl or optionally substituted 5- to 18-membered heteroaryl.

In certain embodiments, Cy₂ is independently selected from: H, optionally substituted C₆₋₁₄ aryl, optionally substituted 5- to 10-membered heteroaryl, optionally substituted 5- to 10-membered heterocyclyl, optionally substituted bi-C₆₋₁₄ aryl or optionally substituted 5- to 10-membered heteroaryl.

In certain embodiments, Cy₂ is independently selected from: H, optionally substituted C₆₋₁₀ aryl, optionally substituted 5- to 7-membered heteroaryl, optionally substituted 5- to 7-membered heterocyclyl, optionally substituted bi-C₆₋₁₀ aryl or optionally substituted 5- to 7-membered heteroaryl.

In certain embodiments, Cy₂ is independently selected from: H, optionally substituted C₆₋₁₀ aryl, or optionally substituted 5- to 6-membered heteroaryl.

In certain embodiments, Cy₂ is independently selected from: H, or optionally substituted phenyl, naphthyl, anthracyl, phenanthrenyl, triphenylene, fluorenyl, biphenyl, furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyrimidinyl, pyridazinyl, pyrazinyl, triazinyl, indolyl, isoindolyl, benzoimidazolyl, benzopyrazolyl, benzofuranyl, benzothienyl, benzoxazolyl, benzothiazolyl, carbazolyl, azacarbazolyl, quinolinyl, isoquinolinyl, indolizinyl, azaindolizinyl, dibenzofuranyl, dibenzothienyl, naphthyridinyl, azetidinyl, oxacyclobutyl, pyrrolidinyl, pyrrolinyl, pyrazolidinyl, pyrazolinyl, imidazolidinyl, imidazolinyl, oxazolidinyl, oxazolinyl, isoxazolidinyl, isoxazolinyl, thiazolidinyl, thiazolinyl, isothiazolidinyl, isothiazolinyl, dihydrofuranyl, dihydrothienyl, tetrahydrofuranyl, tetrahydrothienyl, dioxacyclopentyl, dithiacyclopentyl, oxathiacyclopentyl, oxanyl, thianyl, dihydropyranyl, piperidinyl, morpholinyl, thiomorpholinyl, piperazinyl, N-methylpiperazinyl, dithianyl, hexahydropyridazinyl, hexahydropyrimidinyl, homopiperidinyl, homopiperazinyl, tetrahydroquinolinyl, indolinyl, pyridonyl, 2-pyrrolidonyl, 1,4-benzodioxanyl, 1,3-benzodioxolyl, ethyleneureido, phthalimidyl, or succinimidyl.
preferably, Cy₂ is selected from: H, or optionally substituted phenyl, naphthyl, anthracyl, phenanthrenyl, triphenylene, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyrimidinyl, pyridazinyl, pyrazinyl, or triazinyl.
preferably, the "optionally substituted" refers to being unsubstituted or mono- or poly-substituted with a group selected from the following: halogen, hydroxyl, thiol, carboxyl, cyano, nitro, amino, C₁₋₆ alkyl optionally substituted with halogen, C₁₋₆ alkoxy optionally substituted with halogen, C₁₋₆ alkylthio optionally substituted with halogen, C₃₋₆ cycloalkyl, C₆₋₁₄ aryl, C₆₋₁₄ arylC₁₋₂ alkyl-, 5- to 7-membered heteroaryl, 5- to 7-membered heterocyclyl, C₁₋₆ ester group, C₁₋₆ acyl, C₁₋₆ alkylamino, di(C₁₋₆ alkyl)amino, or amido substituted with 0, 1 or 2 C₁₋₆ alkyl, wherein the aryl, heteroaryl, and heterocyclyl, alone or as part of a group, can be optionally substituted with a group selected from halogen, hydroxyl, thiol, carboxyl, cyano, nitro, C₁₋₆ alkyl optionally substituted with halogen, C₁₋₆ alkoxy optionally substituted with halogen, or C₃₋₆ cycloalkyl.
preferably, the "optionally substituted" refers to being unsubstituted or mono- or poly-substituted with a group selected from the following: halogen, hydroxyl, cyano, nitro, C₁₋₆ alkyl optionally substituted with halogen, C₁₋₆ alkoxy optionally substituted with halogen, C₃₋₆ cycloalkyl, C₆₋₁₀ aryl, C₆₋₁₀ arylC₁₋₂ alkyl-, 5- to 6-membered heteroaryl, 5- to 7-membered heterocyclyl, C₁₋₆ ester group, C₁₋₆ acyl, C₁₋₆ alkylamino, di(C₁₋₆ alkyl)amino, or amido substituted with 0, 1 or 2 C₁₋₆ alkyl, wherein the aryl, heteroaryl, and heterocyclyl, alone or as part of a group, can be optionally substituted with a group selected from cyano, nitro, halogen, C₁₋₄ alkyl optionally substituted with halogen, or C₁₋₄ alkoxy optionally substituted with halogen.
preferably, the "optionally substituted" refers to being unsubstituted or mono- or poly-substituted with a group selected from the following: halogen, hydroxyl, cyano, nitro, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, cyclohexyl, phenyl, naphthyl, pyridyl, pyrimidinyl, - COOMe, -COOEt, -COMe, -COEt, amino, methylamino, dimethylamino, ethylamino, diethylamino, -CONH₂, -CONHMe, -CONHEt, -CON(Me)₂, or - CON(Et)₂.

In certain embodiments, Cy₂ is selected from: H, or one of the following structures: 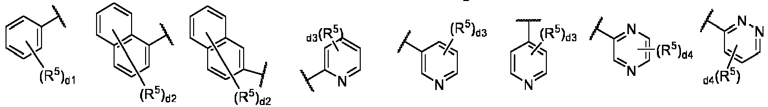 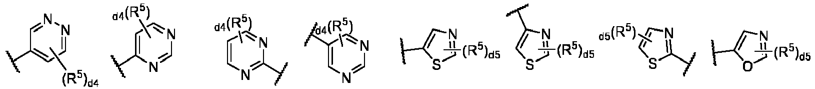 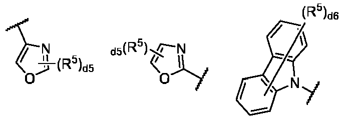
each R⁵ is independently selected from: halogen, hydroxyl, thiol, carboxyl, cyano, nitro, amino, C₁₋₆ alkyl optionally substituted with halogen, C₁₋₆ alkoxy optionally substituted with halogen, C₁₋₆ alkylthio optionally substituted with halogen, C₃₋₆ cycloalkyl, C₆₋₁₄ aryl, C₆₋₁₄ arylC₁₋₂ alkyl-, 5- to 7-membered heteroaryl, 5- to 7-membered heterocyclyl, C₁₋₆ ester group, C₁₋₆ acyl, C₁₋₆ alkylamino, di(C₁₋₆ alkyl)amino, or amido substituted with 0, 1 or 2 C₁₋₆ alkyl, wherein the aryl, heteroaryl, and heterocyclyl, alone or as part of a group, can be optionally substituted with a group selected from halogen, hydroxyl, thiol, carboxyl, cyano, nitro, C₁₋₆ alkyl optionally substituted with halogen, C₁₋₆ alkoxy optionally substituted with halogen, or C₃₋₆ cycloalkyl.
preferably, each R⁵ is independently selected from: halogen, hydroxyl, cyano, nitro, C₁₋₆ alkyl optionally substituted with halogen, C₁₋₆ alkoxy optionally substituted with halogen, C₃₋₆ cycloalkyl, C₆₋₁₀ aryl, C₆₋₁₀ arylC₁₋₂ alkyl-, 5- to 6-membered heteroaryl, 5- to 7-membered heterocyclyl, C₁₋₆ ester group, C₁₋₆ acyl, C₁₋₆ alkylamino, di(C₁₋₆ alkyl)amino, or amido substituted with 0, 1 or 2 C₁₋₆ alkyl, wherein the aryl, heteroaryl, and heterocyclyl, alone or as part of a group, can be optionally substituted with a group selected from cyano, nitro, halogen, C₁₋₄ alkyl optionally substituted with halogen, or C₁₋₄ alkoxy optionally substituted with halogen.
preferably, each R⁵ is independently selected from: halogen, hydroxyl, cyano, nitro, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, cyclohexyl, phenyl, naphthyl, pyridyl, pyrimidinyl, -COOMe, - COOEt, -COMe, -COEt, amino, methylamino, dimethylamino, ethylamino, diethylamino, -CONH₂, -CONHMe, -CONHEt, -CON(Me)₂, or -CON(Et)₂.
each d1 is independently selected from 0, 1, 2, 3, 4, and 5, each d2 is independently selected from 0, 1, 2, 3, 4, 5, 6, and 7, each d3 is independently selected from 0, 1, 2, 3, and 4, each d4 is independently selected from 0, 1, 2, and 3, each d5 is independently selected from 0, 1, and 2, each d6 is independently selected from 0, 1, 2, 3, 4, 5, 6, 7, and 8.
preferably, Cy₂ is selected from: H, or one of the following structures: 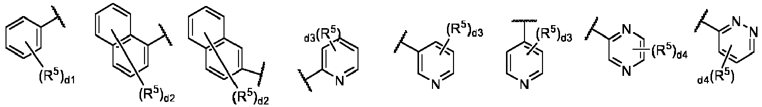 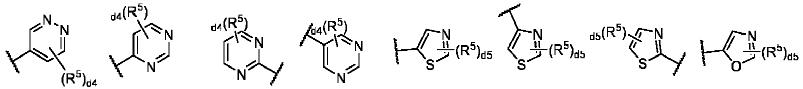 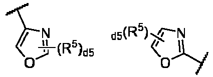
each R⁵ is independently selected from: halogen, hydroxyl, thiol, carboxyl, cyano, nitro, amino, C₁₋₆ alkyl optionally substituted with halogen, C₁₋₆ alkoxy optionally substituted with halogen, C₁₋₆ alkylthio optionally substituted with halogen, C₃₋₆ cycloalkyl, C₆₋₁₄ aryl, C₆₋₁₄ arylC₁₋₂ alkyl-, 5- to 7-membered heteroaryl, 5- to 7-membered heterocyclyl, C₁₋₆ ester group, C₁₋₆ acyl, C₁₋₆ alkylamino, di(C₁₋₆ alkyl)amino, or amido substituted with 0, 1 or 2 C₁₋₆ alkyl, wherein the aryl, heteroaryl, and heterocyclyl, alone or as part of a group, can be optionally substituted with a group selected from halogen, hydroxyl, thiol, carboxyl, cyano, nitro, C₁₋₆ alkyl optionally substituted with halogen, C₁₋₆ alkoxy optionally substituted with halogen, or C₃₋₆ cycloalkyl.
preferably, each R⁵ is independently selected from: halogen, hydroxyl, cyano, nitro, C₁₋₆ alkyl optionally substituted with halogen, C₁₋₆ alkoxy optionally substituted with halogen, C₃₋₆ cycloalkyl, C₆₋₁₀ aryl, C₆₋₁₀ arylC₁₋₂ alkyl-, 5- to 6-membered heteroaryl, 5- to 7-membered heterocyclyl, C₁₋₆ ester group, C₁₋₆ acyl, C₁₋₆ alkylamino, di(C₁₋₆ alkyl)amino, or amido substituted with 0, 1 or 2 C₁₋₆ alkyl, wherein the aryl, heteroaryl, and heterocyclyl, alone or as part of a group, can be optionally substituted with a group selected from cyano, nitro, halogen, C₁₋₄ alkyl optionally substituted with halogen, or C₁₋₄ alkoxy optionally substituted with halogen.
preferably, each R⁵ is independently selected from: halogen, hydroxyl, cyano, nitro, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, cyclohexyl, phenyl, naphthyl, pyridyl, pyrimidinyl, -COOMe, - COOEt, -COMe, -COEt, amino, methylamino, dimethylamino, ethylamino, diethylamino, -CONH₂, -CONHMe, -CONHEt, -CON(Me)₂, or -CON(Et)₂.

Each d1 is independently selected from 0, 1, 2, 3, 4, and 5, each d2 is independently selected from 0, 1, 2, 3, 4, 5, 6, and 7, each d3 is independently selected from 0, 1, 2, 3, and 4, each d4 is independently selected from 0, 1, 2, and 3, and each d5 is independently selected from 0, 1, and 2.

In certain embodiments, 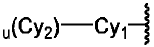 is selected from one of the following structures: 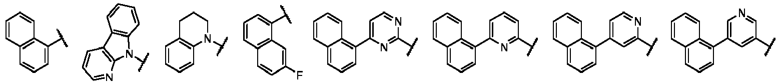 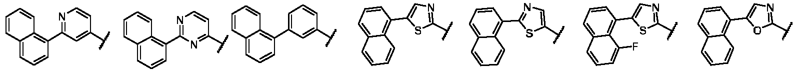 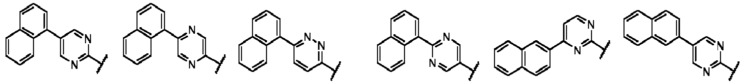 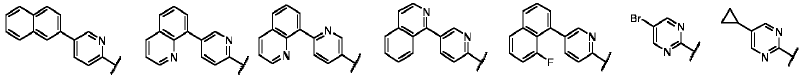 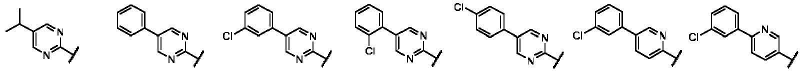 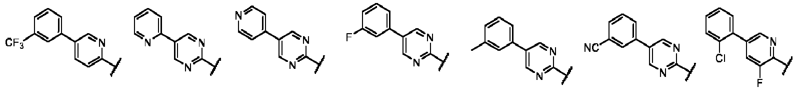 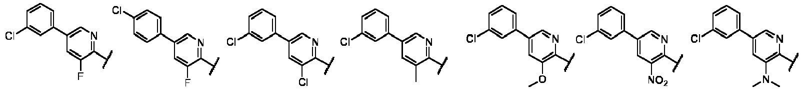 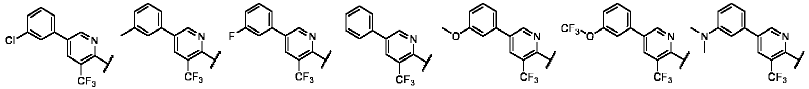 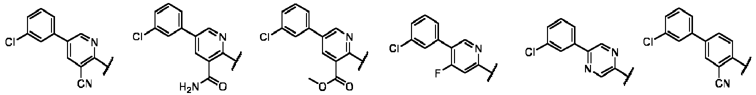 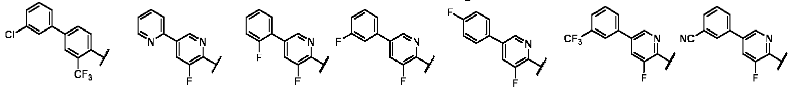 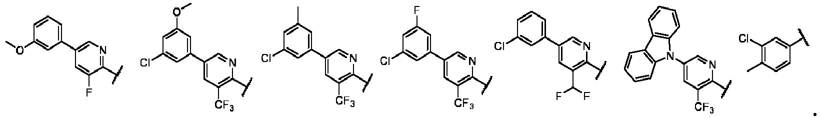 preferably, 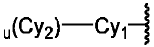 is selected from one of the following structures:

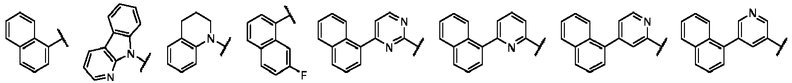

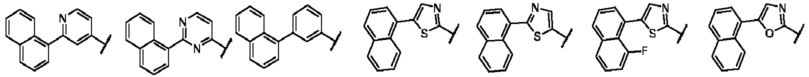

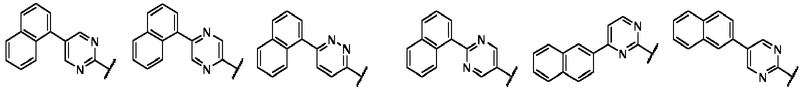

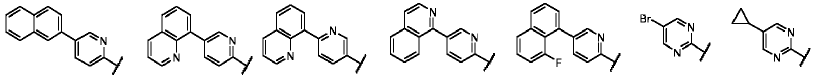

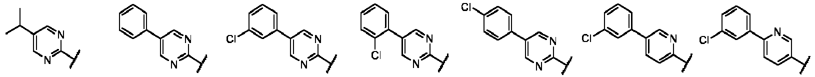

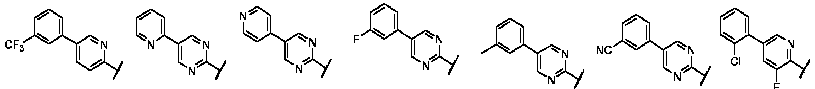

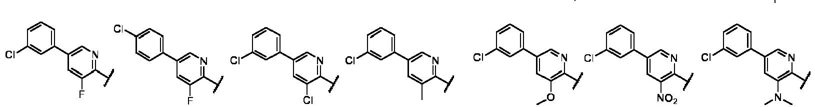

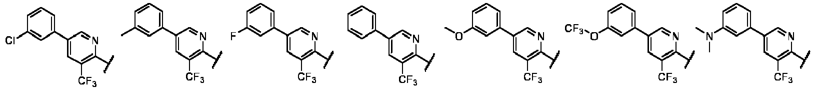

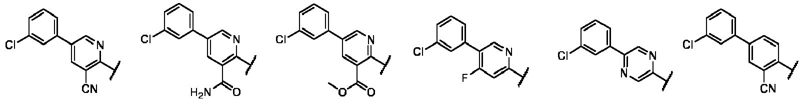

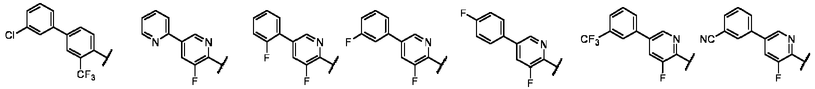

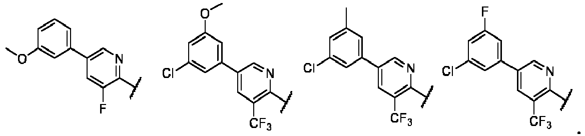

In certain embodiments, the compound of Formula (I) is preferably a compound of Formula (I-A). Therefore, the present invention provides a compound of formula (I-A) or a pharmaceutically acceptable salt, solvate, hydrate, stereoisomer, tautomer, or prodrug thereof:

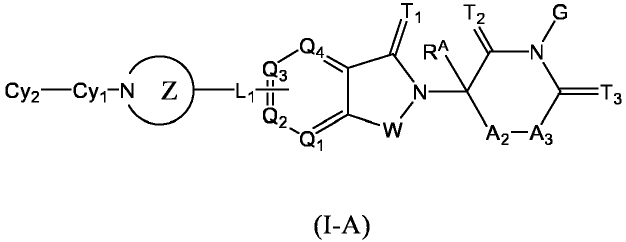

Among them, W, G, T₁-T₃, A₂-A₃, R^{A}, Q₁-Q₄, L₁, ring Z, Cy₁, and Cy₂ are as defined above.

In certain embodiments, each group below may also have the meaning defined above in addition to the preferred embodiments.

In certain embodiments, the compound of formula (I-A) of the present invention is selected from the following compound of formula (I-Aa) or formula (I-Ab):

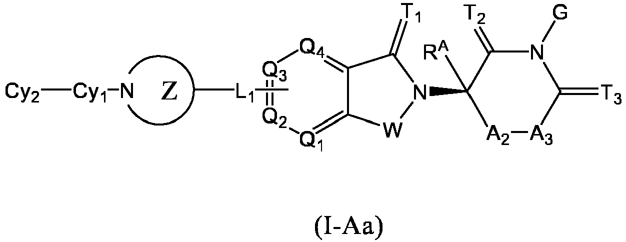

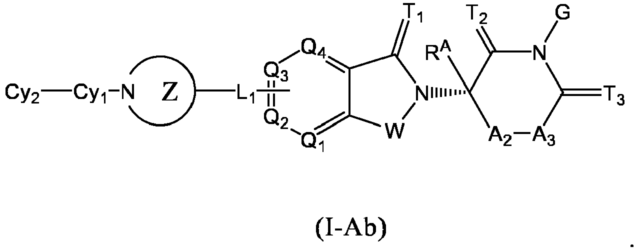

In certain embodiments, in the aforementioned compounds:
W is selected from: C(R^{W})₂, C=O, or C=S;
R^{W} is selected from: H, halogen, C₁₋₆ alkyl optionally substituted with halogen, C₁₋₆ alkoxy optionally substituted with halogen, or C₃₋₆ cycloalkyl;
G is selected from: H or C₁₋₆ alkyl, wherein the C₁₋₆ alkyl is optionally substituted with a group selected from the following: hydroxyl, -OPO(OR^{G})₂, - OCOR^{G}, or -OS(O)₁₋₂R^{G}, wherein each R^{G} is independently selected from: H, or C₁₋₄ alkyl;
T₁-T₃ are independently selected from: O or S;
A₂-A₃ are independently selected from: C(R^{A})₂;
each R^{A} is independently selected from: H, halogen, C₁₋₆ alkyl optionally substituted with halogen, C₁₋₆ alkoxy optionally substituted with halogen, or C₃₋₆ cycloalkyl;
Q₁-Q₄ are independently selected from: CR^{Q} or N;
R^{Q} is selected from: H, halogen, cyano, nitro, hydroxyl, C₁₋₆ alkyl optionally substituted with halogen, C₁₋₆ alkoxy optionally substituted with halogen, or C₃₋₆ cycloalkyl;
L₁ represents a linking group;
ring Z represents an optionally substituted heterocycloalkyl containing at least one N atom as a heteroatom, and is linked to Cy₁ through the N;
Cy¹ is selected from: optionally substituted aryl, optionally substituted heteroaryl, optionally substituted heterocyclyl, optionally substituted biaryl or optionally substituted biheteroaryl;
Cy² is selected from: H, optionally substituted aryl, optionally substituted heteroaryl, optionally substituted heterocyclyl, optionally substituted biaryl or optionally substituted biheteroaryl.

Preferably, in the aforementioned compounds:
W is selected from: C(R^{W})₂, or C=O;
R^{W} is selected from: H, halogen, C₁₋₆ alkyl optionally substituted with halogen, C₁₋₆ alkoxy optionally substituted with halogen, or C₃₋₆ cycloalkyl;
G is selected from: H, or C₁₋₆ alkyl, wherein the C₁₋₆ alkyl is substituted with a group selected from the following: -OPO(OR^{G})₂, wherein each R^{G} is independently selected from: H, or C₁₋₄ alkyl;
T₁-T₃ are independently selected from: O;
A₂-A₃ are independently selected from: C(R^{A})₂;
each R^{A} is independently selected from: H, halogen, cyano, C₁₋₆ alkyl optionally substituted with halogen, or C₁₋₆ alkoxy optionally substituted with halogen;
Q₁-Q₄ are independently selected from: CR^{Q} or N;
R^{Q} is selected from: H, halogen, cyano, C₁₋₆ alkyl optionally substituted with halogen, or C₁₋₆ alkoxy optionally substituted with halogen;
L₁ is selected from: -O-, -S-, -N(R¹)-, -CO-, -CO-O-, -O-CO-, -O-CO-O-, - CO-N (R¹)-, -N(R¹)-CO-, -N(R¹)-CO-N(R¹)-, -CO-N(R¹)-CO and optionally substituted C₁₋₁₅ alkylene-, where the head, tail or middle of the alkylene is optionally inserted by 1, 2, 3, 4, 5 or more groups selected from: -O-, -S-, -N(R¹)-, -CO-, -CO-O-, -O-CO-, -O-CO-O-, -CO-N (R¹)-, -N(R¹)-CO-, -N(R¹)-CO-N(R¹)-, -CO-N(R¹)-CO, C₂ alkenylene, C₂ alkynylene, C₃₋₆ cycloalkylene, C₆₋₁₀ arylene, 5-to 6-membered heteroarylene, 5- to 7-membered heterocyclylene or any combination thereof; wherein each R¹ is independently selected from: H, or C₁₋₆ alkyl optionally substituted with halogen; 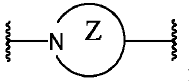 is selected from one of the following structures: 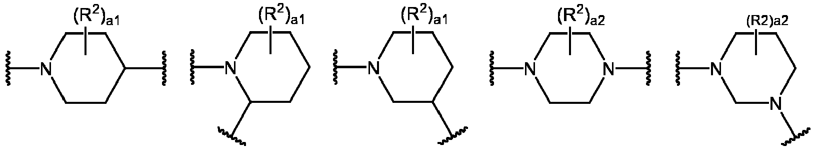 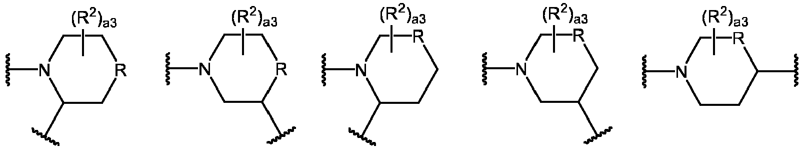 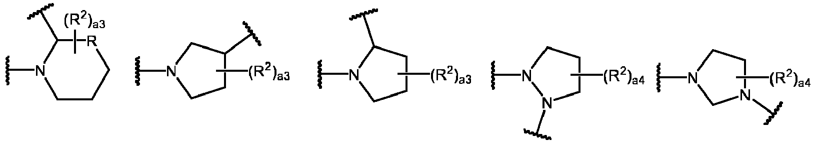 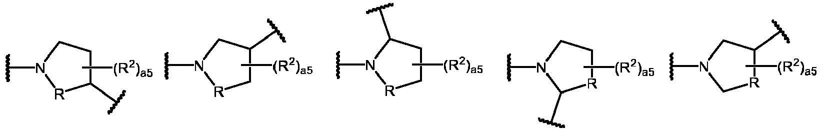 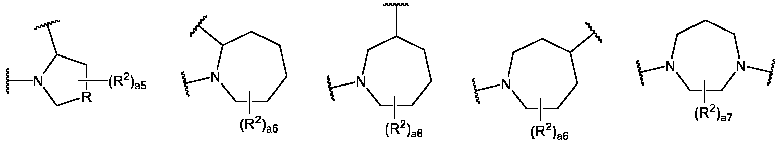 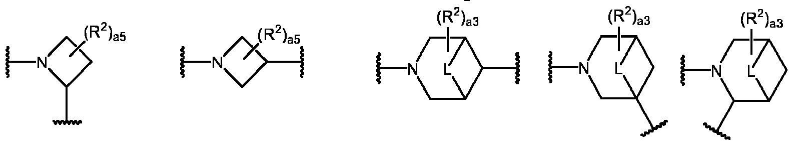 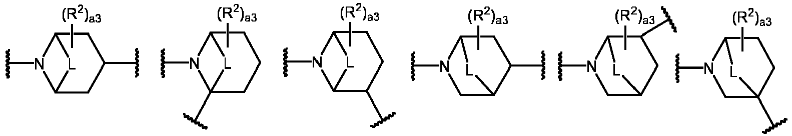 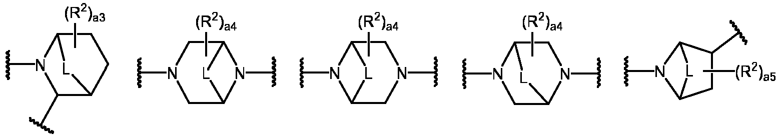 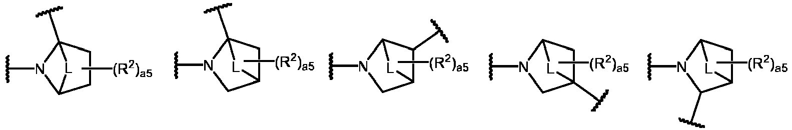 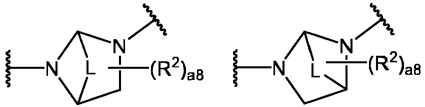
wherein each R² is independently selected from: H, halogen, cyano, nitro, C₁₋₁₅ alkyl optionally substituted with halogen, C₃₋₁₅ cycloalkyl, -COOR', -CONR' R", -OR', or -NR'R";
R' and R" are independently selected from: H, C₁₋₆ alkyl optionally substituted with halogen, or C₃₋₆ cycloalkyl;
each a1 is independently selected from: 0, 1, 2, 3, 4, 5, 6, 7, 8, and 9, each a2 is independently selected from: 0, 1, 2, 3, 4, 5, 6, 7, and 8, each a3 is independently selected from: 0, 1, 2, 3, 4, 5, 6, and 7, each a4 is independently selected from: 0, 1, 2, 3, 4, 5, and 6, each a5 is independently selected from: 0, 1, 2, 3, 4, and 5, each a6 is independently selected from: 0, 1, 2, 3, 4, 5, 6, 7, 8, 9, 10, and 11, each a7 is independently selected from: 0, 1, 2, 3, 4, 5, 6, 7, 8, 9, and 10, and each a8 is independently selected from 0, 1, 2, 3, and 4;
each R is independently selected from: O, S, or NR³, wherein R³ is selected from: H, C₁₋₆ alkyl, or -COC₁₋₆ alkyl;
each L is independently selected from: -CH₂-, -CH₂CH₂-, -C(CH₃)₂-, or - C(CH₃)₂CH₂-;
Cy¹ is selected from optionally substituted phenyl, naphthyl, anthracyl, phenanthrenyl, furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyrimidinyl, pyridazinyl, pyrazinyl, triazinyl, indolyl, isoindolyl, benzoimidazolyl, benzopyrazolyl, benzofuranyl, benzothienyl, benzoxazolyl, benzothiazolyl, quinolinyl, isoquinolinyl, indolizinyl, or azaindolizinyl;
Cy² is selected from H, and optionally substituted phenyl, naphthyl, anthracyl, phenanthrenyl, furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyrimidinyl, pyridazinyl, pyrazinyl, triazinyl, indolyl, isoindolyl, benzoimidazolyl, benzopyrazolyl, benzofuranyl, benzothienyl, benzoxazolyl, benzothiazolyl, quinolinyl, isoquinolinyl, indolizinyl, azaindolizinyl, or carbazolyl;
the "optionally substituted" refers to being unsubstituted or mono- or polysubstituted with a group selected from the following: halogen, hydroxyl, thiol, carboxyl, cyano, nitro, amino, C₁₋₆ alkyl optionally substituted with halogen, C₁₋₆ alkoxy optionally substituted with halogen, C₁₋₆ alkylthio optionally substituted with halogen, C₃₋₆ cycloalkyl, C₆₋₁₄ aryl, C₆₋₁₄ arylC₁₋₂ alkyl-, 5- to 7-membered heteroaryl, 5- to 7-membered heterocyclyl, C₁₋₆ ester group, C₁₋₆ acyl, C₁₋₆ alkylamino, di(C₁₋₆ alkyl)amino, or amido substituted with 0, 1 or 2 C₁₋₆ alkyl, wherein the aryl, heteroaryl, and heterocyclyl, alone or as part of a group, can be optionally substituted with a group selected from halogen, hydroxyl, thiol, carboxyl, cyano, nitro, C₁₋₆ alkyl optionally substituted with halogen, C₁₋₆ alkoxy optionally substituted with halogen, or C₃₋₆ cycloalkyl.

Preferably, in the aforementioned compounds:
W is selected from: CH₂, or C=O;
G is selected from: H, or C₁₋₆ alkyl, wherein the C₁₋₆ alkyl is substituted with a group selected from the following: -OPO(OR^{G})₂, wherein each R^{G} is independently selected from: H;
T₁-T₃ are independently selected from: O;
A₂-A₃ are independently selected from: CH₂;
R^{A} is selected from: H;
Q₁-Q₄ are independently selected from: CR^{Q} or N;
R^{Q} is selected from: H, or halogen;
L₁ is selected from: -O-, -S-, -N(R¹)-, -CO-, -CO-O-, -O-CO-, -O-CO-O-, - CO-N (R¹)-, -N(R¹)-CO-, -N(R¹)-CO-N(R¹)-, -CO-N(R¹)-CO and optionally substituted C₁₋₁₅ alkylene-, where the head, tail or middle of the alkylene is optionally inserted by 1, 2, 3, 4, 5 or more groups selected from: -O-, -S-, -N(R¹)-, -CO-, -CO-O-, -O-CO-, -O-CO-O-, -CO-N (R¹)-, -N(R¹)-CO-, -N(R¹)-CO-N(R¹)-, -CO-N(R¹)-CO or any combination thereof; wherein each R¹ is independently selected from: H, or C₁₋₆ alkyl optionally substituted with halogen; 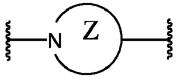 is selected from one of the following structures: 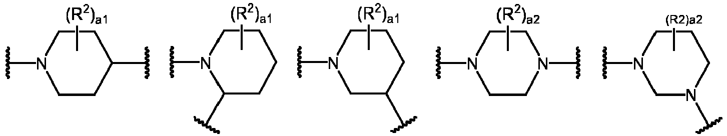 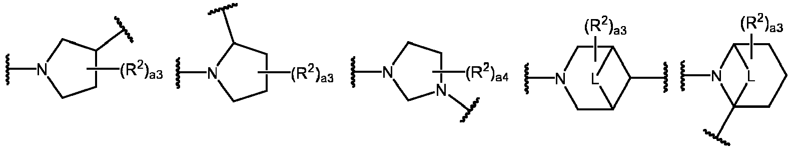
wherein each R² is independently selected from: H, halogen, cyano, nitro, hydroxyl, C₁₋₆ alkyl optionally substituted with halogen, or C₁₋₆ alkoxy optionally substituted with halogen;
each a1 is independently selected from: 0, 1, 2, 3, 4, 5, 6, 7, 8, and 9, each a2 is independently selected from: 0, 1, 2, 3, 4, 5, 6, 7, and 8, each a3 is independently selected from: 0, 1, 2, 3, 4, 5, 6, and 7, each a4 is independently selected from: 0, 1, 2, 3, 4, 5, and 6;
each L is independently selected from: -CH₂-, or -CH₂CH₂-;
Cy¹ is selected from optionally substituted phenyl, naphthyl, anthracyl, phenanthrenyl, furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyrimidinyl, pyridazinyl, pyrazinyl, or triazinyl;
Cy² is selected from H, and optionally substituted phenyl, naphthyl, anthracyl, phenanthrenyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyrimidinyl, pyridazinyl, pyrazinyl, triazinyl, or carbazol-9-yl;
the "optionally substituted" refers to being unsubstituted or mono- or polysubstituted with a group selected from the following: halogen, hydroxyl, cyano, nitro, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, cyclohexyl, phenyl, naphthyl, pyridyl, pyrimidinyl, -COOMe, - COOEt, -COMe, -COEt, amino, methylamino, dimethylamino, ethylamino, diethylamino, -CONH₂, -CONHMe, -CONHEt, -CON(Me)₂, or -CON(Et)₂.

Preferably, in the aforementioned compounds:
W is selected from: CH₂, or C=O;
G is selected from: H;
T₁-T₃ are independently selected from: O;
A₂-A₃ are independently selected from: CH₂;
R^{A} is selected from: H;
Q₁-Q₄ are independently selected from: CR^{Q};
R^{Q} is selected from: H, or halogen;
L₁ is selected from a group represented by the structure of the following general formula:

   -(CHR¹)ₖ-;

   -(CHR¹)ₘ-O-(CHR¹)ₙ-;

   -(CHR¹)ₘ-S-(CHR¹)ₙ-;

   -(CHR¹)ₘ-N(R¹)-(CHR¹)ₙ-;

   -(CHR¹)ₘ-CO-(CHR¹)ₙ-;

   -(CHR¹)ₘ-CO-O-(CHR¹)ₙ-;

   -(CHR¹)ₘ-O-CO-(CHR¹)ₙ-;

   -(CHR¹)ₘ-O-CO-O-(CHR¹)ₙ-;

   -(CHR¹)ₘ-CO-N(R¹)-(CHR¹)ₙ-;

   -(CHR¹)ₘ-N(R¹)-CO-(CHR¹)ₙ-;

   -(CHR¹)ₘ-CO-N(R¹)-(CHR¹)ₙ-;

   -(CHR¹)ₘ-N(R¹)-CO-N(R¹)-(CHR¹)ₙ-;

   -(CHR¹)ₘ-CO-N(R¹)-CO-(CHR¹)ₙ-;

   -(CHR¹)ₘ-O-(CHR¹)ₗ-O-(CHR¹)ₙ-;

   -(CHR¹)ₘ-O-(CHR¹)ₗ-N(R¹)-(CHR¹)ₙ-;

   -(CHR¹)ₘ-N(R¹)-(CHR¹)ₗ-O-(CHR¹)ₙ-;

   -(CHR¹)ₘ-O-(CHR¹)ₗ-CO-(CHR¹)ₙ-;

   -(CHR¹)ₘ-CO-(CHR¹)ₗ-O-(CHR¹)ₙ-;

   -(CHR¹)ₘ-O-(CHR¹)ₗ-CO-N(R¹)-(CHR¹)ₙ-;

   -(CHR¹)ₘ-CO-N(R¹)-(CHR¹)ₗ-O-(CHR¹)ₙ-;

   -(CHR¹)ₘ-O-(CHR¹)ₗ-N(R¹)-CO-(CHR¹)ₙ-;

   -(CHR¹)ₘ-N(R¹)-CO-(CHR¹)ₗ-O-(CHR¹)ₙ-;

   -(CHR¹)ₘ-N(R¹)-(CHR¹)ₗ-N(R¹)-(CHR¹)ₙ-;

   -(CHR¹)ₘ-N(R¹)-(CHR¹)ₗ-CO-(CHR¹)ₙ-;

   -(CHR¹)ₘ-CO-(CHR¹)ₗ-N(R¹)-(CHR¹)ₙ-;

   -(CHR¹)ₘ-N(R¹)-(CHR¹)ₗ-CO-N(R¹)-(CHR¹)ₙ-;

   -(CHR¹)ₘ-CO-N(R¹)-(CHR¹)ₗ-N(R¹)-(CHR¹)ₙ-;

   -(CHR¹)ₘ-N(R¹)-(CHR¹)ₗ-N(R¹)-CO-(CHR¹)ₙ-;

   -(CHR¹)ₘ-N(R¹)-CO-(CHR¹)ₗ-N(R¹)-(CHR¹)ₙ-;

   -(CHR¹)ₘ-CO-(CHR¹)ₗ-CO-N(R¹)-(CHR¹)ₙ-;

   -(CHR¹)ₘ-CO-N(R¹)-(CHR¹)ₗ-CO-(CHR¹)ₙ-;

   -(CHR¹)ₘ-CO-(CHR¹)ₗ-N(R¹)-CO-(CHR¹)ₙ-;

   -(CHR¹)ₘ-N(R¹)-CO-(CHR¹)ₗ-CO-(CHR¹)ₙ-;

   -(CHR¹)ₘ-N(R¹)-CO-(CHR¹)ₗ-CO-N(R¹)-(CHR¹)ₙ-;

   -(CHR¹)ₘ-N(R¹)-CO-(CHR¹)ₗ-N(R¹)-CO-(CHR¹)ₙ-;

   -(CHR¹)ₘ-CO-N(R¹)-(CHR¹)ₗ-CO-N(R¹)-(CHR¹)ₙ-;

   -(CHR¹)ₘ-CO-N(R¹)-(CHR¹)ₗ-N(R¹)-CO- (CHR¹)ₙ-;

   -(CHR¹)ₘ-C₆₋₁₀arylene-CO-O-(CHR¹)ₙ-;

   -(CHR¹)ₘ-C₆₋₁₀arylene-O-CO-(CHR¹)ₙ-;

   -(CHR¹)ₘ-CO-O-C₆₋₁₀arylene-(CHR¹)ₙ-;

   -(CHR¹)ₘ-O-CO-C₆₋₁₀arylene-(CHR¹)ₙ-;

   -(CHR¹)ₘ-C₆₋₁₀arylene-N(R¹)-CO-(CHR¹)ₙ-;

   -(CHR¹)ₘ-C₆₋₁₀arylene-CO-N(R¹)-(CHR¹)ₙ-;

   -(CHR¹)ₘ-N(R¹)-CO-C₆₋₁₀arylene-(CHR¹)ₙ-;

   -(CHR¹)ₘ-CO-N(R¹)-C₆₋₁₀arylene-(CHR¹)ₙ-;
wherein k is selected from: 0, 1, 2, 3, 4, 5, 6, 7, 8, 9, 10, 11, and 12, m and n are independently selected from: 0, 1, 2, 3, 4, 5, and 6, 1 is selected from 1, 2, 3, 4, 5, and 6, and each R¹ is independently selected from: H, or C₁₋₆ alkyl; 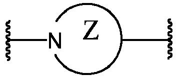 is selected from one of the following structures: 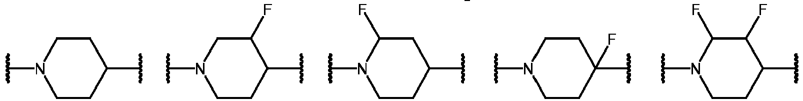 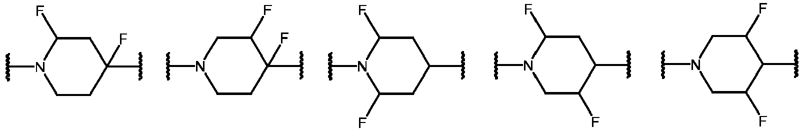 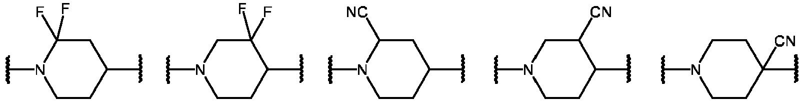 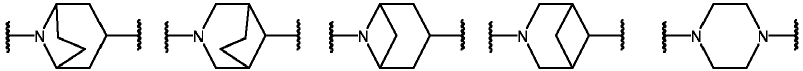
Cy¹ is selected from one of the following structures: 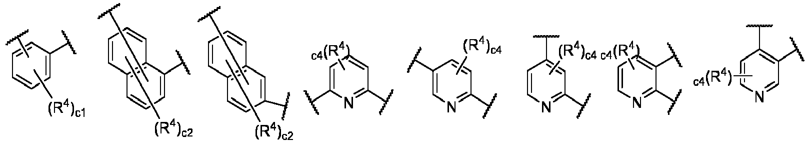 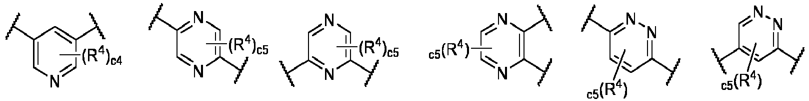 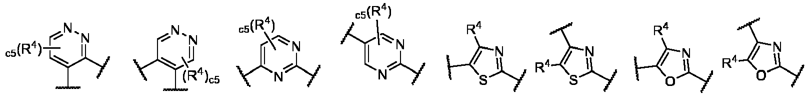
each R⁴ is independently selected from: halogen, hydroxyl, cyano, nitro, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, cyclohexyl, phenyl, naphthyl, pyridyl, pyrimidinyl, -COOMe, - COOEt, -COMe, -COEt, amino, methylamino, dimethylamino, ethylamino, diethylamino, -CONH₂, -CONHMe, -CONHEt, -CON(Me)₂, or -CON(Et)₂;
each c1 is independently selected from 0, 1, 2, 3, and 4, each c2 is independently selected from 0, 1, 2, 3, 4, 5, and 6, each c3 is independently selected from 0, 1, 2, 3, 4, 5, 6, 7, 8, and 9, each c4 is independently selected from 0, 1, 2, and 3, and each c5 is independently selected from 0, 1, and 2;
Cy² is selected from H and one of the following structures: 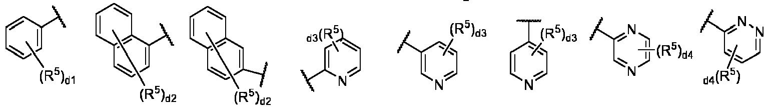 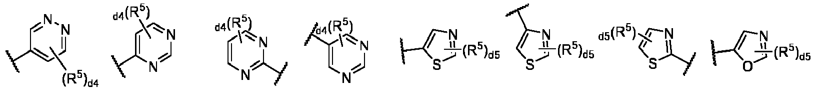 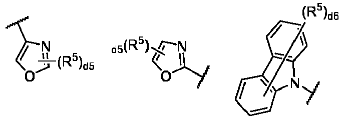
each R⁵ is independently selected from: halogen, hydroxyl, cyano, nitro, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, cyclohexyl, phenyl, naphthyl, pyridyl, pyrimidinyl, -COOMe, - COOEt, -COMe, -COEt, amino, methylamino, dimethylamino, ethylamino, diethylamino, -CONH₂, -CONHMe, -CONHEt, -CON(Me)₂, or -CON(Et)₂;
Each d1 is independently selected from 0, 1, 2, 3, 4, and 5, each d2 is independently selected from 0, 1, 2, 3, 4, 5, 6, and 7, each d3 is independently selected from 0, 1, 2, 3, and 4, each d4 is independently selected from 0, 1, 2, and 3, and each d5 is independently selected from 0, 1, and 2.

Preferably, in the aforementioned compounds:
W is selected from: CH₂, or C=O;
G is selected from: H;
T₁-T₃ are independently selected from: O;
A₂-A₃ are independently selected from: CH₂;
R^{A} is selected from: H;
Q₁-Q₄ are independently selected from: CH;
L₁ is selected from a group represented by the structure of the following general formula:

   -(CHR¹)ₖ-;

   -O-; -(CHR¹)ₘ-O-; -O-(CHR¹)ₙ-; -(CHR¹)ₘ-O-(CHR¹)ₙ-;

   -N(R¹)-; -(CHR¹)ₘ-N(R¹)-; -N(R¹)-(CHR¹)ₙ-; -(CHR¹)ₘ-N(R¹)-(CHR¹)ₙ-;

   -CO-; -(CHR¹)ₘ-CO-; -CO-(CHR¹)ₙ-; -(CHR¹)ₘ-CO-(CHR¹)ₙ-;

   -CO-N(R¹)-; -(CHR¹)ₘ-CO-N(R¹)-; -CO-N(R¹)-(CHR¹)ₙ-; -(CHR¹)ₘ-CO-N(R¹)-(CHR¹)ₙ-;

   -N(R¹)-CO-; -(CHR¹)ₘ-N(R¹)-CO-; -N(R¹)-CO-(CHR¹)ₙ-; -(CHR¹)ₘ-N(R¹)-CO-(CHR¹)ₙ-;

   -CO-O-; -(CHR¹)ₘ-CO-O; -CO-O-(CHR¹)ₙ-; -(CHR¹)ₘ-CO-O-(CHR¹)ₙ-;

   -O-CO-; -(CHR¹)ₘ-O-CO-; -O-CO-(CHR¹)ₙ-; -(CHR¹)ₘ-O-CO-(CHR¹)ₙ-;

   -(CHR¹)ₘ-C₆arylene-N(R¹)-CO-(CHR¹)ₙ-;

   -(CHR¹)ₘ-C₆arylene-CO-N(R¹)-(CHR¹)ₙ-;
wherein k, m and n are independently selected from: 1, 2, 3, 4, 5, and 6, each R¹ is independently selected from: H, methyl, or ethyl; 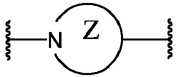 is selected from one of the following structures: 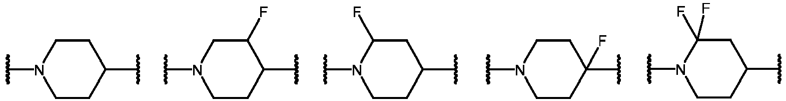 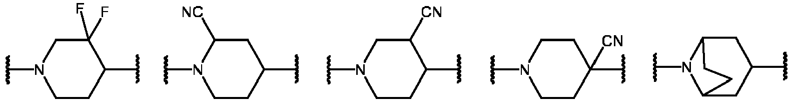 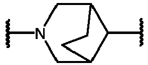 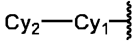 is selected from one of the following structures:

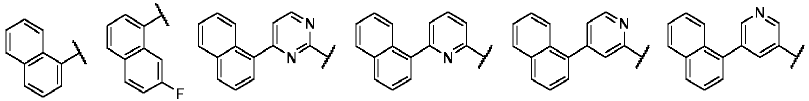

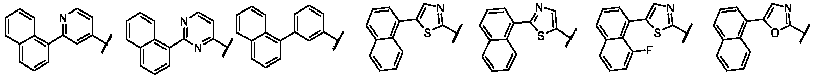

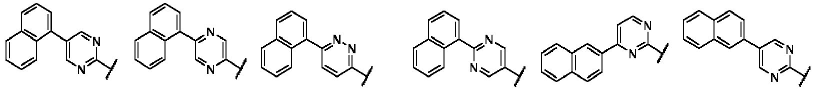

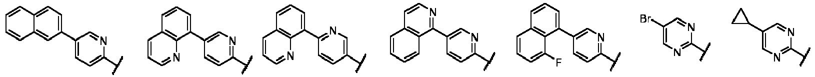

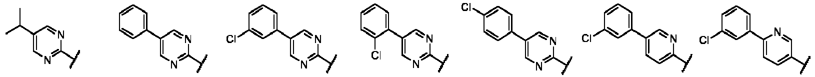

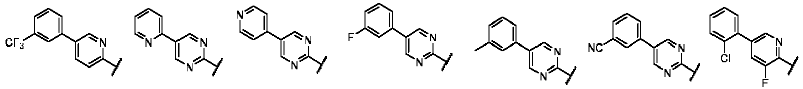

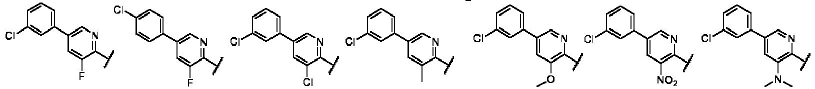

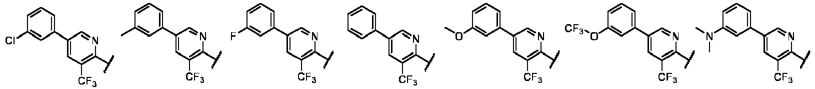

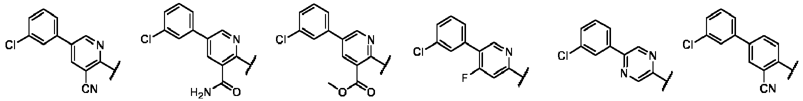

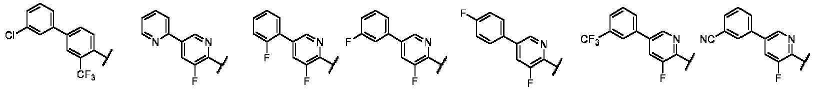

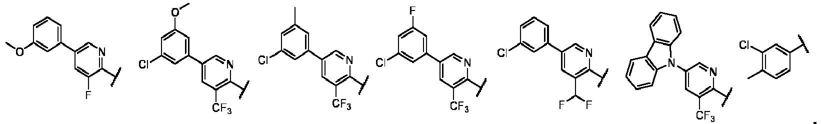

In certain embodiments, the compound of the present invention is selected from:

| | | | |
|---|---|---|---|
| A1 | 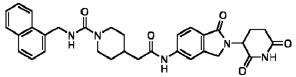 | A90 | 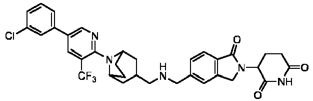 |
| A2 | 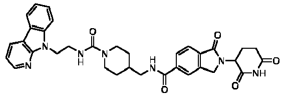 | A91 | 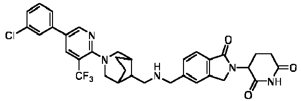 |
| A3 | 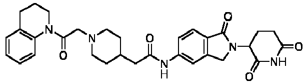 | A92 | 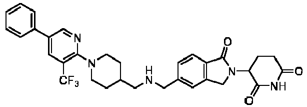 |
| A4 | 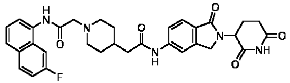 | A93 | 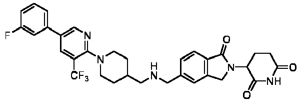 |
| A5 | 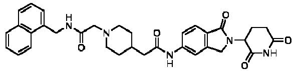 | A94 | 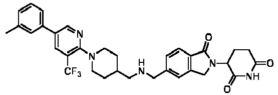 |
| A6 | 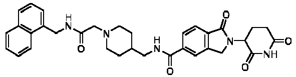 | A95 | 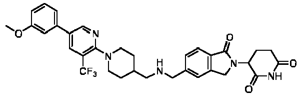 |
| A7 | 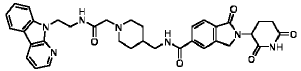 | A96 | 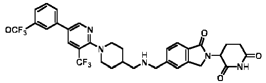 |
| A8 | 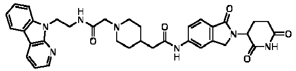 | A97 | 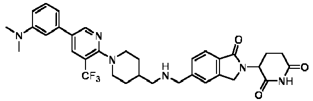 |
| A9 | 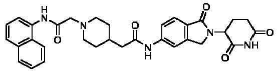 | A98 | 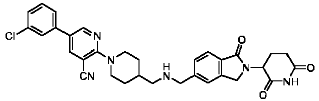 |
| A10 | 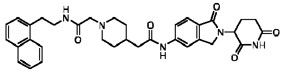 | A99 | 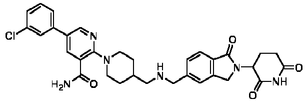 |
| A11 | 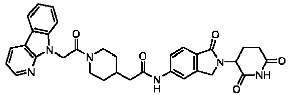 | A100 | 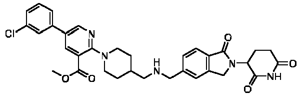 |
| A12 | 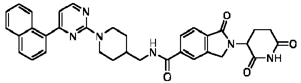 | A101 | 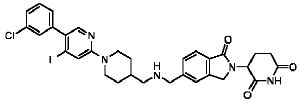 |
| A13 | 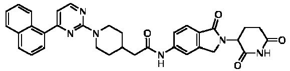 | A102 | 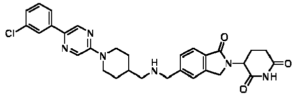 |
| A14 | 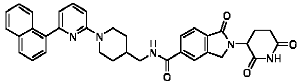 | A103 | 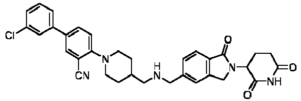 |
| A15 | 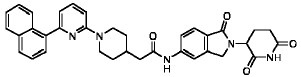 | A104 | 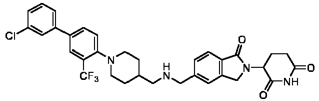 |
| A16 | 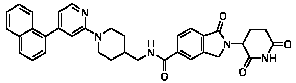 | A105 | 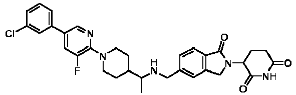 |
| A17 | 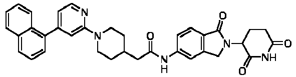 | A106 | 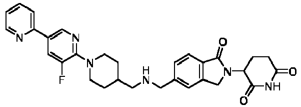 |
| A18 | 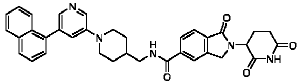 | A107 | 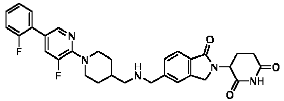 |
| A19 | 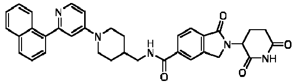 | A108 | 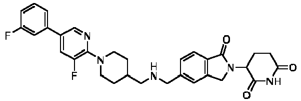 |
| A20 | 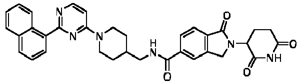 | A109 | 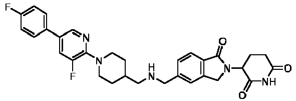 |
| A21 | 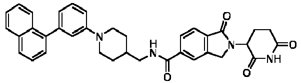 | A110 | 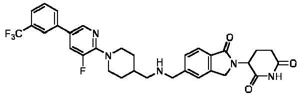 |
| A22 | 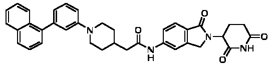 | A111 | 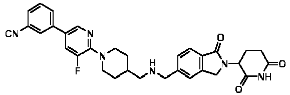 |
| A23 | 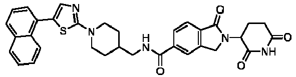 | A112 | 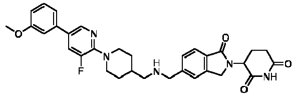 |
| A24 | 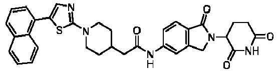 | A113 | 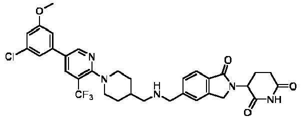 |
| A25 | 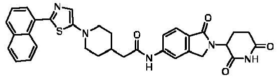 | A114 | 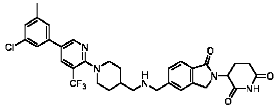 |
| A26 | 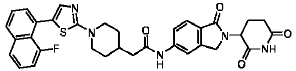 | A115 | 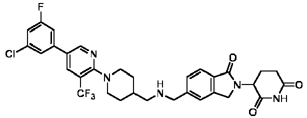 |
| A27 | 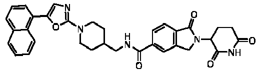 | A116 | 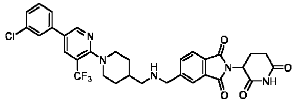 |
| A28 | 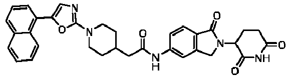 | A117 | 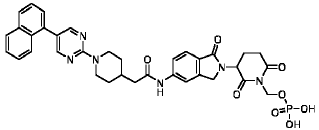 |
| A29 | 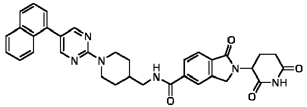 | A118 | 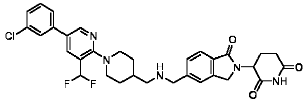 |
| A30 | 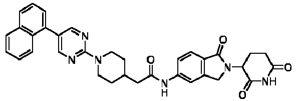 | A119 | 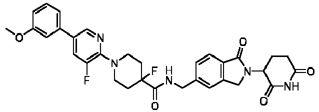 |
| A31 | 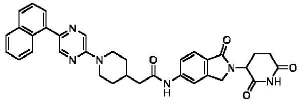 | A120 | 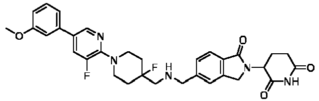 |
| A32 | 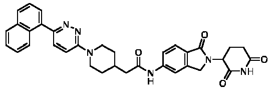 | A121 | 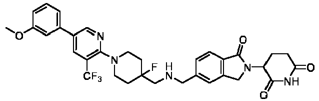 |
| A33 | 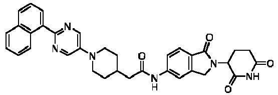 | A122 | 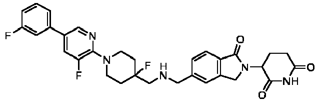 |
| A34 | 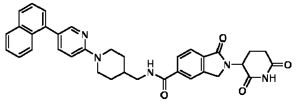 | A123 | 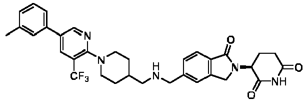 |
| A35 | 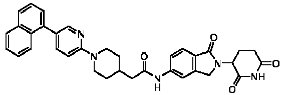 | A124 | 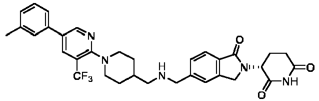 |
| A36 | 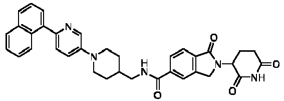 | A125 | 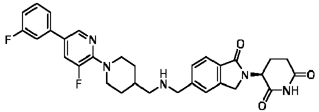 |
| A37 | 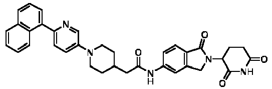 | A126 | 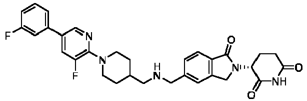 |
| A38 | 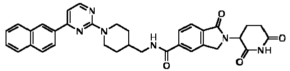 | A127 | 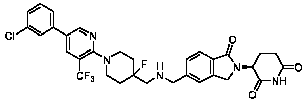 |
| A39 | 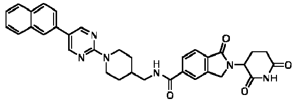 | A128 | 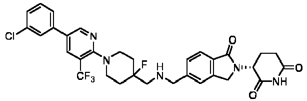 |
| A40 | 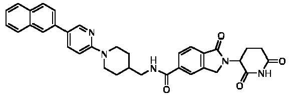 | A129 | 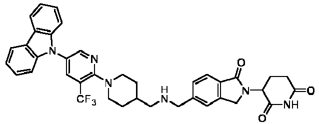 |
| A41 | 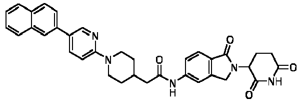 | A130 | 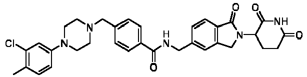 |
| A42 | 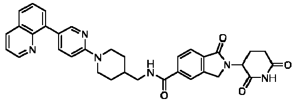 | A131 | 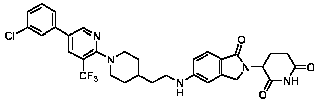 |
| A43 | 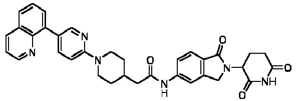 | A132 | 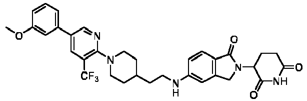 |
| A44 | 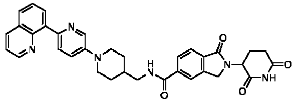 | A133 | 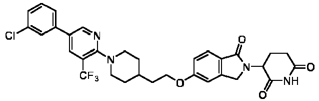 |
| A45 | 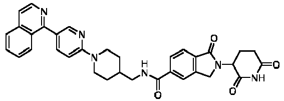 | A134 | 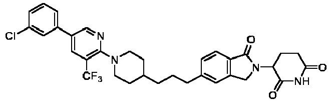 |
| A46 | 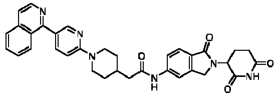 | A135 | 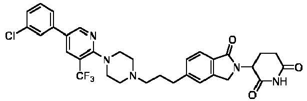 |
| A47 | 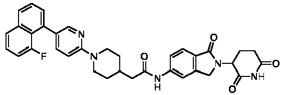 | A136 | 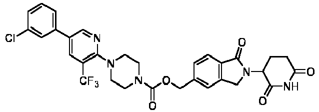 |
| A48 | 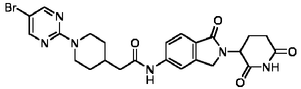 | A137 | 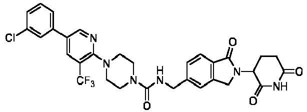 |
| A49 | 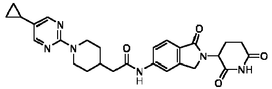 | A138 | 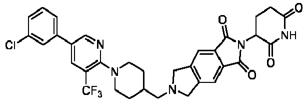 |
| A50 | 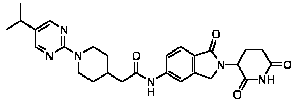 | A139 | 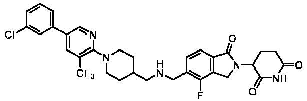 |
| A51 | 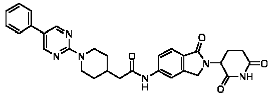 | A140 | 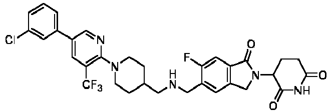 |
| A52 | 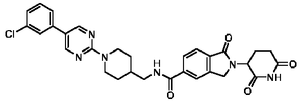 | A141 | 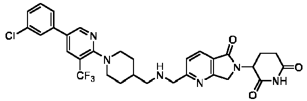 |
| A53 | 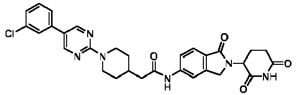 | A142 | 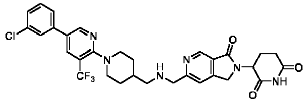 |
| A54 | 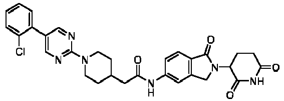 | A143 | 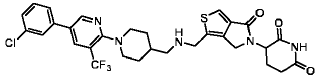 |
| A55 | 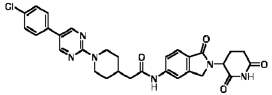 | A144 | 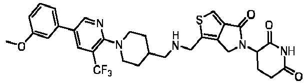 |
| A56 | 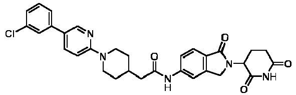 | A145 | 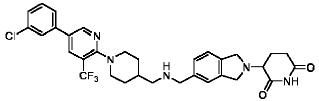 |
| A57 | 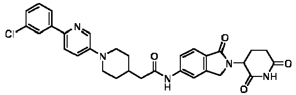 | A146 | 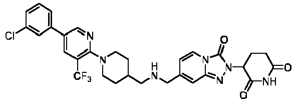 |
| A58 | 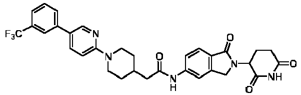 | A147 | 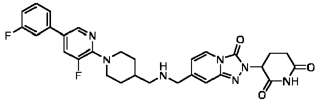 |
| A59 | 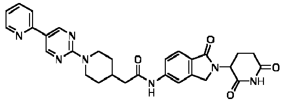 | A148 | 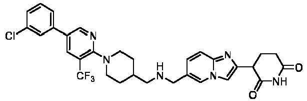 |
| A60 | 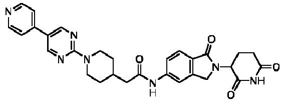 | A149 | 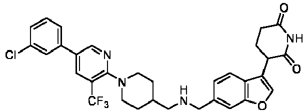 |
| A61 | 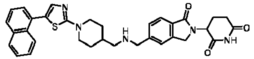 | A150 | 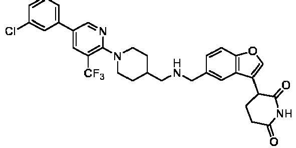 |
| A62 | 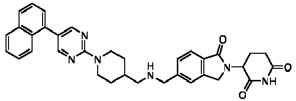 | A151 | 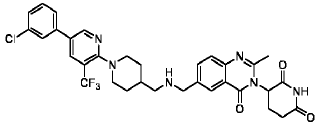 |
| A63 | 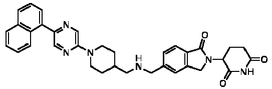 | A152 | 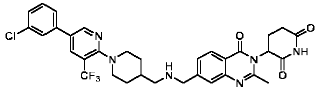 |
| A64 | 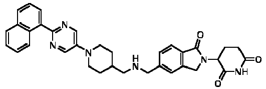 | A153 | 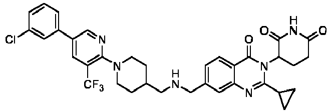 |
| A65 | 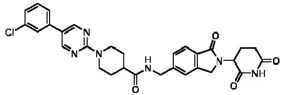 | A154 | 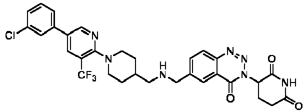 |
| A66 | 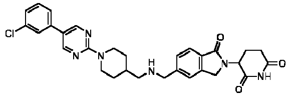 | A155 | 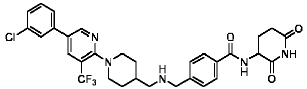 |
| A67 | 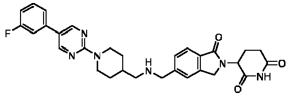 | A156 | 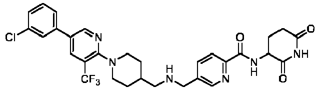 |
| A68 | 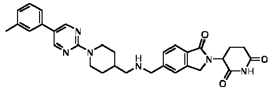 | A157 | 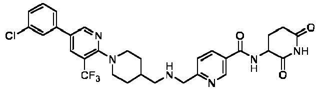 |
| A69 | 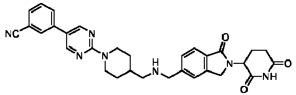 | A158 | 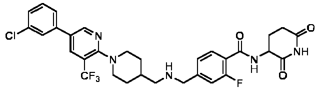 |
| A70 | 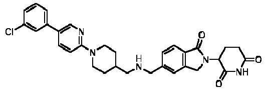 | A159 | 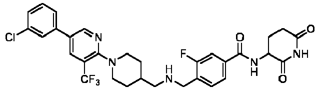 |
| A71 | 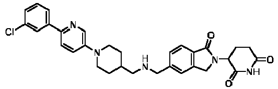 | A160 | 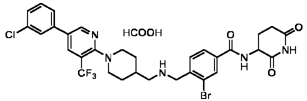 |
| A72 | 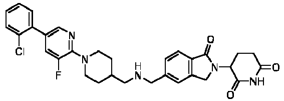 | A161 | 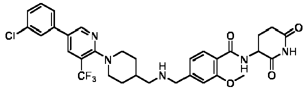 |
| A73 | 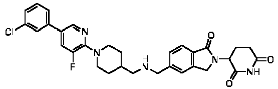 | A162 | 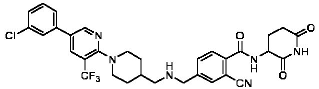 |
| A74 | 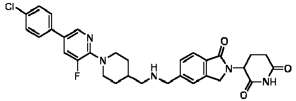 | A163 | 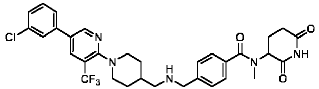 |
| A75 | 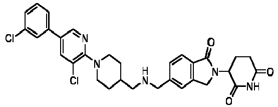 | A164 | 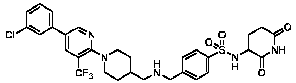 |
| A76 | 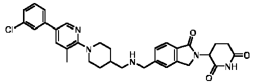 | A165 | 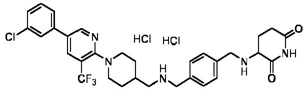 |
| A77 | 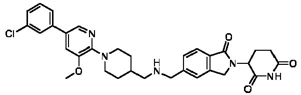 | A166 | 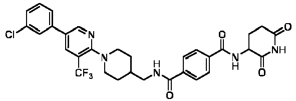 |
| A78 | 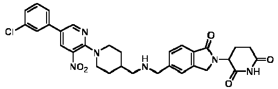 | A167 | 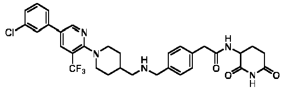 |
| A79 | 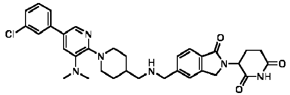 | A168 | 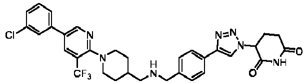 |
| A80 | 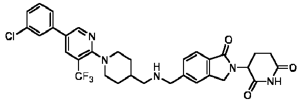 | A169 | 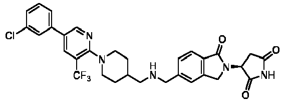 |
| A81 | 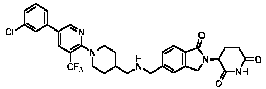 | A170 | 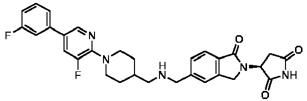 |
| A82 | 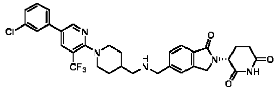 | A171 | 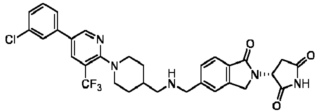 |
| A83 | 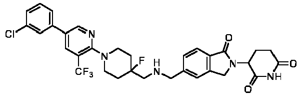 | A172 | 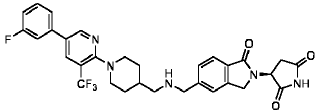 |
| A84 | 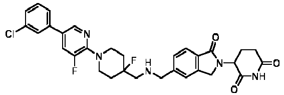 | A173 | 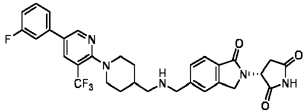 |
| A85 | 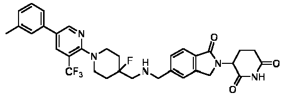 | A174 | 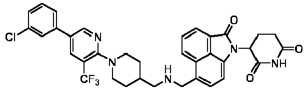 |
| A86 | 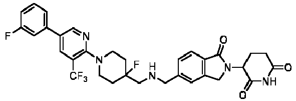 | A175 | 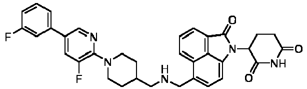 |
| A87 | 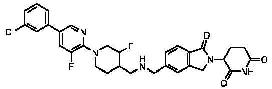 | A176 | 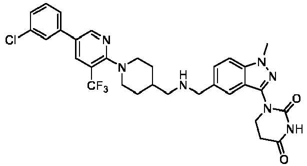 |
| A88 | 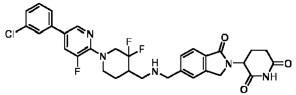 | A177 | 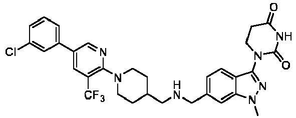 |
| A89 | 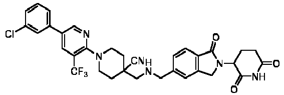 | | |

The above lists some specific chemical structures of the compounds of formula (I₀) and formula (I) of the present invention, but the present invention is not limited to these listed chemical structures. All compounds based on formula (I₀) and formula (I) whose substituents are the groups defined above should be included.

Another aspect of the present invention provides a pharmaceutical composition comprising an effective amount of a compound of the present invention or the pharmaceutically acceptable salt, solvate, hydrate, stereoisomer, tautomer, or prodrug thereof and a pharmaceutically acceptable carrier and/or an excipient.

Pharmaceutically acceptable carriers of the present invention include ion exchangers, aluminum oxide, aluminum stearate, lecithin, serum proteins, buffer substances, partial glyceride mixtures of saturated plant fatty acids, water, salts or electrolytes, etc., but are not limited to these.

In certain embodiments, the active compounds are prepared with carriers that will protect the compounds against rapid elimination from the body, such as controlled release formulations, including implants and microencapsulated delivery systems. Methods for preparing such formulations will be apparent to those skilled in the art.

Liposome suspensions can also be pharmaceutically acceptable carriers and/or excipients, and liposome formulations can be prepared as follows: the appropriate lipid is dissolved in an inorganic solvent and then evaporated, leaving a thin film of dry lipid on the surface of the container. An aqueous solution of the active compound is then introduced into the container. The container is then rotated by hand to separate the lipid material from the sides of the container and disperse the lipid aggregates, forming a liposome suspension.

In embodiments of the present invention, the pharmaceutical composition further comprises at least one additional bioactive agent.

In embodiments of the present invention, the bioactive agent is helpful in the treatment of cancer.

In embodiments of the present invention, the bioactive agent is an anti-cancer agent.

Preferably, the anti-cancer agent includes: platinum agents, such as cisplatin, carboplatin, oxaliplatin, JM-216 or satraplatin, CI-973; anti-microtubule agents, such as vinca alkaloids such as vincristine and vinblastine, taxanes such as paclitaxel and docetaxel; antimetabolites, such as 5-fluorouracil, methotrexate, and fludarabine; alkylating agents such as cyclophosphamide, melphalan, carmustine, nitrosoureas such as bischloroethylnitrosourea and hydroxyurea; anthracyclines, such as doxorubicin and daunorubicin; anti-tumor antibiotics, such as mitomycin, idarubicin, adriamycin, and daunorubicin; topoisomerase inhibitors, such as etoposide and camptothecin; anti-angiogenic agents, such as bevacizumab; or any other cytotoxic agents such as estramustine phosphate, prednimustine, hormones or hormone agonists, antagonists, partial agonists or partial antagonists, kinase inhibitors and radiation therapy.

Therefore, the present invention further provides a combination drug comprising an effective amount of the compound of the present invention or the pharmaceutically acceptable salt, solvate, hydrate, stereoisomer, tautomer, prodrug thereof, and at least one additional bioactive agent as described above.

Another aspect of the present invention provides a therapeutic composition comprising an effective amount of at least two different compounds of the present invention or the pharmaceutically acceptable salt, solvate, hydrate, stereoisomer, tautomer, or prodrug thereof and a pharmaceutically acceptable carrier and/or an excipient.

The compound of the present invention or the pharmaceutically acceptable salt, solvate, hydrate, stereoisomer, tautomer, and prodrug thereof can modulate protein degradation in patients or subjects, and can be used to treat or improve a disease state or condition modulated by degraded proteins. In certain embodiments, the compound of the present invention can be used to effect degradation of proteins of interest in order to treat or improve a disease. the protein in the protein degradation of the present invention is any one or more of c-Myc, GSPT1, CK1α and IKZF (1/2/3) proteins.

Therefore, another aspect of the present invention provides the use of the compound of the present invention or the pharmaceutically acceptable salt, solvate, hydrate, stereoisomer, tautomer, or prodrug thereof in the preparation of a protein degradation agent.

Another aspect of the present invention provides the use of the compound of the present invention or the pharmaceutically acceptable salt, solvate, hydrate, stereoisomer, tautomer, or prodrug thereof in the preparation of a drug, characterized in that the drug is used to prevent or treat a disease or a disorder associated with protein degradation.

In certain embodiments, the protein in the protein degradation is any one or more of c-Myc, GSPT1, CK1α and IKZF (1/2/3) proteins.

In certain embodiments, the diseases or disorders that can be treated by the protein degradation agent or the diseases or disorders associated with protein degradation include cancers, cardiovascular and cerebrovascular diseases, viral infections and immune diseases.

Preferably, the cancer comprises: leukemia, lymphoma, malignant glioma, medulloblastoma, melanoma, multiple myeloma, malignant glioma, osteosarcoma, liver cancer, lung cancer, kidney cancer, pancreatic cancer, oral cancer, gastric cancer, esophageal cancer, laryngeal cancer, nasopharyngeal cancer, skin cancer, breast cancer, colon cancer, rectal cancer, cervical cancer, bladder cancer, ovarian cancer, prostate cancer, rhabdomyosarcoma, osteoblastic sarcoma, chondrosarcoma and small cell lung cancer;
preferably, the viral infection-related disease comprises: HIV, hepatitis B virus (HBV), hepatitis B, hepatitis C, hepatitis A, influenza, epidemic encephalitis B and herpes.

Preferably, the immune disease comprises: systemic lupus erythematosus, rheumatoid arthritis, scleroderma, hyperthyroidism, juvenile diabetes, idiopathic thrombocytopenic purpura, autoimmune hemolytic anemia, ulcerative colitis, and chronic liver disease.

Another aspect of the present invention provides a method of treating a disease or a disorder in a subject, comprising the steps of administering the compound of the present invention or the pharmaceutically acceptable salt, solvate, hydrate, stereoisomer, tautomer, or prodrug thereof to the subject in need thereof.

In certain embodiments, the disease or the disorder is associated with protein degradation.

In certain embodiments, the protein in the protein degradation is any one or more of c-Myc, GSPT1, CK1α and IKZF (1/2/3) proteins.

In certain embodiments, the diseases or disorders comprise cancers, cardiovascular and cerebrovascular diseases, viral infections, and immune diseases.

Preferably, the cancer comprises: leukemia, lymphoma, malignant glioma, medulloblastoma, melanoma, multiple myeloma, malignant glioma, osteosarcoma, liver cancer, lung cancer, kidney cancer, pancreatic cancer, oral cancer, gastric cancer, esophageal cancer, laryngeal cancer, nasopharyngeal cancer, skin cancer, breast cancer, colon cancer, rectal cancer, cervical cancer, bladder cancer, ovarian cancer, prostate cancer, rhabdomyosarcoma, osteoblastic sarcoma, chondrosarcoma and small cell lung cancer;
preferably, the viral infection-related disease comprises: HIV, hepatitis B virus (HBV), hepatitis B, hepatitis C, hepatitis A, influenza, epidemic encephalitis B and herpes.

Preferably, the immune disease comprises: systemic lupus erythematosus, rheumatoid arthritis, scleroderma, hyperthyroidism, juvenile diabetes, idiopathic thrombocytopenic purpura, autoimmune hemolytic anemia, ulcerative colitis, and chronic liver disease.

Another aspect of the present invention provides the use of the compound of the present invention or the pharmaceutically acceptable salt, solvate, hydrate, stereoisomer, tautomer, or prodrug thereof in the preparation of a proteolysis-targeting chimera (Protac) or an antibody-drug conjugate (ADC).

The present invention further provides a method for preparing the compound of the present invention, but the preparation method of the present invention is not limited thereto. The core structure of the compound of formula (I) can be prepared by the reaction route shown below, the substituents can be bonded by methods known in the art, and the type and position of the substituents or the number of substituents can be changed based on techniques known in the art.

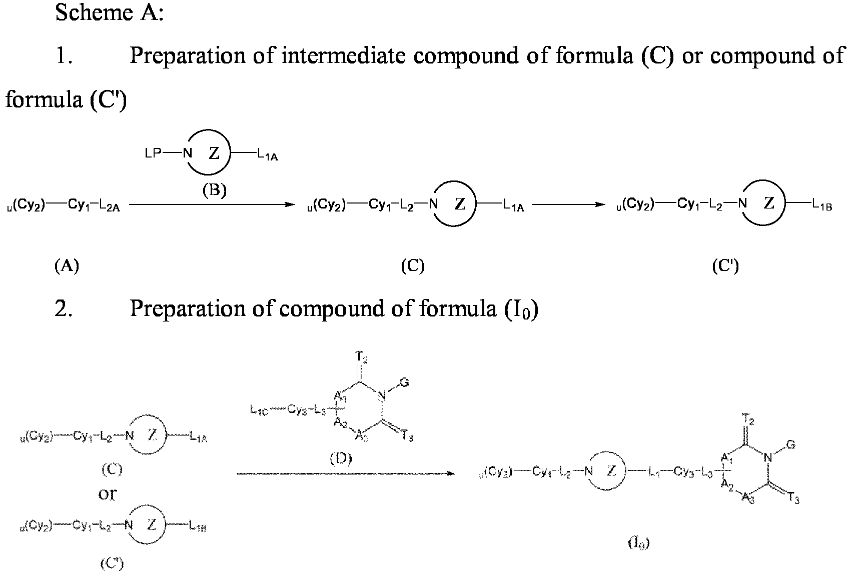

A compound of formula (A) reacts with a compound of formula (B) to form a compound of formula (C). Optionally, the compound of formula (C) is converted into a compound of formula (C'), and then the compound of formula (C) or the compound of formula (C') reacts with a compound of formula (D) to generate the compound of formula (I₀);
wherein, LP represents H or a leaving group; L_{2A} represents a group that can react with LP to form L₂;
L_{1A} and L_{1B} are different, and indicate a group that can react with L_{1C} to jointly form L₁, or the group that is protected.

Preferably, L_{2A} is selected from halogen, such as fluorine, chlorine, bromine or iodine.

Preferably, L_{1A} and L_{1B} contain functional groups for reaction with L_{1C}, such as -NH₂, - COOH, -CONH₂, -CHO, -OH, halogen, etc., or the group that is protected, but are not limited thereto.

Preferably, L_{1C} contains a functional group for reaction with L_{1B}, such as - NH₂, -COOH, -CONH₂, -CHO, -OH, halogen, etc., or the group that is protected, but are not limited thereto; in addition, L_{1C} may also be H.

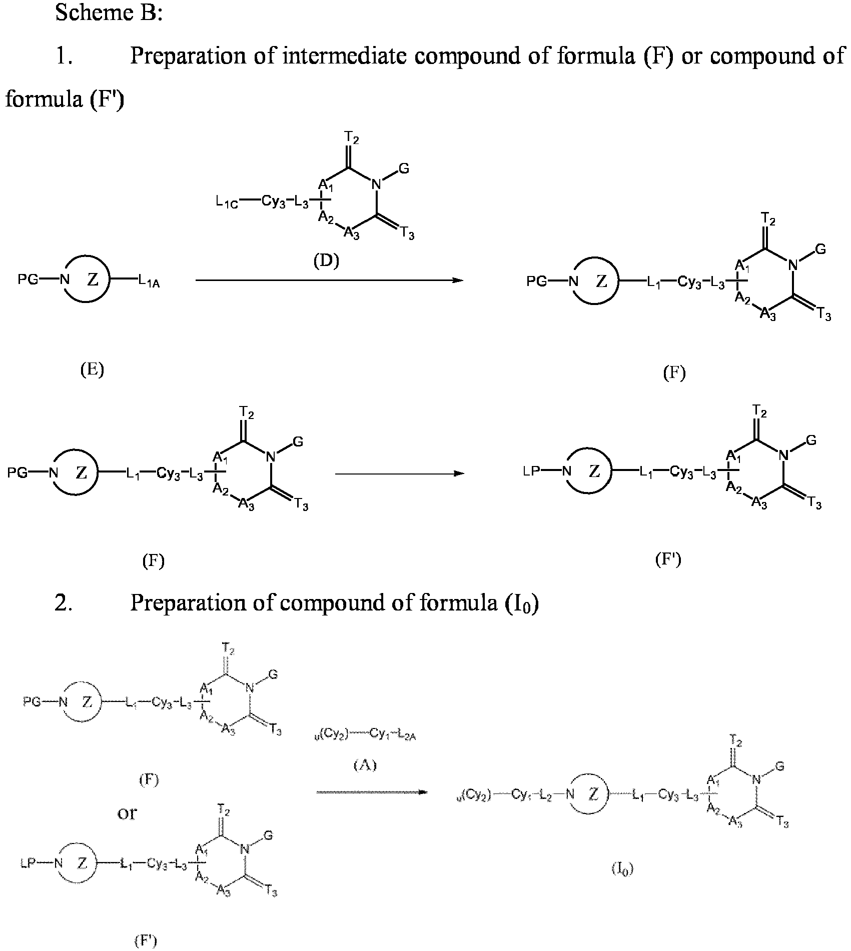

A compound of formula (E) reacts with the compound of formula (D) to form a compound of formula (F). Optionally, the compound of formula (F) is converted into a compound of formula (F'), and then the compound of formula (F) or the compound of formula (F') reacts with the compound of formula (A) to generate the compound of formula (I);
wherein, PG represents H or a protecting group; L_{1A} represents a group that can react with L_{1C} to jointly form L₁, or the group that is protected.

LP represents H or a leaving group; L_{2A} represents a group that can react with PG/LP to form L₂.

Preferably, L_{1A} contains a functional group for reaction with L_{1C}, such as - NH₂, - COOH, -CONH₂, -CHO, -OH, halogen, etc., or the group that is protected, but are not limited thereto.

Preferably, L_{1C} contains a functional group for reaction with L_{1A}, such as - NH₂, -COOH, -CONH₂, -CHO, -OH, halogen, etc., or the group that is protected, but are not limited thereto; in addition, L_{1C} may also be H.

Preferably, L_{2A} is selected from halogen, such as fluorine, chlorine, bromine or iodine.

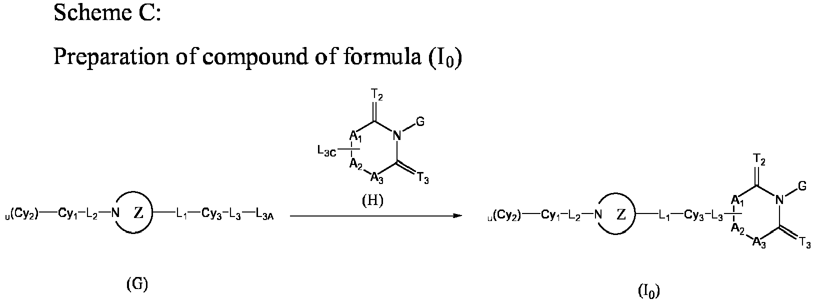

A compound of formula (G) reacts with a compound of formula (H) to generate the compound of formula (I₀);

L_{3A} represents a group that can react with L_{3C} to jointly form L₃, or the group that is protected.

Preferably, L_{3A} contains a functional group for reaction with L_{3C}, such as - NH₂, - COOH, -CONH₂, -CHO, -OH, halogen, etc., or the group that is protected, but are not limited thereto.

Preferably, L_{3C} contains a functional group for reaction with L_{3A}, such as - NH₂, -COOH, -CONH₂, -CHO, -OH, halogen, etc., or the group that is protected, but are not limited thereto; in addition, L_{3C} may also be H.

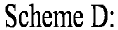

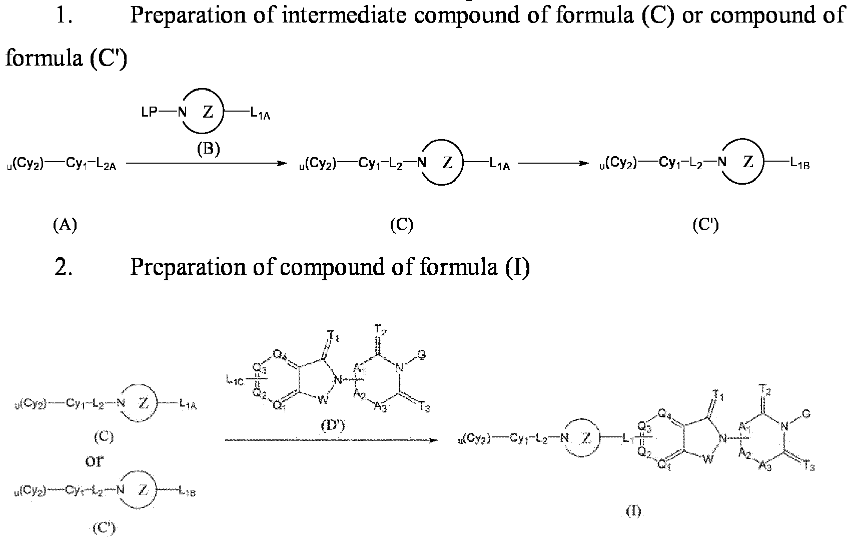

A compound of formula (A) reacts with a compound of formula (B) to form a compound of formula (C). Optionally, the compound of formula (C) is converted into a compound of formula (C'), and then the compound of formula (C) or the compound of formula (C') reacts with a compound of formula (D') to generate the compound of formula (I);
wherein, LP represents H or a leaving group; L_{2A} represents a group that can react with LP to form L₂;
L_{1A} and L_{1B} are different, and indicate a group that can react with L_{1C} to jointly form L₁, or the group that is protected.

Preferably, L_{2A} is selected from halogen, such as fluorine, chlorine, bromine or iodine.

Preferably, L_{1A} and L_{1B} contain functional groups for reaction with L_{1C}, such as -NH₂, - COOH, -CONH₂, -CHO, -OH, halogen, etc., or the group that is protected, but are not limited thereto.

Preferably, L_{1C} contains a functional group for reaction with L_{1B}, such as - NH₂, -COOH, -CONH₂, -CHO, -OH, halogen, etc., or the group that is protected, but are not limited thereto; in addition, L_{1C} may also be H.

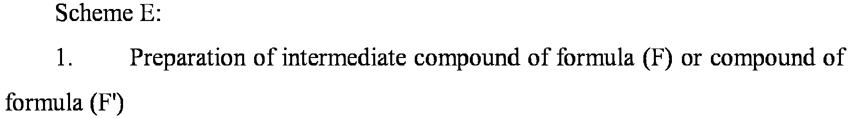

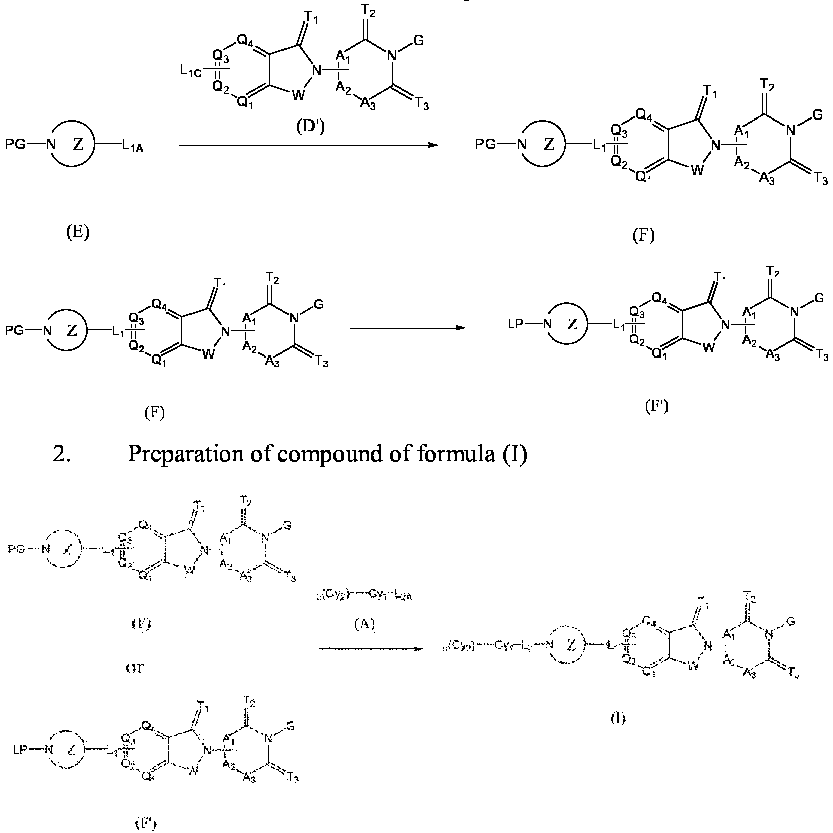

A compound of formula (E) reacts with the compound of formula (D') to form a compound of formula (F). Optionally, the compound of formula (F) is converted into a compound of formula (F'), and then the compound of formula (F) or the compound of formula (F') reacts with the compound of formula (A) to generate the compound of formula (I);
wherein, PG represents H or a protecting group; L_{1A} represents a group that can react with L_{1C} to jointly form L₁, or the group that is protected.

LP represents H or a leaving group; L_{2A} represents a group that can react with PG/LP to form L₂.

Preferably, L_{1A} contains a functional group for reaction with L_{1C}, such as - NH₂, - COOH, -CONH₂, -CHO, -OH, halogen, etc., or the group that is protected, but are not limited thereto.

Preferably, L_{1C} contains a functional group for reaction with L_{1A}, such as - NH₂, -COOH, -CONH₂, -CHO, -OH, halogen, etc., or the group that is protected, but are not limited thereto; in addition, L_{1C} may also be H.

Preferably, L_{2A} is selected from halogen, such as fluorine, chlorine, bromine or iodine.

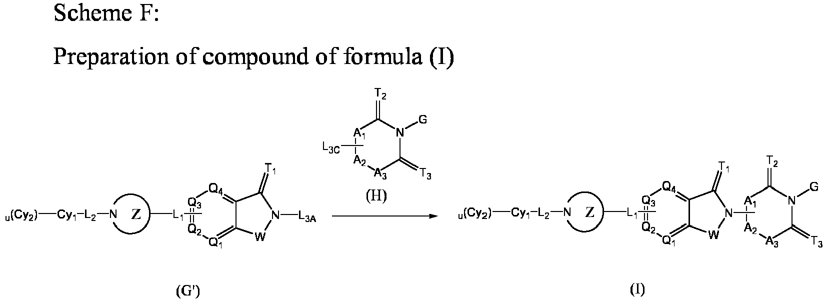

A compound of formula (G') reacts with a compound of formula (H) to generate the compound of formula (I);

L_{3A} represents a group that can react with L_{3C} to jointly form L₃, or the group that is protected.

Preferably, L_{3A} contains a functional group for reaction with L_{3C}, such as - NH₂, - COOH, -CONH₂, -CHO, -OH, halogen, ethynyl, azido, etc., or the group that is protected, but are not limited thereto.

Preferably, L_{3C} contains a functional group for reaction with L_{3A}, such as - NH₂, - COOH, -CONH₂, -CHO, -OH, halogen, ethynyl, azido, etc., or the group that is protected, but are not limited thereto; in addition, L_{3C} may also be H.

Preferred examples of the present invention will be described in detail below. The examples are given to better understand the content of the present invention, and the content of the present invention is not limited to the examples. Non-essential improvements and modifications to the embodiments based on the content of the present invention still fall within the scope of the present invention.

The experimental methods in the following examples are all conventional methods unless otherwise specified. If specific techniques or conditions are not specified in the examples, techniques or conditions described in the literature in the art or techniques or conditions according to the product instructions are followed. The abbreviations of the reagents used in the examples are as follows:
Cbz-Cl: Benzyl chloroformate;
DAST: Diethylamine sulfur trifluoride;
Dess-Martin: 1,1,1,-triacetoxy-1,1-dihydro-1,2-benzeniodoyl-3-(1H)-one];
DIPEA: diisopropylethylamine;
DME: Dimethoxyethane;
DMF: N,N-dimethylformamide;
DMSO: dimethyl sulfoxide;
HCl: hydrogen chloride
HATU: 2-(7-azabenzotriazol-1-yl)-N,N,N',N'-tetramethyluronium hexafluorophosphate;
LiHMDS: lithium hexamethyldisilazide;
LiOH: lithium hydroxide;
NaBF₄: sodium tetrafluoroborate;
NaH: sodium hydride;
NBS: N-bromosuccinimide;
Pd/C: palladium/carbon;
Pd₂(dba)₃: tris(dibenzylideneacetone)dipalladium;
Pd(dppf)Cl₂: [1,1'-Bis(diphenylphosphino)ferrocene]palladium dichloride;
Pd(PPh₃)₄: tetrakis triphenylphosphine palladium;
TLC: thin layer chromatography;
Xphos: 2-bicyclohexylphosphine-2',4',6'-triisopropylbiphenyl;
(Boc)₂O: Di-tert-butyl dicarbonate
AIBN: Azobisisobutyronitrile
DPPF: Bis(diphenylphosphino)ferrocene
Xantphos: 4,5-bis(diphenylphosphine)-9,9-dimethylxanthene
Pd(PPh₃)₂Cl₂: Bis(triphenylphosphine)palladium chloride
XphosPd(G2): (2-dicyclohexylphosphino-2',4',6'-triisopropyl-1,1'-biphenyl) (2'-amino -1,1 '-biphenyl-2-yl)palladium chloride

### 1. Preparation examples

The compounds of the present invention are prepared by referring to the corresponding methods in schemes 1-1, 1-2, 2-1, 2-2, 3, 4-1, 4-2 and 5 to 16, as follows:

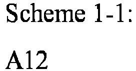

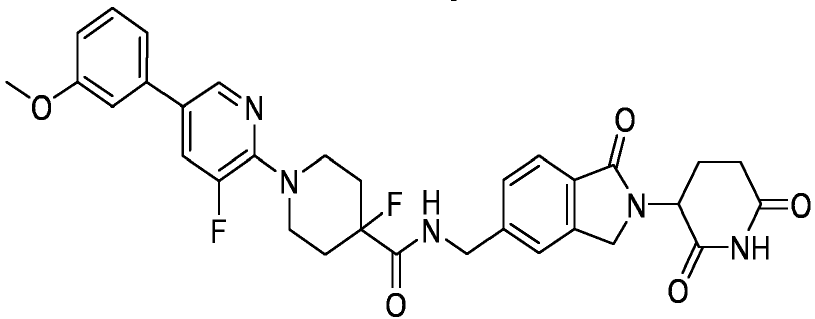

### Synthesis of compound A12-3

A12-1 (1.49 g, 10.0 mmol), A12-2 (1.72 g, 10.0 mmol) and potassium carbonate (1.38 g, 10.0 mmol) were dissolved in DME/water (18 mL/3 mL), and Pd (PPh₃)₄ (1.15 g, 0.10 mmol) was added. The mixture was replaced with nitrogen three times and then reacted at 90 °C for 4 h. TLC showed that the reaction was completed. The reaction liquid was filtered and the filtrate was diluted with ethyl acetate (20 mL). The organic phase was washed with saturated saline solution (10 ml*3), and the solution was dried by rotary evaporation. The solute was separated and purified by column chromatography (petroleum ether: ethyl acetate = 10: 1 - 7: 1) to obtain compound A12-3 (2.27 g, yield 94%). ¹H NMR (400 MHz, DMSO) δ 8.92 (d, *J* = 5.1 Hz, 1H), 8.21 - 8.11 (m, 2H), 8.11 - 8.02 (m, 1H), 7.91 (d, *J* = 5.1 Hz, 1H), 7.77 (dd, *J* = 7.1, 1.2 Hz, 1H), 7.71 - 7.56 (m, 3H).

### Synthesis of compound A12-5

A12-3 (482 mg, 2.0 mmol), A12-4 (471 mg, 2.2 mmol) and DIPEA (310 mg, 2.4 mmol) were dissolved in 2 mL of DMSO. The mixture was reacted at 80 °C for 4 h, and then TLC showed that the reaction was completed. The reaction liquid was diluted with ethyl acetate (20 mL), and then the organic phase was washed with saturated saline solution (10 ml*3). The solution was dried by rotary evaporation. The solute was separated and purified by column chromatography (petroleum ether: ethyl acetate = 10: 1 - 7: 1) to obtain compound A12-5 (750 mg, yield 90%). ¹H NMR (400 MHz, DMSO) δ 8.48 (d, *J* = 4.9 Hz, 1H), 8.27 - 8.20 (m, 1H), 8.03 (dd, *J* = 10.8, 8.3 Hz, 2H), 7.70 - 7.51 (m, 4H), 6.89 (t, *J* = 5.7 Hz, 1H), 6.86 (d, *J* = 4.9 Hz, 1H), 4.70 (d, *J* = 12.8 Hz, 2H), 2.98 - 2.79 (m, 4H), 1.68 (d, *J* = 10.3 Hz, 3H), 1.38 (s, 9H), 1.08 (dd, *J* = 22.0, 12.0 Hz, 2H).

### Synthesis of compound A12-6

A12-5 (160 mg, 0.38 mmol) was dissolved in 1 mL of dichloromethane, and HCl/ethyl acetate (2 mL, 4 M) was added. The mixture was reacted at ambient temperature for 2 h, and then TLC showed that the reaction was completed. The reaction liquid was dried by rotary evaporation to obtain compound A12-6 (140 mg, crude product).

### Synthesis of compound A12

A12-6 (140 mg, 0.38 mmol), A12-7 (90 mg, 0.38 mmol) and triethylamine (97 mg, 0.96 mmol) were dissolved in 3 mL of dichloromethane, and HATU (218 mg, 0.57 mmol) was added. The mixture was reacted at ambient temperature for 2 h, and then TLC showed that the reaction was completed. The reaction liquid was diluted with ethyl acetate (20 mL), and then the organic phase was washed with saturated saline solution (10 ml*3), and the solution was dried by rotary evaporation. The solute was separated and purified by column chromatography (dichloromethane: methanol = 100: 1 - 50: 1) to obtain compound A12 (60 mg, yield 26%).

¹H NMR (400 MHz, DMSO) δ 11.01 (s, 1H), 8.72 (t, *J* = 5.8 Hz, 1H), 8.49 (d, *J* = 4.9 Hz, 1H), 8.30 - 8.18 (m, 1H), 8.09 - 7.90 (m, 4H), 7.80 (d, *J* = 7.9 Hz, 1H), 7.68 - 7.64 (m, 1H), 7.63 - 7.50 (m, 3H), 6.87 (d, *J* = 4.9 Hz, 1H), 5.13 (dd, *J* = 13.3, 5.1 Hz, 1H), 4.73 (d, *J* = 13.1 Hz, 2H), 4.45 (dd, *J* = 50.9, 17.6 Hz, 2H), 3.23 (t, *J* = 6.2 Hz, 2H), 2.99 - 2.85 (m, 3H), 2.68 - 2.56 (m, 1H), 2.46 - 2.32 (m, 1H), 2.08 - 1.85 (m, 2H), 1.79 (d, *J* = 10.7 Hz, 2H), 1.24 - 1.12 (m, 2H). ESI-MS(M+H)⁺: 589.2.

The preparation methods of compounds A18, A21, A27, A36, A38, A44 and A45 refer to A12. The characterization results are as follows:

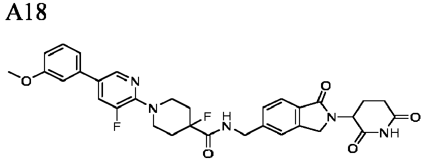

¹H NMR (400 MHz, DMSO) δ 11.01 (s, 1H), 8.72 (t, *J* = 5.7 Hz, 1H), 8.40 (s, 1H), 8.11 - 7.93 (m, 5H), 7.82 - 7.76 (m, 2H), 7.63 - 7.44 (m, 4H), 7.37 (s, 1H), 5.14 (dd, *J* = 13.3, 5.1 Hz, 1H), 4.46 (dd, *J* = 48.8, 17.6 Hz, 2H), 3.92 - 3.80 (m, 2H), 3.25 (t, *J* = 5.8 Hz, 2H), 2.97 - 2.86 (m, 1H), 2.77 (t, *J* = 11.4 Hz, 2H), 2.66 - 2.56 (m, 1H), 2.47 - 2.36 (m, 1H), 2.09 - 1.95 (m, 1H), 1.87 - 1.72 (m, 3H), 1.41 - 1.26 (m, 2H). ESI-MS(M+H)⁺: 588.2.

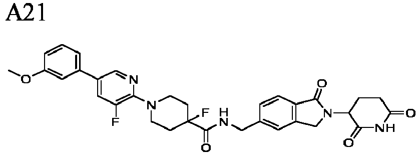

¹H NMR (400 MHz, DMSO) δ 11.00 (s, 1H), 8.71 (t, *J* = 5.7 Hz, 1H), 8.06 (s, 1H), 8.01 - 7.90 (m, 3H), 7.85 - 7.79 (m, 2H), 7.58 - 7.40 (m, 4H), 7.38 - 7.29 (m, 1H), 7.03 (d, *J* = 8.1 Hz, 1H), 6.97 (s, 1H), 6.84 (d, *J* = 7.5 Hz, 1H), 5.13 (dd, *J* = 13.3, 5.1 Hz, 1H), 4.45 (dd, *J* = 50.6, 17.7 Hz, 2H), 3.81 - 3.73 (m, 2H), 3.24 (t, *J* = 6.0 Hz, 2H), 2.97 - 2.85 (m, 1H), 2.77 - 2.57 (m, 3H), 2.45 - 2.33 (m, 1H), 2.09 - 1.99 (m, 1H), 1.85 - 1.72 (m, 3H), 1.40 - 1.26 (m, 2H). ESI-MS(M+H)⁺: 587.2.

The corresponding intermediates were prepared according to the following conditions:

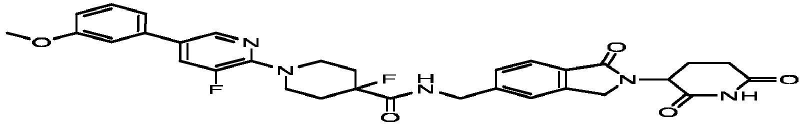

### Synthesis of compound A21-2

A21-1 (4.12 g, 17.4 mmol), A12-2 (1.5 g, 8.7 mmol) and potassium tert-butoxide (1.95 g, 17.4 mmol) were dissolved in 30 mL of dioxane, and Pd(dppf)Cl₂ (636 mg, 0.87 mmol) was added. The mixture was replaced with nitrogen three times and then reacted at 100 °C overnight, and then TLC showed that the reaction was completed. The reaction liquid was filtered, and the filtrate was dried by rotary evaporation. The solute was separated and purified by column chromatography (pure petroleum ether) to obtain compound A21-2 (1.2 g, yield 49%).

### Synthesis of compound A21-3

A21-2 (282 mg, 1.0 mmol), A12-4 (214 mg, 1.0 mmol) and sodium tert-butoxide (189 mg, 2.0 mmol) was dissolved in 15 mL of dioxane, and Pd₂(dba)₃ (92 mg, 0.1 mmol) and Xphos (48 mg, 0.1 mmol) were added. The mixture was replaced with nitrogen three times and then reacted at 90 °C overnight, and then TLC showed that the reaction was completed. The reaction liquid was filtered, and the filtrate was dried by rotary evaporation. The solute was separated and purified by a preparative TLC (petroleum ether: ethyl acetate = 4 : 1) to obtain compound A21-3 (360 mg, yield 86%).

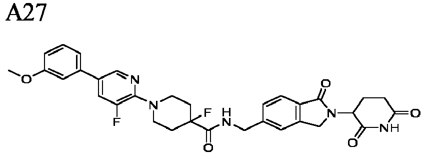

¹H NMR (400 MHz, DMSO) δ 11.00 (s, 1H), 8.74 (t, *J* = 5.7 Hz, 1H), 8.29 (d, *J* = 8.0 Hz, 1H), 8.07 (s, 1H), 8.01 - 7.95 (m, 2H), 7.87 (d, *J* = 8.2 Hz, 1H), 7.81 (d, *J* = 7.9 Hz, 1H), 7.72 (d, *J* = 6.5 Hz, 1H), 7.64 - 7.50 (m, 3H), 7.40 (s, 1H), 5.14 (dd, *J* = 13.2, 5.1 Hz, 1H), 4.46 (dd, *J* = 49.9, 17.6 Hz, 2H), 4.15 - 4.05 (m, 2H), 3.25 (t, *J* = 6.0 Hz, 2H), 3.05 (t, *J* = 11.6 Hz, 2H), 2.98 - 2.86 (m, 1H), 2.67 - 2.56 (m, 1H), 2.48 - 2.33 (m, 1H), 2.07 - 1.97 (m, 1H), 1.91 - 1.77 (m, 3H), 1.36 - 1.25 (m, 2H). ESI-MS(M+H)⁺: 578.2.

The corresponding intermediates were prepared according to the following conditions:

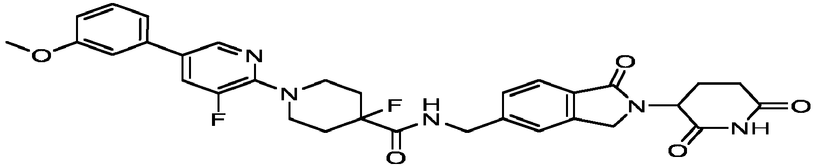

### Synthesis of compound A27-3

A27-1 (1.56 g, 10.0 mmol) and A27-2 (1.95 g, 10.0 mmol) were dissolved in 100 mL of methanol, and potassium carbonate (4.14 g, 30.0 mmol) was added. The mixture was reacted at 65 °C for 3 h, and then TLC showed that the reaction was completed. 200 mL of water was added to quench the reaction. The aqueous phase was extracted with ethyl acetate (100 ml*3). The organic phases were combined and dried by rotary evaporation. The solute was separated and purified by column chromatography (petroleum ether: ethyl acetate = 1: 1) to obtain compound A27-3 (1.1 g, yield 56%).

### Synthesis of compound A27-4

A27-3 (0.98 g, 5.0 mmol) was dissolved in 40 mL of anhydrous tetrahydrofuran, LiHMDS (7.5 mL, 1 M, 7.5 mmol) was added dropwise at -78 °C and the mixture was reacted for 3 h. TLC showed that the reaction was completed. 200 mL of water was added to quench the reaction. The aqueous phase was extracted with ethyl acetate (100 ml*3). The organic phases were combined and dried by rotary evaporation. The solute was separated and purified by column chromatography (petroleum ether: ethyl acetate = 1: 1) to obtain compound A27-4 (650 mg, yield 56%).

### Synthesis of compound A27-5

A27-4 (458 mg, 2.0 mmol) and A12-4 (471 g, 2.2 mmol) were dissolved in 10 mL of DMF, and potassium carbonate (828 mg, 6.0 mmol) was added. The mixture was reacted at 80 °C for 3 h, and then TLC showed that the reaction was completed. 100 mL of water was added to quench the reaction. The aqueous phase was extracted with ethyl acetate (60 ml*3), then the organic phases were combined, washed with saturated saline solution (20 ml*3), and dried. The solute was separated and purified by column chromatography (petroleum ether: ethyl acetate = 1: 1) to obtain compound A27-5 (575 mg, yield 71%).

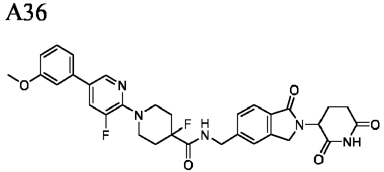

¹H NMR (400 MHz, DMSO) δ 11.00 (s, 1H), 8.74 (t, *J* = 5.7 Hz, 1H), 8.48 (s, 1H), 8.17 (d, *J* = 8.2 Hz, 1H), 8.08 (s, 1H), 8.00 - 7.96 (m, 2H), 7.93 (dd, *J* = 6.6, 2.8 Hz, 1H), 7.82 (d, *J* = 7.9 Hz, 1H), 7.60 - 7.43 (m, 6H), 5.14 (dd, *J* = 13.3, 5.1 Hz, 1H), 4.46 (dd, *J* = 49.1, 17.6 Hz, 2H), 3.92 - 3.88 (m, 2H), 3.27 (t, *J* = 5.9 Hz, 2H), 2.97 - 2.85 (m, 1H), 2.86 - 2.75 (m, 2H), 2.66 - 2.56 (m, 1H), 2.47 - 2.35 (m, 1H), 2.06 - 1.97 (m, 1H), 1.90 - 1.77 (m, 3H), 1.44 - 1.40 (m, 2H). ESI-MS(M+H)⁺: 588.2.

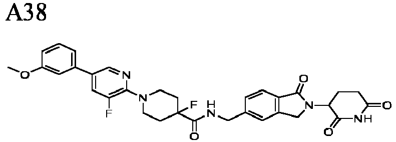

¹H NMR (400 MHz, DMSO) δ 11.00 (s, 1H), 8.74 - 8.71 (m, 2H), 8.47 (d, *J* = 5.1 Hz, 1H), 8.27 (dd, *J* = 8.6, 1.4 Hz, 1H), 8.12 - 8.02 (m, 3H), 8.00 - 7.94 (m, 2H), 7.82 (d, *J* = 7.9 Hz, 1H), 7.61 - 7.56 (m, 2H), 7.33 (d, *J* = 5.2 Hz, 1H), 5.15 (dd, *J* = 13.2, 5.0 Hz, 1H), 4.91 - 4.81 (m, 2H), 4.47 (dd, *J* = 49.6, 17.5 Hz, 2H), 3.25 (t, *J* = 6.1 Hz, 2H), 3.06 - 2.87 (m, 3H), 2.67 - 2.57 (m, 1H), 2.47 - 2.36 (m, 1H), 2.10 - 2.00 (m, 1H), 1.96 - 1.89 (m, 1H), 1.88 - 1.78 (m, 2H), 1.30 - 1.19 (m, 2H). ESI-MS(M+H)⁺: 589.2.

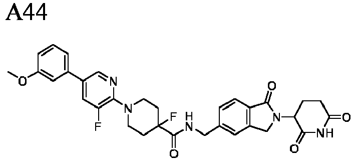

¹H NMR (400 MHz, DMSO) δ 11.00 (s, 1H), 8.94 (dd, *J* = 4.1, 1.8 Hz, 1H), 8.74 (t, *J* = 5.7 Hz, 1H), 8.48 - 8.40 (m, 2H), 8.19 - 8.10 (m, 2H), 8.07 (s, 1H), 8.02 - 7.95 (m, 2H), 7.82 (d, *J* = 7.9 Hz, 1H), 7.72 - 7.65 (m, 1H), 7.57 (dd, *J* = 8.3, 4.1 Hz, 1H), 7.43 (dd, *J* = 8.9, 2.9 Hz, 1H), 5.14 (dd, *J* = 13.3, 5.1 Hz, 1H), 4.46 (dd, *J* = 50.1, 17.6 Hz, 2H), 3.94 - 3.83 (m, 2H), 3.28 - 3.22 (m, 2H), 2.97 - 2.87 (m, 1H), 2.80 (t, *J* = 11.5 Hz, 2H), 2.68 - 2.56 (m, 1H), 2.45 - 2.36 (m, 1H), 2.09 - 1.98 (m, 1H), 1.89 - 1.73 (m, 3H), 1.43 - 1.30 (m, 2H). ESI-MS(M+H)⁺: 589.2.

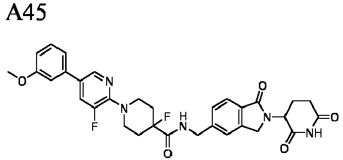

¹H NMR (400 MHz, DMSO) δ 11.00 (s, 1H), 8.73 (t, *J* = 5.7 Hz, 1H), 8.55 (d, *J* = 5.6 Hz, 1H), 8.45 (d, *J* = 2.3 Hz, 1H), 8.12 (d, *J* = 8.5 Hz, 1H), 8.07 (s, 1H), 8.01 (dd, *J* = 13.1, 8.6 Hz, 2H), 7.88 (dd, *J* = 8.8, 2.4 Hz, 1H), 7.82 (d, *J* = 8.0 Hz, 1H), 7.79 - 7.76 (m, 2H), 7.70 - 7.61 (m, 1H), 7.01 (d, *J* = 8.9 Hz, 1H), 5.14 (dd, *J* = 13.3, 5.1 Hz, 1H), 4.58 - 4.35 (m, 4H), 3.26 - 3.21 (m, 2H), 2.98 - 2.85 (m, 3H), 2.66 - 2.57 (m, 1H), 2.46 - 2.36 (m, 1H), 2.04 - 1.91 (m, 2H), 1.86 - 1.77 (m, 2H), 1.34 - 1.24 (m, 2H). ESI-MS(M+H)⁺: 589.2.

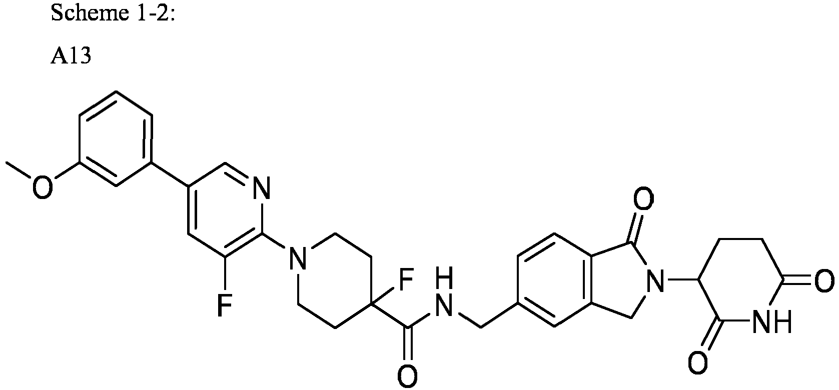

### Synthesis of compound A13-2

A12-3 (241 mg, 1.0 mmol), A13-1 (232 mg, 1.2 mmol) and DIPEA (322 mg, 2.5 mmol) were dissolved in 2 mL of DMSO. The mixture was reacted at 80 °C for 4 h, and then TLC showed that the reaction was completed. The reaction liquid was diluted with ethyl acetate (20 mL), and then the organic phase was washed with saturated saline solution (10 ml*3), and the solution was dried by rotary evaporation. The solute was separated and purified by column chromatography (petroleum ether: ethyl acetate = 10: 1 - 7: 1) to obtain compound A13-2 (350 mg, yield 97%).

### Synthesis of compound A13-3

A13-2 (350 mg, 0.97 mmol) was dissolved in methanol/water (3 mL/1 mL), and LiOH (122 mg, 2.90 mmol) was added. The mixture was reacted at ambient temperature for 2 h, and then TLC showed that the reaction was completed. After adjusting pH = 4 with 1 N dilute hydrochloric acid, the aqueous phase was extracted with dichloromethane (10 ml*3). The organic phases were combined and dried by rotary evaporation to obtain compound A13-3 (310 mg, yield 92%).

### Synthesis of compound A13

A13-3 (104 mg, 0.30 mmol), A13-4 (77 mg, 0.30 mmol) and triethylamine (45 mg, 0.45 mmol) were dissolved in 2 mL of DMF, HATU (171 mg, 0.45 mmol) was added. The mixture was reacted overnight at ambient temperature, and then TLC showed that the reaction was completed. The reaction liquid was diluted with ethyl acetate (20 mL), and then the organic phase was washed with saturated saline solution (10 ml*3), and the solution was dried by rotary evaporation. The solute was separated and purified by column chromatography (dichloromethane: methanol = 100: 1 - 30: 1) to obtain compound A13 (40 mg, yield 22%).

¹H NMR (400 MHz, DMSO) δ 10.97 (s, 1H), 10.26 (s, 1H), 8.49 (d, *J* = 4.9 Hz, 1H), 8.29 - 8.20 (m, 1H), 8.08 - 7.94 (m, 3H), 7.71 - 7.50 (m, 6H), 6.87 (d, *J* = 4.9 Hz, 1H), 5.08 (dd, *J* = 13.3, 5.0 Hz, 1H), 4.75 - 4.66 (m, 2H), 4.35 (dd, *J* = 56.4, 17.3 Hz, 2H), 3.04 - 2.82 (m, 3H), 2.70 - 2.54 (m, 1H), 2.44 - 2.30 (m, 3H), 2.18 - 2.05 (m, 1H), 2.03 - 1.91 (m, 1H), 1.82 - 1.73 (m, 2H), 1.28 - 1.17 (m, 2H). ESI-MS(M+H)⁺: 589.2.

The preparation methods of compounds A22, A28, A31, A33, A37, A46 and A57 refer to A13. The characterization results are as follows:

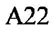

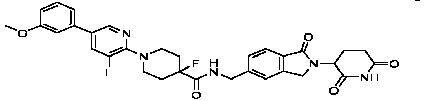

¹H NMR (400 MHz, DMSO) δ 10.96 (s, 1H), 10.26 (s, 1H), 8.00 - 7.97 (m, 2H), 7.93 (d, *J* = 8.2 Hz, 1H), 7.84 (d, *J* = 8.3 Hz, 1H), 7.65 (d, *J* = 8.3 Hz, 1H), 7.61 - 7.46 (m, 4H), 7.42 (dd, *J* = 7.0, 1.1 Hz, 1H), 7.37 - 7.29 (m, 1H), 7.03 (dd, *J* = 8.3, 1.9 Hz, 1H), 6.97 (s, 1H), 6.84 (d, *J* = 7.5 Hz, 1H), 5.07 (dd, *J* = 13.2, 5.1 Hz, 1H), 4.35 (dd, *J* = 55.8, 17.3 Hz, 2H), 3.79 - 3.71 (m, 2H), 2.97 - 2.85 (m, 1H), 2.79 - 2.69 (m, 2H), 2.67 - 2.55 (m, 1H), 2.44 - 2.29 (m, 3H), 2.05 - 1.90 (m, 2H), 1.82 - 1.73 (m, 2H), 1.43 - 1.31 (m, 2H). ESI-MS(M+H)⁺: 587.2.

The corresponding intermediates were prepared according to the following conditions:

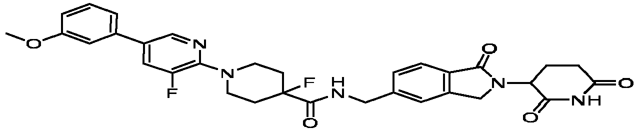

### Synthesis of compound A22-1

A21-2 (127 mg, 1.0 mmol), A13-1 (88 mg, 0.45 mmol) and cesium carbonate (441 mg, 1.35 mmol) were dissolved in 20 mL of dioxane, and Pd₂(dba)₃ (41 mg, 0.045 mmol) and Xphos (22 mg, 0.045 mmol) were added. The mixture was replaced with nitrogen three times and then reacted at 90 °C overnight, and then TLC showed that the reaction was completed. The reaction liquid was filtered, and the filtrate was dried by rotary evaporation. The solute was separated and purified by a preparative TLC (petroleum ether: ethyl acetate = 5 : 1) to obtain compound A22-1 (150 mg, yield 93%).

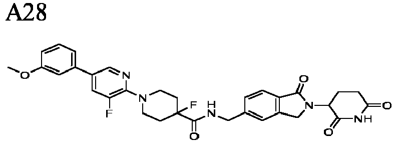

¹H NMR (400 MHz, DMSO) δ 10.97 (s, 1H), 10.27 (s, 1H), 8.29 (d, *J* = 8.2 Hz, 1H), 8.01 - 7.96 (m, 2H), 7.87 (d, *J* = 8.1 Hz, 1H), 7.72 (d, *J* = 6.3 Hz, 1H), 7.66 (d, *J* = 8.3 Hz, 1H), 7.63 - 7.51 (m, 4H), 7.40 (s, 1H), 5.08 (dd, *J* = 13.3, 5.2 Hz, 1H), 4.36 (dd, *J* = 56.0, 17.3 Hz, 2H), 4.11 - 4.03 (m, 2H), 3.09 (t, *J* = 11.6 Hz, 2H), 2.97 - 2.85 (m, 1H), 2.69 - 2.54 (m, 1H), 2.43 - 2.28 (m, 3H), 2.12 - 2.04 (m, 1H), 2.03 - 1.91 (m, 1H), 1.86 - 1.79 (m, 2H), 1.45 - 1.26 (m, 2H). ESI-MS(M+H)⁺: 578.2.

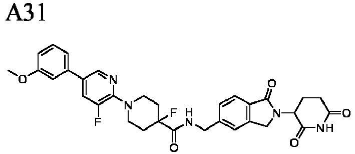

¹H NMR (400 MHz, DMSO) δ 10.96 (s, 1H), 10.26 (s, 1H), 8.50 (d, *J* = 1.2 Hz, 1H), 8.35 (d, *J* = 1.3 Hz, 1H), 8.14 (d, *J* = 8.3 Hz, 1H), 8.04 - 7.93 (m, 3H), 7.66 (d, *J* = 8.3 Hz, 1H), 7.63 - 7.47 (m, 5H), 5.08 (dd, *J* = 13.3, 5.1 Hz, 1H), 4.50 - 4.22 (m, 4H), 3.09 - 2.82 (m, 3H), 2.69 - 2.53 (m, 1H), 2.44 - 2.31 (m, 3H), 2.20 - 2.09 (m, 1H), 2.05 - 1.95 (m, 1H), 1.88 - 1.79 (m, 2H), 1.40 - 1.25 (m, 2H). ESI-MS(M+H)⁺: 589.2.

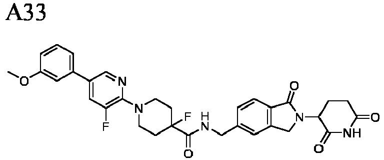

¹H NMR (400 MHz, DMSO) δ 10.96 (s, 1H), 10.28 (s, 1H), 8.79 - 8.64 (m, 3H), 8.03 - 7.93 (m, 4H), 7.66 (d, *J* = 8.3 Hz, 1H), 7.62 - 7.58 (m, 2H), 7.56 - 7.50 (m, 2H), 5.08 (dd, *J* = 13.3, 5.1 Hz, 1H), 4.36 (dd, *J* = 55.5, 17.3 Hz, 2H), 4.00 - 3.97 (m, 2H), 2.96 - 2.83 (m, 3H), 2.65 - 2.55 (m, 1H), 2.43 - 2.30 (m, 3H), 2.11 - 1.99 (m, 2H), 1.89 - 1.78 (m, 2H), 1.47 - 1.35 (m, 2H). ESI-MS(M+H)⁺: 589.2.

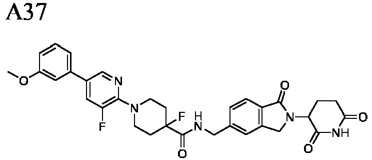

¹H NMR (400 MHz, DMSO) δ 10.96 (s, 1H), 10.28 (s, 1H), 8.48 (s, 1H), 8.17 (d, *J* = 8.3 Hz, 1H), 8.01 (s, 1H), 7.99 - 7.95 (m, 1H), 7.93 (dd, *J* = 6.8, 2.5 Hz, 1H), 7.67 (d, *J* = 8.3 Hz, 1H), 7.63 - 7.59 (m, 1H), 7.57 - 7.44 (m, 6H), 5.08 (dd, *J* = 13.3, 5.1 Hz, 1H), 4.36 (dd, *J* = 55.4, 17.4 Hz, 2H), 3.92 - 3.83 (m, 2H), 2.95 - 2.80 (m, 3H), 2.67 - 2.55 (m, 1H), 2.45 - 2.31 (m, 3H), 2.06 - 1.94 (m, 2H), 1.87 - 1.79 (m, 2H), 1.48 - 1.34 (m, 2H). ESI-MS(M+H)⁺: 588.2.

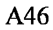

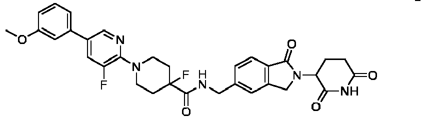

¹H NMR (400 MHz, DMSO) δ 10.96 (s, 1H), 10.26 (s, 1H), 8.55 (d, *J* = 5.6 Hz, 1H), 8.45 (d, *J* = 2.3 Hz, 1H), 8.12 (d, *J* = 8.5 Hz, 1H), 8.03 - 8.01 (m, 2H), 7.88 (dd, *J* = 8.8, 2.4 Hz, 1H), 7.83 - 7.75 (m, 2H), 7.70 - 7.63 (m, 2H), 7.60 (dd, *J* = 8.3, 1.4 Hz, 1H), 7.00 (d, *J* = 8.9 Hz, 1H), 5.08 (dd, *J* = 13.3, 5.1 Hz, 1H), 4.48 - 4.24 (m, 4H), 3.00 - 2.84 (m, 3H), 2.67 - 2.54 (m, 1H), 2.44 - 2.30 (m, 3H), 2.19 - 2.07 (m, 1H), 2.04 - 1.94 (m, 1H), 1.86 - 1.76 (m, 2H), 1.35 - 1.27 (m, 2H). ESI-MS(M+H)⁺: 589.2.

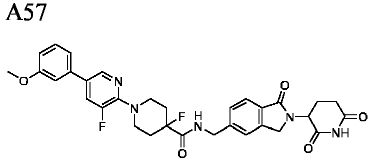

¹H NMR (400 MHz, DMSO) δ 10.96 (s, 1H), 10.26 (s, 1H), 8.39 (d, *J* = 2.8 Hz, 1H), 8.03 (s, 1H), 8.00 (s, 1H), 7.94 (d, *J* = 7.9 Hz, 1H), 7.84 (d, *J* = 8.8 Hz, 1H), 7.66 (d, *J* = 8.3 Hz, 1H), 7.60 (dd, *J* = 8.3, 1.4 Hz, 1H), 7.47 - 7.42 (m, 1H), 7.42 - 7.32 (m, 2H), 5.08 (dd, *J* = 13.3, 5.1 Hz, 1H), 4.36 (dd, *J* = 55.5, 17.3 Hz, 2H), 3.91 - 3.80 (m, 2H), 2.97 - 2.74 (m, 3H), 2.65 - 2.54 (m, 1H), 2.44 - 2.28 (m, 3H), 2.10 - 1.95 (m, 2H), 1.84 - 1.74 (m, 2H), 1.43 - 1.29 (m, 2H). ESI-MS(M+H)⁺: 572.2.

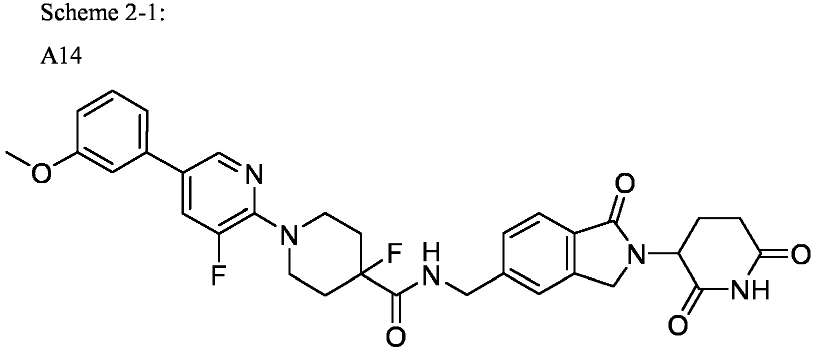

### Synthesis of compound A14-2

A14-1 (350 mg, 2.0 mmol), A12-4 (470 mg, 2.2 mmol) and potassium carbonate (552 mg, 4.0 mmol) were dissolved in 6 mL of acetonitrile. The mixture was reacted with stirring at room temperature for 3 h, and then TLC showed that the reaction was completed. The reaction liquid was diluted with water (20 mL), the organic phase was extracted with ethyl acetate (20 ml*3). The organic phases were combined, and dried by rotary evaporation. The solute was separated and purified by a preparative TLC (petroleum ether: ethyl acetate = 10:1) to obtain compound A14-2 (640 mg, yield 86%).

### Synthesis of compound A14-3

A14-2 (640 mg, 1.73 mmol), A12-2 (328 mg, 1.91 mmol) and potassium carbonate (263 mg, 3.82 mmol) were dissolved in dioxane/water (4 mL/1 mL), and Pd(PPh₃)₄(100 mg, 0.08 mmol) was added. The mixture was replaced with nitrogen three times and then reacted at 100 °C overnight, and then TLC showed that the reaction was completed. The reaction liquid was filtered, the filtrate was diluted with ethyl acetate (40 mL). The organic phase was washed with saturated saline solution (10 ml*3), and the solution was dried by rotary evaporation. The solute was separated and purified by column chromatography (petroleum ether: ethyl acetate = 5:1) to obtain compound A14-3 (690 mg, yield 95%).

### Synthesis of compound A14-4

A14-3 (150 mg, 0.36 mmol) was dissolved in 6 mL of dichloromethane, and 2 mL of trifluoroacetic acid was added. The mixture was reacted at ambient temperature for 2 h, and then TLC showed that the reaction was completed. The reaction liquid was dried by rotary evaporation to obtain compound A14-4 (180 mg, crude product).

### Synthesis of compound A14

A14-4 (180 mg, 0.36 mmol), A12-7 (103 mg, 0.36 mmol) and triethylamine (80 mg, 0.79 mmol) were dissolved in 6 mL of dichloromethane, and HATU (150 mg, 0.40 mmol) was added. The mixture was reacted at ambient temperature for 2 h, and then TLC showed that the reaction was completed. The reaction liquid was diluted with ethyl acetate (20 mL), and then the organic phase was washed with saturated saline solution (10 ml*3), and the solution was dried by rotary evaporation. The solute was separated and purified by a preparative TLC (dichloromethane : methanol = 10 : 1) and then subjected to medium pressure preparation to obtain compound A14 (40 mg, yield 18%).

¹H NMR (400 MHz, DMSO) δ 11.01 (s, 1H), 8.71 (t, *J* = 5.7 Hz, 1H), 8.21 (d, *J* = 8.2 Hz, 1H), 8.06 (s, 1H), 8.01 -7.95 (m, 3H), 7.81 (d, *J* = 7.9 Hz, 1H), 7.71 - 7.65 (m, 1H), 7.59 - 7.56 (m, 2H), 7.54 - 7.47 (m, 2H), 6.88 (dd, *J* = 15.4, 7.9 Hz, 2H), 5.14 (dd, *J* = 13.3, 5.1 Hz, 1H), 4.52 (d, *J* = 17.6 Hz, 1H), 4.43 - 4.31 (m, 3H), 3.23 (t, *J* = 6.2 Hz, 2H), 3.01 - 2.78 (m, 3H), 2.66 - 2.57 (m, 1H), 2.47 - 2.35 (m, 1H), 2.06 - 1.99 (m, 1H), 1.93 - 1.84 (m, 1H), 1.83 - 1.72 (m, 2H), 1.28 - 1.18 (m, 2H). ESI-MS(M+H)⁺: 588.2.

The preparation methods of compounds A16, A19, A20, A23, A29, A34, A39, A40, A42, and A52 refer to A14. The characterization results are as follows:

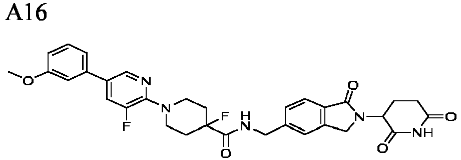

¹H NMR (400 MHz, DMSO) δ 11.01 (s, 1H), 8.71 (t, *J* = 5.7 Hz, 1H), 8.22 (d, *J* = 5.0 Hz, 1H), 8.07 (s, 1H), 8.04-7.96 (m, 3H), 7.82 (t, *J* = 8.5 Hz, 2H), 7.63 - 7.44 (m, 4H), 6.87 (s, 1H), 6.71 (dd, *J* = 5.0, 1.0 Hz, 1H), 5.15 (dd, *J* = 13.3, 5.1 Hz, 1H), 4.52 (d, *J* = 17.6 Hz, 1H), 4.46 - 4.34 (m, 3H), 3.24 (t, *J* = 6.2 Hz, 2H), 2.99 - 2.79 (m, 3H), 2.66 - 2.57 (m, 1H), 2.47-2.36 (m, 1H), 2.07 - 1.98 (m, 1H), 1.93-1.83 (m, 1H), 1.78 (d, *J* = 12.1 Hz, 2H), 1.29-1.17 (m, 2H). ESI-MS(M+H)⁺: 588.2.

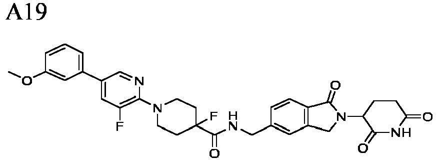

¹H NMR (400 MHz, DMSO) δ 11.00 (s, 1H), 8.73 (t, *J* = 5.7 Hz, 1H), 8.63 (s, 1H), 8.32 - 8.23 (m, 2H), 8.06 (s, 1H), 8.04 - 8.00 (m, 1H), 8.00 - 7.90 (m, 3H), 7.81 (d, *J* = 7.9 Hz, 1H), 7.58 - 7.50 (m, 2H), 7.49 (d, *J* = 2.3 Hz, 1H), 6.84 (dd, *J* = 6.0, 2.4 Hz, 1H), 5.14 (dd, *J* = 13.3, 5.1 Hz, 1H), 4.45 (dd, *J* = 49.8, 17.6 Hz, 2H), 4.15 - 4.11 (m, 2H), 3.24 (t, *J* = 6.2 Hz, 2H), 2.99 - 2.84 (m, 3H), 2.67 - 2.59 (m, 1H), 2.47 - 2.33 (m, 1H), 2.07 - 1.98 (m, 1H), 1.95 - 1.87 (m, 1H), 1.86 - 1.77 (m, 2H), 1.36 - 1.25 (m, 2H). ESI-MS(M+H)⁺: 588.2.

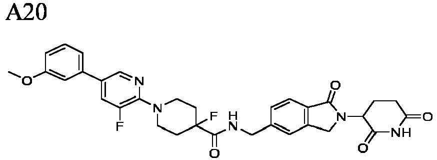

¹H NMR (400 MHz, DMSO) δ 11.02 (s, 1H), 8.73 (t, *J* = 5.7 Hz, 1H), 8.70 - 8.64 (m, 1H), 8.38 (d, *J* = 6.2 Hz, 1H), 8.06 (s, 1H), 8.04 - 7.93 (m, 4H), 7.82 (d, *J* = 7.9 Hz, 1H), 7.60 (dd, *J* = 8.0, 7.4 Hz, 1H), 7.56 - 7.50 (m, 2H), 6.87 (d, *J* = 6.3 Hz, 1H), 5.14 (dd, *J* = 13.3, 5.1 Hz, 1H), 4.58 - 4.44 (m, 3H), 4.40 (d, *J* = 17.6 Hz, 1H), 3.24 (t, *J* = 6.2 Hz, 2H), 3.02 - 2.88 (m, 3H), 2.62 (d, *J* = 16.5 Hz, 1H), 2.46 - 2.36 (m, 1H), 2.08 - 1.90 (m, 2H), 1.82 (d, *J* = 11.1 Hz, 2H), 1.28 - 1.15 (m, 2H). ESI-MS(M+H)⁺: 589.2.

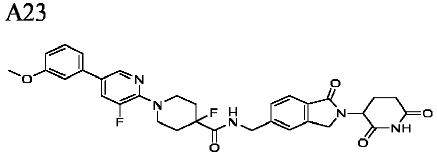

¹H NMR (400 MHz, DMSO) δ 11.01 (s, 1H), 8.76 (t, *J* = 5.7 Hz, 1H), 8.24 - 8.15 (m, 1H), 8.07 (s, 1H), 8.02 - 7.97 (m, 2H), 7.93 - 7.92 (m, 1H), 7.82 (d, *J* = 7.9 Hz, 1H), 7.61 - 7.48 (m, 4H), 7.33 (s, 1H), 5.14 (dd, *J* = 13.3, 5.1 Hz, 1H), 4.46 (dd, *J* = 49.2, 17.6 Hz, 2H), 4.02 - 3.92 (m, 2H), 3.26 (t, *J* = 6.0 Hz, 2H), 3.13 - 3.02 (m, 2H), 2.98 - 2.84 (m, 1H), 2.66 - 2.56 (m, 1H), 2.46 - 2.32 (m, 1H), 2.07 - 1.96 (m, 1H), 1.92 - 1.78 (m, 3H), 1.42 - 1.25 (m, 2H). ESI-MS(M+H)⁺: 594.2.

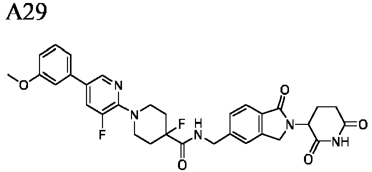

¹H NMR (400 MHz, DMSO) δ 11.01 (s, 1H), 8.75 (t, *J* = 5.7 Hz, 1H), 8.49 (s, 2H), 8.08 (s, 1H), 8.04 - 7.98 (m, 2H), 7.96 (d, *J* = 8.2 Hz, 1H), 7.86 - 7.79 (m, 2H), 7.62 - 7.51 (m, 3H), 7.46 (dd, *J* = 7.0, 1.0 Hz, 1H), 5.14 (dd, *J* = 13.3, 5.1 Hz, 1H), 4.81 - 4.73 (m, 2H), 4.46 (dd, *J* = 50.3, 17.6 Hz, 2H), 3.25 (t, *J* = 6.3 Hz, 2H), 3.03 - 2.84 (m, 3H), 2.67 - 2.57 (m, 1H), 2.48 - 2.37 (m, 1H), 2.08 - 2.01 (m, 1H), 1.97 - 1.90 (m, 1H), 1.87 - 1.79 (m, 2H), 1.28 - 1.22 (m, 2H). ESI-MS(M+H)⁺: 589.2.

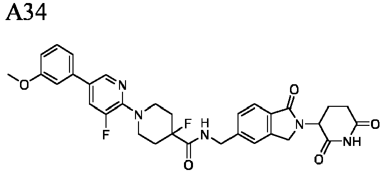

¹H NMR (400 MHz, DMSO) δ 11.00 (s, 1H), 8.73 (t, *J* = 5.6 Hz, 1H), 8.21 (d, *J* = 2.3 Hz, 1H), 8.07 (s, 1H), 7.98 (d, *J* = 3.2 Hz, 2H), 7.93 (d, *J* = 8.4 Hz, 1H), 7.86 - 7.80 (m, 2H), 7.65 (dd, *J* = 8.8, 2.4 Hz, 1H), 7.58 - 7.48 (m, 3H), 7.42 (d, *J* = 6.3 Hz, 1H), 6.99 (d, *J* = 8.8 Hz, 1H), 5.14 (dd, *J* = 13.2, 5.0 Hz, 1H), 4.55 - 4.38 (m, 4H), 3.25 (t, *J* = 6.0 Hz, 2H), 2.98 - 2.81 (m, 3H), 2.67 - 2.56 (m, 1H), 2.46 - 2.34 (m, 1H), 2.10 - 1.97 (m, 1H), 1.95 - 1.86 (m, 1H), 1.85 - 1.77 (m, 2H), 1.30 - 1.24 (m, 2H). ESI-MS(M+H)⁺: 588.2.

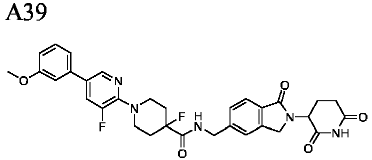

¹H NMR (400 MHz, DMSO) δ 11.00 (s, 1H), 8.84 (s, 2H), 8.72 (t, *J* = 5.7 Hz, 1H), 8.18 (s, 1H), 8.07 (s, 1H), 7.99 (d, *J* = 8.6 Hz, 2H), 7.95 - 7.91 (m, 2H), 7.86 - 7.78 (m, 2H), 7.58 - 7.46 (m, 2H), 5.14 (dd, *J* = 13.3, 5.1 Hz, 1H), 4.75 (d, *J* = 13.2 Hz, 2H), 4.46 (dd, *J* = 49.6, 17.5 Hz, 2H), 3.24 (t, *J* = 6.2 Hz, 2H), 3.01 - 2.88 (m, 3H), 2.67 - 2.55 (m, 1H), 2.47 - 2.34 (m, 1H), 2.09 - 1.99 (m, 1H), 1.98 - 1.89 (m, 1H), 1.87 - 1.77 (m, 2H), 1.22 - 1.11 (m, 2H). ESI-MS(M+H)⁺: 589.2.

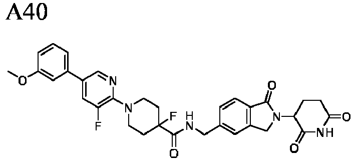

¹H NMR (400 MHz, DMSO) δ 11.00 (s, 1H), 8.72 (t, *J* = 5.7 Hz, 1H), 8.59 (d, *J=* 2.5 Hz, 1H), 8.14 (s, 1H), 8.06 (s, 1H), 8.02 - 7.87 (m, 5H), 7.81 (d, *J=* 8.0 Hz, 2H), 7.56 - 7.43 (m, 2H), 6.97 (d, *J =* 9.0 Hz, 1H), 5.13 (dd, *J =* 13.3, 5.1 Hz, 1H), 4.54 - 4.37 (m, 4H), 3.23 (t, *J* = 6.2 Hz, 2H), 2.97 - 2.81 (m, 3H), 2.67 - 2.56 (m, 1H), 2.47 - 2.36 (m, 1H), 2.09 - 1.96 (m, 1H), 1.94 - 1.84 (m, 1H), 1.83 - 1.75 (m, 2H), 1.29 - 1.18 (m, 2H). ESI-MS(M+H)⁺: 588.2.

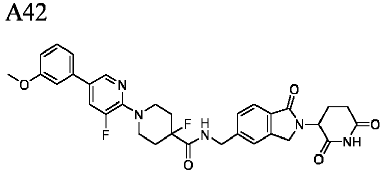

¹H NMR (400 MHz, DMSO) δ 11.01 (s, 1H), 8.91 (dd, *J* = 4.1, 1.8 Hz, 1H), 8.73 (t, *J=* 5.7 Hz, 1H), 8.46 - 8.38 (m, 2H), 8.07 (s, 1H), 7.99 (d, *J=* 7.9 Hz, 1H), 7.95 - 7.89 (m, 2H), 7.82 (d, *J* = 7.9 Hz, 1H), 7.78 (dd, *J* = 7.2, 1.4 Hz, 1H), 7.70 - 7.63 (m, 1H), 7.57 (dd, *J* = 8.3, 4.1 Hz, 1H), 6.95 (d, *J* = 8.9 Hz, 1H), 5.14 (dd, *J* = 13.3, 5.1 Hz, 1H), 4.55 - 4.37 (m, 4H), 3.24 (t, *J* = 6.3 Hz, 2H), 2.97 - 2.82 (m, 3H), 2.67 - 2.56 (m, 1H), 2.47 - 2.35 (m, 1H), 2.06 - 1.98 (m, 1H), 1.97 - 1.85 (m, 1H), 1.85 - 1.77 (m, 2H), 1.30 - 1.19 (m, 2H). ESI-MS(M+H)⁺: 589.2.

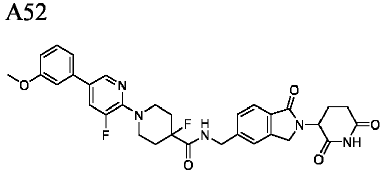

¹H NMR (400 MHz, DMSO) δ 11.00 (s, 1H), 8.73 - 8.68 (m, 3H), 8.06 (s, 1H), 7.98 (d, *J* = 7.9 Hz, 1H), 7.81 (d, *J* = 7.9 Hz, 1H), 7.74 - 7.72 (m, 1H), 7.61 (d, *J* = 8.0 Hz, 1H), 7.48 - 7.39 (m, 1H), 7.37 (d, *J* = 8.0 Hz, 1H), 5.14 (dd, *J* = 13.3, 5.1 Hz, 1H), 4.74 - 4.70 (m, 2H), 4.46 (dd, *J* = 50.2, 17.6 Hz, 2H), 3.22 (t, *J* = 6.2 Hz, 2H), 3.01 - 2.86 (m, 3H), 2.67 - 2.57 (m, 1H), 2.47 - 2.35 (m, 1H), 2.09 - 1.99 (m, 1H), 1.98 - 1.87 (m, 1H), 1.84 - 1.75 (m, 2H), 1.21 - 1.09 (m, 2H). ESI-MS(M+H)⁺: 573.2.

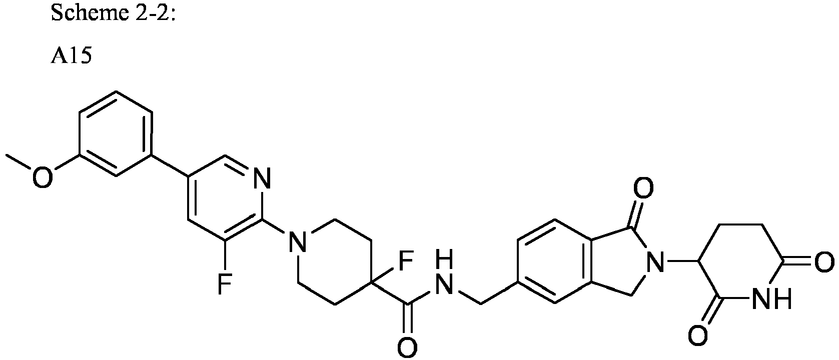

### Synthesis of compound A15-1

A14-1 (150 mg, 0.86 mmol), A13-1 (148 mg, 0.94 mmol) and potassium carbonate (237 mg, 1.71 mmol) were dissolved in 6 mL of acetonitrile. The mixture was reacted with stirring at 60 °C for 3 h, and then TLC showed that the reaction was completed. The reaction liquid was diluted with water (20 mL), the organic phase was extracted with ethyl acetate (20 ml*3). Then the organic phases were combined, dried by rotary evaporation. The solute was separated and purified by a preparative TLC (petroleum ether: ethyl acetate = 5 : 1) to obtain compound A15-1 (250 mg, yield 93%).

### Synthesis of compound A15-2

A15-1 (250 mg, 0.80 mmol), A12-2 (152 mg, 0.88 mmol) and potassium carbonate (122 mg, 0.88 mmol) were dissolved in dioxane/water (5 mL/1 mL), and Pd(PPh₃)₄(65 mg, 0.05 mmol) was added. The mixture was replaced with nitrogen three times and then reacted at 100 °C overnight, and then TLC showed that the reaction was completed. The reaction liquid was filtered, the filtrate was diluted with ethyl acetate (40 mL). The organic phase was washed with saturated saline solution (10 ml*3), and the solution was dried by rotary evaporation. The solute was separated and purified by the preparative TLC (petroleum ether: ethyl acetate = 5 : 1) to obtain compound A15-2 (250 mg, yield 87%).

### Synthesis of compound A15-3

A15-2 (250 mg, 0.69 mmol) was dissolved in methanol/water (6 mL/1 mL), and LiOH (58 mg, 1.39 mmol) was added. The mixture was reacted at 50 °C for 2 h, and then TLC showed that the reaction was completed. The methanol was spun off, 1 N hydrochloric acid aqueous solution was used to adjust pH = 4. The aqueous phase was extracted with dichloromethane (20 ml*3). The organic phases were combined and dried by rotary evaporation to obtain compound A15-3 (240 mg, yield 100%).

### Synthesis of compound A15

A15-3 (240 mg, 0.69 mmol), A13-4 (180 mg, 0.69 mmol) and triethylamine (154 mg, 1.53 mmol) were dissolved in 6 mL of DMF, and HATU (290 mg, 0.76 mmol) was added. The mixture was reacted overnight at ambient temperature, and then TLC showed that the reaction was completed. The reaction liquid was diluted with ethyl acetate (40 mL), and then the organic phase was washed with saturated saline solution (10 ml*3), and the solution was dried by rotary evaporation. The solute was separated and purified by a preparative TLC (dichloromethane : methanol = 10 : 1) and then subjected to medium pressure preparation to obtain compound A15 (55 mg, yield 13%).

¹H NMR (400 MHz, DMSO) δ 10.96 (s, 1H), 10.23 (s, 1H), 8.20 (d, *J* = 8.3 Hz, 1H), 8.03 - 7.92 (m, 3H), 7.72 - 7.62 (m, 2H), 7.62 - 7.55 (m, 3H), 7.55 - 7.43 (m, 2H), 6.89 (d, *J* = 8.6 Hz, 1H), 6.86 (d, *J* = 7.2 Hz, 1H), 5.08 (dd, *J* = 13.3, 5.1 Hz, 1H), 4.44 - 4.25 (m, 4H), 2.98 - 2.80 (m, 3H), 2.65 - 2.54 (m, 1H), 2.43-2.27 (m, 3H), 2.13 - 2.03 (m, 1H), 2.04 - 1.92 (m, 1H), 1.80 - 1.70 (m, 2H), 1.34 - 1.24 (m, 2H). ESI-MS(M+H)⁺: 588.2.

The preparation methods of compounds A17, A24, A26, A30, A32, A35, A41, A43, A47, A48, A49, A50, A51, A53, A54, A55, A56, A58, A59, A60, A65, and A119 refer to A15. The characterization results are as follows:

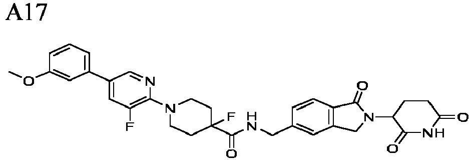

¹H NMR (400 MHz, DMSO) δ 10.95 (s, 1H), 10.25 (s, 1H), 8.21 (d, *J* = 4.3 Hz, 1H), 8.04 - 7.99 (m, 3H), 7.83 (d, *J* = 7.8 Hz, 1H), 7.70 - 7.41 (m, 6H), 6.86 (s, 1H), 6.70 (d, *J* = 4.1 Hz, 1H), 5.08 (d, *J* = 8.8 Hz, 1H), 4.47 - 4.22 (m, 4H), 3.00 - 2.75 (m, 3H), 2.68 - 2.54 (m, 1H), 2.43 - 2.26 (m, 3H), 2.13 - 1.92 (m, 2H), 1.82 - 1.69 (m, 2H), 1.36 - 1.22 (m, 2H). ESI-MS(M+H)⁺: 588.2.

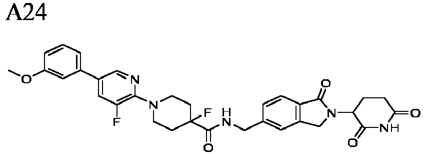

¹H NMR (400 MHz, DMSO) δ 10.98 (s, 1H), 10.29 (s, 1H), 8.23 - 8.17 (m, 1H), 8.04 - 7.96 (m, 2H), 7.94 - 7.90 (m, 1H), 7.66 (d, *J* = 8.3 Hz, 1H), 7.62 - 7.50 (m, 5H), 7.33 (s, 1H), 5.08 (dd, *J* = 13.3, 5.1 Hz, 1H), 4.36 (dd, *J* = 56.1, 17.4 Hz, 2H), 4.01 - 3.90 (m, 2H), 3.16 - 3.06 (m, 2H), 2.99 - 2.82 (m, 1H), 2.67 - 2.54 (m, 1H), 2.45 - 2.27 (m, 3H), 2.16 - 2.04 (m, 1H), 2.04 - 1.94 (m, 1H), 1.87 - 1.77 (m, 2H), 1.44 - 1.30 (m, 2H). ESI-MS(M+H)⁺: 594.2.

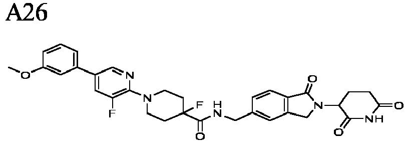

¹H NMR (400 MHz, DMSO) δ 10.97 (s, 1H), 10.28 (s, 1H), 8.08 - 7.96 (m, 2H), 7.85 (d, *J* = 8.1 Hz, 1H), 7.66 (d, *J* = 8.3 Hz, 1H), 7.63 - 7.48 (m, 4H), 7.31 (dd, *J* = 13.8, 7.4 Hz, 1H), 7.09 (d, *J* = 1.6 Hz, 1H), 5.08 (dd, *J* = 13.2, 5.1 Hz, 1H), 4.36 (dd, *J* = 55.9, 17.4 Hz, 2H), 3.96 - 2.85 (m, 2H), 3.07 (t, *J* = 11.4 Hz, 2H), 2.95 - 2.84 (m, 1H), 2.70 - 2.54 (m, 1H), 2.43 - 2.29 (m, 3H), 2.14 - 2.05 (m, 1H), 2.03 - 1.96 (m, 1H), 1.86 - 1.76 (m, 2H), 1.43 - 1.30 (m, 2H). ESI-MS(M+H)⁺: 612.2

The corresponding intermediates were prepared according to the following conditions:

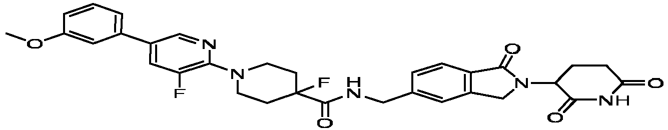

### Synthesis of compound A26-2

A26-1 (2.2 g, 10 mmol) was dissolved in 48% aqueous fluoroboric acid solution/tetrahydrofuran (2 mL/5 mL). 4 mL sodium nitrite (2.07 g, 30 mmol) aqueous solution was added dropwise in an ice bath. The mixture was reacted with stirring for 1 h at 0 °C, and then NaBF₄ (5.5 g, 50 mmol) was added. The mixture was reacted at ambient temperature for 1 h, and then TLC showed that the reaction was completed. The reaction liquid was filtered, and the filter cake was washed with methyl tert-butyl ether (5 mL*2) and dried to obtain the intermediate. This intermediate was dissolved in 8 mL of chlorobenzene and the mixture was heated to reflux. The mixture was reacted for 3 h, and then TLC showed that the reaction was completed. The reaction liquid was dried by rotary evaporation. The solute was separated and purified by column chromatography (petroleum ether: ethyl acetate = 100 : 1) to obtain compound A26-2 (1.32 g, yield 59%).

### Synthesis of compound A26-3

A26-2 (1.32 g, 5.9 mmol), pinacol boronate (3.01 g, 11.8 mmol) and potassium acetate (1.73 g, 17.7 mmol) were dissolved in 15 mL of dioxane, and Pd(dppf)Cl₂ (432 mg, 0.59 mmol) were added. The mixture was replaced with nitrogen three times and then reacted at 90 °C for 18 h. TLC showed that the reaction was completed. The reaction liquid was filtered, the filtrate was diluted with ethyl acetate (50 mL). The organic phase was washed with saturated saline solution (10 ml*3), and the organic phase was dried by rotary evaporation. The solute was separated and purified by column chromatography (petroleum ether: ethyl acetate = 100: 1 - 50: 1) to obtain compound A26-3 (885 mg, yield 55%).

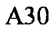

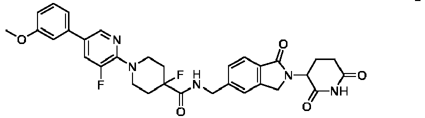

¹H NMR (400 MHz, DMSO) δ 10.97 (s, 1H), 10.27 (s, 1H), 8.50 (s, 2H), 8.02 - 7.99 (m, 2H), 7.96 (d, *J* = 8.3 Hz, 1H), 7.84 (d, *J* = 7.7 Hz, 1H), 7.66 (d, *J* = 8.2 Hz, 1H), 7.63 - 7.51 (m, 4H), 7.46 (d, *J* = 6.1 Hz, 1H), 5.08 (dd, *J* = 13.2, 5.1 Hz, 1H), 4.79 - 4.71 (m, 2H), 4.36 (dd, *J* = 55.9, 17.3 Hz, 2H), 3.01 (t, *J* = 11.8 Hz, 2H), 2.96 - 2.84 (m, 1H), 2.67 - 2.56 (m, 1H), 2.44 - 2.30 (m, 3H), 2.21 - 2.09 (m, 1H), 2.04 - 1.97 (m, 1H), 1.86 - 1.76 (m, 2H), 1.32 - 1.24 (m, 2H). ESI-MS(M+H)⁺: 589.2.

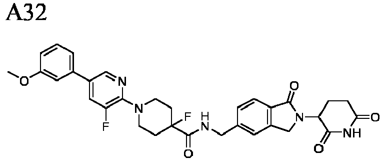

¹H NMR (400 MHz, DMSO) δ 10.97 (s, 1H), 10.27 (s, 1H), 8.05 (d, *J* = 8.4 Hz, 1H), 8.02 - 7.98 (m, 3H), 7.69 - 7.59 (m, 5H), 7.59 - 7.49 (m, 2H), 7.42 (d, *J* = 9.6 Hz, 1H), 5.08 (dd, *J* = 13.3, 5.1 Hz, 1H), 4.54 - 4.23 (m, 4H), 3.02 (t, *J* = 11.7 Hz, 2H), 2.96 - 2.85 (m, 1H), 2.65-2.55 (m, 1H), 2.43 - 2.28 (m, 3H), 2.24 - 2.09 (m, 1H), 2.06 - 1.94 (m, 1H), 1.89 - 1.78 (m, 2H), 1.41 - 1.28 (m, 2H). ESI-MS(M+H)⁺: 589.2.

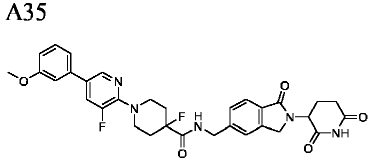

¹H NMR (400 MHz, DMSO) δ 10.97 (s, 1H), 10.27 (s, 1H), 8.21 (d, *J* = 2.3 Hz, 1H), 8.00 (dd, *J* = 8.4, 7.0 Hz, 2H), 7.92 (d, *J* = 8.2 Hz, 1H), 7.85 (d, *J* = 8.1 Hz, 1H), 7.70 - 7.64 (m, 2H), 7.63 - 7.47 (m, 4H), 7.45 - 7.38 (m, 1H), 6.98 (d, *J* = 8.8 Hz, 1H), 5.08 (dd, *J* = 13.3, 5.1 Hz, 1H), 4.49 - 4.22 (m, 4H), 2.97 - 2.84 (m, 3H), 2.66 - 2.55 (m, 1H), 2.44 - 2.29 (m, 3H), 2.17 - 2.05 (m, 1H), 2.05 - 1.92 (m, 1H), 1.84 - 1.75 (m, 2H), 1.37 - 1.24 (m, 2H). ESI-MS(M+H)⁺: 588.2.

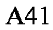

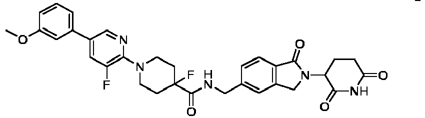

¹H NMR (400 MHz, DMSO) δ 10.97 (s, 1H), 10.25 (s, 1H), 8.59 (d, *J* = 2.4 Hz, 1H), 8.14 (s, 1H), 8.05 - 7.87 (m, 5H), 7.87 - 7.78 (m, 1H), 7.66 (d, *J* = 8.3 Hz, 1H), 7.60 (d, *J* = 8.4 Hz, 1H), 7.54 - 7.45 (m, 2H), 6.97 (d, *J* = 8.9 Hz, 1H), 5.08 (dd, *J* = 13.3, 4.9 Hz, 1H), 4.45 - 4.26 (m, 4H), 2.94 - 2.87 (m, 3H), 2.68 - 2.55 (m, 1H), 2.44 - 2.27 (m, 3H), 2.16 - 2.03 (m, 1H), 2.02 - 1.94 (m, 1H), 1.82 - 1.73 (m, 2H), 1.34 - 1.25 (m, 2H). ESI-MS(M+H)⁺: 588.2.

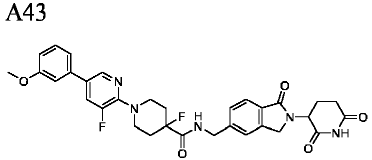

¹H NMR (400 MHz, DMSO) δ 10.96 (s, 1H), 10.25 (s, 1H), 8.90 (dd, *J* = 4.1, 1.8 Hz, 1H), 8.45 - 8.38 (m, 2H), 8.01 (s, 1H), 7.95 - 7.88 (m, 2H), 7.77 (dd, *J* = 7.2, 1.4 Hz, 1H), 7.70 - 7.63 (m, 2H), 7.60 (dd, *J* = 8.3, 1.6 Hz, 1H), 7.56 (dd, *J* = 8.3, 4.1 Hz, 1H), 6.94 (d, *J* = 8.9 Hz, 1H), 5.08 (dd, *J* = 13.3, 5.1 Hz, 1H), 4.45 - 4.27 (m, 4H), 2.98 - 2.85 (m, 3H), 2.66 - 2.55 (m, 1H), 2.44 - 2.29 (m, 3H), 2.17 - 2.07 (m, 1H), 2.05 - 1.94 (m, 1H), 1.83 - 1.74 (m, 2H), 1.35 - 1.25 (m, 2H). ESI-MS(M+H)⁺: 589.2.

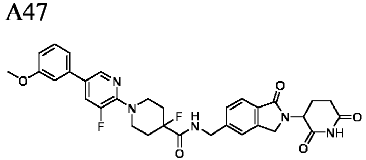

¹H NMR (400 MHz, DMSO) δ 10.98 (s, 1H), 10.28 (s, 1H), 8.13 (s, 1H), 8.02 - 8.00 (m, 2H), 7.86 (d, *J* = 8.1 Hz, 1H), 7.67 (d, *J* = 8.3 Hz, 1H), 7.64 - 7.58 (m, 2H), 7.58 - 7.49 (m, 2H), 7.38 (d, *J* = 6.3 Hz, 1H), 7.26 (dd, *J* = 13.6, 7.1 Hz, 1H), 6.88 (d, *J* = 8.8 Hz, 1H), 5.09 (dd, *J* = 13.3, 5.1 Hz, 1H), 4.46 - 4.28 (m, 4H), 2.98 - 2.82 (m, 3H), 2.68 - 2.55 (m, 1H), 2.44 - 2.30 (m, 3H), 2.17 - 2.05 (m, 1H), 2.05 - 1.94 (m, 1H), 1.84 - 1.75 (m, 2H), 1.37 - 1.25 (m, 2H). ESI-MS(M+H)⁺: 606.2.

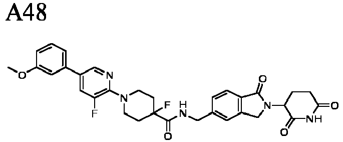

¹H NMR (400 MHz, DMSO) δ 10.96 (s, 1H), 10.24 (s, 1H), 8.42 (s, 2H), 7.99 (s, 1H), 7.65 (d, *J* = 8.3 Hz, 1H), 7.59 (dd, *J* = 8.3, 1.5 Hz, 1H), 5.08 (dd, *J* = 13.3, 5.1 Hz, 1H), 4.60 - 4.50 (m, 2H), 4.35 (dd, *J* = 55.5, 17.3 Hz, 2H), 2.98 - 2.82 (m, 3H), 2.65 - 2.55 (m, 1H), 2.43 - 2.23 (m, 3H), 2.16 - 2.04 (m, 1H), 2.04 - 1.92 (m, 1H), 1.81 - 1.70 (m, 2H), 1.21 - 1.11 (m, 2H). ESI-MS(M+H)⁺: 541.2.

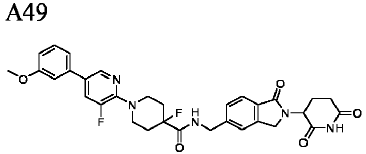

¹H NMR (400 MHz, DMSO) δ 10.95 (s, 1H), 10.21 (s, 1H), 8.14 (s, 2H), 7.99 (s, 1H), 7.65 (d, *J* = 8.3 Hz, 1H), 7.62 - 7.55 (m, 1H), 5.07 (dd, *J* = 13.2, 5.1 Hz, 1H), 4.61 - 4.53 (m, 2H), 4.35 (dd, *J* = 55.1, 17.3 Hz, 2H), 2.96 - 2.80 (m, 3H), 2.65 - 2.55 (m, 1H), 2.43 - 2.27 (m, 3H), 2.14 - 1.93 (m, 2H), 1.82 - 1.66 (m, 3H), 1.21 - 1.07 (m, 2H), 0.89 - 0.79 (m, 2H), 0.64 - 0.55 (m, 2H). ESI-MS(M+H)⁺: 503.2.

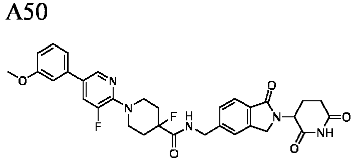

¹H NMR (400 MHz, DMSO) δ 10.96 (s, 1H), 10.23 (s, 1H), 8.25 (s, 2H), 7.99 (s, 1H), 7.65 (d, *J* = 8.3 Hz, 1H), 7.59 (dd, *J* = 8.3, 1.6 Hz, 1H), 5.08 (dd, *J* = 13.3, 5.1 Hz, 1H), 4.64 - 4.54 (m, 2H), 4.35 (dd, *J* = 55.5, 17.3 Hz, 2H), 2.95 - 2.81 (m, 3H), 2.78 - 2.72 (m, 1H), 2.66 - 2.56 (m, 1H), 2.42 - 2.34 (m, 1H), 2.33 - 2.27 (m, 2H), 2.12 - 2.04 (m, 1H), 2.03 - 1.93 (m, 1H), 1.78 - 1.69 (m, 2H), 1.22 - 1.07 (m, 8H). ESI-MS(M+H)⁺: 503.2.

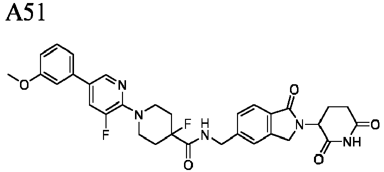

¹H NMR (400 MHz, DMSO) δ 10.96 (s, 1H), 10.25 (s, 1H), 8.69 (s, 2H), 8.00 (s, 1H), 7.68 - 7.58 (m, 4H), 7.48 - 7.40 (m, 2H), 7.36 - 7.30 (m, 1H), 5.08 (dd, *J* = 13.3, 5.1 Hz, 1H), 4.74 - 4.65 (m, 2H), 4.36 (dd, *J* = 55.2, 17.3 Hz, 2H), 3.02 - 2.84 (m, 3H), 2.65 - 2.55 (m, 1H), 2.43 - 2.29 (m, 3H), 2.19 - 2.08 (m, 1H), 2.03 - 1.93 (m, 1H), 1.83 - 1.74 (m, 2H), 1.25 - 1.11 (m, 2H). ESI-MS(M+H)⁺: 539.2.

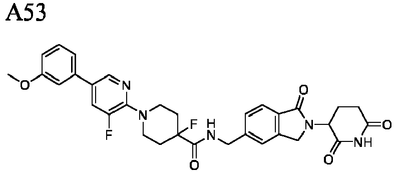

¹H NMR (400 MHz, DMSO) δ 10.97 (s, 1H), 10.25 (s, 1H), 8.72 (s, 2H), 8.00 (s, 1H), 7.73 (s, 1H), 7.66 (d, *J* = 8.3 Hz, 1H), 7.63 - 7.58 (m, 2H), 7.47 - 7.43 (m, 1H), 7.37 (d, *J* = 8.0 Hz, 1H), 5.08 (dd, *J* = 13.3, 5.1 Hz, 1H), 4.75 - 4.64 (m, 2H), 4.36 (dd, *J* = 54.9, 17.3 Hz, 2H), 3.03 - 2.84 (m, 3H), 2.65 - 2.55 (m, 1H), 2.43 - 2.27 (m, 3H), 2.19 - 2.06 (m, 1H), 2.04 - 1.94 (m, 1H), 1.85 - 1.72 (m, 2H), 1.28 - 1.13 (m, 2H). ESI-MS(M+H)⁺: 573.2.

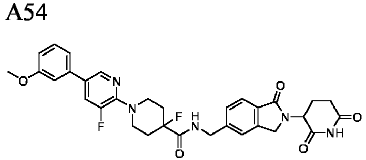

¹H NMR (400 MHz, DMSO) δ 10.97 (s, 1H), 10.26 (s, 1H), 8.46 (s, 2H), 8.00 (s, 1H), 7.66 (d, *J* = 8.3 Hz, 1H), 7.63 - 7.54 (m, 2H), 7.49 - 7.35 (m, 3H), 5.08 (dd, *J* = 13.3, 5.1 Hz, 1H), 4.75 - 4.66 (m, 2H), 4.33 (dd, *J* = 55.7, 17.4 Hz, 2H), 3.03 - 2.83 (m, 3H), 2.69 - 2.54 (m, 1H), 2.43 - 2.28 (m, 3H), 2.19 - 2.07 (m, 1H), 2.03 - 1.93 (m, 1H), 1.84 - 1.74 (m, 2H), 1.27 - 1.15 (m, 2H). ESI-MS(M+H)⁺: 573.2.

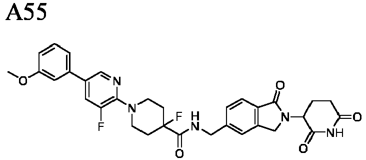

¹H NMR (400 MHz, DMSO) δ 10.96 (s, 1H), 10.25 (s, 1H), 8.70 (s, 2H), 8.00 (s, 1H), 7.68 - 7.64 (m, 3H), 7.59 (dd, *J* = 8.4, 1.6 Hz, 1H), 7.52 - 7.46 (m, 2H), 5.08 (dd, *J* = 13.3, 5.2 Hz, 1H), 4.73 - 4.64 (m, 2H), 4.36 (dd, *J* = 55.9, 17.3 Hz, 2H), 3.02 - 2.85 (m, 3H), 2.64 - 2.54 (m, 1H), 2.42 - 2.29 (m, 3H), 2.18 - 2.07 (m, 1H), 2.04 - 1.95 (m, 1H), 1.83 - 1.74 (m, 2H), 1.22 - 1.13 (m, 2H). ESI-MS(M+H)⁺: 573.2.

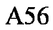

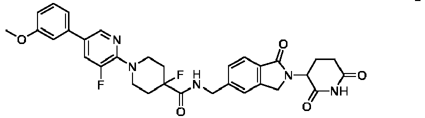

¹H NMR (400 MHz, DMSO) δ 10.96 (s, 1H), 10.24 (s, 1H), 8.46 (d, *J* = 2.5 Hz, 1H), 8.00 (s, 1H), 7.86 (dd, *J* = 9.0, 2.6 Hz, 1H), 7.68 - 7.66 (m, 2H), 7.59 (d, *J* = 8.4 Hz, 2H), 7.45 - 7.41 (m, 1H), 7.33 (dd, *J* = 7.9, 1.1 Hz, 1H), 6.91 (d, *J* = 9.0 Hz, 1H), 5.08 (dd, *J* = 13.2, 5.1 Hz, 1H), 4.45 - 4.26 (m, 4H), 2.97 - 2.82 (m, 3H), 2.67 - 2.55 (m, 1H), 2.43 - 2.27 (m, 3H), 2.14 - 2.04 (m, 1H), 2.02 - 1.95 (m, 1H), 1.81 - 1.71 (m, 2H), 1.30 - 1.17 (m, 2H). ESI-MS(M+H)⁺: 572.2.

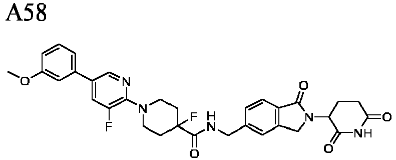

¹H NMR (400 MHz, DMSO) δ 10.96 (s, 1H), 10.24 (s, 1H), 8.51 (d, *J* = 2.5 Hz, 1H), 8.00 (s, 1H), 7.96 - 7.87 (m, 3H), 7.69 - 7.55 (m, 4H), 6.93 (d, *J* = 9.0 Hz, 1H), 5.08 (dd, *J* = 13.3, 5.1 Hz, 1H), 4.45 - 4.15 (m, 4H), 2.99 - 2.79 (m, 3H), 2.68 - 2.53 (m, 1H), 2.43 - 2.29 (m, 3H), 2.18 - 2.03 (m, 1H), 2.04 - 1.93 (m, 1H), 1.82 - 1.71 (m, 2H), 1.30 - 1.24 (m, 2H). ESI-MS(M+H)⁺: 606.2.

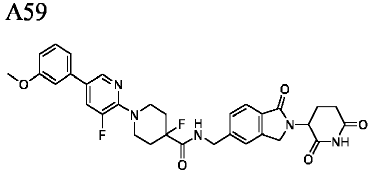

¹H NMR (400 MHz, DMSO) δ 10.96 (s, 1H), 10.25 (s, 1H), 9.02 (s, 2H), 8.62 - 8.59 (m, 1H), 8.00 (s, 1H), 7.90 - 7.80 (m, 2H), 7.65 (d, *J* = 8.2 Hz, 1H), 7.59 (dd, *J* = 8.4, 1.5 Hz, 1H), 7.30 - 7.27 (m, 1H), 5.08 (dd, *J* = 13.3, 5.2 Hz, 1H), 4.78 - 4.68 (m, 2H), 4.36 (dd, *J* = 55.9, 17.3 Hz, 2H), 2.99 (t, *J* = 11.7 Hz, 2H), 2.94 - 2.83 (m, 1H), 2.65 - 2.54 (m, 1H), 2.42 -2.30 (m, 3H), 2.21 - 2.08 (m, 1H), 2.04 - 1.96 (m, 1H), 1.84 - 1.75 (m, 2H), 1.23 - 1.12 (m, 2H). ESI-MS(M+H)⁺: 540.2.

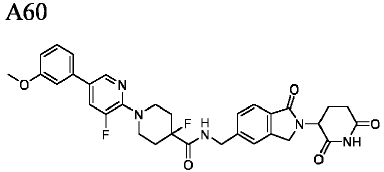

¹H NMR (400 MHz, DMSO) δ 10.96 (s, 1H), 10.25 (s, 1H), 8.84 (s, 2H), 8.57 (dd, *J* = 4.6, 1.6 Hz, 2H), 8.00 (s, 1H), 7.70 (dd, *J* = 4.6, 1.6 Hz, 2H), 7.65 (d, *J* = 8.3 Hz, 1H), 7.59 (dd, *J* = 8.3, 1.5 Hz, 1H), 5.08 (dd, *J* = 13.3, 5.1 Hz, 1H), 4.77 - 4.67 (m, 2H), 4.48 - 4.24 (m, 2H), 3.07 - 2.82 (m, 3H), 2.65 - 2.54 (m, 1H), 2.43 - 2.30 (m, 3H), 2.20 - 2.08 (m, 1H), 2.04 - 1.94 (m, 1H), 1.85 - 1.74 (m, 2H), 1.23 - 1.13 (m, 2H). ESI-MS(M+H)⁺: 540.2.

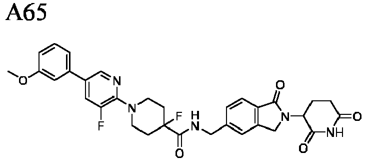

¹H NMR (400 MHz, DMSO) δ 10.92 (s, 1H), 8.73 (s, 2H), 8.49 (t, *J* = 5.9 Hz, 1H), 7.74 (t, *J* = 1.8 Hz, 1H), 7.68 (d, *J* = 7.8 Hz, 1H), 7.62 (d, *J* = 8.2 Hz, 1H), 7.51 - 7.42 (m, 2H), 7.42 - 7.33 (m, 2H), 5.10 (dd, *J* = 13.3, 5.1 Hz, 1H), 4.77 - 4.67 (m, 2H), 4.51 - 4.22 (m, 4H), 3.04 - 2.95 (m, 2H), 2.95 - 2.82 (m, 1H), 2.64 - 2.53 (m, 2H), 2.44 - 2.30 (m, 1H), 2.05 - 1.95 (m, 1H), 1.87 - 1.77 (m, 2H), 1.62 - 1.48 (m, 2H). ESI-MS(M+H)⁺: 573.2.

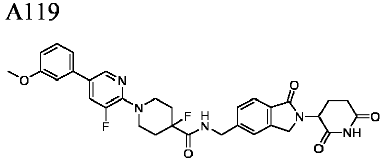

¹H NMR (400 MHz, DMSO) δ 10.98 (s, 1H), 8.94 (s, 1H), 8.39 (s, 1H), 7.92 (dd, *J* = 14.8, 1.9 Hz, 1H), 7.68 (d, *J* = 7.8 Hz, 1H), 7.44 (d, *J* = 14.0 Hz, 1H), 7.42 - 7.32 (m, 2H), 7.25 (d, *J* = 7.9 Hz, 1H), 7.23 (d, *J* = 2.1 Hz, 1H), 6.92 (dd, *J* = 8.1, 2.1 Hz, 1H), 5.10 (dd, *J* = 13.3, 5.1 Hz, 1H), 4.51 - 4.27 (m, 4H), 4.08 - 3.98 (m, 2H), 3.81 (s, 3H), 3.22 (t, *J* = 12.4, 8.7 Hz, 2H), 2.99 - 2.84 (m, 1H), 2.65 - 2.55 (m, 1H), 2.44 - 2.31 (m, 1H), 2.27 - 2.07 (m, 2H), 2.05 - 1.96 (m, 1H), 1.95 - 1.84 (m, 2H).ESI-MS(M+H)⁺: 604.3.

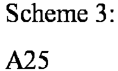

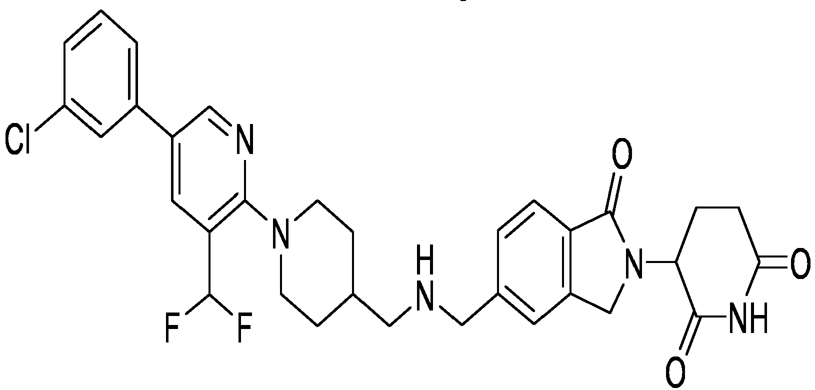

### Synthesis of compound A25-2

A25-1 (6.0 g, 36.6 mmol), A13-1 (8.5 g, 43.9 mmol) and potassium carbonate (15.2 g, 109.7 mmol) were dissolved in 60 mL of DMSO. The mixture was reacted with stirring overnight at 120 °C, and then TLC showed that the reaction was completed. The reaction liquid was diluted with ethyl acetate (250 mL), and the organic phase was washed with saturated saline solution (40 ml*3). Then the organic phase was dried by rotary evaporation. The solute was separated and purified by column chromatography (petroleum ether: ethyl acetate = 4 : 1) to obtain compound A25-2 (3.8 g, yield 43%).

### Synthesis of compound A25-3

A25-2 (3.8 g, 15.8 mmol) was dissolved in 35 mL of acetonitrile, and NBS (3.4 g, 18.9 mmol) was added. The mixture was reacted at ambient temperature for 10 min, and another NBS (840 mg, 4.7 mmol) was added. The mixture was reacted at ambient temperature for another 10 min, and then TLC showed that the reaction was completed. The reaction liquid was diluted with water (60 mL). The mixture was extracted with ethyl acetate (60 ml*3). The organic phases were combined, dried by rotary evaporation, and the solute was separated and purified by column chromatography (petroleum ether: ethyl acetate = 10 : 1) to obtain compound A25-3 (3.2 g, yield 63%).

### Synthesis of compound A25-4

A25-3 (3.2 g, 8.0 mmol), A12-2 (1.38 g, 8.0 mmol) and potassium carbonate (2.2 g, 16.1 mmol) were dissolved in dioxane/water (30 mL/10 mL), and Pd(PPh₃)₄ (930 mg, 0.80 mmol) was added. The mixture was replaced with nitrogen three times and then reacted at 100 °C overnight, and then TLC showed that the reaction was completed. The reaction liquid was filtered, the filtrate was diluted with ethyl acetate (200 mL). The organic phase was washed with saturated saline solution (30 ml*3), and the solution was dried by rotary evaporation to obtain compound A25-4 (5.6 g, crude product).

### Synthesis of compound A25-5

A25-4 (5.6 g, 8.0 mmol) was dissolved in methanol/water (20 mL/6 mL), and LiOH (660 mg, 16.1 mmol) was added. The mixture was reacted at 60 °C for 1 h, and then TLC showed that the reaction was completed. The methanol was spun off, and 1 N hydrochloric acid aqueous solution was used to adjust pH = 4. The aqueous phase was extracted with ethyl acetate (60 ml*3). The organic phases were combined, dried by rotary evaporation, and the solute was separated and purified by column chromatography (dichloromethane : methanol = 50 : 1) to obtain compound A25-5 (1.4 g, yield 41%).

### Synthesis of compound A25-6

A25-5 (1.4 g, 3.3 mmol) was dissolved in 25 mL of methanol, sodium acetate (530 mg, 6.5 mmol) and Pd/C (200 mg, 5% wt) were added. The mixture was reacted at ambient temperature under hydrogen atmosphere for two days, and then TLC showed that the reaction was completed. The reaction liquid was filtered and then dried by rotary evaporation. The solute was separated and purified by column chromatography (petroleum ether: ethyl acetate = 1: 1) to obtain compound A25-6 (460 mg, yield 40%).

### Synthesis of compound A25

A25-6 (460 mg, 1.3 mmol), A13-4 (340 mg, 1.3 mmol) and triethylamine (530 mg, 5.2 mmol) were dissolved in 15 mL of DMF, HATU (740 mg, 1.96 mmol) was added. The mixture was reacted overnight at 50 °C, and then TLC showed that the reaction was completed. The reaction liquid was diluted with ethyl acetate (200 mL), and then the organic phase was washed with saturated saline solution (20 ml*3), and the solution was dried by rotary evaporation. The solute was separated and purified by a preparative TLC (dichloromethane : methanol = 40 : 1) to obtain compound A25 (148 mg, yield 19%).

¹H NMR (400 MHz, DMSO) δ 10.95 (s, 1H), 10.27 (s, 1H), 9.01 - 8.94 (m, 1H), 8.03 - 7.91 (m, 3H), 7.74 (d, *J* = 6.3 Hz, 1H), 7.66 (d, *J* = 8.3 Hz, 1H), 7.63 - 7.51 (m, 4H), 7.22 (s, 1H), 5.08 (dd, *J* = 13.2, 5.1 Hz, 1H), 4.36 (dd, *J* = 55.2, 17.4 Hz, 2H), 3.60 - 3.51 (m, 2H), 3.03 - 2.83 (m, 3H), 2.64 - 2.53 (m, 1H), 2.43 - 2.30 (m, 3H), 2.06 - 1.98 (m, 2H), 1.87 - 1.78 (m, 2H), 1.53 - 1.37 (m, 2H). ESI-MS(M+H)⁺: 594.2.

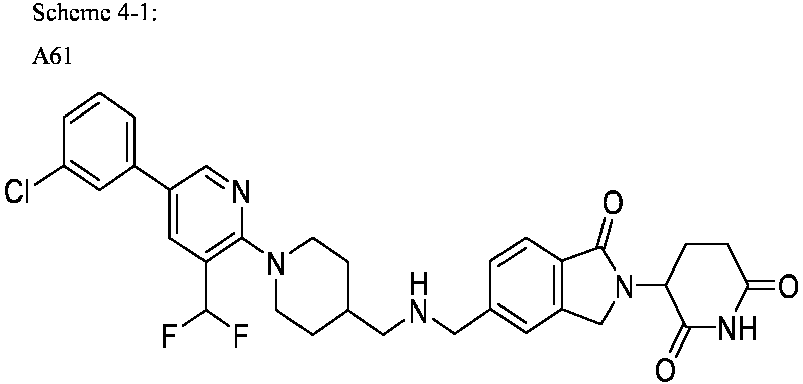

### Synthesis of compound A61-3

A61-1 (600 mg, 2.5 mmol), A61-2 (430 mg, 3.7 mmol) and triethylamine (250 mg, 2.5 mmol) were dissolved in 20 mL of ethanol. The mixture was reacted with stirring under microwave irradiation at 120 °C for 2 h, and then TLC showed that the reaction was completed. The reaction liquid was diluted with water (60 mL), and then the aqueous phase was extracted with ethyl acetate (60 ml*3). The organic phases were combined, and dried by rotary evaporation. The solute was separated and purified by column chromatography (petroleum ether: ethyl acetate = 4 : 1) to obtain compound A61-3 (430 mg, yield 63%).

### Synthesis of compound A61-4

A61-3 (430 mg, 1.6 mmol), A12-2 (320 mg, 1.9 mmol) and potassium carbonate (430 mg, 3.1 mmol) were dissolved in dioxane/water (12 mL/4 mL), and Pd(PPh₃)₄ (180 mg, 0.15 mmol) was added. The mixture was subjected to nitrogen replacement replaced with nitrogen three times and reacted at 100 °C for 4 h, and then TLC showed that the reaction was completed. The reaction liquid was filtered, and the filtrate was diluted with ethyl acetate (60 mL). The organic phase was washed with saturated saline solution (10 ml*3), and the solution was dried by rotary evaporation. The solute was separated and purified by column chromatography (petroleum ether: ethyl acetate = 2 : 1) to obtain compound A61-4 (380 mg, yield 75%).

### Synthesis of compound A61-5

A61-4 (150 mg, 0.46 mmol) was dissolved in 15 mL of dichloromethane, and Dess-Martin reagent (300 mg, 0.70 mmol) was added. The mixture was reacted at ambient temperature for 1 h, and then TLC showed that the reaction was completed. Saturated sodium bicarbonate aqueous solution (60 mL) was added to quench the reaction, and then the aqueous phase was extracted with ethyl acetate (60 ml*3). The organic phases were combined and dried by rotary evaporation. The solute was separated and purified by the preparative TLC (petroleum ether: ethyl acetate = 1 : 1) to obtain compound A61-5 (100 mg, yield 65%).

### Synthesis of compound A61

A61-5 (100 mg, 0.30 mmol) and compound A61-6 (100 mg, 0.30 mmol) were dissolved in methanol/dichloromethane (9 mL/4 mL), and 1 drop of glacial acetic acid was added. The mixture was reacted at ambient temperature for 1 h, and then sodium cyanoborohydride (40 mg, 0.60 mmol) was added. The reaction was carried out at ambient temperature overnight. Saturated sodium bicarbonate solution (20 mL) was added to quench the reaction, and then the aqueous phase was extracted with dichloromethane (30 ml*3). The organic phases were combined and then dried by rotary evaporation. The solute was separated and purified by a preparative TLC (dichloromethane : methanol = 10 : 1) and then subjected to medium pressure preparation to obtain compound A61 (20 mg, yield 14%).

¹H NMR (400 MHz, DMSO) δ 10.98 (s, 1H), 8.25 - 8.15 (m, 1H), 8.00 - 7.96 (m, 1H), 7.95 - 7.88 (m, 1H), 7.71 (d, *J* = 7.8 Hz, 1H), 7.63 (s, 1H), 7.61 - 7.49 (m, 5H), 7.32 (s, 1H), 5.11 (dd, *J* = 13.3, 5.1 Hz, 1H), 4.39 (dd, *J* = 53.2, 17.3 Hz, 2H), 4.01 - 3.90 (m, 4H), 3.07 (t, *J* = 11.4 Hz, 2H), 2.99 - 2.84 (m, 1H), 2.66 - 2.52 (m, 3H), 2.46 - 2.32 (m, 1H), 2.05 - 1.96 (m, 1H), 1.92 - 1.73 (m, 3H), 1.36 - 1.25 (m, 2H). ESI-MS(M+H)⁺: 580.2.

The preparation methods of compounds A62, A66, A67, A68, A69, A70, A72, A73, A74, A75, A76, A77, A78, A79, A80, A81, A82, A83, A84, A85, A86, A87, A88, A89, A90, A91, A92, A93, A94, A95, A96, A97, A98, A99, A100, A103, A105, A106, A107, A108, A109, A110, A111, A112, A113, A114, A115, A118, A120-129, A131, A132, A138-A164, A167-A173, A176, and A177 refer to A61, and the characterization results are as follows:

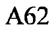

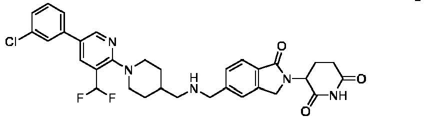

¹H NMR (400 MHz, DMSO) δ 10.99 (s, 1H), 8.49 (s, 2H), 8.05 - 7.99 (m, 1H), 7.96 (d, *J* = 8.3 Hz, 1H), 7.83 (d, *J* = 7.9 Hz, 1H), 7.74 (d, *J* = 7.8 Hz, 1H), 7.69 (s, 1H), 7.62 - 7.49 (m, 4H), 7.45 (dd, *J* = 7.0, 1.1 Hz, 1H), 5.12 (dd, *J* = 13.3, 5.1 Hz, 1H), 4.80 - 4.71 (m, 2H), 4.41 (dd, *J* = 53.4, 17.4 Hz, 2H), 4.04 (s, 2H), 3.03 - 2.85 (m, 3H), 2.69 - 2.53 (m, 3H), 2.46 - 2.33 (m, 1H), 2.07 - 1.91 (m, 2H), 1.90 - 1.81 (m, 2H), 1.21 - 1.08 (m, 2H). ESI-MS(M+H)⁺: 575.2.

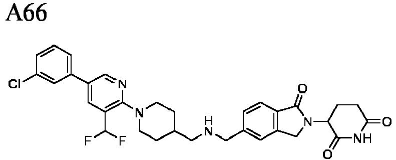

¹H NMR (400 MHz, DMSO) δ 10.99 (s, 1H), 8.73 (s, 2H), 7.79 (d, *J* = 8.1 Hz, 2H), 7.75 - 7.67 (m, 2H), 7.63 - 7.60 (m, 1H), 7.49 - 7.43 (m, 1H), 7.40 - 7.35 (m, 1H), 5.13 (dd, *J* = 13.3, 5.1 Hz, 1H), 4.75 - 4.66 (m, 2H), 4.43 (dd, *J* = 53.8, 17.6 Hz, 2H), 4.25 (s, 2H), 3.00 - 2.86 (m, 3H), 2.81 (s, 2H), 2.67 - 2.55 (m, 1H), 2.47 - 2.36 (m, 1H), 2.15 - 1.98 (m, 2H), 1.91 - 1.81 (m, 2H), 1.21 - 1.01 (m, 2H). ESI-MS(M+H)⁺: 559.2.

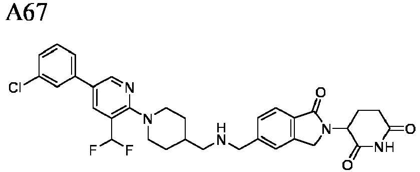

¹H NMR (400 MHz, DMSO) δ 10.96 (s, 1H), 8.71 (s, 2H), 7.66 (d, *J* = 7.8 Hz, 1H), 7.56 (s, 1H), 7.54 - 7.43 (m, 4H), 7.16 -7.08 (m, 1H), 5.10 (dd, *J* = 13.3, 5.1 Hz, 1H), 4.75 - 4.65 (m, 2H), 4.37 (dd, *J* = 53.7, 17.2 Hz, 2H), 3.81 (s, 2H), 2.96 - 2.86 (m, 3H), 2.65 - 2.55 (m, 1H), 2.45 -2.31 (m, 3H), 2.05 - 1.93 (m, 1H), 1.86 - 1.77 (m, 2H), 1.77 - 1.69 (m, 1H), 1.15 - 1.02 (m, 2H). ESI-MS(M+H)⁺: 543.2.

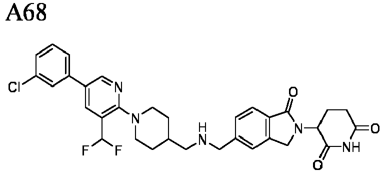

¹H NMR (400 MHz, DMSO) δ 10.96 (s, 1H), 8.64 (s, 2H), 7.66 (d, *J* = 7.8 Hz, 1H), 7.57 (s, 1H), 7.48 (d, *J* = 7.9 Hz, 1H), 7.44 (s, 1H), 7.40 (d, *J* = 8.1 Hz, 1H), 7.34 - 7.29 (m, 1H), 7.14 (d, *J* = 7.4 Hz, 1H), 5.10 (dd, *J* = 13.3, 5.1 Hz, 1H), 4.73 - 4.65 (m, 2H), 4.37 (dd, *J* = 53.9, 17.2 Hz, 2H), 3.81 (s, 2H), 2.96 - 2.84 (m, 3H), 2.65 - 2.52 (m, 1H), 2.43 - 2.28 (m, 6H), 2.05 - 1.91 (m, 1H), 1.85 - 1.77 (m, 2H), 1.75 - 1.69 (m, 1H), 1.16 - 0.98 (m, 2H). ESI-MS(M+H)⁺: 539.2.

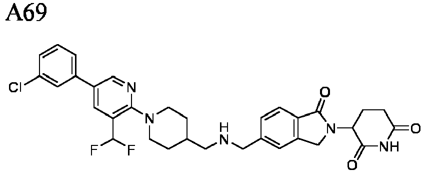

¹H NMR (400 MHz, DMSO) δ 10.99 (s, 1H), 8.78 (s, 2H), 8.16 (s, 1H), 8.00 (d, *J* = 8.0 Hz, 1H), 7.86 - 7.71 (m, 3H), 7.67 - 7.62 (m, 2H), 5.13 (dd, *J* = 13.2, 5.1 Hz, 1H), 4.77 - 4.62 (m, 2H), 4.44 (dd, *J* = 52.7, 17.5 Hz, 2H), 4.27 (s, 2H), 3.04 - 2.75 (m, 5H), 2.67 - 2.56 (m, 1H), 2.45 - 2.32 (m, 1H), 2.08 - 1.97 (m, 2H), 1.90 - 1.79 (m, 2H), 1.22 - 1.12 (m, 2H). ESI-MS(M+H)⁺: 550.2.

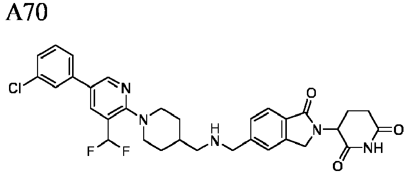

¹H NMR (400 MHz, DMSO) δ 10.98 (s, 1H), 8.45 (d, *J* = 2.5 Hz, 1H), 7.85 (dd, *J* = 9.0, 2.6 Hz, 1H), 7.71 (d, *J* = 7.8 Hz, 1H), 7.67 - 7.64 (m, 2H), 7.59 - 7.54 (m, 2H), 7.45 - 7.41 (m, 1H), 7.33 (dd, *J* = 8.0, 1.1 Hz, 1H), 6.90 (d, *J* = 9.0 Hz, 1H), 5.11 (dd, *J* = 13.3, 5.1 Hz, 1H), 4.53 - 4.25 (m, 4H), 3.97 (s, 2H), 2.98 - 2.79 (m, 3H), 2.65 - 2.52 (m, 3H), 2.45 - 2.32 (m, 1H), 2.05 - 1.95 (m, 1H), 1.87 - 1.76 (m, 3H), 1.21 - 1.06 (m, 2H). ESI-MS(M+H)⁺: 558.2.

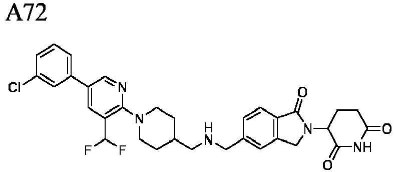

¹H NMR (400 MHz, DMSO) δ 11.00 (s, 1H), 8.07 - 8.06 (m, 1H), 7.80 (d, *J* = 7.8 Hz, 1H), 7.77 (s, 1H), 7.70 - 7.61 (m, 2H), 7.59 - 7.54 (m, 1H), 7.48 - 7.37 (m, 3H), 5.13 (dd, *J* = 13.2, 5.1 Hz, 1H), 4.44 (dd, *J* = 52.9, 17.6 Hz, 2H), 4.26 (s, 2H), 4.14 - 4.03 (m, 2H), 2.99 - 2.82 (m, 5H), 2.67 - 2.56 (m, 1H), 2.47 - 2.36 (m, 1H), 2.07 - 1.94 (m, 2H), 1.91 - 1.80 (m, 2H), 1.41 - 1.26 (m, 2H). ESI-MS(M+H)⁺: 576.2.

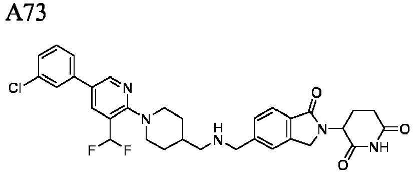

¹H NMR (400 MHz, DMSO) δ 10.98 (s, 1H), 8.38 (t, *J* = 1.7 Hz, 1H), 7.91 (dd, *J* = 15.0, 2.0 Hz, 1H), 7.76 (t, *J* = 1.8 Hz, 1H), 7.70 (d, *J* = 7.8 Hz, 1H), 7.65 (dd, *J* = 7.3, 5.8 Hz, 1H), 7.62 (s, 1H), 7.53 (d, *J* = 7.7 Hz, 1H), 7.46 (t, *J* = 7.9 Hz, 1H), 7.40 - 7.37 (m, 1H), 5.11 (dd, *J* = 13.3, 5.1 Hz, 1H), 4.39 (dd, *J* = 53.5, 17.3 Hz, 2H), 4.13 - 4.03 (m, 2H), 3.94 (s, 2H), 2.98 - 2.82 (m, 3H), 2.69 - 2.53 (m, 3H), 2.44 -2.32 (m, 1H), 2.05 - 1.95 (m, 1H), 1.87 - 1.71 (m, 3H), 1.33 - 1.24 (m, 2H). ESI-MS(M+H)⁺: 576.2.

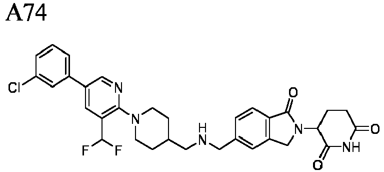

¹H NMR (400 MHz, DMSO) δ 10.99 (s, 1H), 8.36 (d, *J* = 1.7 Hz, 1H), 7.88 (dd, *J* = 14.9, 2.0 Hz, 1H), 7.83 - 7.76 (m, 2H), 7.74 - 7.66 (m, 3H), 7.49 (d, *J* = 8.6 Hz, 2H), 5.13 (dd, *J* = 13.3, 5.1 Hz, 1H), 4.43 (dd, *J* = 52.9, 17.6 Hz, 2H), 4.27 (s, 2H), 4.10 - 4.03 (m, 2H), 3.00 - 2.78 (m, 5H), 2.68 - 2.56 (m, 1H), 2.45 - 2.36 (m, 1H), 2.07 - 1.93 (m, 2H), 1.90 - 1.80 (m, 2H), 1.40 - 1.26 (m, 2H). ESI-MS(M+H)⁺: 576.2.

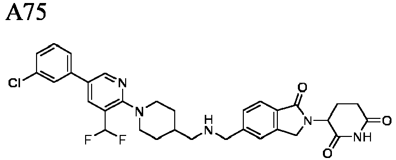

¹H NMR (400 MHz, DMSO) δ 10.98 (s, 1H), 8.54 (d, *J* = 2.2 Hz, 1H), 8.12 (d, *J* = 2.2 Hz, 1H), 7.78 - 7.77 (m, 1H), 7.67 - 7.65 (m, 2H), 7.57 (s, 1H), 7.49 - 7.44 (m, 2H), 7.41 (dd, *J* = 4.9, 3.8 Hz, 1H), 5.10 (dd, *J* = 13.3, 5.1 Hz, 1H), 4.37 (dd, *J* = 52.3, 17.3 Hz, 2H), 3.82 - 3.79 (m, 4H), 2.97 - 2.85 (m, 1H), 2.80 (t, *J* = 11.6 Hz, 2H), 2.66 - 2.65 (m, 1H), 2.46 - 2.32 (m, 3H), 2.06 - 1.93 (m, 1H), 1.89 - 1.78 (m, 2H), 1.71 - 1.56 (m, 1H), 1.35 - 1.24 (m, 2H). ESI-MS(M+H)⁺: 592.2.

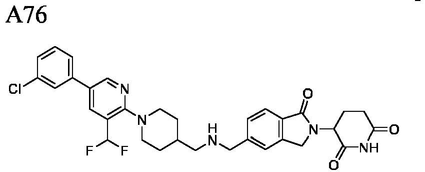

¹H NMR (400 MHz, DMSO) δ 10.99 (s, 1H), 8.42 (d, *J* = 2.3 Hz, 1H), 7.84 (d, *J* = 2.0 Hz, 1H), 7.77 - 7.70 (m, 3H), 7.63 (t, *J* = 8.1 Hz, 2H), 7.46 (t, *J* = 7.9 Hz, 1H), 7.40 - 7.39 (m, 1H), 5.13 (dd, *J* = 13.3, 5.1 Hz, 1H), 4.41 (dd, *J* = 53.2, 17.4 Hz, 2H), 4.10 (s, 2H), 3.52 - 3.43 (m, 2H), 2.96 - 2.87 (m, 1H), 2.79 - 2.67 (m, 4H), 2.63 - 2.55 (m, 1H), 2.47 - 2.34 (m, 1H), 2.28 (s, 3H), 2.06 - 1.96 (m, 1H), 1.92 - 1.75 (m, 3H), 1.40 - 1.26 (m, 2H). ESI-MS(M+H)⁺: 572.2.

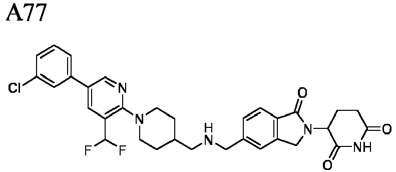

¹H NMR (400 MHz, DMSO) δ 10.98 (s, 1H), 8.11 (d, *J* = 2.0 Hz, 1H), 7.77 - 7.75 (m, 1H), 7.72 (d, *J* = 7.8 Hz, 1H), 7.65 (d, *J* = 8.2 Hz, 1H), 7.63 (s, 1H), 7.54 (d, *J* = 7.8 Hz, 1H), 7.49 - 7.43 (m, 2H), 7.40 - 7.34 (m, 1H), 5.11 (dd, *J* = 13.3, 5.1 Hz, 1H), 4.40 (dd, *J* = 52.6, 17.4 Hz, 2H), 4.08 - 3.94 (m, 4H), 3.89 (s, 3H), 2.96 - 2.83 (m, 1H), 2.73 (t, *J* = 11.6 Hz, 2H), 2.66 - 2.54 (m, 3H), 2.47 - 2.34 (m, 1H), 2.05 - 1.94 (m, 1H), 1.85 - 1.75 (m, 2H), 1.74 - 1.66 (m, 1H), 1.37 - 1.24 (m, 2H). ESI-MS(M+H)⁺: 588.2.

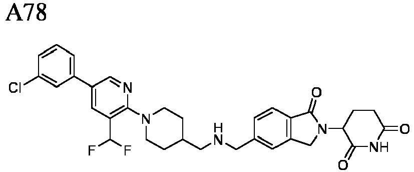

¹H NMR (400 MHz, DMSO) δ 10.98 (s, 1H), 8.85 (d, *J* = 2.0 Hz, 1H), 8.30 (d, *J* = 2.2 Hz, 1H), 7.70 - 7.56 (m, 4H), 7.53 - 7.48 (m, 2H), 7.26 - 7.17 (m, 1H), 5.11 (dd, *J* = 13.3, 5.1 Hz, 1H), 4.38 (dd, *J* = 51.7, 17.2 Hz, 2H), 3.87 (s, 2H), 3.53 - 3.42 (m, 2H), 3.20 (t, *J* = 11.2 Hz, 2H), 2.98 - 2.86 (m, 1H), 2.70 (d, *J* = 20.4 Hz, 2H), 2.62 - 2.57 (m, 1H), 2.44 - 2.33 (m, 2H), 2.05 - 1.74 (m, 5H). ESI-MS(M+H)⁺: 603.2.

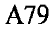

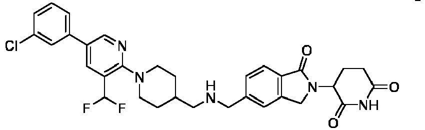

¹H NMR (400 MHz, DMSO) δ 10.99 (s, 1H), 8.12 (d, *J* = 2.0 Hz, 1H), 7.71 (s, 1H), 7.67 (d, *J* = 7.8 Hz, 1H), 7.61 (d, *J* = 7.8 Hz, 1H), 7.57 (s, 1H), 7.51 - 7.42 (m, 2H), 7.37 (d, *J* = 8.0 Hz, 1H), 7.32 (d, *J* = 1.9 Hz, 1H), 5.11 (dd, *J* = 13.3, 5.1 Hz, 1H), 4.38 (dd, *J* = 53.8, 17.3 Hz, 2H), 4.17 - 4.05 (m, 2H), 3.82 (s, 2H), 2.98 - 2.86 (m, 1H), 2.78 (s, 6H), 2.64 - 2.54 (m, 3H), 2.45 - 2.32 (m, 3H), 2.04 - 1.95 (m, 1H), 1.89 - 1.78 (m, 2H), 1.66 - 1.53 (m, 1H), 1.34 - 1.22 (m, 2H). ESI-MS(M+H)⁺: 601.2.

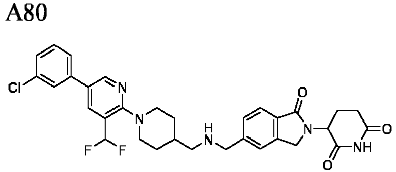

¹H NMR (400 MHz, DMSO) δ 10.98 (s, 1H), 8.80 (d, *J* = 2.1 Hz, 1H), 8.27 (d, *J* = 2.3 Hz, 1H), 7.83 (s, 1H), 7.71 - 7.65 (m, 2H), 7.56 (s, 1H), 7.53 - 7.45 (m, 2H), 7.43 (d, *J* = 8.5 Hz, 1H), 5.11 (dd, *J* = 13.3, 5.1 Hz, 1H), 4.37 (dd, *J* = 51.0, 17.2 Hz, 2H), 3.81 (s, 2H), 3.69 - 3.56 (m, 2H), 2.98 - 2.84 (m, 3H), 2.66 - 2.54 (m, 1H), 2.46 - 2.29 (m, 3H), 2.06 - 1.95 (m, 1H), 1.88 - 1.76 (m, 2H), 1.71 - 1.56 (m, 1H), 1.35 - 1.18 (m, 2H). ESI-MS(M+H)⁺: 626.2.

A80 was subjected to separation by chiral preparation to obtain A81 and A82. The separation conditions are:
Chromatographic column: Superchiral R-IC (Chiralway), 0.46 cm I.D. * 15 cm Length, 5 um; column temperature: 35 °C; Mobile phase: MeOH /ACN/DEA = 50/50/0.05 (v/v/v); UV detection wavelength: 220 nm; flow rate: 0.75 ml/min 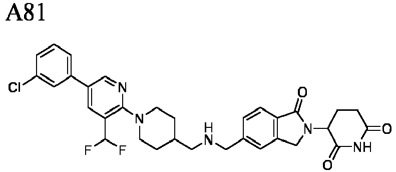 ee value>98%

¹H NMR (400 MHz, DMSO) δ 10.98 (s, 1H), 8.80 (d, *J* = 2.1 Hz, 1H), 8.27 (d, *J* = 2.3 Hz, 1H), 7.83 (s, 1H), 7.71 - 7.65 (m, 2H), 7.56 (s, 1H), 7.53 - 7.45 (m, 2H), 7.43 (d, *J* = 8.5 Hz, 1H), 5.11 (dd, *J* = 13.3, 5.1 Hz, 1H), 4.37 (dd, *J* = 51.0, 17.2 Hz, 2H), 3.81 (s, 2H), 3.69 - 3.56 (m, 2H), 2.98 - 2.84 (m, 3H), 2.66 - 2.54 (m, 1H), 2.46 - 2.29 (m, 3H), 2.06 - 1.95 (m, 1H), 1.88 - 1.76 (m, 2H), 1.71 - 1.56 (m, 1H), 1.35 - 1.18 (m, 2H). 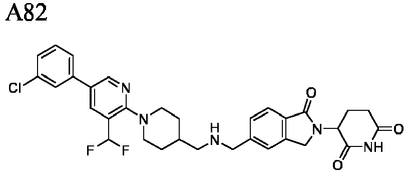 ee value>98%

¹H NMR (400 MHz, DMSO) δ 10.98 (s, 1H), 8.80 (d, *J* = 2.1 Hz, 1H), 8.27 (d, *J* = 2.3 Hz, 1H), 7.83 (s, 1H), 7.71 - 7.65 (m, 2H), 7.56 (s, 1H), 7.53 - 7.45 (m, 2H), 7.43 (d, *J* = 8.5 Hz, 1H), 5.11 (dd, *J* = 13.3, 5.1 Hz, 1H), 4.37 (dd, *J* = 51.0, 17.2 Hz, 2H), 3.81 (s, 2H), 3.69 - 3.56 (m, 2H), 2.98 - 2.84 (m, 3H), 2.66 - 2.54 (m, 1H), 2.46 - 2.29 (m, 3H), 2.06 - 1.95 (m, 1H), 1.88 - 1.76 (m, 2H), 1.71 - 1.56 (m, 1H), 1.35 - 1.18 (m, 2H).

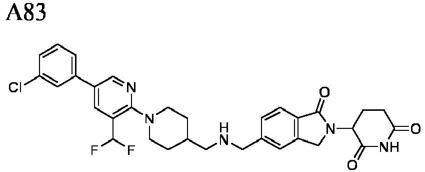

¹H NMR (400 MHz, DMSO) δ 10.97 (s, 1H), 8.84 (d, *J* = 2.3 Hz, 1H), 8.31 (d, *J* = 2.4 Hz, 1H), 7.86 (t, *J* = 1.8 Hz, 1H), 7.76 - 7.70 (m, 1H), 7.67 (d, *J* = 7.8 Hz, 1H), 7.58 (s, 1H), 7.53 - 7.41 (m, 3H), 5.10 (dd, *J* = 13.3, 5.1 Hz, 1H), 4.38 (dd, *J* = 53.1, 17.3 Hz, 2H), 3.87 (s, 2H), 3.53 - 3.43 (m, 2H), 3.25 - 3.13 (m, 2H), 2.97 - 2.86 (m, 1H), 2.70 (d, *J* = 20.4 Hz, 2H), 2.63 - 2.55 (m, 1H), 2.45 - 2.34 (m, 1H), 2.05 - 1.86 (m, 4H), 1.86 - 1.75 (m, 1H). ESI-MS(M+H)⁺: 644.2.

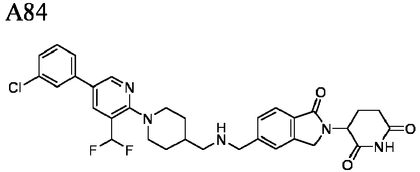

¹H NMR (400 MHz, DMSO) δ 10.98 (s, 1H), 8.40 (s, 1H), 7.93 (dd, *J* = 14.9, 1.9 Hz, 1H), 7.77 (s, 1H), 7.68 - 7.65 (m, 2H), 7.56 (s, 1H), 7.51 - 7.42 (m, 2H), 7.39 (dd, *J* = 8.0, 0.8 Hz, 1H), 5.10 (dd, *J* = 13.3, 5.1 Hz, 1H), 4.37 (dd, *J* = 52.7, 17.3 Hz, 2H), 3.97 - 3.81 (m, 4H), 3.22 (t, *J* = 11.0 Hz, 2H), 2.97 - 2.84 (m, 1H), 2.68 (d, *J=* 20.2 Hz, 2H), 2.65 - 2.54 (m, 1H), 2.46 - 2.32 (m, 1H), 2.04 - 1.72 (m, 5H). ESI-MS(M+H)⁺: 594.2.

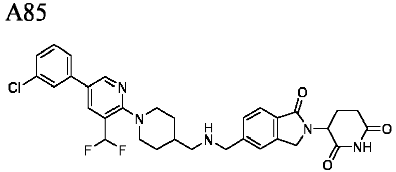

¹H NMR (400 MHz, DMSO) δ 10.99 (s, 1H), 8.83 (d, *J* = 2.2 Hz, 1H), 8.25 (d, *J* = 2.4 Hz, 1H), 7.69 (d, *J* = 7.8 Hz, 1H), 7.59 (d, *J* = 6.5 Hz, 2H), 7.52 (dd, *J* = 12.1, 7.9 Hz, 2H), 7.38 (t, *J* = 7.6 Hz, 1H), 7.23 (d, *J* = 7.5 Hz, 1H), 5.11 (dd, *J* = 13.3, 5.1 Hz, 1H), 4.39 (dd, *J* = 54.2, 17.3 Hz, 2H), 3.89 (s, 2H), 3.48 - 3.39 (m, 2H), 3.19 (t, *J* = 11.0 Hz, 2H), 2.98 - 2.84 (m, 1H), 2.79 - 2.66 (m, 2H), 2.65 - 2.55 (m, 1H), 2.42 - 2.31 (m, 4H), 2.05 - 1.88 (m, 4H), 1.87 - 1.74 (m, 1H). ESI-MS(M+H)⁺: 624.2.

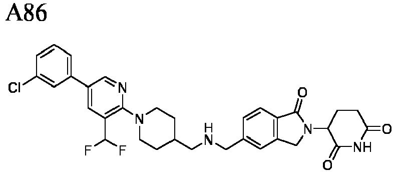

¹H NMR (400 MHz, DMSO) δ 10.98 (s, 1H), 8.75 (d, *J* = 2.1 Hz, 1H), 8.50 (d, *J* = 2.1 Hz, 1H), 7.83 (s, 1H), 7.68 (dd, *J* = 13.4, 7.7 Hz, 2H), 7.57 (s, 1H), 7.51 - 7.44 (m, 2H), 7.42 (d, *J* = 8.0 Hz, 1H), 5.10 (dd, *J* = 13.3, 5.0 Hz, 1H), 4.37 (dd, *J* = 54.3, 17.2 Hz, 2H), 3.85 - 3.73 (m, 4H), 3.03 (t, *J* = 12.0 Hz, 2H), 2.97 - 2.82 (m, 1H), 2.66 - 2.54 (m, 1H), 2.46 - 2.31 (m, 3H), 2.06 - 1.93 (m, 1H), 1.88 - 1.78 (m, 2H), 1.77 - 1.68 (m, 1H), 1.26 - 1.13 (m, 2H). ESI-MS(M+H)⁺: 628.2.

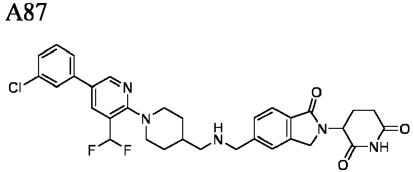

¹H NMR (400 MHz, DMSO) δ 10.97 (s, 1H), 8.45 - 8.31 (m, 1H), 7.99 - 7.84 (m, 1H), 7.76 (dt, *J* = 15.1, 1.8 Hz, 1H), 7.66 (dt, *J* = 9.5, 4.5 Hz, 2H), 7.61 - 7.54 (m, 1H), 7.53 - 7.34 (m, 3H), 5.09 (ddd, *J* = 17.4, 11.2, 5.0 Hz, 1H), 4.81 - 4.11 (m, 5H), 3.97 - 3.49 (m, 4H), 3.08 (ddd, *J* = 21.9, 13.1, 8.9 Hz, 1H), 2.97 - 2.86 (m, 1H), 2.81 - 2.54 (m, 3H), 2.45 - 2.28 (m, 1H), 2.20 - 1.32 (m, 3H). ESI-MS(M+H)⁺: 594.2.

The corresponding intermediates were prepared according to the following conditions:

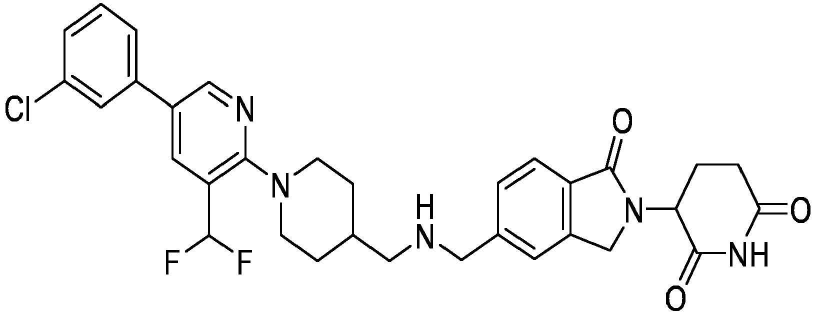

### Synthesis of compound A87-3

A87-1 (1.94 g, 10.0 mmol), A87-2 (1.71 g, 10.0 mmol) and potassium carbonate (4.14 g, 30.0 mmol) were dissolved in 20 mL of DMF. The mixture was reacted at 80 °C for 3 h, and then TLC showed that the reaction was completed. The reaction liquid was diluted with water (100 ml), and then the aqueous phase was extracted with ethyl acetate (50 mL*3). The organic phases were combined and then washed with saturated saline solution (20 ml*3). The solution was dried by rotary evaporation. The solute was separated and purified by column chromatography (petroleum ether: ethyl acetate = 5 : 1) to obtain compound A87-3 (1.65 g, yield 48%).

### Synthesis of compound A87-5

A87-3 (1.65 g, 4.78 mmol), A87-4 (746 mg, 4.78 mmol) and potassium carbonate (1.98 g, 14.3 mmol) were dissolved in dioxane/water (20 mL/5 mL), and Pd(dppf)Cl₂ (162 mg, 0.14 mmol) was added. The mixture was replaced with nitrogen three times and reacted at 80 °C for 3 h, and then TLC showed that the reaction was completed. The reaction liquid was filtered, the filtrate was diluted with ethyl acetate (60 mL). The organic phase was washed with saturated saline solution (10 ml*3), and then dried by rotary evaporation. The solute was separated and purified by column chromatography (petroleum ether: ethyl acetate = 5 : 1) to obtain compound A87-5 (1.05 g, yield 58%).

### Synthesis of compound A87-6

A87-5 (1.05 g, 2.79 mmol) was dissolved in 50 mL of tetrahydrofuran. In an ice bath, sodium borohydride (106 mg, 2.79 mmol) was added. The mixture was reacted at 0 °C for 1 h, and then TLC showed that the reaction was completed. The reaction liquid was quenched by water (100 ml), and then the aqueous phase was extracted with ethyl acetate (50 mL*3). The organic phases were combined and then washed with saturated saline solution (20 ml*3) and the solution was dried by rotary evaporation. The solute was separated and purified by column chromatography (petroleum ether: ethyl acetate = 3 : 1) to obtain compound A87-6 (950 mg, yield 90%).

### Synthesis of compound A87-7

A87-6 (950 mg, 2.50 mmol) was dissolved in 30 mL of dichloromethane. DASF (404 mg, 2.50 mmol) was added in an ice bath. The mixture was reacted at 0 °C for 1 h, and then TLC showed that the reaction was completed. The reaction liquid was quenched by saturated sodium bicarbonate aqueous solution (50 ml), and then the aqueous phase was extracted with dichloromethane (50 mL*3). The organic phases were combined and then washed with saturated saline solution (20 ml*3) . The solution was dried by rotary evaporation. The solute was separated and purified by column chromatography (petroleum ether: ethyl acetate = 5 : 1) to obtain compound A87-7 (770 mg, yield 80%).

### Synthesis of compound A87-8

A87-7 (770 mg, 2.02 mmol) was dissolved in 30 mL of tetrahydrofuran. Lithium aluminium tetrahydride (77 mg, 2.02 mmol) was added in an ice bath. The mixture was reacted at 0 °C for 1 h, and then TLC showed that the reaction was completed. The reaction liquid was quenched by adding saturated sodium bicarbonate solution (50 ml), and then the aqueous phase was extracted with ethyl acetate (50 mL*3). The organic phases were combined and then washed with saturated saline solution (20 ml*3) and the solution was dried by rotary evaporation. The solute was separated and purified by column chromatography (petroleum ether: ethyl acetate = 3 : 1) to obtain compound A87-8 (550 mg, yield 80%).

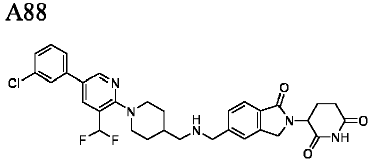

¹H NMR (400 MHz, DMSO) δ 10.99 (s, 1H), 8.41 (s, 1H), 7.99 (dd, *J* = 14.9, 1.8 Hz, 1H), 7.79 (s, 1H), 7.73 - 7.64 (m, 2H), 7.61 (s, 1H), 7.52 (d, *J* = 7.8 Hz, 1H), 7.49 - 7.45 (m, 1H), 7.43 - 7.38 (m, 1H), 5.11 (dd, *J* = 13.3, 5.0 Hz, 1H), 4.39 (dd, *J=* 55.2, 17.4 Hz, 2H), 4.25 - 4.09 (m, 2H), 3.92 (s, 2H), 3.11 - 3.00 (m, 1H), 2.99 - 2.85 (m, 2H), 2.69 - 2.55 (m, 3H), 2.45 - 2.25 (m, 2H), 2.19 - 2.10 (m, 1H), 2.05 - 1.96 (m, 1H), 1.65 - 1.54 (m, 1H). ESI-MS(M+H)⁺: 612.2.

The corresponding intermediates were prepared according to the following conditions:

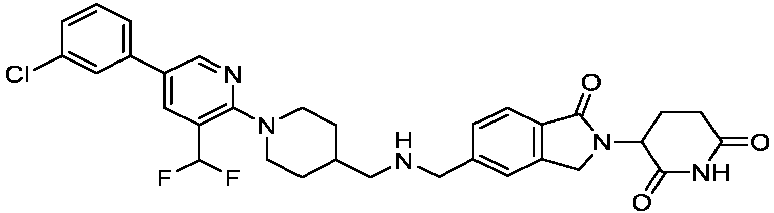

### Synthesis of compound A88-1

A87-5 (942 mg, 2.5 mmol) was dissolved in methanol/tetrahydrofuran (20 mL/20 mL), and sodium borohydride (570 mg, 15.0 mmol) was added in portions at 0 °C. The mixture was reacted overnight at ambient temperature, and then TLC showed that the reaction was completed. Water (50 mL) was added to the reaction liquid to quench the reaction. The aqueous phase was extracted with ethyl acetate (50 ml*3). The organic phases were combined and washed with saturated saline solution (20 ml*3), and then the organic phase was dried by rotary evaporation. The solute was separated and purified by column chromatography (pure ethyl acetate) to obtain compound A88-1 (680 mg, yield 81%).

### Synthesis of compound A88-2

A88-1 (674 mg, 2.0 mmol) and triethylamine (606 mg, 6.0 mmol) were dissolved in 20 mL of dichloromethane, and acetyl chloride (174 mg, 2.2 mmol) was added at 0 °C. The mixture was reacted at 0 °C for 30 min, and then TLC showed that the reaction was completed. Water (50 mL) was added to the reaction liquid to quench the reaction. The aqueous phase was extracted with ethyl acetate (50 ml*3). The organic phases were combined and washed with saturated saline solution (20 ml*3). Then the organic phase was dried by rotary evaporation. The solute was separated and purified by column chromatography (petroleum ether: ethyl acetate = 3 : 1) to obtain compound A88-2 (440 mg, yield 58%).

### Synthesis of compound A88-3

A88-2 (189 mg, 0.5 mmol) was dissolved in 10 mL of dichloromethane, and Dess-Martin reagent (424 mg, 1.0 mmol) was added. The mixture was reacted at ambient temperature for 1 h, and then TLC showed that the reaction was completed. The reaction liquid was diluted by dichloromethane (30 mL). The organic phase was washed with saturated sodium bicarbonate solution (20 ml*3) and then dried by rotary evaporation to obtain compound A88-3 (188 mg, crude product), which was directly used for next step.

### Synthesis of compound A88-4

A88-3 (188 mg, 0.5 mmol) was dissolved in 10 mL of dichloromethane, and DAST reagent (483 mg, 3.0 mmol) was added. The mixture was reacted at ambient temperature for 1 h, and then TLC showed that the reaction was completed. Water (50 mL) was added to the reaction to quench the reaction. The aqueous phase was extracted with dichloromethane (50 ml*3). The organic phases were combined and washed with saturated saline solution (20 ml*3), and then the organic phase was dried by rotary evaporation. The solute was separated and purified by column chromatography (petroleum ether: ethyl acetate = 5 : 1) to obtain compound A88-4 (130 mg, yield 65%).

### Synthesis of compound A88-5

A88-4 (130 mg, 0.33 mmol) was dissolved in methanol/water (10 mL/3 mL), and lithium hydroxide (24 mg, 0.99 mmol) was added. The mixture was reacted at ambient temperature for 1 h, and then TLC showed that the reaction was completed. Water (50 mL) was added to the reaction to quench the reaction. The aqueous phase was extracted with ethyl acetate (50 ml*3). The organic phases were combined and washed with saturated saline solution (20 ml*3), and then the organic phase was dried by rotary evaporation. The solute was separated and purified by column chromatography (petroleum ether: ethyl acetate = 3 : 1) to obtain compound A88-5 (110 mg, yield 65%).

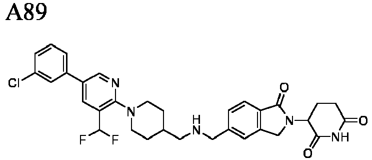

¹H NMR (400 MHz, DMSO) δ 10.98 (s, 1H), 8.90 (d, *J* = 2.2 Hz, 1H), 8.35 (d, *J* = 2.3 Hz, 1H), 7.89 (d, *J* = 1.8 Hz, 1H), 7.74 (d, *J* = 7.6 Hz, 1H), 7.68 (d, *J* = 7.8 Hz, 1H), 7.62 (s, 1H), 7.56 - 7.44 (m, 3H), 5.11 (dd, *J* = 13.3, 5.1 Hz, 1H), 4.38 (dd, *J* = 53.8, 17.3 Hz, 2H), 3.93 (s, 2H), 3.62 - 3.51 (m, 2H), 3.12 (t, *J* = 12.1 Hz, 2H), 3.00 - 2.87 (m, 1H), 2.73 (s, 2H), 2.66 - 2.53 (m, 1H), 2.46 - 2.30 (m, 1H), 2.10 - 1.94 (m, 3H), 1.72 - 1.59 (m, 2H). ESI-MS(M+H)⁺: 651.2.

The corresponding intermediates were prepared according to the following conditions:

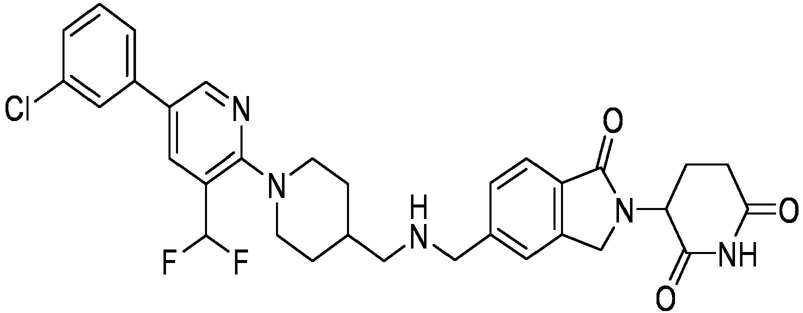

### Synthesis of compound A89-3

A89-1 (4.2 g, 16.4 mmol), A89-2 (1.98 g, 18.0 mmol) and potassium carbonate (6.8 g, 49.2 mmol) were dissolved in 40 mL of DMF. The mixture was reacted overnight at 80 °C, and then TLC showed that the reaction was completed. The reaction liquid was diluted with water (100 ml), and then the aqueous phase was extracted with ethyl acetate (50 mL*3). The organic phases were combined and then washed with saturated saline solution (20 ml*3). The solution was dried by rotary evaporation. The solute was separated and purified by column chromatography (petroleum ether: ethyl acetate = 20: 1 - 4: 1) to obtain compound A89-3 (3.4 g, yield 62%).

### Synthesis of compound A89-4

A89-3 (3.33 g, 10.0 mmol), A87-4 (1.72 g, 11.0 mmol) and potassium carbonate (3.45 g, 25.0 mmol) were dissolved in dioxane/water (30 mL/6 mL), and Pd(PPh₃)₄ (1.15 g, 1.0 mmol) was added. The mixture was replaced with nitrogen three times and then reacted at 90 °C overnight, and then TLC showed that the reaction was completed. The reaction liquid was filtered, and the filtrate was diluted with water (45 mL), and then extracted with ethyl acetate (40 ml*3). The organic phases were combined and dried by rotary evaporation. The solute was separated and purified by column chromatography (petroleum ether: ethyl acetate = 20: 1 - 3: 1) to obtain compound A89-4 (3.5 g, yield 96%).

### Synthesis of compound A89-5

A89-4 (3.06 g, 8.4 mmol) was dissolved in 30 mL of anhydrous tetrahydrofuran, LiHMDS (12.6 mL, 1 M, 12.6 mmol) was added dropwise at - 78 °C. The mixture was reacted at -78 °C for 1.5 h, and then a solution of Cbz-Cl (2.14 g, 12.6 mmol) in anhydrous tetrahydrofuran was added dropwise. The mixture was reacted at -78 °C for 20 min, and then slowly warmed to room temperature. TLC showed that the reaction was completed, and then saturated ammonium chloride (30 mL) was added to the reaction liquid to quench the reaction. The aqueous phase was extracted with ethyl acetate (40 ml*3). The organic phases were combined and dried by rotary evaporation. The solute was separated and purified by column chromatography (petroleum ether: ethyl acetate = 60: 1 - 10: 1) to obtain compound A89-5 (456 mg, yield 11%).

### Synthesis of compound A89-6

A89-5 (230 mg, 0.46 mmol) was dissolved in 4 mL of methanol, and sodium borohydride (35 mg, 0.92 mmol) was added in portions at -10 °C. The mixture was reacted at -10 °C for 3 h, and then TLC showed that the reaction was completed. Water (15 mL) was added to the reaction liquid to quench the reaction. The aqueous phase was extracted with ethyl acetate (15 ml*3). The organic phases were combined and dried by rotary evaporation. The solute was separated and purified by column chromatography (petroleum ether: ethyl acetate = 5 : 1) to obtain compound A89-6 (56 mg, yield 29%).

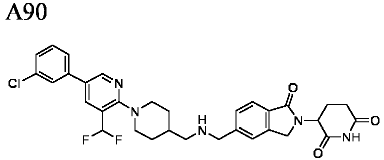

¹H NMR (400 MHz, DMSO) δ 11.00 (s, 1H), 8.76 (d, *J=* 2.3 Hz, 1H), 8.25 (d, *J=* 2.4 Hz, 1H), 7.87 - 7.78 (m, 2H), 7.75 (s, 1H), 7.72 - 7.63 (m, 2H), 7.50 - 7.47 (m, 1H), 7.44 - 7.39 (m, 1H), 5.13 (dd, *J=* 13.3, 5.1 Hz, 1H), 4.56 - 4.30 (m, 4H), 4.24 (s, 2H), 3.00 -2.87 (m, 1H), 2.79 (s, 2H), 2.71 - 2.57 (m, 1H), 2.44 - 2.28 (m, 2H), 2.06 - 1.89 (m, 3H), 1.79 - 1.68 (m, 4H), 1.55 - 1.42 (m, 2H). ESI-MS(M+H)⁺: 652.2.

The corresponding intermediates were prepared according to the following conditions:

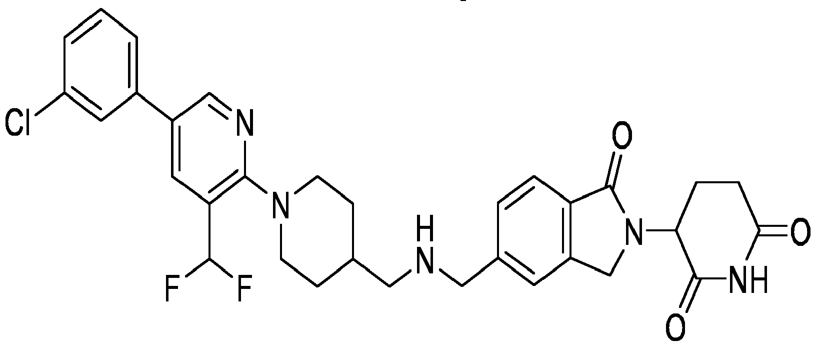

### Synthesis of compound A90-2

A90-1 (3.3 g, 24.0 mmol), A89-1 (4.14 g, 15.9 mmol) and potassium carbonate (8.28 g, 60.0 mmol) were dissolved in 30 mL of DMF. The mixture was reacted overnight at 100 °C, and then TLC showed that the reaction was completed. The reaction liquid was diluted with water (100 ml), and then the aqueous phase was extracted with ethyl acetate (50 mL*3). The organic phases were combined and then washed with saturated saline solution (20 ml*3) and the solution was dried by rotary evaporation. The solute was separated and purified by column chromatography (petroleum ether: ethyl acetate = 80: 1 - 10: 1) to obtain compound A90-2 (1.56 g, yield 28%).

### Synthesis of compound A90-3

A90-2 (1.51 g, 4.34 mmol), A87-4 (744 mg, 4.77 mmol) and potassium carbonate (1.49 g, 10.9 mmol) were dissolved in dioxane/water (16 mL/2 mL), and Pd(PPh₃)₄ (501 mg, 0.43 mmol) was added. The mixture was replaced with nitrogen three times and then reacted at 90 °C overnight, and then TLC showed that the reaction was completed. The reaction liquid was filtered, and the filtrate was diluted with water (30 mL), and then extracted with ethyl acetate (30 ml*3). The organic phases were combined and dried by rotary evaporation. The solute was separated and purified by column chromatography (petroleum ether: ethyl acetate = 80: 1 - 15: 1) to obtain compound A90-3 (1.2 g, yield 73%).

### Synthesis of compound A90-5

A90-4 (1.84 g, 6.0 mmol) was dissolved in 8 mL of anhydrous tetrahydrofuran, n-butyllithium (3.8 mL, 1.6 M, 6.0 mmol) was added dropwise at -20 °C. The mixture was reacted at -28 °C for 1.5 h, and then a solution of A90-3 (570 mg, 1.5 mmol) in anhydrous tetrahydrofuran was added dropwise. The mixture was reacted at -20 °C for 30 min, and then slowly warmed to room temperature. TLC showed that the reaction was completed, and then saturated ammonium chloride (20 mL) was added to the reaction to quench the reaction. The aqueous phase was extracted with ethyl acetate (25 ml*3). The organic phases were combined and dried by rotary evaporation. The solute was separated and purified by column chromatography (petroleum ether: ethyl acetate = 60: 1 - 10: 1) to obtain compound A90-5 (280 mg, yield 46%).

### Synthesis of compound A90-6

A90-5 (196 mg, 0.48 mmol) was dissolved in 4 mL of tetrahydrofuran, and 6 N hydrochloric acid (2 mL) was added. The mixture was reacted at ambient temperature for 2 h, and then TLC showed that the reaction was completed. Saturated sodium bicarbonate aqueous solution was added to adjust the pH to 6-7, and then the aqueous phase was extracted with ethyl acetate (15 ml*3). The organic phases were combined and dried by rotary evaporation. The solute was separated and purified by the preparative TLC (petroleum ether: ethyl acetate = 5 : 1) to obtain compound A90-6 (90 mg, yield 47%).

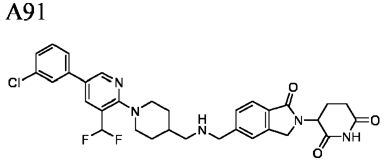

¹H NMR (400 MHz, DMSO) δ 10.98 (s, 1H), 8.83 (d, *J* = 2.3 Hz, 1H), 8.28 (d, *J* = 2.4 Hz, 1H), 7.86 - 7.85 (m, 1H), 7.76 - 7.69 (m, 2H), 7.65 (s, 1H), 7.56 (d, *J* = 7.8 Hz, 1H), 7.52 - 7.47 (m, 1H), 7.47 - 7.41 (m, 1H), 5.12 (dd, *J* = 13.3, 5.1 Hz, 1H), 4.40 (dd, *J* = 53.2, 17.3 Hz, 2H), 3.97 (s, 2H), 3.52 (dd, *J* = 11.7, 3.3 Hz, 2H), 3.08 (d, *J* = 11.4 Hz, 2H), 2.98 - 2.85 (m, 1H), 2.66 - 2.53 (m, 1H), 2.48 - 2.33 (m, 3H), 2.24 (s, 2H), 2.06 - 1.92 (m, 2H), 1.61 (s, 4H). ESI-MS(M+H)⁺: 652.2.

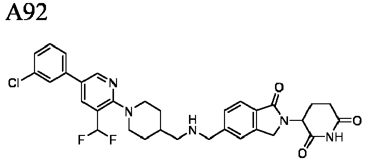

¹H NMR (400 MHz, DMSO) δ 10.99 (s, 1H), 8.82 (d, *J* = 2.2 Hz, 1H), 8.24 (d, *J* = 2.4 Hz, 1H), 7.82 - 7.76 (m, 2H), 7.76 - 7.70 (m, 2H), 7.68 (d, *J* = 7.6 Hz, 1H), 7.54 - 7.45 (m, 2H), 7.43 - 7.35 (m, 1H), 5.12 (dd, *J* = 13.3, 5.1 Hz, 1H), 4.43 (dd, *J=* 52.8, 17.6 Hz, 2H), 4.24 (s, 2H), 3.63 - 3.55 (m, 2H), 2.97 - 2.80 (m, 5H), 2.66 - 2.56 (m, 1H), 2.46 - 2.34 (m, 1H), 2.07 - 1.98 (m, 1H), 1.96 - 1.82 (m, 3H), 1.41 - 1.25 (m, 2H). ESI-MS(M+H)⁺: 592.2.

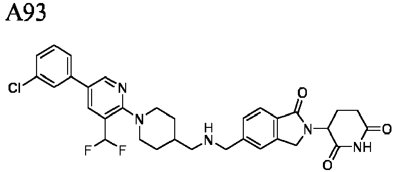

¹H NMR (400 MHz, DMSO) δ 10.98 (s, 1H), 8.84 (d, *J* = 1.8 Hz, 1H), 8.28 (d, *J* = 2.1 Hz, 1H), 7.72 - 7.64 (m, 2H), 7.60 (s, 2H), 7.55 - 7.47 (m, 2H), 7.26 - 7.17 (m, 1H), 5.11 (dd, *J* = 13.2, 5.1 Hz, 1H), 4.39 (dd, *J* = 53.2, 17.2 Hz, 2H), 3.88 (s, 2H), 3.67 - 3.60 (m, 2H), 2.99 - 2.85 (m, 3H), 2.67 - 2.54 (m, 1H), 2.49 - 2.29 (m, 3H), 2.08 - 1.94 (m, 1H), 1.89 - 1.79 (m, 2H), 1.75 - 1.64 (m, 1H), 1.34 - 1.22 (m, 2H). ESI-MS(M+H)⁺: 610.2.

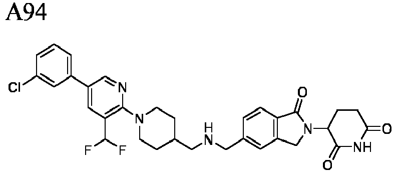

¹H NMR (400 MHz, DMSO) δ 10.98 (s, 1H), 8.79 (d, *J* = 2.1 Hz, 1H), 8.21 (d, *J* = 2.3 Hz, 1H), 7.71 (d, *J* = 7.8 Hz, 1H), 7.65 (s, 1H), 7.57 - 7.55 (m, 2H), 7.52 (d, *J* = 7.9 Hz, 1H), 7.38 - 7.34 (m, 1H), 7.21 (d, *J* = 7.6 Hz, 1H), 5.12 (dd, *J* = 13.3, 5.1 Hz, 1H), 4.40 (dd, *J* = 53.2, 17.4 Hz, 2H), 3.98 (s, 2H), 3.63 - 3.52 (m, 2H), 2.98 - 2.83 (m, 3H), 2.66 - 2.53 (m, 3H), 2.46 - 2.32 (m, 4H), 2.06 - 1.97 (m, 1H), 1.89 - 1.81 (m, 2H), 1.80 - 1.67 (m, 1H), 1.37 - 1.24 (m, 2H). ESI-MS(M+H)⁺: 606.2.

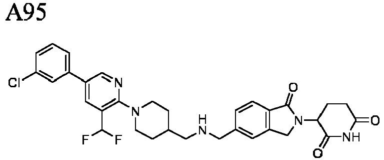

¹H NMR (400 MHz, DMSO) δ 10.97 (s, 1H), 8.79 (d, *J* = 2.2 Hz, 1H), 8.21 (d, *J* = 2.3 Hz, 1H), 7.66 (d, *J* = 7.8 Hz, 1H), 7.57 (s, 1H), 7.48 (d, *J* = 7.9 Hz, 1H), 7.38 (t, *J* = 7.9 Hz, 1H), 7.32 - 7.21 (m, 2H), 6.96 (dd, *J* = 8.2, 1.8 Hz, 1H), 5.09 (dd, *J* = 13.3, 5.1 Hz, 1H), 4.37 (dd, *J* = 52.9, 17.3 Hz, 2H), 3.82 (s, 5H), 3.64 - 3.53 (m, 2H), 2.96 - 2.82 (m, 3H), 2.65 - 2.55 (m, 1H), 2.46 - 2.30 (m, 3H), 2.05 - 1.95 (m, 1H), 1.88 - 1.77 (m, 2H), 1.70 - 1.57 (m, 1H), 1.32 - 1.19 (m, 2H). ESI-MS(M+H)⁺: 622.2.

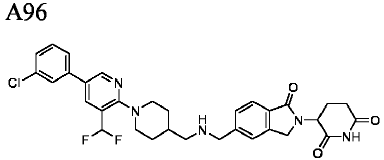

¹H NMR (400 MHz, DMSO) δ 10.98 (s, 1H), 8.84 (d, *J* = 2.2 Hz, 1H), 8.31 (d, *J* = 2.3 Hz, 1H), 7.82 - 7.76 (m, 2H), 7.70 (d, *J* = 7.8 Hz, 1H), 7.65 - 7.57 (m, 2H), 7.54 (d, *J* = 7.8 Hz, 1H), 7.38 (d, *J* = 7.7 Hz, 1H), 5.11 (dd, *J* = 13.3, 5.1 Hz, 1H), 4.39 (dd, *J* = 53.3, 17.3 Hz, 2H), 3.93 (s, 2H), 3.70 - 3.60 (m, 2H), 3.01 - 2.83 (m, 3H), 2.66 -2.52 (m, 3H), 2.44 - 2.33 (m, 1H), 2.06 - 1.97 (m, 1H), 1.89 - 1.80 (m, 2H), 1.78 - 1.66 (m, 1H), 1.35 - 1.22 (m, 2H). ESI-MS(M+H)⁺: 676.2.

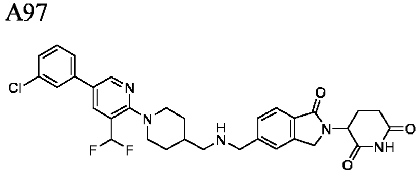

¹H NMR (400 MHz, DMSO) δ 10.96 (s, 1H), 8.77 (d, *J* = 2.2 Hz, 1H), 8.16 (d, *J* = 2.3 Hz, 1H), 7.67 (d, *J* = 7.8 Hz, 1H), 7.57 (s, 1H), 7.49 (d, *J* = 8.0 Hz, 1H), 7.27 (dd, *J* = 10.2, 6.1 Hz, 1H), 6.99 - 6.90 (m, 2H), 6.75 (dd, *J* = 8.3, 1.8 Hz, 1H), 5.10 (dd, *J* = 13.3, 5.1 Hz, 1H), 4.37 (dd, *J* = 53.5, 17.3 Hz, 2H), 3.82 (s, 2H), 3.61 - 3.54 (m, 2H), 2.95 (s, 6H), 2.93 - 2.83 (m, 3H), 2.66 - 2.55 (m, 1H), 2.46 - 2.31 (m, 3H), 2.05 - 1.95 (m, 1H), 1.88 - 1.79 (m, 2H), 1.69 - 1.58 (m, 1H), 1.33 - 1.22 (m, 2H). ESI-MS(M+H)⁺: 635.2.

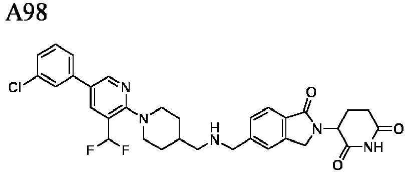

¹H NMR (400 MHz, DMSO) δ 10.97 (s, 1H), 8.74 (d, *J* = 2.6 Hz, 1H), 8.41 (d, *J* = 2.6 Hz, 1H), 7.81 (t, *J* = 1.8 Hz, 1H), 7.71 - 7.63 (m, 2H), 7.57 (s, 1H), 7.52 - 7.44 (m, 2H), 7.43 - 7.37 (m, 1H), 5.10 (dd, *J* = 13.3, 5.1 Hz, 1H), 4.49 - 4.24 (m, 4H), 3.82 (s, 2H), 3.05 (t, *J* = 11.7 Hz, 2H), 2.95 - 2.86 (m, 1H), 2.65 - 2.54 (m, 1H), 2.46 - 2.29 (m, 3H), 2.05 - 1.94 (m, 1H), 1.91 - 1.81 (m, 2H), 1.80 - 1.69 (m, 1H), 1.32 - 1.17 (m, 2H). ESI-MS(M+H)⁺: 583.2.

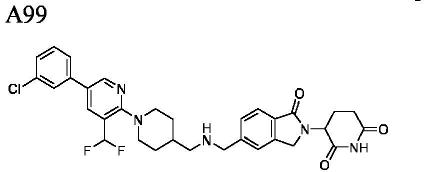

¹H NMR (400 MHz, DMSO) δ 11.00 (s, 1H), 8.55 (d, *J* = 2.3 Hz, 1H), 7.98 - 7.91 (m, 2H), 7.80 (d, *J* = 6.9 Hz, 2H), 7.77 - 7.67 (m, 2H), 7.64 (d, *J* = 7.7 Hz, 1H), 7.56 (s, 1H), 7.47 (t, *J* = 7.8 Hz, 1H), 7.39 (d, *J* = 7.5 Hz, 1H), 5.14 (dd, *J* = 13.2, 5.0 Hz, 1H), 4.43 (dd, *J* = 54.0, 17.5 Hz, 2H), 4.27 (s, 2H), 3.93 - 3.82 (m, 2H), 2.97 - 2.79 (m, 5H), 2.66 - 2.55 (m, 1H), 2.45 - 2.31 (m, 1H), 2.09 - 1.92 (m, 2H), 1.89 - 1.76 (m, 2H), 1.42 - 1.24 (m, 2H). ESI-MS(M+H)⁺: 601.2.

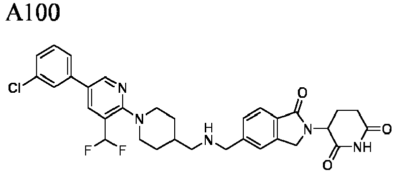

¹H NMR (400 MHz, DMSO) δ 10.97 (s, 1H), 8.59 (d, *J* = 2.5 Hz, 1H), 8.14 (d, *J* = 2.5 Hz, 1H), 7.72 (s, 1H), 7.66 (d, *J* = 7.8 Hz, 1H), 7.61 (d, *J* = 7.8 Hz, 1H), 7.56 (s, 1H), 7.50 - 7.46 (m, 1H), 7.44 (d, *J* = 7.8 Hz, 1H), 7.37 (d, *J* = 7.9 Hz, 1H), 5.09 (dd, *J* = 13.3, 5.1 Hz, 1H), 4.37 (dd, *J* = 53.3, 17.3 Hz, 2H), 3.92 - 3.74 (m, 7H), 2.99 - 2.83 (m, 3H), 2.65 - 2.55 (m, 1H), 2.46 - 2.32 (m, 3H), 2.04 - 1.97 (m, 1H), 1.83 - 1.74 (m, 2H), 1.73 - 1.62 (m, 1H), 1.25 - 1.12 (m, 2H). ESI-MS(M+H)⁺: 616.2.

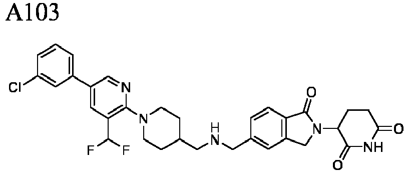

¹H NMR (400 MHz, DMSO) δ 10.99 (s, 1H), 8.07 (d, *J* = 2.3 Hz, 1H), 7.93 (dd, *J* = 8.7, 2.4 Hz, 1H), 7.81 - 7.70 (m, 3H), 7.65 (t, *J* = 9.0 Hz, 2H), 7.47 (t, *J* = 7.8 Hz, 1H), 7.41 (d, *J* = 7.9 Hz, 1H), 7.23 (d, *J* = 8.8 Hz, 1H), 5.13 (dd, *J* = 13.3, 5.1 Hz, 1H), 4.42 (dd, *J* = 53.6, 17.4 Hz, 2H), 4.09 (dd, *J* = 16.7, 11.5 Hz, 2H), 3.60 (d, *J* = 12.0 Hz, 2H), 3.00 - 2.69 (m, 5H), 2.65 - 2.54 (m, 1H), 2.46 - 2.32 (m, 1H), 2.08 - 1.97 (m, 1H), 1.96 - 1.77 (m, 3H), 1.46 - 1.33 (m, 2H). ESI-MS(M+H)⁺: 582.2.

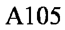

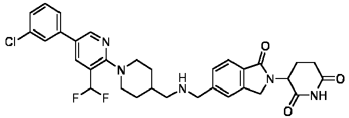

¹H NMR (400 MHz, DMSO) δ 10.97 (s, 1H), 8.39 - 8.38 (m, 1H), 7.90 (dd, *J* = 15.0, 2.0 Hz, 1H), 7.77 - 7.75 (m, 1H), 7.70 (d, *J* = 7.8 Hz, 1H), 7.68 - 7.62 (m, 2H), 7.55 (d, *J* = 7.9 Hz, 1H), 7.49 - 7.42 (m, 1H), 7.40 - 7.37 (m, 1H), 5.11 (dd, *J* = 13.3, 5.1 Hz, 1H), 4.39 (dd, *J* = 52.9, 17.3 Hz, 2H), 4.19 - 4.09 (m, 2H), 3.96 (dd, *J* = 42.3, 14.1 Hz, 2H), 2.99 - 2.80 (m, 3H), 2.67 - 2.55 (m, 2H), 2.46 - 2.31 (m, 1H), 2.07 - 1.96 (m, 1H), 1.83 - 1.64 (m, 3H), 1.44 - 1.28 (m, 2H), 1.03 (d, *J* = 6.4 Hz, 3H). ESI-MS(M+H)⁺: 590.2.

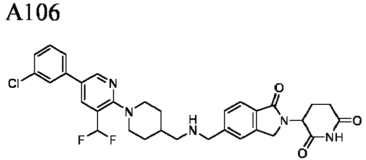

¹H NMR (400 MHz, DMSO) δ 10.98 (s, 1H), 8.72 - 8.70 (m, 1H), 8.64 - 8.57 (m, 1H), 8.09 (dd, *J* = 15.2, 1.9 Hz, 1H), 7.94 (d, *J* = 8.0 Hz, 1H), 7.84 (td, *J* = 7.8, 1.8 Hz, 1H), 7.72 (d, *J* = 7.8 Hz, 1H), 7.67 (s, 1H), 7.57 (d, *J* = 7.9 Hz, 1H), 7.32 - 7.28 (m, 1H), 5.11 (dd, *J* = 13.3, 5.1 Hz, 1H), 4.40 (dd, *J* = 52.9, 17.4 Hz, 2H), 4.18 - 4.07 (m, 2H), 4.01 (s, 2H), 3.02 - 2.85 (m, 3H), 2.66 - 2.55 (m, 3H), 2.46 - 2.33 (m, 1H), 2.07 - 1.98 (m, 1H), 1.91 - 1.78 (m, 3H), 1.35 - 1.19 (m, 2H). ESI-MS(M+H)⁺: 543.2.

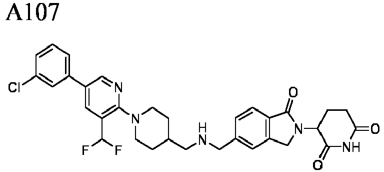

¹H NMR (400 MHz, DMSO) δ 10.98 (s, 1H), 8.20 (d, *J* = 1.6 Hz, 1H), 7.74 - 7.65 (m, 2H), 7.61 (s, 1H), 7.59 - 7.55 (m, 1H), 7.52 (d, *J* = 7.8 Hz, 1H), 7.44 - 7.37 (m, 1H), 7.34 - 7.25 (m, 2H), 5.11 (dd, *J* = 13.3, 5.1 Hz, 1H), 4.38 (dd, *J* = 53.0, 17.3 Hz, 2H), 4.13 - 4.02 (m, 2H), 3.89 (s, 2H), 2.98 - 2.84 (m, 3H), 2.64 - 2.56 (m, 1H), 2.49 (d, *J* = 4.2 Hz, 2H), 2.46 - 2.35 (m, 1H), 2.05 - 1.94 (m, 1H), 1.88 - 1.70 (m, 3H), 1.33 - 1.19 (m, 2H). ESI-MS(M+H)⁺: 560.2.

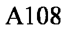

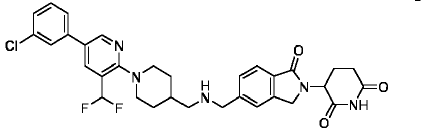

¹H NMR (400 MHz, DMSO) δ 10.98 (s, 1H), 8.40 (s, 1H), 7.90 (dd, *J* = 15.0, 1.9 Hz, 1H), 7.70 (d, *J* = 7.8 Hz, 1H), 7.63 (s, 1H), 7.57 - 7.53 (m, 3H), 7.47 (dd, *J* = 14.3, 8.1 Hz, 1H), 7.20 - 7.09 (m, 1H), 5.11 (dd, *J* = 13.3, 5.1 Hz, 1H), 4.39 (dd, *J* = 53.4, 17.3 Hz, 2H), 4.10 - 4.06 (m, 2H), 3.94 (s, 2H), 2.99 - 2.83 (m, 3H), 2.66 - 2.51 (m, 3H), 2.45 - 2.32 (m, 1H), 2.07 - 1.95 (m, 1H), 1.87 - 1.71 (m, 3H), 1.30 - 1.17 (m, 2H). ESI-MS(M+H)⁺: 560.2.

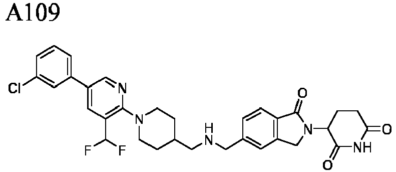

¹H NMR (400 MHz, DMSO) δ 10.98 (s, 1H), 8.33 - 8.31 (m, 1H), 7.83 (dd, *J* = 14.9, 2.0 Hz, 1H), 7.75 - 7.67 (m, 3H), 7.62 (s, 1H), 7.53 (d, *J* = 7.8 Hz, 1H), 7.31 - 7.23 (m, 2H), 5.11 (dd, *J* = 13.3, 5.1 Hz, 1H), 4.39 (dd, *J* = 53.3, 17.3 Hz, 2H), 4.07 - 4.00 (m, 2H), 3.93 (s, 2H), 2.96 - 2.85 (m, 3H), 2.65 - 2.56 (m, 1H), 2.52 (d, *J* = 7.5 Hz, 2H), 2.45 - 2.32 (m, 1H), 2.05 - 1.93 (m, 1H), 1.87 - 1.67 (m, 3H), 1.35 - 1.19 (m, 2H). ESI-MS(M+H)⁺: 560.2.

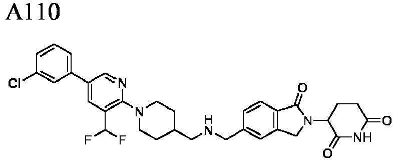

¹H NMR (400 MHz, DMSO) δ 10.97 (s, 1H), 8.43 (t, *J* = 1.7 Hz, 1H), 8.04 - 7.94 (m, 3H), 7.72 - 7.63 (m, 3H), 7.57 (s, 1H), 7.48 (d, *J* = 7.9 Hz, 1H), 5.10 (dd, *J* = 13.3, 5.1 Hz, 1H), 4.37 (dd, *J* = 53.5, 17.2 Hz, 2H), 4.14 - 4.08 (m, 2H), 3.82 (s, 2H), 2.97 - 2.85 (m, 3H), 2.66 - 2.54 (m, 1H), 2.45 - 2.33 (m, 3H), 2.06 - 1.95 (m, 1H), 1.88 - 1.78 (m, 2H), 1.76 - 1.61 (m, 1H), 1.33 - 1.16 (m, 2H). ESI-MS(M+H)⁺: 610.2.

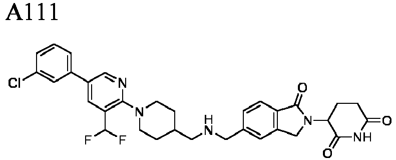

¹H NMR (400 MHz, DMSO) δ 11.00 (s, 1H), 8.44 (s, 1H), 8.19 (s, 1H), 8.04 (d, *J* = 8.2 Hz, 1H), 7.99 (dd, *J* = 15.0, 2.0 Hz, 1H), 7.80 (t, *J* = 7.8 Hz, 2H), 7.73 (s, 1H), 7.64 (t, *J* = 7.8 Hz, 2H), 5.13 (dd, *J* = 13.2, 5.0 Hz, 1H), 4.44 (dd, *J* = 52.4, 17.5 Hz, 2H), 4.26 (s, 2H), 4.16 - 4.06 (m, 2H), 3.00 - 2.81 (m, 5H), 2.67 - 2.56 (m, 1H), 2.45 - 2.35 (m, 1H), 2.09 - 1.93 (m, 2H), 1.88 - 1.79 (m, 2H), 1.38 - 1.26 (m, 2H). ESI-MS(M+H)⁺: 567.2.

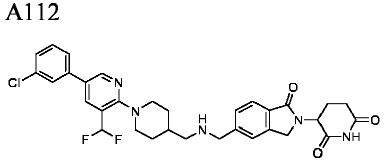

¹H NMR (400 MHz, DMSO) δ 10.97 (s, 1H), 8.35 (t, *J* = 1.7 Hz, 1H), 7.84 (dd, *J* = 14.9, 2.0 Hz, 1H), 7.66 (d, *J* = 7.8 Hz, 1H), 7.57 (s, 1H), 7.48 (d, *J* = 7.9 Hz, 1H), 7.35 (t, *J* = 7.9 Hz, 1H), 7.23 (d, *J* = 7.8 Hz, 1H), 7.22 - 7.18 (m, 1H), 6.94 - 6.86 (m, 1H), 5.10 (dd, *J* = 13.3, 5.1 Hz, 1H), 4.37 (dd, *J* = 53.3, 17.2 Hz, 2H), 4.09 - 3.89 (m, 2H), 3.85 - 3.77 (m, 5H), 2.96 - 2.80 (m, 3H), 2.65 - 2.54 (m, 1H), 2.44 - 2.33 (m, 3H), 2.05 - 1.95 (m, 1H), 1.87 - 1.77 (m, 2H), 1.76 - 1.61 (m, 1H), 1.32 - 1.21 (m, 2H). ESI-MS(M+H)⁺: 572.2.

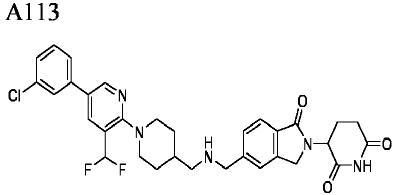

¹H NMR (400 MHz, DMSO) δ 10.97 (s, 1H), 8.81 (d, *J* = 2.1 Hz, 1H), 8.27 (d, *J* = 2.3 Hz, 1H), 7.66 (d, *J* = 7.8 Hz, 1H), 7.57 (s, 1H), 7.48 (d, *J* = 7.8 Hz, 1H), 7.40 (t, *J* = 1.5 Hz, 1H), 7.28 - 7.23 (m, 1H), 7.03 (t, *J* = 1.9 Hz, 1H), 5.11 (dd, *J* = 13.3, 5.1 Hz, 1H), 4.37 (dd, *J* = 52.9, 17.2 Hz, 2H), 3.84 (s, 3H), 3.82 (s, 2H), 3.70 - 3.58 (m, 2H), 2.99 - 2.84 (m, 3H), 2.65 - 2.54 (m, 1H), 2.46 - 2.34 (m, 3H), 2.06 - 1.94 (m, 1H), 1.89 - 1.77 (m, 2H), 1.72 - 1.56 (m, 1H), 1.32 - 1.19 (m, 2H). ESI-MS(M+H)⁺: 656.2.

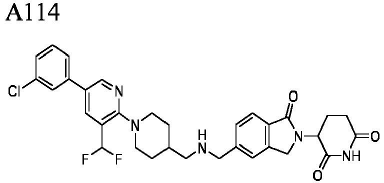

¹H NMR (400 MHz, DMSO) δ 10.98 (s, 1H), 8.78 (d, *J* = 2.2 Hz, 1H), 8.25 (d, *J* = 2.3 Hz, 1H), 7.66 (d, *J* = 7.8 Hz, 1H), 7.61 (s, 1H), 7.56 (s, 1H), 7.53 (s, 1H), 7.48 (d, *J* = 7.8 Hz, 1H), 7.26 (s, 1H), 5.11 (dd, *J* = 13.3, 5.1 Hz, 1H), 4.37 (dd, *J* = 51.5, 17.3 Hz, 2H), 3.81 (s, 2H), 3.62 (d, *J* = 12.6 Hz, 2H), 2.99 - 2.83 (m, 3H), 2.68 - 2.55 (m, 1H), 2.46 - 2.28 (m, 6H), 2.05 - 1.95 (m, 1H), 1.88 - 1.77 (m, 2H), 1.72 - 1.56 (m, 1H), 1.34 - 1.18 (m, 2H). ESI-MS(M+H)⁺: 640.2.

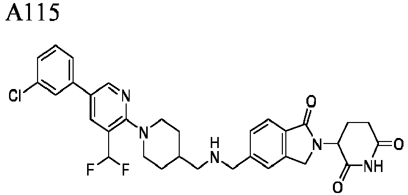

¹H NMR (400 MHz, DMSO) δ 10.98 (s, 1H), 8.85 (d, *J* = 2.3 Hz, 1H), 8.35 (d, *J* = 2.4 Hz, 1H), 7.76 (d, *J* = 1.5 Hz, 1H), 7.73 - 7.65 (m, 2H), 7.59 (s, 1H), 7.50 (d, *J* = 7.9 Hz, 1H), 7.44 (dt, *J* = 8.7, 2.0 Hz, 1H), 5.11 (dd, *J* = 13.3, 5.1 Hz, 1H), 4.38 (dd, *J* = 53.8, 17.3 Hz, 2H), 3.86 (s, 2H), 3.73 - 3.63 (m, 2H), 3.00 - 2.81 (m, 3H), 2.68 - 2.55 (m, 1H), 2.47 - 2.35 (m, 3H), 2.06 - 1.96 (m, 1H), 1.89 - 1.80 (m, 2H), 1.77 - 1.62 (m, 1H), 1.34 - 1.24 (m, 2H). ESI-MS(M+H)⁺: 644.2.

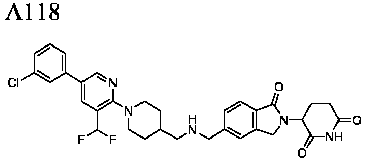

¹H NMR (400 MHz, DMSO) δ 11.00 (s, 1H), 8.74 (d, *J* = 4.4 Hz, 1H), 8.24 - 8.14 (m, 2H), 7.82 (t, *J* = 1.8 Hz, 1H), 7.69 (d, *J* = 7.8 Hz, 2H), 7.61 (s, 1H), 7.55 - 7.38 (m, 3H), 7.06 (t, *J* = 54.2 Hz, 1H), 5.12 (dd, *J* = 13.3, 5.1 Hz, 1H), 4.39 (dd, *J* = 54.5, 17.3 Hz, 2H), 3.90 (s, 2H), 3.64 - 3.49 (m, 4H), 2.97 - 2.88 (m, 3H), 2.64 - 2.55 (m, 1H), 2.45 - 2.34 (m, 1H), 2.05 - 1.95 (m, 1H), 1.89 - 1.78 (m, 2H), 1.74 - 1.61 (m, 1H), 1.44 - 1.29 (m, 2H).ESI-MS(M+H)⁺: 608.3.

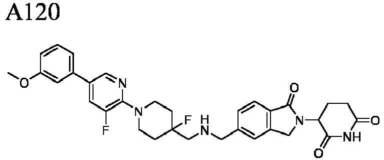

¹H NMR (400 MHz, DMSO) δ 10.99 (s, 1H), 8.38 (s, 1H), 7.88 (dd, *J* = 14.9, 1.7 Hz, 1H), 7.67 (d, *J* = 7.8 Hz, 1H), 7.57 (s, 1H), 7.48 (d, *J* = 7.8 Hz, 1H), 7.37 - 7.33 (m, 1H), 7.28 - 7.19 (m, 2H), 6.91 (dd, *J* = 8.1, 1.9 Hz, 1H), 5.10 (dd, *J* = 13.3, 5.0 Hz, 1H), 4.37 (dd, *J* = 52.4, 17.3 Hz, 2H), 3.93 - 3.82 (m, 4H), 3.81 (s, 3H), 3.21 (t, *J* = 11.1 Hz, 2H), 2.97 - 2.83 (m, 1H), 2.69 (d, *J* = 20.2 Hz, 2H), 2.62 - 2.56 (m, 1H), 2.43 - 2.32 (m, 1H), 2.04 - 1.74 (m, 5H).ESI-MS(M+H)⁺: 590.3.

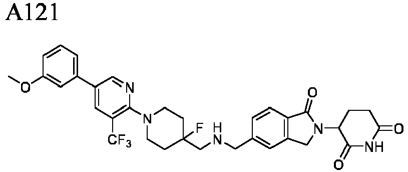

¹H NMR (400 MHz, DMSO) δ 10.98 (s, 1H), 8.84 (d, *J* = 2.1 Hz, 1H), 8.26 (d, *J* = 2.3 Hz, 1H), 7.67 (d, *J* = 7.8 Hz, 1H), 7.58 (s, 1H), 7.50 (d, *J* = 7.9 Hz, 1H), 7.42 - 7.37 (m, 1H), 7.31 - 7.28 (m, 2H), 6.97 (dd, *J* = 8.1, 1.8 Hz, 1H), 5.11 (dd, *J* = 13.3, 5.1 Hz, 1H), 4.38 (dd, *J* = 53.6, 17.2 Hz, 2H), 3.88 (s, 2H), 3.83 (s, 3H), 3.48 - 3.38 (m, 2H), 3.19 (t, *J* = 10.9 Hz, 2H), 2.96 - 2.85 (m, 1H), 2.70 (d, *J* = 20.3 Hz, 2H), 2.65 - 2.54 (m, 1H), 2.44 - 2.31 (m, 1H), 2.04 - 1.87 (m, 4H), 1.86 - 1.73 (m, 1H).ESI-MS(M+H)⁺: 540.3.

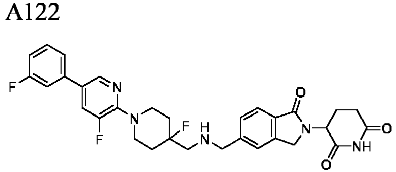

¹H NMR (400 MHz, DMSO) δ 10.98 (s, 1H), 8.42 - 8.41 (t, *J* = 1.7 Hz, 1H), 7.93 (dd, *J* = 15.0, 2.0 Hz, 1H), 7.67 (d, *J* = 7.8 Hz, 1H), 7.60 - 7.52 (m, 3H), 7.50 - 7.44 (m, 2H), 7.21 - 7.12 (m, 1H), 5.10 (dd, *J* = 13.3, 5.1 Hz, 1H), 4.37 (dd, *J* = 53.7, 17.3 Hz, 2H), 3.96 - 3.78 (m, 4H), 3.22 (t, *J* = 11.0 Hz, 2H), 2.98 - 2.85 (m, 1H), 2.68 (d, *J* = 20.2 Hz, 2H), 2.62 - 2.54 (m, 1H), 2.45 - 2.31 (m, 1H), 2.05 - 1.94 (m, 1H), 1.94 - 1.73 (m, 4H).ESI-MS(M+H)⁺: 578.2.

A123 and A124 can be obtained by separation using A94. Separation methods refer to A81 and A82.

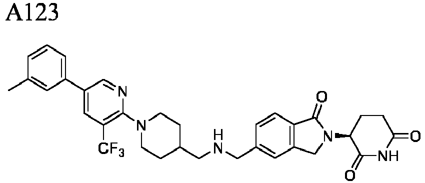

¹H NMR (400 MHz, DMSO) δ 10.98 (s, 1H), 8.79 (d, *J* = 2.1 Hz, 1H), 8.21 (d, *J* = 2.3 Hz, 1H), 7.71 (d, *J* = 7.8 Hz, 1H), 7.65 (s, 1H), 7.57 - 7.55 (m, 2H), 7.52 (d, *J* = 7.9 Hz, 1H), 7.38 - 7.34 (m, 1H), 7.21 (d, *J* = 7.6 Hz, 1H), 5.12 (dd, *J* = 13.3, 5.1 Hz, 1H), 4.40 (dd, *J* = 53.2, 17.4 Hz, 2H), 3.98 (s, 2H), 3.63 - 3.52 (m, 2H), 2.98 - 2.83 (m, 3H), 2.66 - 2.53 (m, 3H), 2.46 - 2.32 (m, 4H), 2.06 - 1.97 (m, 1H), 1.89 - 1.81 (m, 2H), 1.80 - 1.67 (m, 1H), 1.37 - 1.24 (m, 2H).
ee value>98%

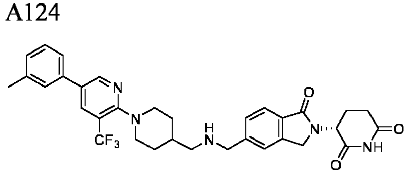

¹H NMR (400 MHz, DMSO) δ 10.98 (s, 1H), 8.79 (d, *J* = 2.1 Hz, 1H), 8.21 (d, *J* = 2.3 Hz, 1H), 7.71 (d, *J* = 7.8 Hz, 1H), 7.65 (s, 1H), 7.57 - 7.55 (m, 2H), 7.52 (d, *J* = 7.9 Hz, 1H), 7.38 - 7.34 (m, 1H), 7.21 (d, *J* = 7.6 Hz, 1H), 5.12 (dd, *J* = 13.3, 5.1 Hz, 1H), 4.40 (dd, *J* = 53.2, 17.4 Hz, 2H), 3.98 (s, 2H), 3.63 - 3.52 (m, 2H), 2.98 - 2.83 (m, 3H), 2.66 - 2.53 (m, 3H), 2.46 - 2.32 (m, 4H), 2.06 - 1.97 (m, 1H), 1.89 - 1.81 (m, 2H), 1.80 - 1.67 (m, 1H), 1.37 - 1.24 (m, 2H).
ee value>98%

A125 and A126 can be obtained by separation using A108. Separation methods refer to A81 and A82.

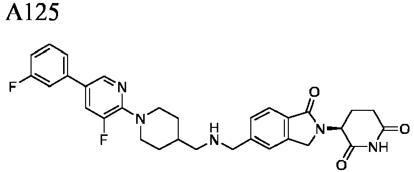

¹H NMR (400 MHz, DMSO) δ 10.98 (s, 1H), 8.40 (s, 1H), 7.90 (dd, *J* = 15.0, 1.9 Hz, 1H), 7.70 (d, *J* = 7.8 Hz, 1H), 7.63 (s, 1H), 7.57 - 7.53 (m, 3H), 7.47 (dd, *J* = 14.3, 8.1 Hz, 1H), 7.20 - 7.09 (m, 1H), 5.11 (dd, *J* = 13.3, 5.1 Hz, 1H), 4.39 (dd, *J* = 53.4, 17.3 Hz, 2H), 4.10 - 4.06 (m, 2H), 3.94 (s, 2H), 2.99 - 2.83 (m, 3H), 2.66 - 2.51 (m, 3H), 2.45 - 2.32 (m, 1H), 2.07 - 1.95 (m, 1H), 1.87 - 1.71 (m, 3H), 1.30 -1.17 (m, 2H).
ee value>98%

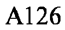

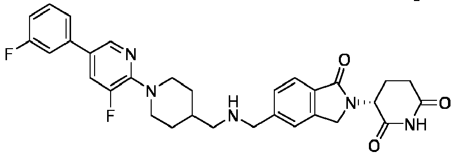

¹H NMR (400 MHz, DMSO) δ 10.98 (s, 1H), 8.40 (s, 1H), 7.90 (dd, *J* = 15.0, 1.9 Hz, 1H), 7.70 (d, *J* = 7.8 Hz, 1H), 7.63 (s, 1H), 7.57 - 7.53 (m, 3H), 7.47 (dd, *J* = 14.3, 8.1 Hz, 1H), 7.20 - 7.09 (m, 1H), 5.11 (dd, *J* = 13.3, 5.1 Hz, 1H), 4.39 (dd, *J* = 53.4, 17.3 Hz, 2H), 4.10 - 4.06 (m, 2H), 3.94 (s, 2H), 2.99 - 2.83 (m, 3H), 2.66 - 2.51 (m, 3H), 2.45 - 2.32 (m, 1H), 2.07 - 1.95 (m, 1H), 1.87 - 1.71 (m, 3H), 1.30 -1.17 (m, 2H).
ee value>98%

A127 and A128 can be obtained by separation using A83. Separation methods refer to A81 and A82.

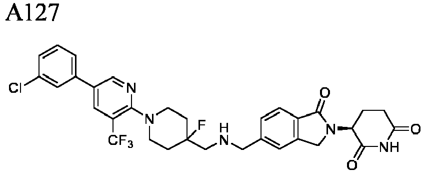

¹H NMR (400 MHz, DMSO) δ 10.97 (s, 1H), 8.84 (d, *J* = 2.3 Hz, 1H), 8.31 (d, *J* = 2.4 Hz, 1H), 7.86 (t, *J* = 1.8 Hz, 1H), 7.76 - 7.70 (m, 1H), 7.67 (d, *J* = 7.8 Hz, 1H), 7.58 (s, 1H), 7.53 - 7.41 (m, 3H), 5.10 (dd, *J* = 13.3, 5.1 Hz, 1H), 4.38 (dd, *J* = 53.1, 17.3 Hz, 2H), 3.87 (s, 2H), 3.53 - 3.43 (m, 2H), 3.25 - 3.13 (m, 2H), 2.97 - 2.86 (m, 1H), 2.70 (d, *J* = 20.4 Hz, 2H), 2.63 - 2.55 (m, 1H), 2.45 - 2.34 (m, 1H), 2.05 - 1.86 (m, 4H), 1.86 - 1.75 (m, 1H).
ee value>98%

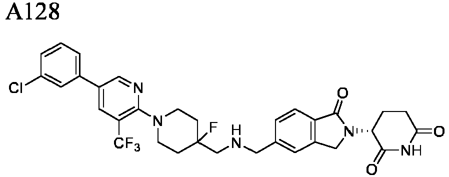

¹H NMR (400 MHz, DMSO) δ 10.97 (s, 1H), 8.84 (d, *J* = 2.3 Hz, 1H), 8.31 (d, *J* = 2.4 Hz, 1H), 7.86 (t, *J* = 1.8 Hz, 1H), 7.76 - 7.70 (m, 1H), 7.67 (d, *J* = 7.8 Hz, 1H), 7.58 (s, 1H), 7.53 - 7.41 (m, 3H), 5.10 (dd, *J* = 13.3, 5.1 Hz, 1H), 4.38 (dd, *J* = 53.1, 17.3 Hz, 2H), 3.87 (s, 2H), 3.53 - 3.43 (m, 2H), 3.25 - 3.13 (m, 2H), 2.97 - 2.86 (m, 1H), 2.70 (d, *J* = 20.4 Hz, 2H), 2.63 - 2.55 (m, 1H), 2.45 - 2.34 (m, 1H), 2.05 - 1.86 (m, 4H), 1.86 - 1.75 (m, 1H).
ee value>98%

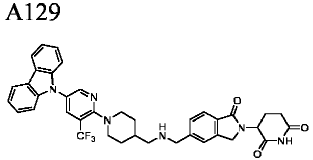

¹H NMR (400 MHz, DMSO) δ 10.99 (s, 1H), 8.74 (d, *J* = 2.5 Hz, 1H), 8.29 (d, *J* = 2.5 Hz, 1H), 8.25 (d, *J* = 7.7 Hz, 2H), 7.69 (d, *J* = 7.8 Hz, 1H), 7.61 (s, 1H), 7.52 (d, *J* = 8.1 Hz, 1H), 7.46 - 7.42 (m, 2H), 7.35 - 7.27 (m, 4H), 5.12 (dd, *J* = 13.3, 5.1 Hz, 1H), 4.39 (dd, *J* = 54.4, 17.3 Hz, 2H), 3.88 (s, 2H), 3.78 - 3.70 (m, 2H), 3.00 (t, *J* = 11.6 Hz, 2H), 2.94 - 2.83 (m, 1H), 2.70 - 2.57 (m, 1H), 2.45 - 2.32 (m, 3H), 2.05 - 1.95 (m, 1H), 1.93 - 1.84 (m, 2H), 1.78 - 1.66 (m, 1H), 1.39 - 1.26 (m, 2H).ESI-MS(M+H)⁺: 681.3.

The corresponding intermediates were prepared according to the following conditions:

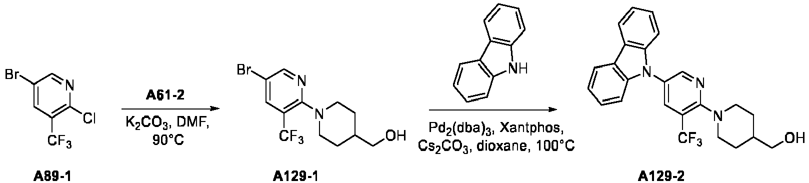

### Synthesis of compound A129-1

A89-1 (30 g, 115.9 mmol), A61-2 (16.0 g, 139.0 mmol) and potassium carbonate (32.0 g, 231.7 mmol) were dissolved in 100 mL of DMF. The mixture was reacted at 90 °C for 2 h, and then TLC showed that the reaction was completed. The reaction liquid was diluted with water (200 ml), and then the aqueous phase was extracted with ethyl acetate (100 mL*3). The organic phases were combined, washed with saturated saline solution (50 ml*3), and dried by rotary evaporation. The crude product was separated and purified by column chromatography (petroleum ether: ethyl acetate = 3 : 1) to obtain compound A129-1 (34.8 g, yield 89%).

### Synthesis of compound A129-2

A129-1 (130 mg, 0.66 mmol), 9*H*-carbazole (300 mg, 0.66 mmol) and cesium carbonate (429 mg, 1.32 mmol) was dissolved in dioxane (8 mL), Pd₂(dba)₃ (60 mg, 0.07 mmol) and Xantphos (38 mg, 0.07 mmol) were added. The mixture was replaced with nitrogen three times and then reacted at 90 °C overnight, and then TLC showed that the reaction was completed. The reaction liquid was filtered, and the filtrate was diluted with water (45 mL), and then extracted with ethyl acetate (40 ml*3). The organic phases were combined and dried by rotary evaporation. The crude product was separated and purified by column chromatography (petroleum ether: ethyl acetate = 10 : 1) to obtain compound A129-2 (170 mg, yield 44%).

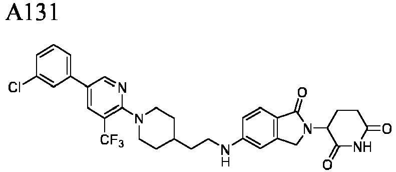

¹H NMR (400 MHz, DMSO) δ 10.93 (s, 1H), 8.83 (d, *J* = 2.1 Hz, 1H), 8.29 (d, *J* = 2.3 Hz, 1H), 7.85 (s, 1H), 7.72 (d, *J* = 7.7 Hz, 1H), 7.51 - 7.47 (m, 1H), 7.44 (d, *J* = 8.2 Hz, 1H), 7.39 (d, *J* = 8.3 Hz, 1H), 6.67 (d, *J* = 8.4 Hz, 1H), 6.64 (s, 1H), 6.37 (t, *J* = 5.1 Hz, 1H), 5.01 (dd, *J* = 13.3, 5.1 Hz, 1H), 4.21 (dd, *J* = 52.0, 16.8 Hz, 2H), 3.70 - 3.56 (m, 2H), 3.14 (dd, *J* = 12.1, 6.3 Hz, 2H), 3.01 - 2.83 (m, 3H), 2.63 - 2.53 (m, 1H), 2.41 - 2.59 (m, 1H), 1.98 - 1.90 (m, 1H), 1.87 - 1.76 (m, 2H), 1.71 - 1.52 (m, 3H), 1.38 - 1.23 (m, 2H).ESI-MS(M+H)⁺: 626.3.

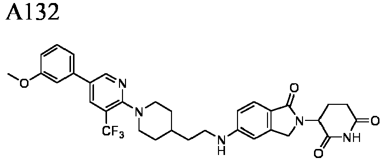

¹H NMR (400 MHz, DMSO) δ 10.93 (s, 1H), 8.82 (d, *J* = 2.2 Hz, 1H), 8.24 (d, *J* = 2.4 Hz, 1H), 7.43 - 7.34 (m, 2H), 7.33 - 7.23 (m, 2H), 6.96 (dd, *J* = 8.2, 1.6 Hz, 1H), 6.70 - 6.60 (m, 2H), 6.38 (t, *J* = 5.2 Hz, 1H), 5.01 (dd, *J* = 13.3, 5.1 Hz, 1H), 4.21 (dd, *J* = 53.4, 16.8 Hz, 2H), 3.83 (s, 3H), 3.66 - 3.56 (m, 2H), 3.14 (dd, *J* = 12.1, 6.5 Hz, 2H), 2.98 - 2.80 (m, 3H), 2.62 - 2.54 (m, 1H), 2.40 - 2.22 (m, 1H), 2.03 - 1.88 (m, 1H), 1.87 - 1.77 (m, 2H), 1.69 - 1.52 (m, 3H), 1.38 - 1.25 (m, 2H).ESI-MS(M+H)⁺: 622.3.

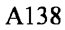

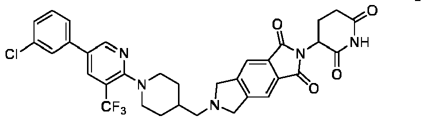

¹H NMR (400 MHz, DMSO) δ 11.13 (s, 1H), 8.84 (d, *J* = 2.3 Hz, 1H), 8.30 (d, *J* = 2.3 Hz, 1H), 7.87 (s, 1H), 7.80 (s, 2H), 7.73 (d, *J* = 7.8 Hz, 1H), 7.52 - 7.48 (m, 1H), 7.45 (d, *J* = 8.5 Hz, 1H), 5.14 (dd, *J* = 12.9, 5.3 Hz, 1H), 3.99 (s, 4H), 3.72 - 3.63 (m, 2H), 3.03 - 2.83 (m, 3H), 2.69 - 2.52 (m, 4H), 2.12 - 2.02 (m, 1H), 1.93 - 1.84 (m, 2H), 1.83 - 1.70 (m, 1H), 1.38 - 1.25 (m, 2H).ESI-MS(M+H)⁺: 652.2.

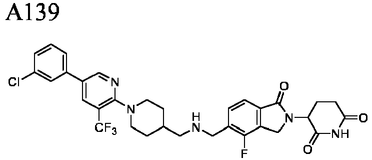

¹H NMR (400 MHz, DMSO) δ 11.00 (s, 1H), 8.83 (d, *J* = 2.1 Hz, 1H), 8.29 (d, *J* = 2.3 Hz, 1H), 7.87 (s, 1H), 7.70 (dd, *J* = 17.3, 7.2 Hz, 2H), 7.59 (d, *J* = 7.7 Hz, 1H), 7.50 (t, *J* = 7.8 Hz, 1H), 7.44 (d, *J* = 8.1 Hz, 1H), 5.12 (dd, *J* = 13.3, 5.1 Hz, 1H), 4.47 (dd, *J* = 69.0, 17.3 Hz, 2H), 3.91 (s, 2H), 3.70 - 3.57 (m, 2H), 2.98 - 2.84 (m, 3H), 2.66 - 2.56 (m, 1H), 2.55 - 2.50 (m, 2H), 2.46 - 2.36 (m, 1H), 2.06 - 1.95 (m, 1H), 1.88 - 1.79 (m, 2H), 1.74 - 1.62 (m, 1H), 1.35 - 1.22 (m, 2H).ESI-MS(M+H)⁺: 644.3.

The corresponding intermediates were prepared according to the following conditions:

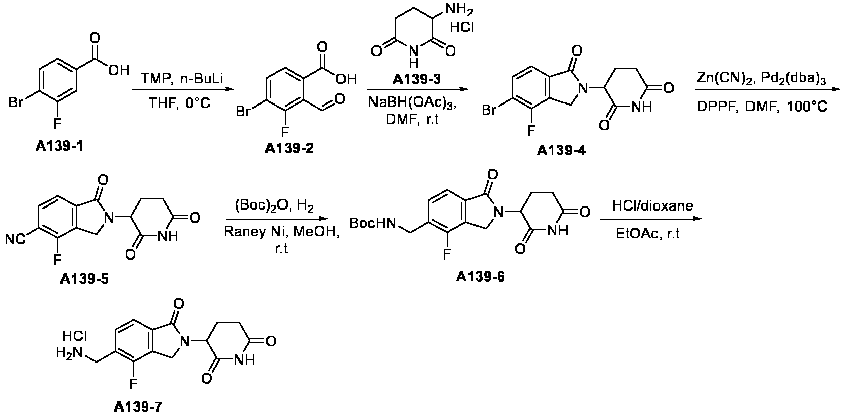

### Synthesis of compound A139-2

2,2,6,6-tetramethylpiperidine (3.53 g, 25.0 mmol) was dissolved in 50 mL of anhydrous tetrahydrofuran, and n-butyllithium (16 mL, 1.5 M, 24 mmol) was added dropwise in an ice bath. The mixture was reacted in an ice bath for 30 min, and a solution of A139-1 (2.19 g, 10.0 mmol) in anhydrous tetrahydrofuran (5 mL) was added dropwise. The mixture was reacted continuously in an ice bath for 3 h, and 5 mL of anhydrous DMF was added. The mixture was reacted overnight at ambient temperature, and then TLC showed that the reaction was completed. The reaction liquid was quenched with 1 M hydrochloric acid (50 mL), and then the aqueous phase was extracted with dichloromethane (100 mL*3). The organic phases were combined, washed with saturated saline solution (50 ml*3), and dried by rotary evaporation to obtain compound A139-2 (1.95 g, yield 79%).

### Synthesis of compound A139-4

A139-2 (1.5 g, 6.0 mmol) and A139-3 (0.99 g, 6.0 mmol) were dissolved in 20 mL of DMF, and sodium triacetoxyborohydride (3.8 g, 18.0 mmol) was added. The mixture was reacted overnight at ambient temperature, and then TLC showed that the reaction was completed. The reaction liquid was poured into 100 mL of water, and the precipitated product was filtered. The filter cake was rinsed with water and dried to obtain compound A139-4 (1.45 g, yield 71%).

### Synthesis of compound A139-5

A139-4 (1.36 g, 4.0 mmol) and zinc cyanide (0.99 g, 6.0 mmol) were dissolved in 20 mL of DMF, and Pd₂(dba)₃ (366 mg, 0.40 mmol) and DPPF (332 mg, 0.60 mmol) were added. The mixture was reacted at 100 °C under nitrogen protection for 3 h, and then TLC showed that the reaction was completed. The reaction liquid was filtered, and the filtrate was diluted with water (60 mL), and then extracted with ethyl acetate (60 mL*3). The organic phases were combined and washed with saturated saline solution (20 mL*3), and then dried by rotary evaporation. The crude product was separated and purified by column chromatography (dichloromethane : methanol = 20 : 1) to obtain compound A139-5 (1.0 g, yield 87%).

### Synthesis of compound A139-6

A139-5 (861 mg, 3.0 mmol) and (Boc)₂O (972 mg, 4.5 mmol) were dissolved in 50 mL of methanol, and Raney Nickel (1 g) was added. The mixture was reacted overnight at ambient temperature under hydrogen atmosphere, and then TLC showed that the reaction was completed. The reaction liquid was filtered, and the filter cake was rinsed with methanol. The filtrate was dried by rotary evaporation. The crude product was separated and purified by column chromatography (dichloromethane : methanol = 20 : 1) to obtain compound A139-6 (550 mg, yield 47%).

### Synthesis of compound A139-7

A139-6 (391 mg, 1.0 mmol) was dissolved in 5 mL of ethyl acetate, and HCl/dioxane (5 mL, 4 M) was added. The mixture was reacted at ambient temperature for 2 h, and then TLC showed that the reaction was completed. The reaction liquid was dried by rotary evaporation to obtain compound A139-7 (400 mg, crude product), which was directly used for next step.

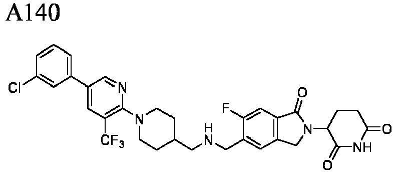

¹H NMR (400 MHz, DMSO) δ 11.02 (s, 1H), 8.84 (d, *J* = 2.0 Hz, 1H), 8.31 (d, *J* = 2.2 Hz, 1H), 7.86 (s, 1H), 7.79 (d, *J* = 6.1 Hz, 1H), 7.72 (d, *J* = 7.6 Hz, 1H), 7.59 (d, *J* = 8.7 Hz, 1H), 7.54 - 7.43 (m, 2H), 6.53 (s, 1H), 5.13 (dd, *J* = 13.3, 5.0 Hz, 1H), 4.41 (dd, *J* = 52.7, 17.4 Hz, 2H), 4.11 (s, 2H), 3.70 - 3.59 (m, 2H), 2.98 - 2.87 (m, 3H), 2.77 (s, 2H), 2.66 - 2.56 (m, 1H), 2.45 - 2.34 (m, 1H), 2.07 - 1.99 (m, 1H), 1.91 - 1.76 (m, 3H), 1.38 - 1.27 (m, 2H).ESI-MS(M+H)⁺: 644.3.

The corresponding intermediates were prepared according to the following conditions:

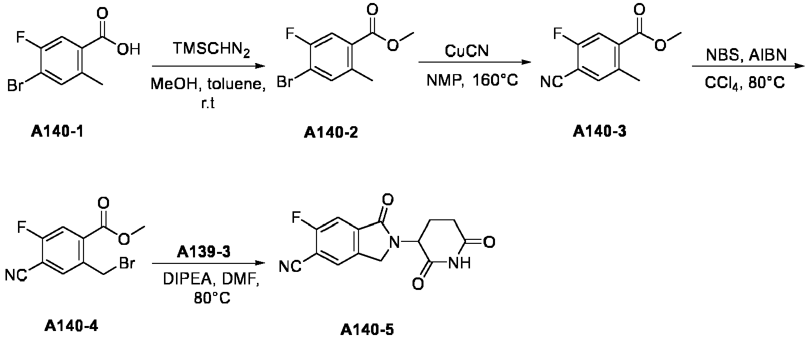

### Synthesis of compound A140-2

A140-1 (5.01 g, 21.5 mmol) was dissolved in a mixed solvent of methanol/toluene (45 mL/10 mL), and trimethylsilyldiazomethane (43 mL, 2 M, 86 mmol) was added dropwise in an ice bath. The mixture was reacted at ambient temperature for 1 h, and then TLC showed that the reaction was completed. The solvent was dried by rotary evaporation, and the crude product was separated and purified by column chromatography (petroleum ether : ethyl acetate = 15 : 1) to obtain compound A140-2 (3.48 g, yield 66%).

### Synthesis of compound A140-3

A140-2 (3.1 g, 12.6 mmol) and CuCN (1.7 g, 18.9 mmol) were dissolved in 30 mL of A-methylpyrrolidone. The mixture was reacted at 100 °C under nitrogen protection for 6 h, and then TLC showed that the reaction was completed. The reaction liquid was filtered, and the filtrate was diluted with water (60 mL), and then extracted with ethyl acetate (40 ml*3). The organic phases were combined, washed with saturated saline solution (40 ml*3) and then dried by rotary evaporation. The crude product was separated and purified by column chromatography (petroleum ether: ethyl acetate = 10 : 1) to obtain compound A140-3 (1.35 g, yield 55%).

### Synthesis of compound A140-4

A140-3 (906 mg, 4.66 mol) was dissolved in 15 mL of carbon tetrachloride, and NBS (913 mg, 5.13 mmol) and AIBN (382 mg, 2.33 mmol) were added. The mixture was reacted overnight at 80 °C, and then TLC showed that the reaction was completed. The reaction liquid was diluted with water (20 mL), and extracted with dichloromethane (25 ml*3). The organic phases were combined, dried by rotary evaporation. The crude product was separated and purified by column chromatography (petroleum ether : ethyl acetate = 10 : 1) to obtain compound A140-4 (680 mg, yield 54%).

### Synthesis of compound A140-5

Compounds A140-5 (552 mg, 3.37 mmol), A139-3 (830 mg, 3.06 mol) and DIPEA (1304 mg, 10.1 mmol) were dissolved in 10 mL of DMF. The mixture was reacted overnight at 80 °C, and then TLC showed that the reaction was completed. The reaction liquid was diluted with water (20 mL), and then extracted with ethyl acetate (25 ml*3). The organic phases were combined and washed with saturated saline solution (20 ml*3) and then dried by rotary evaporation. The crude product was separated and purified by column chromatography (dichloromethane : methanol = 20 : 1) to obtain compound A140-5 (360 mg, yield 41%).

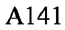

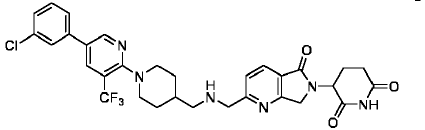

¹H NMR (400 MHz, DMSO) δ 11.01 (s, 1H), 8.82 (d, *J* = 2.2 Hz, 1H), 8.29 (d, *J* = 2.3 Hz, 1H), 8.13 (d, *J* = 7.9 Hz, 1H), 7.85 (s, 1H), 7.72 (d, *J* = 7.8 Hz, 1H), 7.64 (d, *J* = 7.9 Hz, 1H), 7.53 - 7.46 (m, 1H), 7.44 (d, *J* = 8.0 Hz, 1H), 5.17 (dd, *J* = 13.3, 5.1 Hz, 1H), 4.42 (dd, *J* = 65.8, 17.7 Hz, 2H), 3.98 (s, 2H), 3.69 - 3.59 (m, 2H), 2.98 - 2.85 (m, 3H), 2.68 - 2.55 (m, 1H), 2.54 - 2.52 (m, 2H), 2.46 - 2.38 (m, 1H), 2.08 - 1.95 (m, 1H), 1.89 - 1.80 (m, 2H), 1.74 - 1.63 (m, 1H), 1.34 - 1.22 (m, 2H).ESI-MS(M+H)⁺: 627.3.

The corresponding intermediates were prepared according to the following conditions:

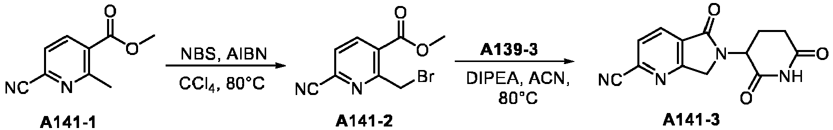

### Synthesis of compound A141-2

A141-1 (220 mg, 1.25 mol) was dissolved in 15 mL of carbon tetrachloride, and NBS (267 mg, 1.5 mmol) and AIBN (62 mg, 0.38 mmol) were added. The mixture was reacted overnight at 80 °C, and then TLC showed that the reaction was completed. The reaction liquid was diluted with water (30 mL), and extracted with dichloromethane (30 ml*3). The organic phases were combined, dried by rotary evaporation. The crude product was separated and purified by column chromatography (petroleum ether : ethyl acetate = 3 : 1) to obtain compound A141-2 (170 mg, yield 54%).

### Synthesis of compound A141-3

Compounds A141-2 (170 mg, 0.67 mmol), A139-3 (131 mg, 0.80 mol) and DIPEA (338 mg, 3.35 mmol) were dissolved in 10 mL of DMF. The mixture was reacted overnight at 80 °C, and then TLC showed that the reaction was completed. The reaction liquid was diluted with water (20 mL), and then extracted with ethyl acetate (25 ml*3). The organic phases were combined, washed with saturated saline solution (20 ml*3) and then dried by rotary evaporation. The crude product was separated and purified by column chromatography (ethyl acetate : petroleum ether = 2 : 1) to obtain compound A141-3 (100 mg, yield 56%).

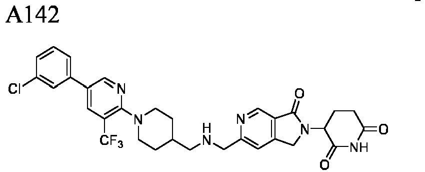

¹H NMR (400 MHz, DMSO) δ 11.04 (s, 1H), 9.03 (s, 1H), 8.85 (d, *J* = 2.2 Hz, 1H), 8.32 (d, *J* = 2.3 Hz, 1H), 7.86 (s, 1H), 7.84 (s, 1H), 7.73 (d, *J* = 7.6 Hz, 1H), 7.55 - 7.43 (m, 2H), 5.15 (dd, *J* = 13.3, 5.1 Hz, 1H), 4.68 - 4.42 (m, 4H), 3.69 - 3.58 (m, 2H), 3.02 - 2.83 (m, 5H), 2.67 - 2.56 (m, 1H), 2.47 - 2.32 (m, 1H), 2.09 - 1.97 (m, 2H), 1.94 - 1.85 (m, 2H), 1.43 - 1.28 (m, 2H).ESI-MS(M+H)⁺: 627.3.

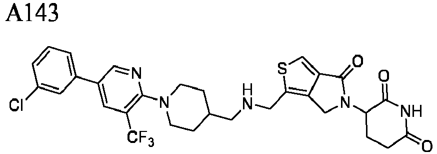

¹H NMR (400 MHz, DMSO) δ 10.98 (s, 1H), 8.82 (d, *J* = 1.9 Hz, 1H), 8.29 (d, *J* = 2.2 Hz, 1H), 7.85 (s, 2H), 7.71 (d, *J* = 7.7 Hz, 1H), 7.49 (t, *J* = 7.8 Hz, 1H), 7.44 (d, *J* = 8.0 Hz, 1H), 5.02 (dd, *J* = 13.2, 5.0 Hz, 1H), 4.23 (dd, *J* = 56.4, 15.4 Hz, 2H), 3.86 (s, 2H), 3.69 - 3.56 (m, 2H), 3.00 - 2.81 (m, 3H), 2.64 - 2.53 (m, 1H), 2.47 (d, *J* = 6.5 Hz, 2H), 2.41 - 2.25 (m, 1H), 2.02 - 1.92 (m, 1H), 1.88 - 1.77 (m, 2H), 1.69 - 1.56 (m, 1H), 1.34 - 1.18 (m, 2H).ESI-MS(M+H)⁺: 632.3.

The corresponding intermediates were prepared according to the following conditions:

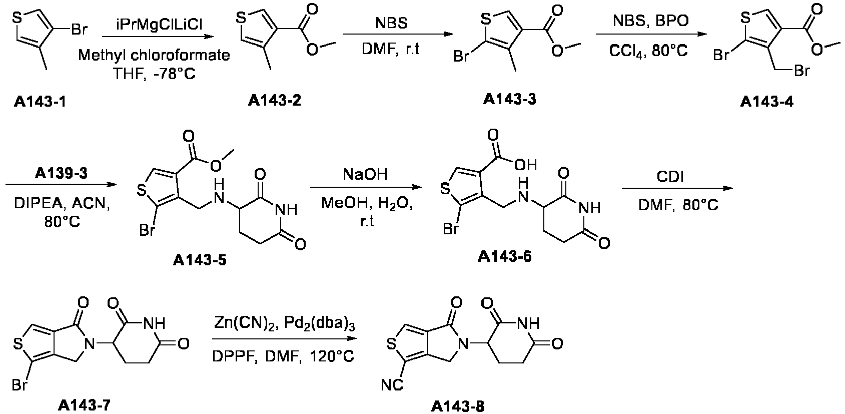

### Synthesis of compound A143-2

A143-1 (10 g, 56.5 mmol) was dissolved in 80 mL of anhydrous tetrahydrofuran, and isopropyl magnesium chloride lithium chloride complex (65 mL, 1.3 M, 84.7 mmol) was added dropwise at -78 °C. The mixture was reacted at ambient temperature overnight, and then cooled to -78 °C. Methyl chloroformate (6.6 mL, 84.7 mmol) was added dropwise at this temperature. After completing, the mixture was warmed to ambient temperature and reacted continuously for another 3 h, and then TLC showed that the reaction was completed. 200 mL of saturated sodium bicarbonate solution was added to quench the reaction. The aqueous phase was extracted with ethyl acetate (80 ml*3). The organic phases were combined and dried by rotary evaporation. The crude product was separated and purified by column chromatography (petroleum ether : ethyl acetate = 10 : 1) to obtain compound A143-2 (7.6 g, yield 86%).

### Synthesis of compound A143-3

A143-2 (7.6 g, 48.6 mmol) was dissolved in 30 mL of DMF, and NBS (9.1 g, 51.0 mmol) was added. The mixture was reacted overnight at ambient temperature, and then TLC showed that the reaction was completed. 60 mL of water was added to quench the reaction. The aqueous phase was extracted with ethyl acetate (60 ml*3). The organic phases were combined, washed with saturated saline solution (20 ml*3), and dried by rotary evaporation. The crude product was separated and purified by column chromatography (petroleum ether : ethyl acetate = 10 : 1) to obtain compound A143-3 (9.3 g, yield 81%).

### Synthesis of compound A143-4

A143-3 (9.3 g, 39.6 mol) was dissolved in 80 mL of carbon tetrachloride, and NBS (7.75 g, 43.5 mmol) and dibenzoyl peroxide (960 mg, 3.9 mmol) were added. The mixture was reacted overnight at 80 °C, and then TLC showed that the reaction was completed. The reaction liquid was diluted by adding water (60 mL), and extracted with dichloromethane (60 ml*3). The organic phases were combined, dried by rotary evaporation. The crude product was separated and purified by column chromatography (petroleum ether : ethyl acetate = 10 : 1) to obtain compound A143-4 (10 g, yield 80%).

### Synthesis of compound A143-5

Compounds A143-4 (10 g, 31.8 mmol), A139-3 (5.8 g, 35 mol) and DIPEA (12.4 g, 95.5 mmol) were dissolved in 80 mL of acetonitrile. The mixture was reacted at 80 °C for 3 h, and then TLC showed that the reaction was completed. The reaction liquid was directly dried by rotary evaporation. The crude product was separated and purified by column chromatography (ethyl acetate : petroleum ether = 1 : 1) to obtain compound A143-5 (6 g, yield 52%).

### Synthesis of compound A143-6

Compound A143-5 (3.5 g, 9.7 mmol) was dissolved in methanol/water (36 mL/12 mL). Sodium hydroxide (1.2 g, 29 mmol) was added in an ice bath. The mixture was reacted at room temperature for 3 h, and then TLC showed that the reaction was completed. The reaction liquid was adjusted to pH = 4 with 2 M hydrochloric acid, the aqueous phase was extracted with dichloromethane (30 ml*3). The organic phases were combined and washed with saturated saline solution (20 mL*3), and then dried by rotary evaporation. The crude product was separated and purified by column chromatography (dichloromethane : methanol = 10 : 1) to obtain compound A143-6 (1.5 g, yield 42%).

### Synthesis of compound A143-7

Compound A143-6 (1.5 g, 4.1 mmol) was dissolved in 60 mL of DMF, and *N,N*'-carbonyldiimidazole (2.7 g, 16.4 mmol) was added. The mixture was reacted overnight at 80 °C, and then TLC showed that the reaction was completed. Water (60 mL) was added to quench the reaction. The aqueous phase was extracted with ethyl acetate (60 mL*3). The combined organic phases were washed with saturated saline solution (20 mL*3), dried and concentrated. The crude product was separated by column chromatography (dichloromethane : methanol = 20 : 1) to obtain compound A143-7 (410 mg, yield 30%).

### Synthesis of compound A143-8

A143-7 (410 mg, 1.3 mmol) and zinc cyanide (290 mg, 2.5 mmol) were dissolved in 20 mL of DMF, and Pd₂(dba)₃ (180 mg, 0.20 mmol) and DPPF (200 mg, 0.40 mmol) were added. The mixture was reacted overnight at 120 °C under nitrogen protection, and then TLC showed that the reaction was completed. The reaction liquid was filtered, and the filtrate was diluted with water (60 mL), and then extracted with ethyl acetate (60 mL*3). The organic phases were combined and washed with saturated saline solution (20 mL*3), and then dried by rotary evaporation. The crude product was separated and purified by column chromatography (dichloromethane : methanol = 20 : 1) to obtain compound A143-8 (340 mg, yield 99%).

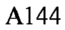

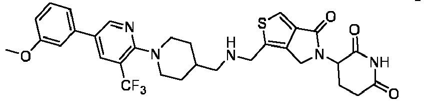

¹H NMR (400 MHz, DMSO) δ 10.98 (s, 1H), 8.81 (d, *J* = 2.1 Hz, 1H), 8.23 (d, *J* = 2.3 Hz, 1H), 7.86 (s, 1H), 7.39 (t, *J* = 7.9 Hz, 1H), 7.30 - 7.27 (m, 2H), 6.96 (dd, *J* = 8.2, 1.8 Hz, 1H), 5.02 (dd, *J* = 13.3, 5.0 Hz, 1H), 4.24 (dd, *J* = 56.1, 15.4 Hz, 2H), 3.88 (s, 2H), 3.83 (s, 3H), 3.64 - 3.55 (m, 2H), 2.96 - 2.83 (m, 3H), 2.62 - 2.53 (m, 1H), 2.47 (d, J = 6.5 Hz, 2H), 2.40 - 2.26 (m, 1H), 2.03 - 1.92 (m, 1H), 1.88 - 1.77 (m, 2H), 1.69 - 1.56 (m, 1H), 1.34 - 1.23 (m, 2H).ESI-MS(M+H)⁺: 628.3.

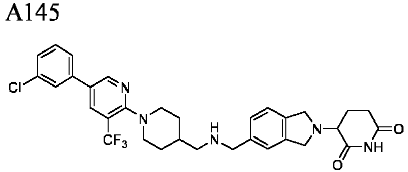

¹H NMR (400 MHz, DMSO) δ 10.73 (s, 1H), 8.82 (d, *J* = 2.3 Hz, 1H), 8.29 (d, *J* = 2.4 Hz, 1H), 7.86 (t, *J* = 1.8 Hz, 1H), 7.75 - 7.67 (m, 1H), 7.53 - 7.46 (m, 1H), 7.46 - 7.40 (m, 1H), 7.20 (s, 1H), 7.16 (s, 2H), 4.13 - 3.96 (m, 4H), 3.70 - 3.56 (m, 5H), 2.91 (t, *J* = 11.6 Hz, 2H), 2.57 (t, *J* = 6.4 Hz, 2H), 2.39 (d, *J* = 6.6 Hz, 2H), 2.13 - 1.95 (m, 2H), 1.87 - 1.77 (m, 2H), 1.68 - 1.54 (m, 1H), 1.32 - 1.18 (m, 2H).ESI-MS(M+H)⁺: 612.3.

The corresponding intermediates were prepared according to the following conditions:

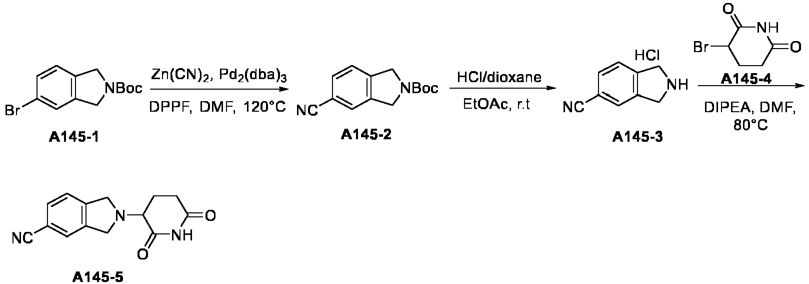

### Synthesis of compound A145-2

A145-1 (1 g, 3.4 mmol) and zinc cyanide (790 mg, 6.7 mmol) were dissolved in 20 mL of DMF, and Pd₂(dba)₃ (450 mg, 0.50 mmol) and DPPF (550 mg, 1 mmol) were added. The mixture was reacted overnight at 120 °C under nitrogen protection, and then TLC showed that the reaction was completed. The reaction liquid was filtered, and the filtrate was diluted with water (60 mL), and then extracted with ethyl acetate (60 mL*3). The organic phases were combined and washed with saturated saline solution (20 mL*3), and then dried by rotary evaporation. The crude product was separated and purified by column chromatography (petroleum ether: ethyl acetate = 5 : 1) to obtain compound A145-2 (800 mg, yield 97%).

### Synthesis of compound A145-3

Compound A145-2 (800 mg, 3.3 mmol) was dissolved in 5 mL of ethyl acetate, and HCl/dioxane (5 mL, 4 M) was added. The mixture was reacted at ambient temperature for 2 h, and then TLC showed that the reaction was completed. The reaction liquid was dried by rotary evaporation to obtain compound A145-3 (780 mg, crude product), which was directly used for next step.

### Synthesis of compound A145-5

Compound A145-3 (780 mg, 2.9 mmol) and compound A145-4 (660 mg, 3.5 mmol) were dissolved in 20 mL of DMF, and DIPEA (1.1 g, 8.6 mmol) was added. The mixture was reacted overnight at 80 °C, and then TLC showed that the reaction was completed. Water (60 mL) was added to quench the reaction. The aqueous phase was extracted with ethyl acetate (60 mL*3). The combined organic phases were washed with saturated saline solution (20 mL*3), dried and concentrated. The crude product was separated by column chromatography (dichloromethane : methanol = 20 : 1) to obtain compound A145-5 (750 mg, yield 99%).

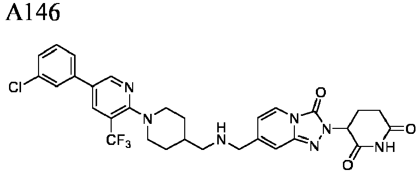

¹H NMR (400 MHz, DMSO) δ 11.09 (s, 1H), 8.83 (d, *J* = 2.2 Hz, 1H), 8.29 (d, *J* = 2.4 Hz, 1H), 7.86 - 7.84 (m, 2H), 7.72 (d, *J* = 7.7 Hz, 1H), 7.52 - 7.43 (m, 2H), 7.10 (s, 1H), 6.66 (dd, *J* = 7.3, 1.0 Hz, 1H), 5.35 (dd, *J* = 12.6, 5.2 Hz, 1H), 3.70 - 3.57 (m, 4H), 2.99 - 2.88 (m, 3H), 2.69 - 2.51 (m, 2H), 2.41 (d, *J* = 6.3 Hz, 2H), 2.21 - 2.12 (m, 1H), 1.89 - 1.79 (m, 2H), 1.70 - 1.59 (m, 1H), 1.33 - 1.24 (m, 2H).ESI-MS(M+H)⁺: 628.2.

The corresponding intermediates were prepared according to the following conditions:

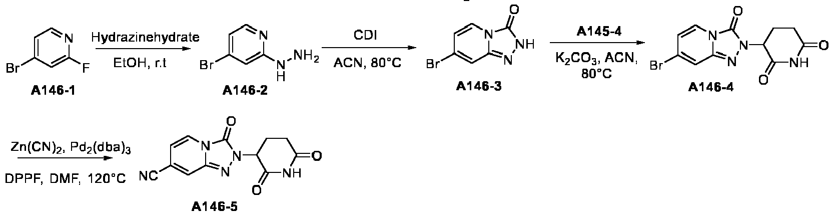

### Synthesis of compound A146-2

A146-1 (2.01 g, 11.49 mmol) was dissolved in 10 mL of ethanol, and hydrazine hydrate (80%, 10 mL) was added in an ice bath. The mixture was reacted overnight at ambient temperature, and then TLC showed that the reaction was completed. The reaction liquid was filtered. The filter cake was rinsed with a small amount of ethanol and dried to obtain compound A146-2 (2.04 g, yield 95%).

### Synthesis of compound A146-3

A146-2 (2.04 g, 10.91 mmol) was dissolved in 20 mL of acetonitrile, and *N,N*'-carbonyldiimidazole (2.65 g, 16.36 mmol) was added. The mixture was reacted at 80 °C for 2 h, and then TLC showed that the reaction was completed. The reaction liquid was cooled to room temperature, and the precipitated solid was filtered. The filter cake was rinsed with a small amount of acetonitrile and dried to obtain compound A146-3 (2.3 g, yield 99%).

### Synthesis of compound A146-4

Compound A146-3 (1.3 g, 6.10 mmol) and compound A145-4 (2.91 g, 15.3 mmol) were dissolved in 15 mL of acetonitrile, and potassium carbonate (1.68 g, 12.2 mmol) was added. The mixture was reacted overnight at 80 °C, and then TLC showed that the reaction was completed. Water (40 mL) was added to quench the reaction. The aqueous phase was extracted with ethyl acetate (40 mL*3). The combined organic phases were washed with saturated saline solution (20 mL*3), dried and concentrated. The crude product was separated by column chromatography (dichloromethane : methanol = 20 : 1) to obtain compound A146-4 (450 mg, yield 23%).

### Synthesis of compound A146-5

A146-4 (450 mg, 1.39 mmol) and zinc cyanide (163 mg, 1.39 mmol) were dissolved in 5 mL of DMF, and Pd₂(dba)₃ (127 mg, 0.14 mmol) and DPPF (154 mg, 0.28 mmol) were added. The mixture was reacted overnight at 120 °C under nitrogen protection, and then TLC showed that the reaction was completed. The reaction liquid was filtered. The filtrate was diluted with water (60 mL), and then extracted with ethyl acetate (60 mL*3). The organic phases were combined and washed with saturated saline solution (20 mL*3), and then dried by rotary evaporation. The crude product was separated and purified by column chromatography (dichloromethane : methanol = 20 : 1) to obtain compound A146-5 (290 mg, yield 64%).

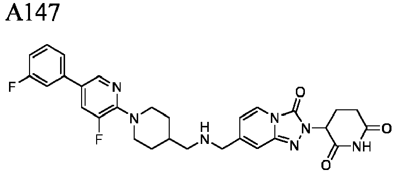

¹H NMR (400 MHz, DMSO) δ 11.09 (s, 1H), 8.40 (s, 1H), 7.90 (dd, *J* = 15.0, 1.9 Hz, 1H), 7.85 (d, *J* = 7.2 Hz, 1H), 7.58 - 7.53 (m, 2H), 7.47 (dd, *J* = 14.3, 8.1 Hz, 1H), 7.20 - 7.11 (m, 1H), 7.09 (s, 1H), 6.65 (d, *J* = 7.3 Hz, 1H), 5.35 (dd, *J* = 12.6, 5.2 Hz, 1H), 4.11 - 4.03 (m, 2H), 3.63 (s, 2H), 2.99 - 2.81 (m, 3H), 2.71 - 2.59 (m, 1H), 2.57 - 2.45 (m, 1H), 2.39 (d, *J* = 6.5 Hz, 2H), 2.21 - 2.11 (m, 1H), 1.86 - 1.77 (m, 2H), 1.74 - 1.63 (m, 1H), 1.31 - 1.20 (m, 2H).ESI-MS(M+H)⁺: 562.3.

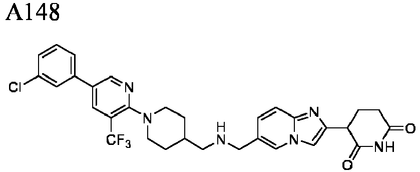

¹H NMR (400 MHz, DMSO) δ 10.86 (s, 1H), 8.85 (d, *J* = 2.3 Hz, 1H), 8.43 (s, 1H), 8.31 (d, *J* = 2.4 Hz, 1H), 7.89 - 7.80 (m, 1H), 7.84 (s, 1H), 7.78 - 7.72 (m, 1H), 7.56 - 7.45 (m, 3H), 7.27 (dd, *J* = 9.3, 1.5 Hz, 1H), 4.02 (t, *J* = 6.6 Hz, 1H), 3.74 - 3.60 (m, 4H), 2.94 (t, *J* = 11.7 Hz, 2H), 2.72 - 2.55 (m, 2H), 2.44 (d, *J* = 6.5 Hz, 2H), 2.25 (q, *J* = 6.6 Hz, 2H), 1.91 - 1.81 (m, 2H), 1.74 - 1.70 (m, 1H), 1.34 - 1.27 (m, 2H).ESI-MS(M+H)⁺: 611.3.

The corresponding intermediates were prepared according to the following conditions:

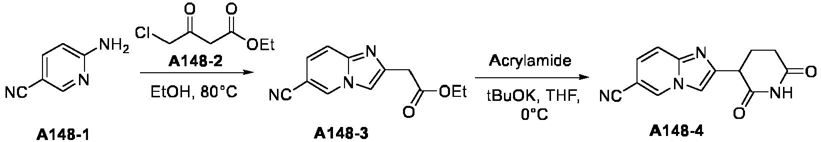

### Synthesis of compound A148-3

A148-1 (3.0 g, 25.2 mmol) was dissolved in 40 mL of ethanol, and A148-2 (10.4 g, 63.0 mmol) was added. The mixture was reacted overnight at 80 °C, and then TLC showed that the reaction was completed. The reaction liquid was filtered. The filter cake was rinsed with a small amount of ethanol and dried to obtain compound A148-3 (2.4 g, yield 41%).

### Synthesis of compound A148-4

A148-3 (1.0 g, 4.4 mmol) and potassium tert-butoxide (490 mg, 4.4 mmol) were dissolved in 20 mL of anhydrous tetrahydrofuran, and acrylamide (310 mg, 4.4 mmol) was added in an ice bath. The mixture was reacted for 2 h in an ice bath, and then TLC showed that the reaction was completed. 30 mL of saturated ammonium chloride solution was added to quench the reaction. The aqueous phase was extracted with ethyl acetate (60 ml*3). The organic phases were combined and dried by rotary evaporation. The crude product was slurried in 10 mL of dichloromethane to obtain compound A148-4 (450 mg, yield 40%).

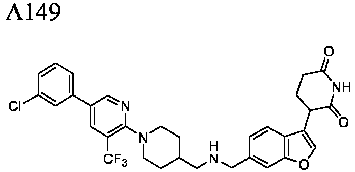

¹H NMR (400 MHz, DMSO) δ 10.90 (s, 1H), 8.82 (d, *J* = 2.0 Hz, 1H), 8.29 (d, *J* = 2.2 Hz, 1H), 7.87 - 7.82 (m, 2H), 7.72 (d, *J* = 7.6 Hz, 1H), 7.57 - 7.42 (m, 4H), 7.22 (d, *J* = 7.9 Hz, 1H), 4.12 (dd, *J* = 11.9, 4.8 Hz, 1H), 3.81 (s, 2H), 3.70 - 3.59 (m, 2H), 2.92 (t, *J* = 11.8 Hz, 2H), 2.77 - 2.67 (m, 1H), 2.61 - 2.53 (m, 1H), 2.42 (d, *J* = 6.6 Hz, 2H), 2.37 - 2.26 (m, 1H), 2.16 - 2.07 (m, 1H), 1.86 - 1.78 (m, 2H), 1.71 - 1.58 (m, 1H), 1.32 - 1.24 (m, 2H).ESI-MS(M+H)⁺: 611.3.

The corresponding intermediates were prepared according to the following conditions:

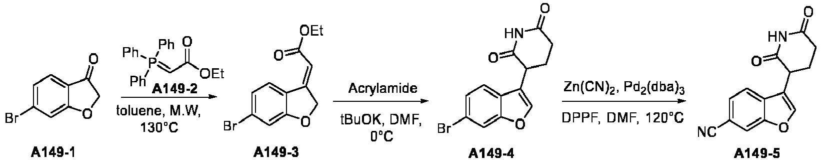

### Synthesis of compound A149-3

A149-1 (848 mg, 4.0 mmol) and A149-2 (8.36 g, 24.0 mmol) were dissolved in 60 mL of toluene. The mixture was reacted at 130 °C for 2 h under microwave irradiation, and then TLC showed that the reaction was completed. Water (50 mL) was added to quench the reaction. The aqueous phase was extracted with ethyl acetate (40 mL*3). The organic phases were combined, dried and concentrated. The crude product was separated by column chromatography (petroleum ether : ethyl acetate = 15 : 1) to obtain compound A149-3 (730 mg, yield 65%).

### Synthesis of compound A149-4

A149-3 (400 mg, 1.42 mmol) and potassium tert-butoxide (175 mg, 1.56 mmol) were dissolved in 5 mL of anhydrous DMF, and acrylamide (110 mg, 1.56 mmol) was added in an ice bath. The mixture was reacted for 2 h in an ice bath, and then TLC showed that the reaction was completed. 10 mL of saturated ammonium chloride solution was added to quench the reaction. The aqueous phase was extracted with ethyl acetate (60 ml*3). The organic phases were combined, washed with saturated saline solution (20 ml*3), and dried by rotary evaporation. The crude product was separated by column chromatography (dichloromethane : methanol = 20 : 1) to obtain compound A149-4 (200 mg, yield 46%).

### Synthesis of compound A149-5

A149-4 (200 mg, 0.65 mmol) and zinc cyanide (76 mg, 0.65 mmol) were dissolved in 5 mL of DMF, and Pd₂(dba)₃ (59 mg, 0.065 mmol) and DPPF (72 mg, 0.13 mmol) were added. The mixture was reacted overnight at 120 °C under nitrogen protection, and then TLC showed that the reaction was completed. The reaction liquid was filtered. The filtrate was diluted with water (60 mL), and then extracted with ethyl acetate (30 mL*3). The organic phases were combined and washed with saturated saline solution (10 mL*3), and then dried by rotary evaporation. The crude product was separated and purified by column chromatography (dichloromethane : methanol = 10 : 1) to obtain compound A149-5 (86 mg, yield 52%).

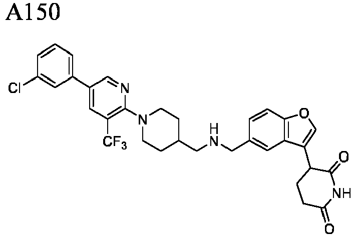

¹H NMR (400 MHz, DMSO) δ 10.94 (s, 1H), 8.83 (d, *J* = 2.1 Hz, 1H), 8.31 (d, *J* = 2.3 Hz, 1H), 7.96 (s, 1H), 7.86 (s, 1H), 7.75 - 7.67 (m, 2H), 7.63 (d, *J* = 8.5 Hz, 1H), 7.53 - 7.42 (m, 3H), 4.20 - 4.05 (m, 3H), 3.68 - 3.58 (m, 2H), 2.92 (t, *J* = 11.9 Hz, 2H), 2.84 - 2.69 (m, 3H), 2.65 - 2.55 (m, 1H), 2.41 - 2.26 (m, 1H), 2.18 - 2.09 (m, 1H), 1.91 - 1.80 (m, 3H), 1.40 - 1.27 (m, 2H).ESI-MS(M+H)⁺: 611.3.

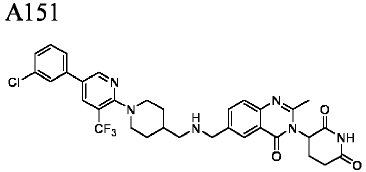

¹H NMR (400 MHz, DMSO) δ 11.05 (s, 1H), 8.83 (d, *J* = 2.3 Hz, 1H), 8.31 (d, *J=* 2.4 Hz, 1H), 8.14 (s, 1H), 7.89 (d, *J* = 8.4 Hz, 1H), 7.86 (t, *J* = 1.8 Hz, 1H), 7.76 - 7.68 (m, 1H), 7.64 (d, *J* = 8.3 Hz, 1H), 7.52 - 7.41 (m, 2H), 5.29 (dd, *J* = 11.5, 5.6 Hz, 1H), 4.11 (s, 2H), 3.67 - 3.57 (m, 2H), 2.98 - 2.81 (m, 3H), 2.77 - 2.56 (m, 7H), 2.23 - 2.13 (m, 1H), 1.91 - 1.75 (m, 3H), 1.38 - 1.25 (m, 2H).ESI-MS(M+H)⁺: 653.3.

The corresponding intermediates were prepared according to the following conditions:

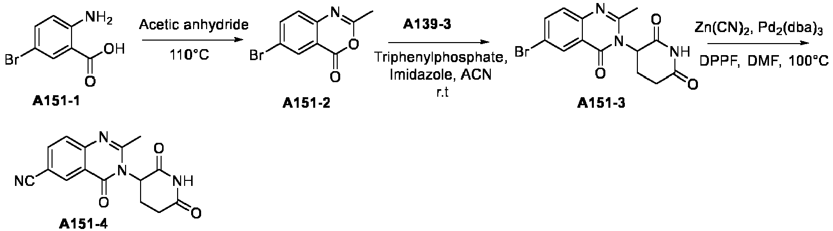

### Synthesis of compound A151-2

A151-1 (2.16 g, 10.0 mmol) was dissolved in 10 mL of acetic anhydride. The mixture was reacted overnight at 110 °C, and then TLC showed that the reaction was completed. Ice water (50 mL) was added to quench the reaction, and the precipitated solid was filtered. The filter cake was rinsed with a small amount of petroleum ether and dried to obtain compound A151-2 (2.05 g, yield 85%).

### Synthesis of compound A151-3

Compounds A151-2 (1.44 g, 6.0 mmol), A139-3 (990 mg, 6.0 mol) and imidazole (612 mg, 9.0 mmol) were dissolved in 20 mL of acetonitrile, and triphenyl phosphate (2.93 g, 6.0 mmol) was added. The mixture was reacted overnight at ambient temperature, and then TLC showed that the reaction was completed. The reaction liquid was diluted with water (20 mL), and then extracted with ethyl acetate (25 ml*3). The organic phases were combined, washed with saturated saline solution (20 ml*3) and then dried by rotary evaporation. The crude product was separated and purified by column chromatography (dichloromethane : methanol = 20 : 1) to obtain compound A151-3 (950 mg, yield 45%).

### Synthesis of compound A151-4

A151-3 (700 mg, 2.0 mmol) and zinc cyanide (234 mg, 2.0 mmol) were dissolved in 20 mL of DMF, and Pd₂(dba)₃ (234 mg, 0.2 mmol) and DPPF (166 mg, 0.3 mmol) were added. The mixture was reacted overnight at 100 °C under nitrogen protection, and then TLC showed that the reaction was completed. The reaction liquid was filtered, and the filtrate was diluted with water (60 mL), and then extracted with ethyl acetate (30 mL*3). The organic phases were combined and washed with saturated saline solution (10 mL*3), and then dried by rotary evaporation. The crude product was separated and purified by column chromatography (dichloromethane : methanol = 20 : 1) to obtain compound A151-4 (350 mg, yield 59%).

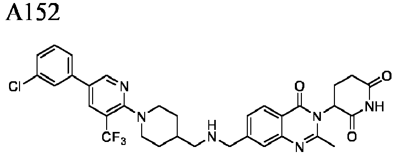

¹H NMR (400 MHz, DMSO) δ 11.04 (s, 1H), 8.83 (d, *J* = 2.1 Hz, 1H), 8.30 (d, *J=* 2.3 Hz, 1H), 8.01 (d, *J* = 8.2 Hz, 1H), 7.86 (s, 1H), 7.72 (d, *J* = 7.7 Hz, 1H), 7.68 (s, 1H), 7.54 (d, *J* = 8.1 Hz, 1H), 7.50 (t, *J* = 7.8 Hz, 1H), 7.45 (d, *J* = 8.1 Hz, 1H), 5.27 (dd, *J* = 11.6, 5.6 Hz, 1H), 4.03 (s, 2H), 3.70 - 3.58 (m, 2H), 2.92 (t, *J* = 11.9 Hz, 2H), 2.88 - 2.79 (m, 1H), 2.71 - 2.54 (m, 7H), 2.23 - 2.09 (m, 1H), 1.90 - 1.81 (m, 2H), 1.80 - 1.70 (m, 1H), 1.36 - 1.24 (m, 2H).ESI-MS(M+H)⁺: 653.3.

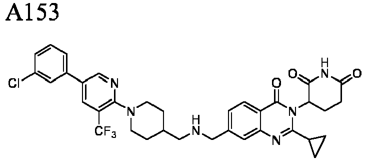

¹H NMR (400 MHz, DMSO) δ 11.07 (s, 1H), 8.85 (d, *J* = 2.1 Hz, 1H), 8.32 (d, *J* = 2.3 Hz, 1H), 8.07 (d, *J* = 8.1 Hz, 1H), 7.87 (s, 1H), 7.81 (s, 1H), 7.73 (d, *J* = 7.6 Hz, 1H), 7.62 (d, *J* = 8.4 Hz, 1H), 7.53 - 7.44 (m, 2H), 5.75 (dd, *J* = 11.5, 5.7 Hz, 1H), 4.32 (s, 2H), 3.70 - 3.56 (m, 2H), 3.01 - 2.82 (m, 5H), 2.75 - 2.58 (m, 2H), 2.36 - 2.28 (m, 1H), 2.24 - 2.11 (m, 1H), 2.04 - 1.93 (m, 1H), 1.92 - 1.80 (m, 2H), 1.42 - 1.31 (m, 2H), 1.30 - 1.24 (m, 2H), 1.11 - 1.05 (m, 1H), 1.03 - 0.97 (m, 1H).ESI-MS(M+H)⁺: 678.3.

The corresponding intermediates were prepared according to the following conditions:

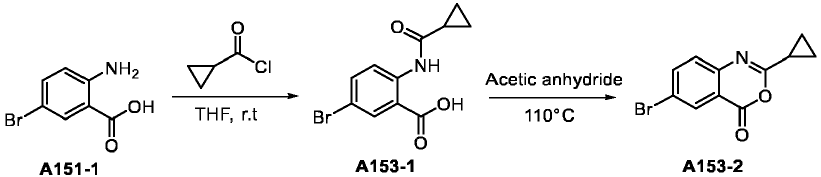

### Synthesis of compound A153-1

A151-1 (1.0 g, 4.63 mmol) was dissolved in 20 mL of tetrahydrofuran, and cyclopropyl formyl chloride (532 mg, 5.10 mmol) was added dropwise. The mixture was reacted overnight at ambient temperature, and then TLC showed that the reaction was completed. The reaction liquid was dried by rotary evaporation, and the crude product was slurried in 10 mL of dichloromethane and filtered to obtain compound A153-1 (1.2 g, yield 92%).

### Synthesis of compound A153-2

A153-1 (1.2 g, 4.23 mmol) was dissolved in 10 mL of acetic anhydride. The mixture was reacted at 110 °C for 1 h, and then TLC showed that the reaction was completed. Ice water (50 mL) was added to quench the reaction, and the precipitated solid was filtered. The filter cake was rinsed with a small amount of petroleum ether and dried to obtain compound A153-2 (800 mg, yield 72%).

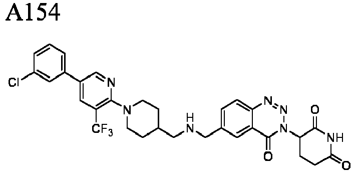

¹H NMR (400 MHz, DMSO) δ 11.20 (s, 1H), 8.83 (d, *J* = 2.3 Hz, 1H), 8.32 - 8.25 (m, 2H), 8.22 (d, *J* = 8.3 Hz, 1H), 8.12 (d, *J* = 8.4 Hz, 1H), 7.86 (d, *J* = 1.8 Hz, 1H), 7.72 (d, *J* = 7.8 Hz, 1H), 7.53 - 7.42 (m, 2H), 5.99 (dd, *J* = 12.5, 5.3 Hz, 1H), 3.99 (s, 2H), 3.68 - 3.59 (m, 2H), 3.02 - 2.86 (m, 3H), 2.76 - 2.62 (m, 2H), 2.49 - 2.43 (m, 2H), 2.34 -2.22 (m, 1H), 1.89 - 1.80 (m, 2H), 1.75 - 1.62 (m, 1H), 1.34 - 1.25 (m, 2H).ESI-MS(M+H)⁺: 640.3.

The corresponding intermediates were prepared according to the following conditions:

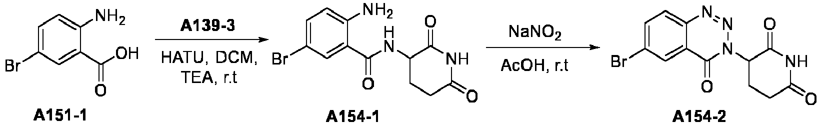

### Synthesis of compound A154-1

A151-1 (216 mg, 1.0 mmol), HATU (456 mg, 1.2 mmol) and triethylamine (252 mg, 2.5 mmol) were dissolved in 10 mL of dichloromethane, and A139-3 (197 mg, 1.2 mmol) was added. The mixture was reacted at ambient temperature for 2 h, and then TLC showed that the reaction was completed. The reaction liquid was quenched with water (10 mL). The aqueous phase was extracted with ethyl acetate (20 mL*3). The organic phase were combined and washed with saturated saline solution (10 ml*3) and then dried by rotary evaporation. The crude product was separated and purified by column chromatography (dichloromethane : methanol = 30 : 1) to obtain compound A154-1 (301 mg, yield 93%).

### Synthesis of compound A154-2

A154-1 (301 mg, 0.923 mmol) was dissolved in 10 mL of glacial acetic acid, and sodium nitrite (115 mg, 1.66 mmol) was added in an ice bath. The mixture was reacted at ambient temperature for 1 h, and then TLC showed that the reaction was completed. The reaction liquid was quenched with water (40 mL). The precipitated solid was filtered. The filter cake was rinsed with a small amount of water and dried to obtain compound A154-2 (282 mg, yield 91%).

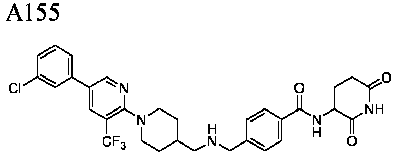

¹H NMR (400 MHz, DMSO) δ 10.86 (s, 1H), 8.81 (d, *J* = 2.2 Hz, 1H), 8.72 (d, *J* = 8.4 Hz, 1H), 8.28 (d, *J* = 2.3 Hz, 1H), 7.88 - 7.80 (m, 3H), 7.75 - 7.67 (m, 1H), 7.52 - 7.40 (m, 4H), 4.83 - 4.69 (m, 1H), 3.77 (s, 2H), 3.68 - 3.57 (m, 2H), 2.93 - 2.88 (m, 2H), 2.85 - 2.73 (m, 1H), 2.61 - 2.52 (m, 1H), 2.41 (d, *J* = 6.5 Hz, 2H), 2.19 - 2.06 (m, 1H), 2.03 - 1.92 (m, 1H), 1.88 - 1.78 (m, 2H), 1.72 - 1.58 (m, 1H), 1.32 - 1.21 (m, 2H).ESI-MS(M+H)⁺: 614.3.

The corresponding intermediates were prepared according to the following conditions:

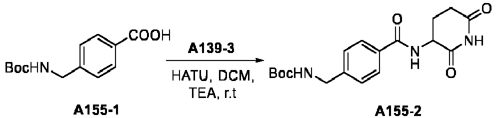

### Synthesis of compound A155-2

A155-1 (200 mg, 0.8 mmol), HATU (440 mg, 1.2 mmol) and triethylamine (320 mg, 3.2 mmol) were dissolved in 20 mL of dichloromethane, and A139-3 (130 mg, 0.8 mmol) was added. The mixture was reacted at ambient temperature for 3 h, and then TLC showed that the reaction was completed. The reaction liquid was filtered. The filter cake was washed with a small amount of dichloromethane to obtain compound A155-2 (320 mg, yield 100%).

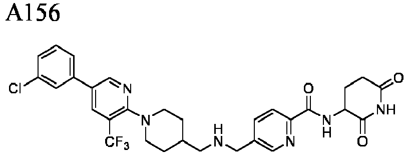

¹H NMR (400 MHz, DMSO) δ 10.87 (s, 1H), 9.05 (d, *J* = 8.5 Hz, 1H), 8.83 (d, *J* = 2.2 Hz, 1H), 8.62 (s, 1H), 8.29 (d, *J* = 2.3 Hz, 1H), 8.02 (d, *J* = 7.9 Hz, 1H), 7.96 (dd, *J* = 8.1, 1.8 Hz, 1H), 7.86 (s, 1H), 7.72 (d, *J* = 7.7 Hz, 1H), 7.54 - 7.41 (m, 2H), 4.85 -4.73 (m, 1H), 3.82 (s, 2H), 3.68 - 3.56 (m, 2H), 2.92 (t, *J* = 11.7 Hz, 2H), 2.86 -2.72 (m, 1H), 2.58 -2.51 (m, 1H), 2.42 (d, *J* = 6.5 Hz, 2H), 2.23 - 2.15 (m, 1H), 2.04 - 1.95 (m, 1H), 1.88 - 1.78 (m, 2H), 1.71 - 1.56 (m, 1H), 1.31 - 1.24 (m, 2H).ESI-MS(M+H)⁺: 615.3.

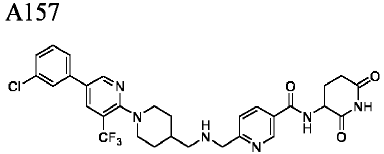

¹H NMR (400 MHz, DMSO) δ 10.91 (s, 1H), 8.95 (s, 1H), 8.83 (d, *J* = 2.2 Hz, 1H), 8.29 (d, *J* = 2.4 Hz, 1H), 8.22 - 8.19 (m, 2H), 7.86 (t, *J* = 1.7 Hz, 1H), 7.72 (d, *J* = 7.7 Hz, 1H), 7.60 (d, *J* = 8.2 Hz, 1H), 7.53 - 7.46 (m, 1H), 7.46 - 7.39 (m, 1H), 4.88 - 4.76 (m, 1H), 3.94 (s, 2H), 3.68 - 2.59 (m, 2H), 2.92 (t, *J* = 12.0 Hz, 2H), 2.87 - 2.76 (m, 1H), 2.59 - 2.51 (m, 3H), 2.19 - 2.07 (m, 1H), 2.05 - 1.96 (m, 1H), 1.89 - 1.79 (m, 2H), 1.75 - 1.64 (m, 1H), 1.34 - 1.21 (m, 2H).ESI-MS(M+H)⁺: 615.3.

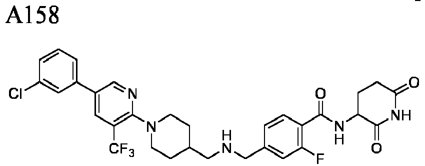

¹H NMR (400 MHz, DMSO) δ 10.87 (s, 1H), 8.82 (d, *J* = 2.2 Hz, 1H), 8.49 (dd, *J* = 8.1, 3.4 Hz, 1H), 8.29 (d, *J* = 2.3 Hz, 1H), 7.85 (t, *J* = 1.7 Hz, 1H), 7.72 (d, *J* = 7.7 Hz, 1H), 7.64 (t, *J* = 7.9 Hz, 1H), 7.51 - 7.43 (m, 2H), 7.30 - 7.26 (m, 2H), 4.83 - 4.70 (m, 1H), 3.76 (s, 2H), 3.69 - 3.57 (m, 2H), 2.92 (t, *J* = 11.7 Hz, 2H), 2.86 - 2.68 (m, 1H), 2.61 - 2.52 (m, 1H), 2.39 (d, *J* = 6.5 Hz, 2H), 2.17 - 1.96 (m, 2H), 1.88 - 1.78 (m, 2H), 1.69 - 1.57 (m, 1H), 1.34 - 1.19 (m, 2H).ESI-MS(M+H)⁺: 632.3.

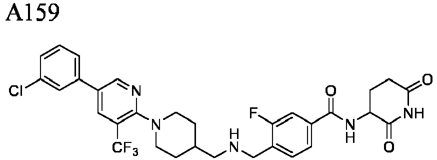

¹H NMR (400 MHz, DMSO) δ 10.89 (s, 1H), 8.84 - 8.81 (m, 2H), 8.29 (d, *J* = 2.4 Hz, 1H), 7.86 - 7.85 (m, 1H), 7.74 - 7.67 (m, 2H), 7.67 - 7.58 (m, 2H), 7.52 - 7.47 (m, 1H), 7.46 - 7.41 (m, 1H), 4.84 - 4.71 (m, 1H), 3.79 (s, 2H), 3.68 - 3.58 (m, 2H), 2.91 (t, *J* = 11.7 Hz, 2H), 2.85 - 2.73 (m, 1H), 2.60 - 2.52 (m, 1H), 2.43 (d, *J* = 6.5 Hz, 2H), 2.18 - 2.05 (m, 1H), 2.03 - 1.94 (m, 1H), 1.88 - 1.78 (m, 2H), 1.71 - 1.58 (m, 1H), 1.32 - 1.89 (m, 2H).ESI-MS(M+H)⁺: 632.3.

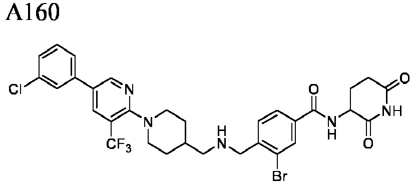

¹H NMR (400 MHz, DMSO) δ 10.88 (s, 1H), 8.86 (d, *J* = 8.3 Hz, 1H), 8.83 (d, *J* = 2.1 Hz, 1H), 8.29 (d, *J* = 2.3 Hz, 1H), 8.08 (d, *J* = 1.4 Hz, 1H), 7.91 - 7.83 (m, 2H), 7.72 (d, *J* = 7.7 Hz, 1H), 7.67 (d, *J* = 8.0 Hz, 1H), 7.52 - 7.48 (m, 1H), 7.45 (d, *J* = 8.1 Hz, 1H), 4.85 - 4.71 (m, 1H), 3.82 (s, 2H), 3.659 - 3.60 (m, 2H), 2.93 (t, *J* = 11.8 Hz, 2H), 2.84 - 2.72 (m, 1H), 2.60 - 2.51 (m, 1H), 2.47 (d, *J* = 6.6 Hz, 2H), 2.17 - 2.05 (m, 1H), 2.03 - 1.93 (m, 1H), 1.91 - 1.80 (m, 2H), 1.74 - 1.62 (m, 1H), 1.34 - 1.25 (m, 2H).ESI-MS(M+H)⁺: 692.3.

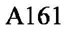

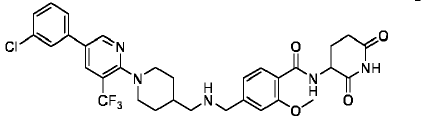

¹H NMR (400 MHz, DMSO) δ 10.91 (s, 1H), 8.85 (d, *J* = 2.3 Hz, 1H), 8.64 (d, *J* = 7.5 Hz, 1H), 8.32 (d, *J* = 2.4 Hz, 1H), 7.93 - 7.83 (m, 2H), 7.78 - 7.68 (m, 1H), 7.53 - 7.44 (m, 3H), 7.21 (d, *J* = 7.9 Hz, 1H), 4.83 - 4.70 (m, 1H), 4.21 (s, 2H), 3.96 (s, 3H), 3.68 - 3.58 (m, 2H), 2.99 - 2.84 (m, 4H), 2.82 - 2.71 (m, 1H), 2.57 - 2.53 (m, 1H), 2.18 - 2.05 (m, 2H), 2.03 - 1.93 (m, 1H), 1.91 - 1.83 (m, 2H), 1.42 - 1.27 (m, 2H).ESI-MS(M+H)⁺: 644.3.

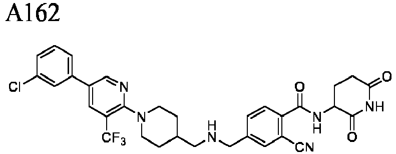

¹H NMR (400 MHz, DMSO) δ 10.93 (s, 1H), 9.12 (d, *J* = 8.3 Hz, 1H), 8.85 (d, *J* = 2.2 Hz, 1H), 8.33 (d, *J* = 2.3 Hz, 1H), 7.97 - 7.89 (m, 2H), 7.87 (s, 1H), 7.73 (d, *J* = 7.7 Hz, 1H), 7.53 - 7.49 (m, 1H), 7.48 - 7.43 (m, 1H), 4.86 - 4.76 (m, 1H), 4.30 (s, 2H), 3.64 (d, *J* = 11.4 Hz, 2H), 3.01 - 2.88 (m, 4H), 2.87 - 2.74 (m, 1H), 2.64 - 2.54 (m, 1H), 2.17 - 1.92 (m, 3H), 1.91 - 1.81 (m, 2H), 1.45 - 1.29 (m, 2H).ESI-MS(M+H)⁺: 639.2.

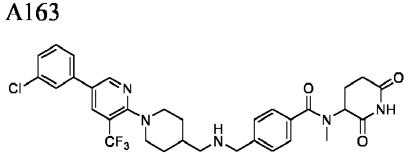

¹H NMR (400 MHz, DMSO) δ 10.89 (s, 1H), 8.83 (d, *J* = 2.2 Hz, 1H), 8.29 (d, *J* = 2.4 Hz, 1H), 7.86 - 7.85 (m, 1H), 7.72 (d, *J* = 7.7 Hz, 1H), 7.54 - 7.46 (m, 1H), 7.46 - 7.27 (m, 5H), 5.15 - 4.50 (m, 1H), 3.78 - 3.72 (m, 2H), 3.69 - 3.59 (m, 2H), 2.98 - 2.77 (m, 6H), 2.69 - 2.53 (m, 1H), 2.47 - 2.29 (m, 3H), 2.03 - 1.93 (m, 1H), 1.88 - 1.78 (m, 2H), 1.70 - 1.58 (m, 1H), 1.33 - 1.23 (m, 2H).ESI-MS(M+H)⁺: 628.3.

The corresponding intermediates were prepared according to the following conditions:

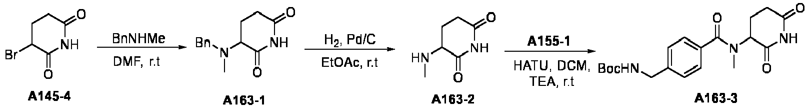

### Synthesis of compound A163-1

A145-4 (400 mg, 2.1 mmol) and N-methylbenzylamine (277 mg, 2.3 mmol) were dissolved in 8 mL of DMF. The mixture was reacted at ambient temperature for 2 h, and then TLC showed that the reaction was completed. The reaction liquid was diluted with water (60 mL), and extracted with ethyl acetate (30 mL*3). The organic phases were combined and washed with saturated saline solution (10 mL*3), and then dried by rotary evaporation. The crude product was separated and purified by column chromatography (petroleum ether : ethyl acetate = 2 : 1) to obtain compound A163-1 (390 mg, yield 80%).

### Synthesis of compound A163-2

A163-1 (360 mg, 1.55 mmol) and palladium on carbon (30 mg, 5%) were dissolved in 5 mL of ethyl acetate. The mixture was reacted overnight at ambient temperature under hydrogen atmosphere, and then TLC showed that the reaction was completed. The reaction liquid was filtered. The filtrate was dried by rotary evaporation to obtain compound A163-2 (160 mg, yield 73%).

### Synthesis of compound A163-3

A155-1 (120 mg, 0.84 mmol), HATU (353 mg, 0.93 mmol) and triethylamine (128 mg, 1.2 mmol) were dissolved in 20 mL of dichloromethane, and A139-3 (212 mg, 0.84 mmol) was added. The mixture was reacted at ambient temperature for 3 h, and then TLC showed that the reaction was completed. The reaction liquid was filtered. The filter cake was washed with a small amount of dichloromethane to obtain compound A163-3 (300 mg, yield 95%).

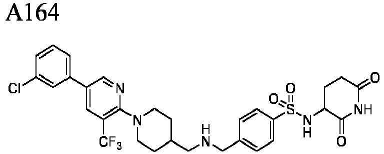

¹H NMR (400 MHz, DMSO) δ 10.79 (s, 1H), 8.83 (d, *J* = 2.3 Hz, 1H), 8.29 (d, *J* = 2.3 Hz, 1H), 8.09 (s, 1H), 7.86 (t, *J* = 1.7 Hz, 1H), 7.79 (d, *J* = 8.3 Hz, 2H), 7.72 (d, *J* = 7.7 Hz, 1H), 7.54 - 7.45 (m, 3H), 7.47 - 7.41 (m, 1H), 4.23 (t, *J* = 8.5 Hz, 1H), 3.80 (s, 2H), 3.68 - 3.58 (m, 2H), 2.91 (t, *J* = 11.7 Hz, 2H), 2.70 - 2.58 (m, 1H), 2.48 - 2.37 (m, 3H), 1.88 - 1.73 (m, 4H), 1.71 - 1.58 (m, 1H), 1.34 - 1.21 (m, 2H).ESI-MS(M+H)⁺: 650.3.

The corresponding intermediates were prepared according to the following conditions:

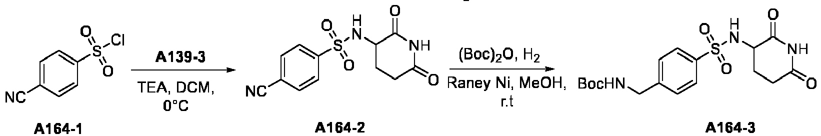

### Synthesis of compound A164-2

A164-1 (600 mg, 3.0 mmol) and triethylamine (606 mg, 6.0 mmol) were dissolved in 20 mL of dichloromethane, and A139-3 (541 mg, 3.3 mmol) was added in an ice bath. The mixture was reacted for 30 min in an ice bath, and then TLC showed that the reaction was completed. The reaction liquid was filtered. The filter cake was washed with a small amount of dichloromethane to obtain compound A164-2 (215 mg, yield 24%).

### Synthesis of compound A164-3

A164-2 (215 mg, 0.73 mmol) and (Boc)₂O (320 mg, 1.47 mmol) were dissolved in 10 mL of methanol, and Raney Nickel (500 mg) was added. The mixture was reacted overnight at ambient temperature under hydrogen atmosphere, and then TLC showed that the reaction was completed. The reaction liquid was filtered. The filter cake was rinsed with methanol, and then the filtrate was dried by rotary evaporation. The crude product was separated by column chromatography (dichloromethane : methanol = 20 : 1) to obtain compound A164-3 (45 mg, yield 15%).

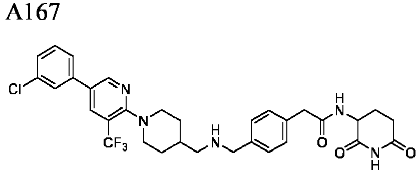

¹H NMR (400 MHz, DMSO) δ 10.83 (s, 1H), 8.52 (d, *J* = 8.1 Hz, 1H), 8.32 (d, *J* = 2.3 Hz, 1H), 7.87 (d, *J* = 1.8 Hz, 1H), 7.73 (d, *J* = 7.7 Hz, 1H), 7.53 - 7.45 (m, 4H), 7.36 (d, *J* = 8.1 Hz, 2H), 4.59 - 4.41 (m, 1H), 4.12 (s, 2H), 3.69 - 3.58 (m, 2H), 3.53 (s, 2H), 2.99 - 2.81 (m, 4H), 2.76 - 2.63 (m, 1H), 2.48 - 2.43 (m, 1H), 2.01 - 1.80 (m, 5H), 1.41 - 1.27 (m, 2H).ESI-MS(M+H)⁺: 628.3.

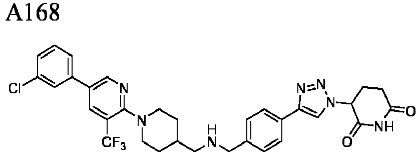

¹H NMR (400 MHz, DMSO) δ 11.28 (s, 1H), 8.84 (d, *J* = 2.1 Hz, 1H), 8.71 (s, 1H), 8.31 (d, *J* = 2.3 Hz, 1H), 7.91 - 7.82 (m, 3H), 7.72 (d, *J* = 7.7 Hz, 1H), 7.55 (d, *J* = 7.9 Hz, 2H), 7.50 (t, *J* = 7.8 Hz, 1H), 7.45 (d, *J* = 8.1 Hz, 1H), 5.87 (dd, *J* = 12.7, 5.1 Hz, 1H), 3.99 (s, 2H), 3.66 - 3.62 (m, 2H), 2.97 - 2.84 (m, 3H), 2.78 - 2.62 (m, 4H), 2.43 - 2.30 (m, 1H), 1.92 - 1.76 (m, 3H), 1.39 - 1.26 (m, 2H).ESI-MS(M+H)⁺: 638.3.

The corresponding intermediates were prepared according to the following conditions:

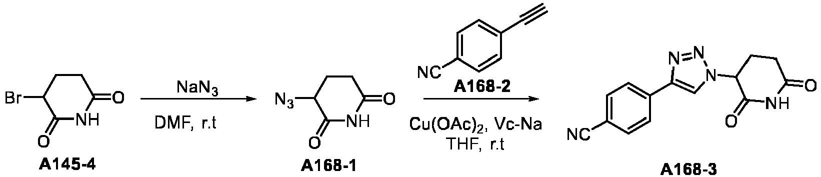

### Synthesis of compound A168-1

A145-4 (340 mg, 1.77 mmol) and sodium azide (460 mg, 7.08 mmol) were dissolved in 8 mL of DMF. The mixture was reacted overnight at ambient temperature, and then TLC showed that the reaction was completed. The reaction liquid was quenched with water (20 mL). The aqueous phase was extracted with ethyl acetate (20 mL*3). The organic phase were combined and washed with saturated saline solution (10 ml*3), and then dried by rotary evaporation to obtain compound A168-1 (270 mg, yield 99%).

### Synthesis of compound A168-3

A168-1 (270 mg, 1.77 mmol), A168-2 (247 mg, 1.95 mmol) and copper acetate (40 mg, 0.2 mmol) were dissolved in 12 mL of tetrahydrofuran, and vitamin C sodium salt (175 mg, 0.89 mmol) was added. The mixture was reacted overnight at ambient temperature, and then TLC showed that the reaction was completed. The reaction liquid was filtered. The filtrate was diluted with water (20 mL). The aqueous phase was extracted with ethyl acetate (20 mL*3). The organic phase were combined and washed with saturated saline solution (10 ml*3) and then dried by rotary evaporation. The crude product was separated by column chromatography (petroleum ether : ethyl acetate = 1 : 1) to obtain compound A168-3 (470 mg, yield 95%).

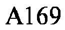

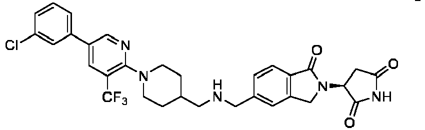

¹H NMR (400 MHz, DMSO) δ 8.84 (d, *J* = 2.1 Hz, 1H), 8.31 (d, *J* = 2.3 Hz, 1H), 7.87 (s, 1H), 7.77 - 7.70 (m, 2H), 7.65 (s, 1H), 7.57 (d, *J* = 8.1 Hz, 1H), 7.55 - 7.42 (m, 2H), 5.25 (dd, *J* = 9.0, 6.4 Hz, 1H), 4.64 (d, *J* = 17.4 Hz, 1H), 4.38 (d, *J* = 17.4 Hz, 1H), 4.03 (s, 2H), 3.69 - 3.58 (m, 2H), 3.03 - 2.87 (m, 3H), 2.70 - 2.58 (m, 2H), 2.05 - 1.95 (m, 1H), 1.90 - 1.81 (m, 2H), 1.80 - 1.71 (m, 1H), 1.37 - 1.24 (m, 2H).ESI-MS(M+H)⁺: 612.3.

The corresponding intermediates were prepared according to the following conditions:

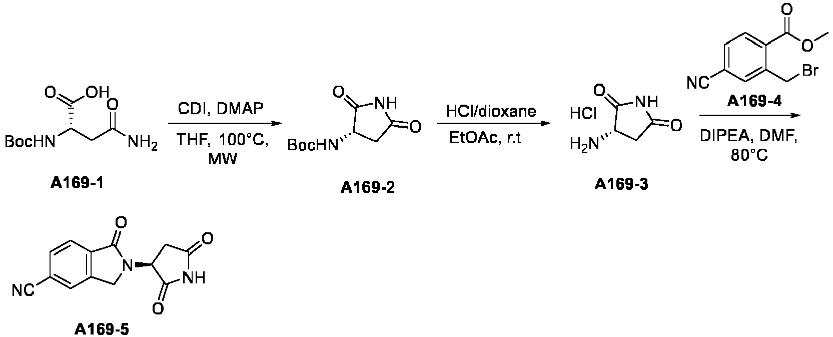

### Synthesis of compound A169-2

Compound A169-1 (2.04 g, 8.8 mmol) and 4-dimethylaminopyridine (105 mg, 0.9 mmol) were dissolved in 30 mL of DMF, and *N,N'*-carbonyldiimidazole (1.43 g, 8.8 mmol) was added. The mixture was reacted under microwave irradiation at 100 °C for 1 h, and then TLC showed that the reaction was completed. The reaction liquid was dried by rotary evaporation. The crude product was separated by column chromatography (dichloromethane : methanol = 20 : 1) to obtain compound A169-2 (1.3 g, yield 69%).

### Synthesis of compound A169-3

Compound A169-2 (1.02 g, 4.77 mmol) was dissolved in 10 mL of ethyl acetate, and HCl/dioxane (10 mL, 4 M) was added. The mixture was reacted at ambient temperature for 2 h, and then TLC showed that the reaction was completed. The reaction liquid was dried by rotary evaporation to obtain compound A169-3 (1 g, crude product), which was directly used for next step.

### Synthesis of compound A169-5

Compounds A169-4 (1.21 g, 4.77 mmol), A169-3 (1 g, 4.77 mol) and DIPEA (922 mg, 7.15 mmol) were dissolved in 10 mL of DMF. The mixture was reacted overnight at 80 °C, and then TLC showed that the reaction was completed. The reaction liquid was diluted with water (30 mL), and then extracted with ethyl acetate (30 ml*3). The organic phases were combined and washed with saturated saline solution (20 ml*3), and then the organic phase was dried by rotary evaporation. The crude product was slurried in 5 mL of ethyl acetate and then filtered. The filter cake was dried to obtain compound A169-5 (390 mg, yield 32%).

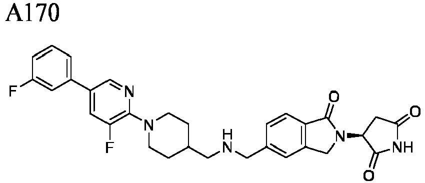

¹H NMR (400 MHz, DMSO) δ 8.41 (s, 1H), 7.93 (dd, *J* = 15.0, 1.9 Hz, 1H), 7.81 - 7.71 (m, 2H), 7.66 (d, *J* = 7.5 Hz, 1H), 7.60 - 7.52 (m, 2H), 7.51 - 7.45 (m, 1H), 7.21 - 7.12 (m, 1H), 5.26 (dd, *J* = 8.9, 6.5 Hz, 1H), 4.53 (dd, *J* = 106.6, 17.6 Hz, 2H), 4.24 (s, 2H), 4.13 - 4.03 (m, 2H), 3.03 - 2.75 (m, 5H), 2.04 - 1.91 (m, 2H), 1.89 - 1.79 (m, 2H), 1.38 - 1.26 (m, 2H).ESI-MS(M+H)⁺: 546.3.

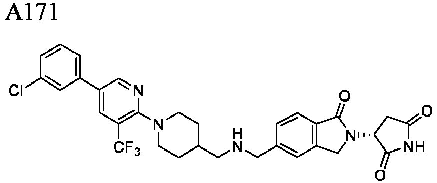

¹H NMR (400 MHz, DMSO) δ 8.82 (d, *J* = 2.3 Hz, 1H), 8.28 (d, *J* = 2.4 Hz, 1H), 7.86 - 7.84 (m, 1H), 7.71 (d, *J* = 7.7 Hz, 1H), 7.64 (d, *J* = 7.8 Hz, 1H), 7.57 (s, 1H), 7.52 - 7.43 (m, 3H), 5.22 (dd, *J* = 9.1, 6.3 Hz, 1H), 4.60 (d, *J* = 17.3 Hz, 1H), 4.35 (d, *J* = 17.3 Hz, 1H), 3.82 (s, 2H), 3.69 - 3.59 (m, 2H), 3.03 - 2.58 (m, 4H), 2.42 (d, *J* = 6.6 Hz, 2H), 1.88 - 1.79 (m, 2H), 1.71 - 1.58 (m, 1H), 1.32 - 1.23 (m, 2H).ESI-MS(M+H)⁺: 612.3.

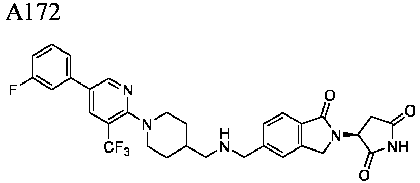

¹H NMR (400 MHz, DMSO) δ 8.84 (d, *J* = 2.2 Hz, 1H), 8.29 (d, *J* = 2.4 Hz, 1H), 7.71 - 7.57 (m, 4H), 7.58 - 7.47 (m, 2H), 7.26 - 7.16 (m, 1H), 5.23 (dd, *J* = 9.0, 6.4 Hz, 1H), 4.62 (d, *J* = 17.4 Hz, 1H), 4.36 (d, *J* = 17.3 Hz, 1H), 3.94 (s, 2H), 3.69 - 3.59 (m, 2H), 3.03 - 2.85 (m, 4H), 2.60 - 2.51 (m, 2H), 1.89 - 1.80 (m, 2H), 1.79 - 1.69 (m, 1H), 1.33 - 1.24 (m, 2H).ESI-MS(M+H)⁺: 546.3.

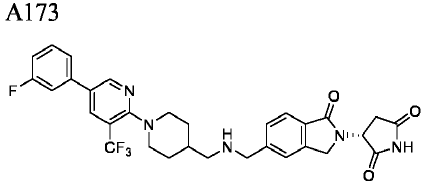

¹H NMR (400 MHz, DMSO) δ 8.83 (d, *J* = 2.2 Hz, 1H), 8.28 (d, *J* = 2.3 Hz, 1H), 7.69 - 7.47 (m, 4H), 7.55 - 7.46 (m, 2H), 7.25 - 7.18 (m, 1H), 5.22 (dd, *J* = 9.1, 6.3 Hz, 1H), 4.61 (d, *J* = 17.3 Hz, 1H), 4.35 (d, *J* = 17.3 Hz, 1H), 3.85 (s, 2H), 3.67 - 3.59 (m, 2H), 3.03 - 2.86 (m, 4H), 2.45 (d, *J* = 6.6 Hz, 2H), 1.88 - 1.79 (m, 2H), 1.74 - 1.62 (m, 1H), 1.34 - 1.24 (m, 2H).ESI-MS(M+H)⁺: 596.3.

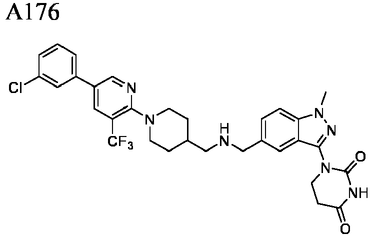

The corresponding intermediates were prepared according to the following conditions:

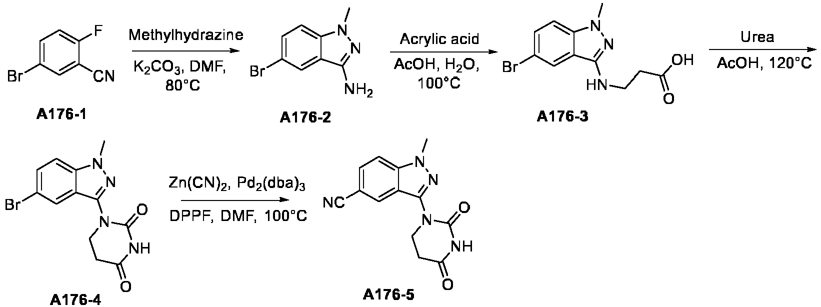

### Synthesis of compound A176-2

Compound A176-1 (2.1 g, 10.55 mmol) and potassium carbonate (4.37 g, 31.66 mmol) were dissolved in 30 mL of DMF, and methylhydrazine (1.82 g, 12.66 mmol) was added. The mixture was reacted overnight at 80 °C, and then TLC showed that the reaction was completed. The reaction liquid was diluted with water (50 mL). The aqueous phase was extracted with ethyl acetate (25 mL*3). The organic phases were combined and washed with saturated saline solution (10 mL*3), and then dried by rotary evaporation. The crude product was separated by column chromatography (petroleum ether : ethyl acetate = 1 : 1) to obtain compound A176-2 (2.13 g, yield 90%).

### Synthesis of compound A176-3

Compound A176-2 (1.1 g, 4.89 mmol) was dissolved in 5 mL of water, and acrylic acid (387 mg, 5.38 mmol) and acetic acid (880 mg, 14.67 mmol) were added. The mixture was reacted overnight at 100 °C, and then TLC showed that the reaction was completed. The reaction liquid was diluted with water (50 mL). The aqueous phase was extracted with ethyl acetate (40 mL*3). The organic phases were combined and washed with saturated saline solution (10 mL*3), and then dried by rotary evaporation. The crude product was separated by column chromatography (dichloromethane : methanol = 10 : 1) to obtain compound A176-3 (1.03 g, yield 71%).

### Synthesis of compound A176-4

Compound A176-3 (1.03 g, 3.47 mmol) and urea (624 mg, 10.4 mmol) were dissolved in 5 mL of acetic acid. The mixture was reacted overnight at 120 °C, and then TLC showed that the reaction was completed. Saturated sodium bicarbonate solution (20 mL) was added to the reaction to quench the reaction. The aqueous phase was extracted with dichloromethane (30 mL*3). The organic phases were combined and washed with saturated saline solution (10 mL*3), and then dried by rotary evaporation. The crude product was separated by column chromatography (dichloromethane : methanol = 10 : 1) to obtain compound A176-4 (295 mg, yield 26%).

### Synthesis of compound A176-5

A176-4 (295 mg, 0.92 mmol) and zinc cyanide (108 mg, 0.92 mmol) were dissolved in 6 mL of DMF, and Pd₂(dba)₃ (84 mg, 0.09 mmol) and DPPF (102 mg, 0.18 mmol) were added. The mixture was reacted overnight at 100 °C under nitrogen protection, and then TLC showed that the reaction was completed. The reaction liquid was filtered. The filtrate was diluted with water (60 mL), and then extracted with ethyl acetate (30 mL*3). The organic phases were combined and washed with saturated saline solution (10 mL*3), and then dried by rotary evaporation. The crude product was slurried in 10 mL of ethyl acetate and filtered to obtain compound A176-4 (174 mg, yield 70%).

¹H NMR (400 MHz, DMSO) δ 10.55 (s, 1H), 8.82 (d, *J* = 2.1 Hz, 1H), 8.29 (d, *J* = 2.3 Hz, 1H), 7.86 (s, 1H), 7.72 (d, *J* = 7.6 Hz, 1H), 7.56 - 7.54 (m, 2H), 7.51 - 7.47 (m, 1H), 7.45 - 7.43 (m, 2H), 3.98 (s, 3H), 3.91 (t, *J* = 6.6 Hz, 2H), 3.78 (s, 2H), 3.68 - 3.58 (m, 2H), 2.91 (t, *J* = 12.1 Hz, 2H), 2.76 (t, *J* = 6.7 Hz, 2H), 2.45 - 2.39 (m, 2H), 1.86 - 1.77 (m, 2H), 1.70 - 1.57 (m, 1H), 1.33 - 1.17 (m, 2H).ESI-MS(M+H)⁺: 626.3.

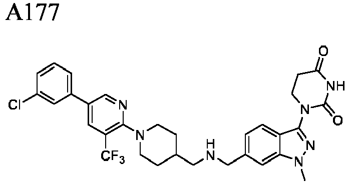

¹H NMR (400 MHz, DMSO) δ 10.57 (s, 1H), 8.83 (d, *J* = 2.2 Hz, 1H), 8.30 (d, *J* = 2.3 Hz, 1H), 7.86 (s, 1H), 7.72 (d, *J* = 7.7 Hz, 1H), 7.70 - 7.61 (m, 2H), 7.52 - 7.43 (m, 2H), 7.21 (d, *J* = 8.4 Hz, 1H), 4.08 (s, 2H), 3.99 (s, 3H), 3.93 (t, *J* = 6.7 Hz, 2H), 3.68 - 3.59 (m, 2H), 2.98 - 2.87 (m, 2H), 2.76 (t, *J* = 6.7 Hz, 2H), 2.72 - 2.63 (m, 2H), 1.92 - 1.75 (m, 3H), 1.37 - 1.25 (m, 2H).ESI-MS(M+H)⁺: 626.3.

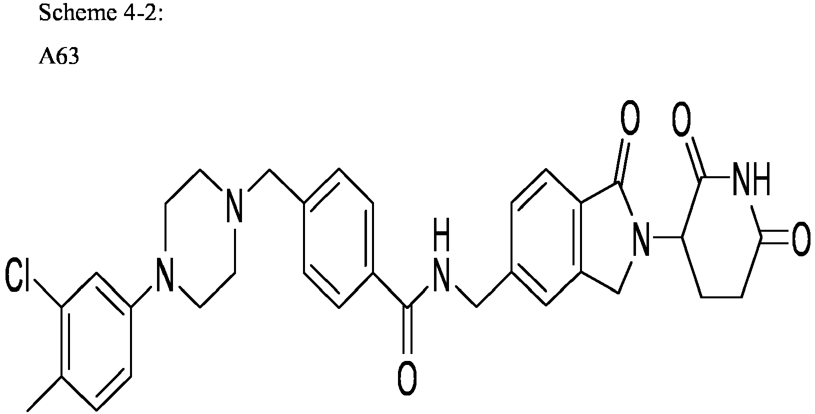

### Synthesis of compound A63-2

A63-1 (300 mg, 2.0 mmol), A12-2 (344 mg, 2.0 mmol) and potassium carbonate (552 mg, 4.0 mmol) were dissolved in dioxane/water (8 mL/2 mL), and Pd(PPh₃)₄ (162 mg, 0.14 mmol) was added. The mixture was replaced with nitrogen three times and reacted at 100 °C for 4 h, and then TLC showed that the reaction was completed. The reaction liquid was filtered, and the filtrate was diluted with ethyl acetate (60 mL). The organic phase was washed with saturated saline solution (10 ml*3), and the solution was dried by rotary evaporation. The solute was separated and purified by column chromatography (petroleum ether: ethyl acetate = 10 : 1) to obtain compound A63-2 (410 mg, yield 85%).

### Synthesis of compound A63-3

A63-2 (350 mg, 1.46 mmol), A61-2 (184 mg, 1.60 mmol) and potassium carbonate (403 mg, 2.92 mmol) were dissolved in 6 mL of acetonitrile. The reaction was stirred at 60 °C for 2 h, and then TLC showed that the reaction was completed. The reaction liquid was diluted with water (20 mL). The organic phase was extracted with ethyl acetate (20 ml*3). The organic phases were combined, and dried by rotary evaporation. The solute was separated and purified by a preparative TLC (petroleum ether: ethyl acetate = 5 : 1) to obtain compound A63-3 (310 mg, yield 66%).

### Synthesis of compound A63-4

A63-3 (310 mg, 0.97 mmol) was dissolved in 6 mL of dichloromethane, and Dess-Martin reagent (495 mg, 1.17 mmol) was added in an ice bath. The mixture was reacted at ambient temperature for 2 h, and then TLC showed that the reaction was completed. Saturated sodium bicarbonate solution (40 mL) was added to quench the reaction. The aqueous phase was extracted with dichloromethane (40 ml*3). The organic phases were combined and then dried by rotary evaporation. The solute was separated and purified by the preparative TLC (petroleum ether: ethyl acetate = 1 : 1) to obtain compound A63-4 (250 mg, yield 80%).

### Synthesis of compound A63

A63-4 (250 mg, 0.78 mmol) and compound A61-6 (240 mg, 0.78 mmol) were dissolved in 6 mL of methanol, and 1 drop of glacial acetic acid was added. The mixture was reacted at ambient temperature for 10 min. Sodium cyanoborohydride (102 mg, 1.57 mmol) was added and the reaction was carried out at ambient temperature for 2 h. Saturated sodium bicarbonate solution (20 mL) was added to quench the reaction. The aqueous phase was extracted with dichloromethane (30 ml*3). The organic phases were combined and dried by rotary evaporation. The solute was separated and purified by a preparative TLC (dichloromethane : methanol = 10 : 1) and then subjected to medium pressure preparation to obtain compound A63 (128 mg, yield 28%). ¹H NMR (400 MHz, DMSO) δ 10.97 (s, 1H), 8.47 (s, 1H), 8.33 (s, 1H), 8.14 (d, *J* = 8.2 Hz, 1H), 7.96 (dd, *J* = 12.2, 6.1 Hz, 2H), 7.67 (d, *J* = 7.8 Hz, 1H), 7.61 - 7.44 (m, 6H), 5.11 (dd, *J* = 13.3, 5.0 Hz, 1H), 4.51 - 4.25 (m, 4H), 3.83 (s, 2H), 3.00 - 2.83 (m, 3H), 2.65 - 2.55 (m, 1H), 2.46 - 2.32 (m, 3H), 2.06 - 1.94 (m, 1H), 1.92 - 1.80 (m, 2H), 1.79 - 1.73 (m, 1H), 1.26 - 1.10 (m, 2H). ESI-MS(M+H)⁺: 575.2.

The preparation methods of compounds A64, A71, A101, A102, and A104 refer to A63. The characterization results are as follows:

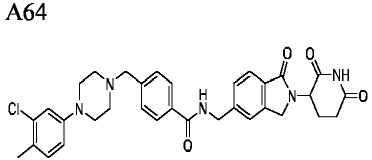

¹H NMR (400 MHz, DMSO) δ 10.97 (s, 1H), 8.73 - 8.65 (m, 3H), 8.03 - 7.94 (m, 3H), 7.67 (d, *J* = 7.8 Hz, 1H), 7.61 - 7.57 (m, 2H), 7.55 - 7.47 (m, 3H), 5.11 (dd, *J* = 13.3, 5.1 Hz, 1H), 4.38 (dd, *J* = 53.0, 17.2 Hz, 2H), 4.00 - 3.90 (m, 2H), 3.83 (s, 2H), 2.97 - 2.80 (m, 3H), 2.64 - 2.55 (m, 1H), 2.45 - 2.33 (m, 3H), 2.04 - 1.95 (m, 1H), 1.92 - 1.83 (m, 2H), 1.73 - 1.62 (m, 1H), 1.34 - 1.24 (m, 2H). ESI-MS(M+H)⁺: 575.2.

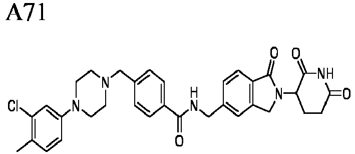

¹H NMR (400 MHz, DMSO) δ 10.99 (s, 1H), 8.40 (d, *J* = 2.9 Hz, 1H), 8.03 (t, *J* = 1.8 Hz, 1H), 7.94 (d, *J* = 8.0 Hz, 1H), 7.87 - 7.76 (m, 3H), 7.69 (d, *J* = 7.8 Hz, 1H), 7.49 - 7.33 (m, 3H), 5.13 (dd, *J* = 13.3, 5.1 Hz, 1H), 4.43 (dd, *J* = 53.1, 17.6 Hz, 2H), 4.26 (s, 2H), 3.94 - 3.82 (m, 2H), 3.01 - 2.72 (m, 5H), 2.67 - 2.57 (m, 1H), 2.46 - 2.35 (m, 1H), 2.04 - 1.92 (m, 2H), 1.91 - 1.82 (m, 2H), 1.39 - 1.27 (m, 2H).

ESI-MS(M+H)⁺: 558.2.

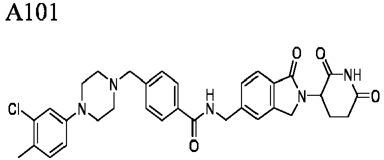

¹H NMR (400 MHz, DMSO) δ 10.97 (s, 1H), 8.26 (d, *J* = 11.9 Hz, 1H), 7.66 (d, *J* = 7.8 Hz, 1H), 7.55 (d, *J* = 8.8 Hz, 2H), 7.51 - 7.44 (m, 3H), 7.42 - 7.36 (m, 1H), 6.78 (d, *J* = 15.0 Hz, 1H), 5.10 (dd, *J* = 13.3, 5.1 Hz, 1H), 4.48 -4.26 (m, 4H), 3.81 (s, 2H), 2.98 - 2.81 (m, 3H), 2.65 - 2.55 (m, 1H), 2.44 - 2.31 (m, 3H), 2.03 - 1.95 (m, 1H), 1.84 - 1.75 (m, 2H), 1.74 - 1.66 (m, 1H), 1.17 - 1.04 (m, 2H). ESI-MS(M+H)⁺: 576.2.

The corresponding intermediates were prepared according to the following conditions:

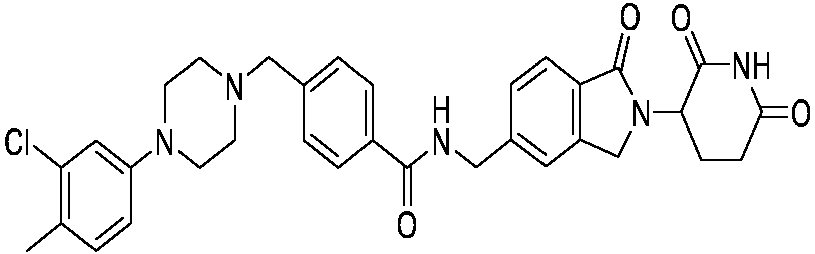

### Synthesis of compound A101-2

A101-1 (1.91 g, 10.0 mmol), A84-4 (1.56 g, 10.0 mmol) and potassium carbonate (4.14 g, 30.0 mmol) were dissolved in dioxane/water (40 mL/10 mL), and Pd(dppf)Cl₂ (732 mg, 1.0 mmol) was added. The mixture was replaced with nitrogen three times and reacted at 80 °C for 3 h, and then TLC showed that the reaction was completed. The reaction liquid was filtered. The filtrate was diluted with water (100 mL). The aqueous phase was extracted with ethyl acetate (100 ml*3. The organic phases were combined, and dried by rotary evaporation. The solute was separated and purified by column chromatography (petroleum ether: ethyl acetate = 10 : 1) to obtain compound A101-2 (1.91 g, yield 86%).

### Synthesis of compound A101-3

A101-2 (2.0 g, 6.0 mmol) was dissolved in acetonitrile (20 mL), and tert-butyl nitrite (2.64 g, 25.7 mmol) and copper bromide (3.7 g, 25.7 mmol) were added. The mixture was reacted overnight at 60 °C, and then TLC showed that the reaction was completed. The reaction liquid was directly dried by rotary evaporation. The solute was separated and purified by column chromatography (petroleum ether: ethyl acetate = 10 : 1) to obtain compound A101-3 (1.05 g, yield 43%).

### Synthesis of compound A 101-4

A101-3 (1.05 g, 3.66 mmol), A104-3 (527 g, 3.66 mmol) and cesium carbonate (3.67 g, 11.0 mmol) were dissolved in toluene (20 mL), and Pd₂(dba)₃ (339 mg, 0.37 mmol) and Xphos (176 mg, 0.37 mmol) were added. The mixture was replaced with nitrogen three times and reacted at 60 °C for 3 h, and then TLC showed that the reaction was completed. The reaction liquid was filtered. The filtrate was diluted with water (60 mL). The aqueous phase was extracted with ethyl acetate (60 ml*3). The organic phases were combined, and dried by rotary evaporation. The solute was separated and purified by column chromatography (petroleum ether: ethyl acetate = 5 : 1) to obtain compound A101-4 (110 mg, yield 9%).

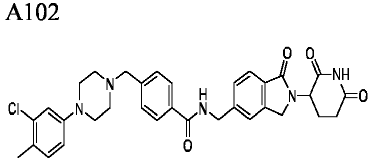

¹H NMR (400 MHz, DMSO) δ 10.97 (s, 1H), 8.70 (d, *J* = 1.4 Hz, 1H), 8.38 (d, *J* = 1.3 Hz, 1H), 7.99 - 7.67 (m, 1H), 7.95 - 7.86 (m, 1H), 7.66 (d, *J* = 7.8 Hz, 1H), 7.56 (s, 1H), 7.51 - 7.41 (m, 2H), 7.39 - 7.34 (m, 1H), 5.10 (dd, *J* = 13.3, 5.1 Hz, 1H), 4.48 - 4.24 (m, 4H), 3.81 (s, 2H), 2.98 - 2.81 (m, 3H), 2.64 - 2.55 (m, 1H), 2.45 - 2.33 (m, 3H), 2.04 - 1.96 (m, 1H), 1.838 - 1.78(d, *J* = 13.1 Hz, 2H), 1.77 - 1.69 (m, 1H), 1.21 - 1.07 (m, 2H). ESI-MS(M+H)⁺: 559.2.

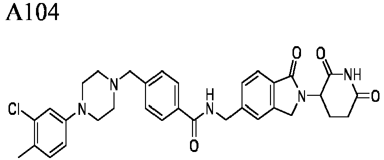

¹H NMR (400 MHz, DMSO) δ 10.98 (s, 1H), 7.95 (dd, *J* = 8.4, 2.0 Hz, 1H), 7.88 (d, *J* = 2.0 Hz, 1H), 7.78 - 7.77 (m, 1H), 7.67 (dd, *J* = 7.7, 2.8 Hz, 2H), 7.60 - 7.57 (m, 2H), 7.53 - 7.41 (m, 3H), 5.11 (dd, *J* = 13.3, 5.1 Hz, 1H), 4.38 (dd, *J* = 54.4, 17.3 Hz, 2H), 3.83 (s, 2H), 3.06 - 2.99 (m, 2H), 2.97 - 2.86 (m, 1H), 2.76 (t, *J* = 10.8 Hz, 2H), 2.65 - 2.55 (m, 1H), 2.46 - 2.31 (m, 3H), 2.05 - 1.95 (m, 1H), 1.88 - 1.77 (m, 2H), 1.65 - 1.52 (m, 1H), 1.35 - 1.22 (m, 2H). ESI-MS(M+H)⁺: 625.2.

The corresponding intermediates were prepared according to the following conditions:

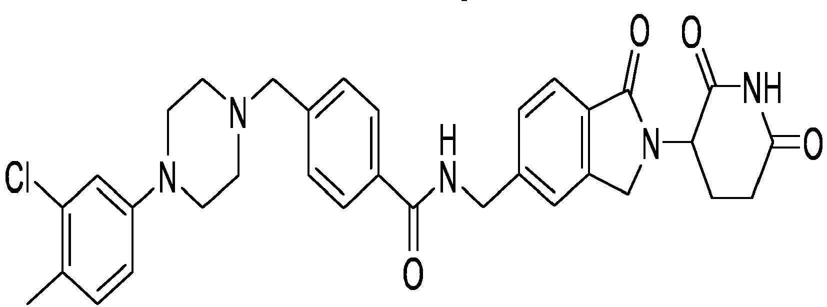

### Synthesis of compound A104-2

A104-1 (5.0 g, 14.2 mmol), A84-4 (2.3 g, 14.3 mmol) and potassium carbonate (4.0 g, 28.5 mmol) were dissolved in dioxane/water (60 mL/20 mL), and Pd(PPh₃)₄ (990 mg, 0.85 mmol) was added. The mixture was replaced with nitrogen three times and then reacted at 100 °C overnight, and then TLC showed that the reaction was completed. The reaction liquid was filtered. The filtrate was diluted with water (60 mL). The aqueous phase was extracted with ethyl acetate (60 ml*3). The organic phases were combined, and dried by rotary evaporation. The solute was separated and purified by column chromatography (petroleum ether: ethyl acetate = 5 : 1) to obtain compound A104-2 (4.4 g, yield 92%).

### Synthesis of compound A104-4

A104-2 (2.0 g, 6.0 mmol), A104-3 (1.7 g, 12.0 mmol) and sodium tert-butoxide (1.15 g, 12.0 mmol) were dissolved in toluene (60 mL), and Pd₂(dba)₃ (1.1 g, 0.6 mmol) and Xphos (1.13 g, 1.2 mmol) were added. The mixture was replaced with nitrogen three times and then reacted at 110 °C overnight, and then TLC showed that the reaction was completed. The reaction liquid was filtered. The filtrate was diluted with water (60 mL). The aqueous phase was extracted with ethyl acetate (60 ml*3). The organic phases were combined, and dried by rotary evaporation. The solute was separated and purified by column chromatography (petroleum ether: ethyl acetate = 5 : 1) to obtain compound A104-4 (120 mg, yield 5%).

### Synthesis of compound A104-5

A104-4 (120 mg, 0.3 mmol) was dissolved in anhydrous tetrahydrofuran (20 mL), and lithium aluminium tetrahydride (23 mg, 0.6 mmol) was added in an ice bath. The mixture was reacted at 0 °C for 1 h, and then TLC showed that the reaction was completed. The reaction liquid was quenched with saturated sodium bicarbonate solution (50 ml). The aqueous phase was extracted with ethyl acetate (50 mL*3). The organic phases were combined and then washed with saturated saline solution (20 ml*3) . The solution was dried by rotary evaporation. The solute was separated and purified by the preparative TLC (petroleum ether: ethyl acetate = 1 : 1) to obtain compound A104-5 (110 mg, yield 98%).

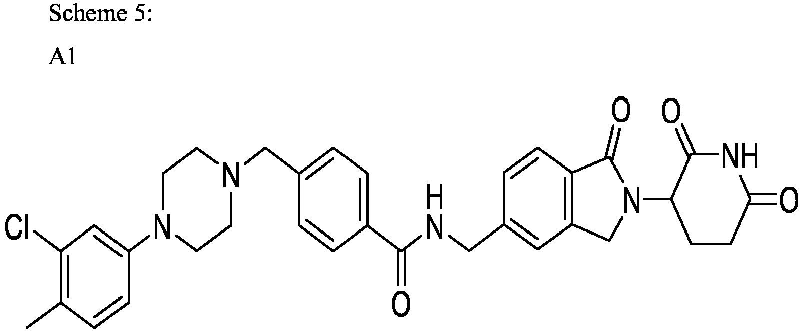

### Synthesis of compound A1-2

A13-4 (150 mg, 0.60 mmol), A13-4 (160 mg, 0.60 mmol) and triethylamine (250 mg, 2.50 mmol) were dissolved in 5 mL of DMF, HATU (350 mg, 0.90 mmol) was added. The mixture was reacted at ambient temperature for 3 h, and then TLC showed that the reaction was completed. The reaction liquid was diluted with ethyl acetate (50 mL). The organic phase was washed with saturated saline solution (20 ml*3), and the solution was dried by rotary evaporation. The solute was separated by a preparative TLC (petroleum ether : ethyl acetate = 1 : 1) to obtain compound A1-2 (140 mg, yield 46%).

### Synthesis of compound A1-3

A1-2 (70 mg, 0.15 mmol) was dissolved in 1 mL of dichloromethane, and HCl/ethyl acetate (2 mL, 4 M) was added. The mixture was reacted at ambient temperature for 2 h, and then TLC showed that the reaction was completed. The reaction liquid was dried by rotary evaporation to obtain compound A1-3 (70 mg, crude product).

### Synthesis of compound A1

Triphosgene (23 mg, 0.08 mmol) was dissolved in 6 mL of dichloromethane, and a solution of compound A1-4 (30 mg, 0.20 mmol) and triethylamine (40 mg, 0.40 mmol) in dichloromethane (4 mL) was added dropwise at -15 °C. The mixture was reacted at -15 °C for 20 min, and then a solution of A1-3 (60 mg, 0.15 mmol) and triethylamine (75 mg, 0.75 mmol) in dichloromethane (6 mL) was added. The mixture was reacted continuously at room temperature for 1 h, and then TLC showed that the reaction was completed. Saturated sodium bicarbonate solution (20 mL) was added to quench the reaction. The aqueous phase was extracted with dichloromethane (30 ml*3). The organic phases were combined and dried by rotary evaporation. The solute was separated by a preparative TLC (dichloromethane : methanol = 10 : 1) and then purified by medium pressure preparation to obtain compound A1 (9 mg, yield 8%).

¹H NMR (400 MHz, DMSO) δ 10.98 (s, 1H), 10.24 (d, *J* = 10.1 Hz, 1H), 8.19 - 8.09 (m, 1H), 7.99 (s, 1H), 7.93 (dd, *J* = 7.0, 2.4 Hz, 1H), 7.81 (d, *J* = 8.0 Hz, 1H), 7.65 (d, *J* = 8.3 Hz, 1H), 7.62 - 7.49 (m, 3H), 7.49 - 7.43 (m, 1H), 7.40 (d, *J* = 6.4 Hz, 1H), 7.04 (t, *J* = 5.6 Hz, 1H), 5.08 (dd, *J* = 13.3, 5.1 Hz, 1H), 4.70 (d, *J* = 5.4 Hz, 2H), 4.35 (dd, *J* = 56.5, 17.4 Hz, 2H), 4.00 (d, *J* = 13.1 Hz, 2H), 2.96 - 2.84 (m, 1H), 2.77 - 2.66 (m, 2H), 2.64 - 2.55 (m, 1H), 2.42 - 2.33 (m, 1H), 2.33 - 2.25 (m, 2H), 2.04 - 1.89 (m, 2H), 1.65 (d, *J* = 10.9 Hz, 2H), 1.18 - 1.03 (m, 2H). ESI-MS(M+H)⁺: 568.2.

The preparation method of A2 compound refers to A1

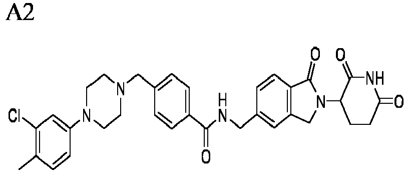

¹H NMR (400 MHz, DMSO) δ 11.01 (s, 1H), 8.66 (t, *J* = 5.7 Hz, 1H), 8.53 (dd, *J* = 7.6, 1.5 Hz, 1H), 8.47 (dd, *J* = 4.8, 1.4 Hz, 1H), 8.19 (d, *J* = 7.7 Hz, 1H), 8.05 (s, 1H), 7.97 (d, *J* = 8.0 Hz, 1H), 7.81 (d, *J* = 7.9 Hz, 1H), 7.65 (d, *J* = 8.2 Hz, 1H), 7.53 (t, *J* = 7.6 Hz, 1H), 7.29 - 7.18 (m, 2H), 6.63 (t, *J* = 5.4 Hz, 1H), 5.14 (dd, *J* = 13.3, 5.1 Hz, 1H), 4.54 - 4.37 (m, 4H), 3.84 (d, *J* = 13.1 Hz, 2H), 3.44 (dd, *J* = 11.6, 5.9 Hz, 2H), 3.15 (t, *J* = 6.2 Hz, 2H), 2.97 - 2.86 (m, 1H), 2.68 - 2.53 (m, 3H), 2.46 - 2.36 (m, 1H), 2.06 - 1.96 (m, 1H), 1.75 - 1.64 (m, 1H), 1.61 - 1.50 (m, 2H), 0.99 - 0.83 (m, 2H).

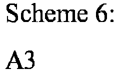

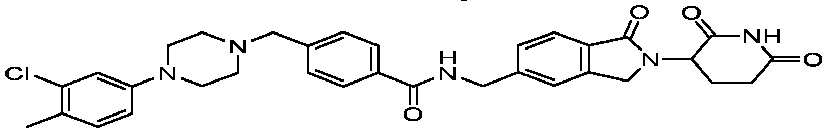

### Synthesis of compound A3-2

A3-1 (200 mg, 1.50 mmol) and triethylamine (303 mg, 3.01 mmol) were dissolved in 6 mL of dichloromethane, and chloroacetyl chloride (200 mg, 1.80 mmol) was added. The mixture was reacted at ambient temperature for 2 h, and then TLC showed that the reaction was completed. Saturated sodium bicarbonate solution (10 mL) was added to quench the reaction. Tthe aqueous phase was extracted with dichloromethane (10 ml*3). The organic phases were combined, and dried by rotary evaporation to obtain compound A3-2 (300 mg, crude product).

### Synthesis of compound A3

A3-2 (100 mg, 0.48 mmol), A1-3 (184 mg, 0.48 mmol) and DIPEA (123 mg, 0.96 mmol) were dissolved in 5 mL of DMF. The mixture was reacted overnight at 80 °C, and then TLC showed that the reaction was completed. Saturated sodium bicarbonate solution (10 mL) was added to quench the reaction. The aqueous phase was extracted with dichloromethane (10 ml*3). The organic phases were combined and then dried by rotary evaporation. The solute was separated by a preparative TLC (dichloromethane : methanol = 10 : 1) and then purified by medium pressure preparation to obtain compound A3 (11 mg, yield 4%).

¹H NMR (400 MHz, DMSO) δ 10.97 (s, 1H), 10.21 (s, 1H), 7.99 (s, 1H), 7.65 (d, *J* = 8.3 Hz, 1H), 7.58 (dd, *J* = 8.3, 1.5 Hz, 2H), 7.18-7.13 (m, 2H), 7.07 (t, *J* = 7.1 Hz, 1H), 5.08 (dd, *J* = 13.3, 5.1 Hz, 1H), 4.42 (d, *J* = 17.3 Hz, 1H), 4.28 (d, *J* = 17.3 Hz, 1H), 3.71 (t, *J* = 6.1 Hz, 2H), 3.25 (s, 2H), 2.97 - 2.87 (m, 1H), 2.78 (d, *J* = 9.9 Hz, 2H), 2.71 (t, *J* = 6.6 Hz, 2H), 2.60 (d, *J* = 17.3 Hz, 2H), 2.42 - 2.33 (m, 1H), 2.26 (d, *J* = 7.0 Hz, 2H), 2.09 - 1.96 (m, 4H), 1.93 - 1.85 (m, 2H), 1.73 (s, 1H), 1.62 (d, *J* = 11.2 Hz, 2H). ESI-MS(M+H)⁺: 558.2.

The preparation methods of compounds A4, A5, A6, A7, A8, A9 and A10 refer to A3. The characterization results are as follows:

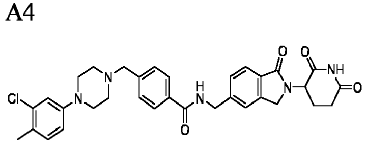

¹H NMR (400 MHz, DMSO) δ 11.03 (s, 1H), 10.31 (s, 1H), 8.05 (s, 1H), 7.94 (dd, *J* = 9.0, 6.1 Hz, 1H), 7.87 (dd, *J* = 11.8, 2.4 Hz, 1H), 7.72 (d, *J* = 8.3 Hz, 1H), 7.65 (d, *J* = 9.7 Hz, 1H), 7.43 (td, *J* = 8.7, 2.5 Hz, 1H), 7.32 (t, *J =* 7.8 Hz, 1H), 7.26 (d, *J* = 8.1 Hz, 1H), 6.61 (d, *J* = 7.5 Hz, 1H), 6.23 (t, *J* = 5.1 Hz, 1H), 5.14 (dd, *J* = 13.3, 5.1 Hz, 1H), 4.55 - 4.31 (m, 3H), 4.21 - 4.02 (m, 3H), 3.16 (t, *J* = 12.0 Hz, 1H), 3.04 - 2.91 (m, 1H), 2.78 - 2.62 (m, 2H), 2.50 - 2.41 (m, 1H), 2.39 (d, *J* = 7.0 Hz, 2H), 2.19 - 2.10 (m, 1H), 2.09 - 2.00 (m, 1H), 1.88 - 1.73 (m, 2H), 1.39 - 1.34 (m, 1H), 1.24 - 1.07 (m, 1H). ESI-MS(M+H)⁺: 586.2.

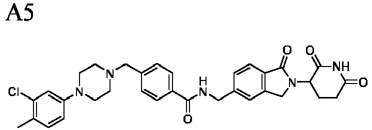

¹H NMR (400 MHz, DMSO) δ 10.97 (s, 1H), 10.21 (s, 1H), 8.19 (t, *J* = 6.0 Hz, 1H), 8.14 - 8.07 (m, 1H), 7.98 (s, 1H), 7.96 - 7.92 (m, 1H), 7.84 (d, *J* = 7.8 Hz, 1H), 7.65 (d, *J=* 8.3 Hz, 1H), 7.60 - 7.49 (m, 3H), 7.49 - 7.41 (m, 2H), 5.08 (dd, *J* = 13.2, 5.1 Hz, 1H), 4.76 (d, *J* = 5.9 Hz, 2H), 4.35 (dd, *J* = 55.9, 17.3 Hz, 2H), 2.96 (s, 2H), 2.94 - 2.85 (m, 1H), 2.77 (d, *J =* 11.5 Hz, 2H), 2.67 - 2.57 (m, 1H), 2.43 - 2.32 (m, 1H), 2.26 (d, *J* = 7.0 Hz, 2H), 2.10 - 1.94 (m, 3H), 1.80 - 1.67 (m, 1H), 1.62 - 1.56 (m, 2H), 1.32 - 1.24 (m, 2H). ESI-MS(M+H)⁺: 585.2.

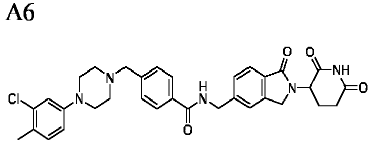

¹H NMR (400 MHz, DMSO) δ 11.00 (s, 1H), 8.63 (t, *J* = 5.7 Hz, 1H), 8.22 (t, *J* = 6.0 Hz, 1H), 8.11 (d, *J* = 7.5 Hz, 1H), 8.04 (s, 1H), 7.94 (dd, *J* = 10.2, 4.6 Hz, 2H), 7.85 - 7.78 (m, 2H), 7.59 - 7.49 (m, 2H), 7.49 - 7.39 (m, 2H), 5.13 (dd, *J* = 13.3, 5.1 Hz, 1H), 4.76 (d, *J* = 6.0 Hz, 2H), 4.45 (dd, *J* = 50.5, 17.6 Hz, 2H), 3.16 (t, *J* = 6.1 Hz, 2H), 2.96 (s, 2H), 2.95 - 2.85 (m, 1H), 2.79 (d, *J* = 11.0 Hz, 2H), 2.67 - 2.56 (m, 1H), 2.47 - 2.32 (m, 1H), 2.07 - 1.95 (m, 3H), 1.66 - 1.56 (m, 2H), 1.55 - 1.47 (m, 1H), 1.30 - 1.18 (m, 2H). ESI-MS(M+H)⁺: 585.2.

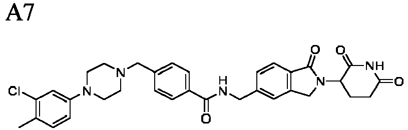

¹H NMR (400 MHz, DMSO) δ 11.03 (s, 1H), 8.65 (t, *J* = 5.7 Hz, 1H), 8.56 - 8.50 (m, 1H), 8.50 - 8.43 (m, 1H), 8.19 (d, *J* = 7.7 Hz, 1H), 8.06 (s, 1H), 7.97 (d, *J* = 8.1 Hz, 1H), 7.83 (t, *J* = 7.1 Hz, 2H), 7.71 (d, *J=* 8.2 Hz, 1H), 7.53 (t, *J* = 7.7 Hz, 1H), 7.31 - 7.18 (m, 2H), 5.15 (dd, *J* = 12.7, 4.5 Hz, 1H), 4.64 - 4.34 (m, 4H), 3.59 (q, *J* = 5.9 Hz, 2H), 3.14 (t, *J* = 6.0 Hz, 2H), 2.98 - 2.83 (m, 1H), 2.70 (s, 2H), 2.65 - 2.52 (m, 3H), 2.46 - 2.35 (m, 1H), 2.07 - 1.97 (m, 1H), 1.85 (t, *J* = 10.9 Hz, 2H), 1.58 - 1.39 (m, 3H), 1.16 - 1.01 (m, 2H). ESI-MS(M+H)⁺: 636.2.

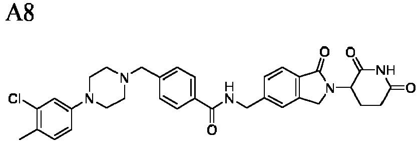

¹H NMR (400 MHz, DMSO) δ 10.98 (s, 1H), 10.22 (s, 1H), 8.52 (d, *J* = 7.7 Hz, 1H), 8.47 (d, *J* = 4.7 Hz, 1H), 8.19 (d, *J* = 7.8 Hz, 1H), 8.00 (s, 1H), 7.80 (t, *J* = 5.8 Hz, 1H), 7.71 (d, *J* = 8.3 Hz, 1H), 7.66 (d, *J* = 8.4 Hz, 1H), 7.59 (d, *J* = 8.3 Hz, 1H), 7.52 (t, *J* = 7.7 Hz, 1H), 7.27 - 7.23 (m, 2H), 5.08 (dd, *J* = 13.3, 5.1 Hz, 1H), 4.58 (t, *J* = 6.0 Hz, 2H), 4.36 (dd, *J* = 56.7, 17.4 Hz, 2H), 3.59 (d, *J* = 5.9 Hz, 2H), 2.98 - 2.81 (m, 1H), 2.70 (s, 2H), 2.68 - 2.52 (m, 3H), 2.42 - 2.30 (m, 1H), 2.24 (d, *J* = 6.9 Hz, 2H), 2.04 - 1.93 (m, 1H), 1.87 (t, *J* = 11.0 Hz, 2H), 1.73 - 1.58 (m, 1H), 1.50 (d, *J* = 12.3 Hz, 2H), 1.11 (dd, *J* = 21.3, 11.1 Hz, 2H). ESI-MS(M+H)⁺: 636.2.

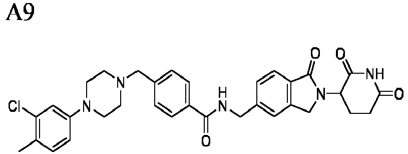

¹H NMR (400 MHz, DMSO) δ 10.98 (s, 1H), 10.29 (s, 1H), 10.02 (s, 1H), 8.01 (s, 1H), 7.96 (dd, *J* = 7.1, 2.2 Hz, 1H), 7.93 - 7.85 (m, 2H), 7.76 (d, *J* = 8.2 Hz, 1H), 7.66 (d, *J* = 8.3 Hz, 1H), 7.63 - 7.46 (m, 4H), 5.08 (dd, *J* = 13.3, 5.1 Hz, 1H), 4.35 (dd, *J* = 55.4, 17.4 Hz, 2H), 3.23 (s, 2H), 2.99 (d, *J* = 11.2 Hz, 2H), 2.95 - 2.86 (m, 1H), 2.65 - 2.55 (m, 1H), 2.43 - 2.32 (m, 3H), 2.27 (t, *J* = 10.9 Hz, 2H), 2.04 - 1.95 (m, 1H), 1.91 - 1.81 (m, 1H), 1.78 - 1.74 (m, 2H), 1.52 - 1.37 (m, 2H). ESI-MS(M+H)⁺: 568.2.

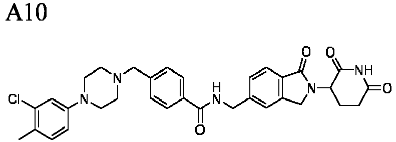

¹H NMR (400 MHz, DMSO) δ 10.98 (s, 1H), 10.24 (s, 1H), 8.22 (d, *J* = 8.3 Hz, 1H), 8.00 (s, 1H), 7.93 - 7.87 (m, 1H), 7.82 - 7.77 (m, 2H), 7.66 (d, *J* = 8.3 Hz, 1H), 7.62 - 7.48 (m, 3H), 7.46 - 7.38 (m, 1H), 7.36 (d, *J* = 6.0 Hz, 1H), 5.08 (dd, *J* = 13.3, 5.1 Hz, 1H), 4.35 (dd, *J* = 56.6, 17.4 Hz, 2H), 3.44 (dd, *J* = 14.2, 6.4 Hz, 2H), 3.21 (t, *J* = 7.3 Hz, 2H), 2.96 - 2.88 (m, 1H), 2.85 (s, 2H), 2.73 - 2.64 (m, 2H), 2.63 - 2.55 (m, 1H), 2.44 - 2.31 (m, 1H), 2.29 (d, *J* = 7.0 Hz, 2H), 2.05 - 1.94 (m, 3H), 1.80 - 1.68 (m, 1H), 1.66 - 1.55 (m, 2H), 1.34 - 1.17 (m, 2H). ESI-MS(M+H)⁺: 596.2.

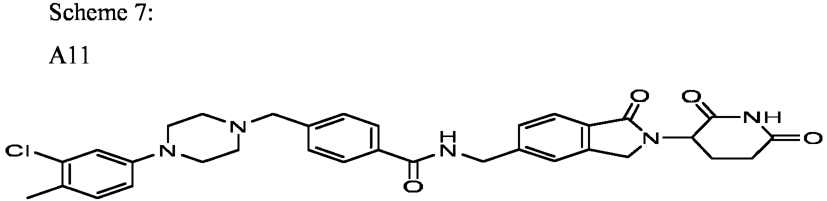

### Synthesis of compound A11-2

Compound A11-1 (3.0 g, 18 mmol) was dissolved in anhydrous tetrahydrofuran (40 ml), and NaH (2.8 g, 60%, 72 mmol) was added in portions in an ice bath. After addition, the reaction was stirred at ambient temperature for 1 h. The compound tert-butyl bromoacetate (3.5 ml, 21.5 mmol) was added, and the reaction was stirred at room temperature for another 2 h. TLC showed the reaction was completed. Water was added to quench the reaction, and ethyl acetate (40 ml*3) was added for extraction. The organic phases were combined and dried by rotary evaporation. The solute was separated and purified by column chromatography (petroleum ether: ethyl acetate = 5 : 1) to obtain compound A11-2 (4.7 g, yield 93%).

### Synthesis of compound A11-3

Compound A11-2 (300 mg, 1.06 mmol) was placed in a 25 mL roundbottomed flask, and 4 mL of (dichloromethane : trifluoroacetic acid =3:1) solution was added. The reaction was stirred at room temperature for 2 h. TLC showed the raw materials were completely reacted. The solvent was dried by rotary evaporation to obtain compound A11-3 (238 mg, crude product), which was directly used for next step.

### Synthesis of compound A11

A11-3 (36 mg, 0.16 mmol), A1-3 (70 mg, 0.14 mmol) and triethylamine (32 mg, 0.32 mmol) were dissolved in 6 mL of dichloromethane, HATU (60 mg, 0.16 mmol) was added. The mixture was reacted overnight at ambient temperature, and then TLC showed that the reaction was completed. The reaction liquid was diluted with water (20 mL). The organic phase was extracted with dichloromethane (40 ml*3). The organic phases were combined, and dried by rotary evaporation. The solute was separated and purified by a preparative TLC (dichloromethane : methanol = 10 : 1) and then subjected to medium pressure preparation to obtain compound A11 (40 mg, yield 46%).

¹H NMR (400 MHz, DMSO) δ 10.98 (s, 1H), 10.29 (s, 1H), 8.54 (dd, *J* = 7.6, 1.6 Hz, 1H), 8.42 (dd, *J* = 4.8, 1.6 Hz, 1H), 8.20 (d, *J* = 7.8 Hz, 1H), 8.01 (s, 1H), 7.66 (d, *J* = 8.3 Hz, 1H), 7.60 (d, *J* = 8.5 Hz, 1H), 7.56 - 7.43 (m, 2H), 7.30 - 7.21 (m, 2H), 5.40 (dd, *J* = 41.9, 17.0 Hz, 2H), 5.09 (dd, *J* = 13.3, 5.1 Hz, 1H), 4.48 - 4.11 (m, 4H), 3.27 - 3.17 (m, 1H), 2.97 - 2.74 (m, 1H), 2.72 - 2.55 (m, 2H), 2.43 - 2.28 (m, 3H), 2.16 - 1.94 (m, 2H), 1.88 - 1.67 (m, 2H), 1.41 - 1.03 (m, 2H). ESI-MS(M+H)⁺: 593.2.

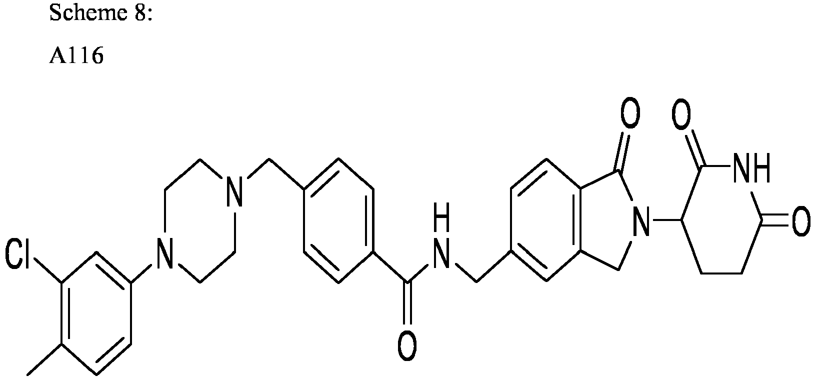

### Synthesis of compound A116-1

A89-1 (1.22 g, 5.0 mmol), A12-4 (1.07 g, 5.0 mmol) and potassium carbonate (2.07 g, 15.0 mmol) were dissolved in 10 mL of acetonitrile. The mixture was reacted at 80 °C for 3 h, and then TLC showed that the reaction was completed. The reaction liquid was diluted with water (50 mL). The organic phase was extracted with ethyl acetate (50 ml*3). The organic phases were combined, washed with saturated saline solution (20 ml*3) and then dried by rotary evaporation. The solute was separated and purified by column chromatography (petroleum ether: ethyl acetate = 5 : 1) to obtain compound A116-1 (1.65 g, yield 75%).

### Synthesis of compound A116-2

A116-1 (1.65 g, 3.78 mmol), A87-4 (588 mg, 3.78 mmol) and potassium carbonate (1.56 g, 11.3 mmol) were dissolved in dioxane/water (20 mL/5 mL), and Pd(dppf)Cl₂ (279 mg, 0.38 mmol) was added. The mixture was replaced with nitrogen three times and reacted at 80 °C for 3 h, and then TLC showed that the reaction was completed. The reaction liquid was filtered. The filtrate was diluted with water (50 mL). The aqueous phase was extracted with ethyl acetate (50 ml*3). The organic phases were combined, and dried by rotary evaporation. The solute was separated and purified by column chromatography (petroleum ether: ethyl acetate = 5 : 1) to obtain compound A116-2 (1.25 g, yield 70%).

### Synthesis of compound A116-3

A116-2 (235 mg, 0.50 mmol) was dissolved in 10 mL of dichloromethane, and HCl/dioxane (3 mL, 4 M) was added. The mixture was reacted at ambient temperature for 2 h, and then TLC showed that the reaction was completed. The reaction liquid was dried by rotary evaporation to obtain compound A116-3 (242 mg, crude product).

### Synthesis of compound A116

A116-3 (242 mg, 0.50 mmol) and DIPEA (194 mg, 1.50 mmol) were dissolved in 20 mL of DMF, and A116-4 (175 mg, 0.50 mmol) was added. The mixture was reacted overnight at ambient temperature, and then TLC showed that the reaction was completed. The reaction liquid was diluted with water (50 mL). The organic phase was extracted with ethyl acetate (50 ml*3). The organic phases were combined, washed with saturated saline solution (20 ml*3) and then dried by rotary evaporation. The solute was prepared under medium-pressure preparative chromatography to obtain compound A116 (48 mg, yield 15%).

¹H NMR (400 MHz, DMSO) δ 11.12 (s, 1H), 8.82 (d, *J* = 2.2 Hz, 1H), 8.28 (d, *J* = 2.3 Hz, 1H), 7.91 (s, 1H), 7.88 - 7.79 (m, 3H), 7.71 (d, *J* = 7.7 Hz, 1H), 7.49 (t, *J* = 7.8 Hz, 1H), 7.47 - 7.41 (m, 1H), 5.14 (dd, *J* = 12.9, 5.4 Hz, 1H), 3.88 (s, 2H), 3.70 - 3.58 (m, 2H), 2.98 - 2.82 (m, 3H), 2.65 - 2.53 (m, 2H), 2.41 (d, *J* = 6.5 Hz, 2H), 2.10 - 1.99 (m, 1H), 1.89 - 1.78 (m, 2H), 1.72 - 1.57 (m, 1H), 1.32 - 1.14 (m, 2H). ESI-MS(M+H)⁺: 640.2.

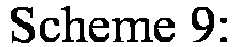

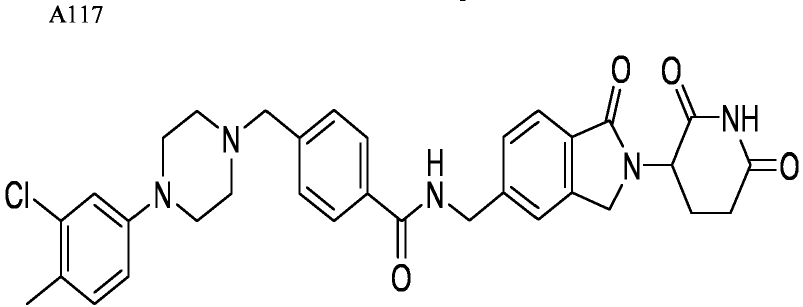

### Synthesis of compound A117-2

A13 (1.15 g, 1.96 mmol) was dissolved in 20 mL of DMF, and NaH (117 mg, 60%, 2.93 mmol) was added in an ice bath. The mixture was reacted in an ice bath for 30 min, and then A117-1 (506 mg, 1.96 mmol) was added. The mixture was reacted overnight at ambient temperature, and then TLC showed that the reaction was completed. Saturated sodium bicarbonate solution (20 mL) was added to quench the reaction. The aqueous phase was extracted with dichloromethane (100 ml*3). The organic phases were combined, washed with saturated saline solution (50 ml*3) and then dried by rotary evaporation. The solute was separated by column chromatography (dichloromethane : methanol = 20 : 1) to obtain compound A117-2 (220 mg, yield 14%).

### Synthesis of compound A117

A117-2 (220 mg, 0.27 mmol) was dissolved in HCl/dioxane (10 mL, 4 M). The mixture was reacted at ambient temperature for 1 h, and then LC-MS showed that the reaction was completed. The solution was dried by rotary evaporation, and the solute was purified by medium-pressure preparative chromatography to obtain compound A117 (11 mg, yield 6%).

¹H NMR (400 MHz, DMSO) δ 10.28 (s, 1H), 8.50 (s, 2H), 8.03 - 8.00 (m, 2H), 7.96 (d, *J* = 8.2 Hz, 1H), 7.84 (d, *J* = 7.8 Hz, 1H), 7.68 (d, *J* = 8.3 Hz, 1H), 7.65 - 7.52 (m, 4H), 7.46 (d, *J* = 6.6 Hz, 1H), 5.42 (d, *J* = 7.3 Hz, 2H), 5.22 (dd, *J* = 13.5, 5.0 Hz, 1H), 4.79 - 4.70 (m, 2H), 4.37 (dd, *J* = 58.9, 17.3 Hz, 2H), 3.07 - 2.96 (m, 3H), 2.86 - 2.78 (m, 1H), 2.42 - 2.30 (m, 3H), 2.21 - 2.10 (m, 1H), 2.07 - 1.99 (m, 1H), 1.87 - 1.77 (m, 2H), 1.35 - 1.25 (m, 2H). ESI-MS(M+H)⁺: 699.2.

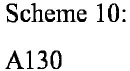

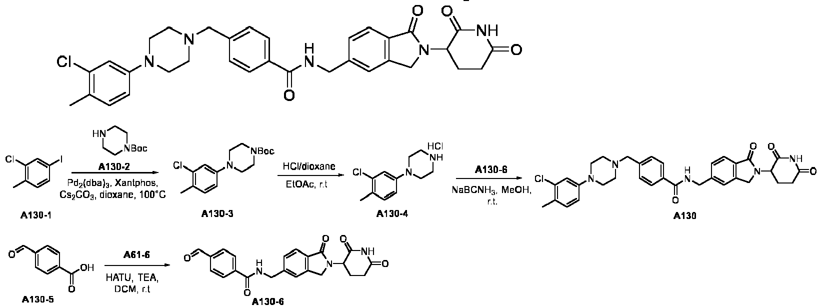

### Synthesis of compound A130-2

A130-1 (200 mg, 0.80 mmol), A130-2 (180 mg, 0.95 mmol) and cesium carbonate (520 mg, 1.60 mmol) were dissolved in dioxane (25 mL), and Pd₂(dba)₃ (140 mg, 0.16 mmol) and Xantphos (185 mg, 0.32 mmol) were added. The mixture was reacted at 100 °C under nitrogen protection for 4 h, and then TLC showed that the reaction was completed. The reaction liquid was filtered. The filtrate was diluted with water (60 mL), and then extracted with ethyl acetate (60 ml*3). The organic phases were combined and dried by rotary evaporation. The crude product was separated and purified by column chromatography (petroleum ether: ethyl acetate = 5 : 1) to obtain compound A130-3 (210 mg, yield 85%).

### Synthesis of compound A130-4

A130-3 (210 mg, 0.67 mmol) was dissolved in 2 mL of ethyl acetate, and HCl/dioxane (2 mL, 4 M) was added. The mixture was reacted at ambient temperature for 2 h, and then TLC showed that the reaction was completed. The reaction liquid was dried by rotary evaporation to obtain compound A130-4 (200 mg, crude product), which was directly used for next step.

### Synthesis of compound A130-6

A130-5 (200 mg, 1.30 mmol), A61-6 (400 mg, 1.50 mmol) and triethylamine (540 mg, 5.30 mmol) were dissolved in 30 mL of dichloromethane, and HATU (750 mg, 2.0 mmol) was added. The mixture was reacted overnight at ambient temperature, and then TLC showed that the reaction was completed. The reaction liquid was filtered. The filter cake was rinsed with a small amount of dichloromethane to obtain compound A130-6 (400 mg, yield 74%).

### Synthesis of compound A130

A130-4 (200 mg, 1.30 mmol) and compound A130-6 (215 mg, 0.53 mmol) were dissolved in methanol/dichloromethane (6 mL/12 mL), and 1 drop of glacial acetic acid was added. The mixture was reacted at ambient temperature for 1 h, sodium cyanoborohydride (85 mg, 1.30 mmol) was added. The mixture was reacted at ambient temperature for another 2 h, and then TLC showed that the reaction was completed. Saturated sodium bicarbonate solution (20 mL) was added to quench the reaction. The aqueous phase was extracted with dichloromethane (80 mL*3). The organic phases were combined and washed with saturated saline solution (20 mL), and then dried by rotary evaporation. The crude product was separated and purified by column chromatography (dichloromethane:methanol = 10:1), and then subjected to medium-pressure preparative chromatography to obtain compound A130 (35 mg, yield 9%).

¹H NMR (400 MHz, DMSO) δ 10.98 (s, 1H), 9.12 (t, *J* = 6.0 Hz, 1H), 7.88 (d, *J* = 8.2 Hz, 2H), 7.69 (d, *J* = 7.8 Hz, 1H), 7.54 (s, 1H), 7.47 - 7.42 (m, 3H), 7.14 (d, *J* = 8.5 Hz, 1H), 6.92 (d, *J* = 2.4 Hz, 1H), 6.81 (dd, *J* = 8.5, 2.4 Hz, 1H), 5.10 (dd, *J* = 13.3, 5.1 Hz, 1H), 4.59 (d, *J* = 5.8 Hz, 2H), 4.37 (dd, *J* = 54.7, 17.4 Hz, 2H), 3.57 (s, 2H), 3.16 - 3.03 (m, 4H), 2.97 - 2.83 (m, 1H), 2.64 - 2.54 (m, 1H), 2.49 - 2.44 (m, 4H), 2.43 - 2.32 (m, 1H), 2.20 (s, 3H), 2.05 - 1.95 (m, 1H).ESI-MS(M+H)⁺: 600.3.

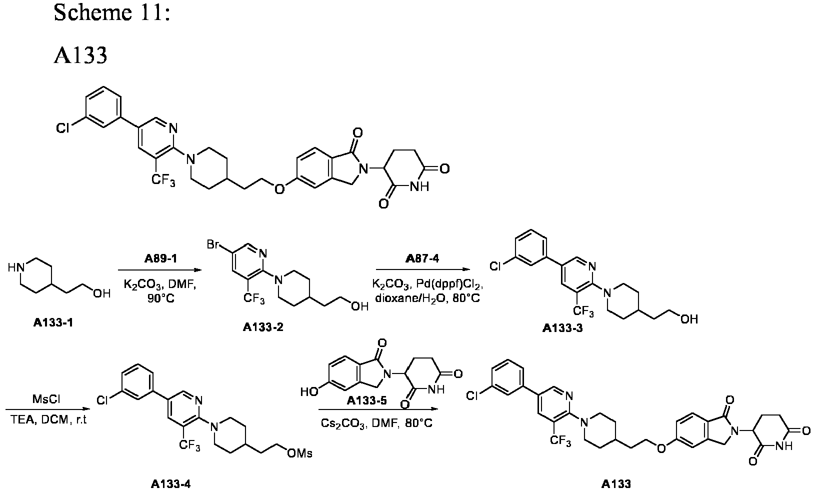

### Synthesis of compound A133-2

A89-1 (300 mg, 1.20 mmol), A133-1 (165 mg, 1.30 mmol) and potassium carbonate (480 mg, 3.50 mmol) were dissolved in 10 mL of DMF. The mixture was reacted at 90 °C for 2 h, and then TLC showed that the reaction was completed. The reaction liquid was diluted by adding water (100 ml). The aqueous phase was extracted with ethyl acetate (60 mL*3). The organic phases were combined, washed with saturated saline solution (20 ml*3) and then dried by rotary evaporation. The crude product was separated and purified by column chromatography (petroleum ether : ethyl acetate = 1 : 1) to obtain compound A133-2 (300 mg, yield 74%).

### Synthesis of compound A133-3

A133-2 (300 mg, 0.85 mmol), A87-4 (140 mg, 0.90 mmol) and potassium carbonate (240 mg, 1.70 mmol) were dissolved in dioxane/water (20 mL/5 mL) and tetrakis triphenylphosphine palladium (100 mg, 0.08 mmol) was added. The mixture was replaced with nitrogen three times and reacted at 80 °C for 3 h, and then TLC showed that the reaction was completed. The reaction liquid was filtered. The filtrate was diluted with ethyl acetate (60 mL). The organic phase was washed with saturated saline solution (20 ml*3), and the solution was dried by rotary evaporation. The crude product was separated and purified by column chromatography (petroleum ether : ethyl acetate = 1 : 1) to obtain compound A133-3 (300 mg, yield 88%).

### Synthesis of compound A133-4

A133-3 (200 mg, 0.52 mmol) and triethylamine (80 mg, 0.80 mmol) were dissolved in 5 mL of dichloromethane. Methanesulfonyl chloride (71 mg, 0.62 mmol) was added in an ice bath. The mixture was reacted at ambient temperature for 1 h, and then TLC showed that the reaction was completed. The reaction was quenched with 10 mL of ice water. The aqueous phase was extracted with dichloromethane (10 ml*3). The organic phases were combined and dried by rotary evaporation. The crude product was separated and purified by column chromatography (petroleum ether : ethyl acetate = 1 : 1) to obtain compound A133-4 (210 mg, yield 88%).

### Synthesis of compound A133

A133-4 (100 mg, 0.22 mmol), A133-5 (56 mg, 0.22 mmol) and cesium carbonate (84 mg, 0.26 mmol) were dissolved in 5 mL of DMF. The mixture was reacted at 80 °C for 3 h, and then TLC showed that the reaction was completed. The reaction liquid was diluted by adding water (40 ml). The aqueous phase was extracted with ethyl acetate (20 mL*3). The organic phases were combined, washed with saturated saline solution (20 ml*3), and dried by rotary evaporation. The crude product was separated and purified by column chromatography (dichloromethane : methanol = 15 : 1) to obtain compound A133 (10 mg, yield 8%).

¹H NMR (400 MHz, DMSO) δ 10.99 (s, 1H), 8.84 (d, *J* = 2.3 Hz, 1H), 8.31 (d, *J* = 2.4 Hz, 1H), 7.88 - 7.86 (m, 1H), 7.73 (d, *J* = 7.7 Hz, 1H), 7.63 (d, *J* = 8.4 Hz, 1H), 7.53 - 7.44 (m, 2H), 7.20 (s, 1H), 7.07 (dd, *J* = 8.4, 2.1 Hz, 1H), 5.08 (dd, *J* = 13.3, 5.1 Hz, 1H), 4.33 (dd, *J* = 51.4, 17.3 Hz, 2H), 4.16 (t, *J* = 6.1 Hz, 2H), 3.71 - 3.61 (m, 2H), 3.01 - 2.86 (m, 3H), 2.66 - 2.55 (m, 1H), 2.44 - 2.31 (m, 1H), 2.03 - 1.94 (m, 1H), 1.90 - 1.81 (m, 2H), 1.80 - 1.65 (m, 3H), 1.42 - 1.30 (m, 2H).ESI-MS(M+H)⁺: 626.2.

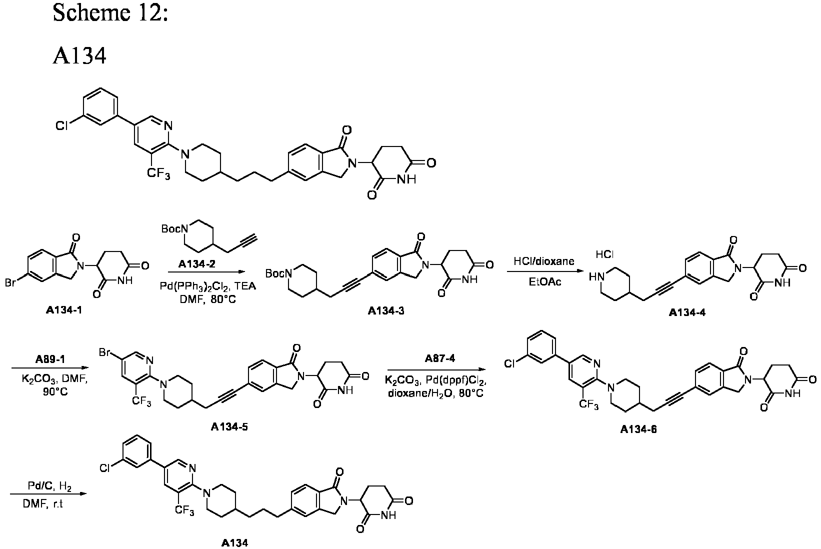

### Synthesis of compound A134-3

A134-1 (970 mg, 3.0 mmol), A134-2 (1.0 g, 4.5 mmol) and copper iodide (57 mg, 0.3 mmol) were dissolved in DMF/triethylamine (20 mL/1 mL), and Pd(PPh₃)₂Cl₂(210 mg, 0.30 mmol) was added. The mixture was replaced with nitrogen three times and reacted at 80 °C for 3 h, and then TLC showed that the reaction was completed. The reaction liquid was filtered. The filtrate was diluted with ethyl acetate (60 mL). The organic phase was washed with saturated saline solution (20 ml*3). The solution was dried by rotary evaporation. The crude product was separated and purified by column chromatography (dichloromethane : methanol = 20 : 1) to obtain compound A134-3 (750 mg, yield 54%).

### Synthesis of compound A134-4

A134-3 (466 mg, 1.0 mmol) was dissolved in 5 mL of ethyl acetate, and HCl/dioxane (5 mL, 4 M) was added. The mixture was reacted at ambient temperature for 2 h, and then TLC showed that the reaction was completed. The reaction liquid was dried by rotary evaporation to obtain compound A134-4 (500 mg, crude product), which was directly used for next step.

### Synthesis of compound A134-5

A89-1 (262 mg, 1.0 mmol), A134-4 (500 mg, 1.0 mmol) and potassium carbonate (414 mg, 3.0 mmol) were dissolved in 10 mL of DMF. The mixture was reacted at 90 °C for 3 h, and then TLC showed that the reaction was completed. The reaction liquid was diluted with water (100 ml). The aqueous phase was extracted with ethyl acetate (30 mL*3). The organic phases were combined, washed with saturated saline solution (30 ml*3), and dried by rotary evaporation. The crude product was separated and purified by column chromatography (petroleum ether: ethyl acetate = 1 : 2) to obtain compound A134-5 (450 mg, yield 76%).

### Synthesis of compound A134-6

A87-4 (119 mg, 0.76 mmol), A134-5 (450 mg, 0.76 mmol) and potassium carbonate (315 mg, 2.28 mmol) were dissolved in dioxane/water (20 mL/5 mL) and tetrakis triphenylphosphine palladium (100 mg, 0.08 mmol) was added. The mixture was replaced with nitrogen three times and reacted at 80 °C for 3 h, and then TLC showed that the reaction was completed. The reaction liquid was filtered. The filtrate was diluted with ethyl acetate (60 mL). The organic phase was washed with saturated saline solution (20 ml*3). The solution was dried by rotary evaporation. The crude product was separated and purified by column chromatography (petroleum ether : ethyl acetate = 1 : 2) to obtain compound A134-6 (225 mg, yield 48%).

### Synthesis of compound A134

A134-6 (122 mg, 0.2 mmol) was dissolved in (20 mL) DMF, and Pd/C (100 mg) was added. The mixture was reacted for 5 h at ambient temperature under hydrogen atmosphere, and then TLC showed that the reaction was completed. The reaction liquid was filtered. The filtrate was evaporated to dryness to obtain a crude product. The crude product was purified by medium-pressure preparative chromatography to obtain A134 (25 mg, yield 20%)

¹H NMR (400 MHz, DMSO) δ 10.98 (s, 1H), 8.82 (s, 1H), 8.29 (s, 1H), 7.85 (s, 1H), 7.72 (d, *J* = 7.5 Hz, 1H), 7.64 (d, *J* = 7.6 Hz, 1H), 7.51 - 7.47 (m, 1H), 7.45 - 7.41 (m, 2H), 7.36 (d, *J* = 7.8 Hz, 1H), 5.10 (dd, *J* = 13.2, 4.8 Hz, 1H), 4.36 (dd, *J* = 53.7, 17.2 Hz, 2H), 3.68 - 3.56 (m, 2H), 2.98 - 2.83 (m, 3H), 2.71 (t, *J* = 7.1 Hz, 2H), 2.65 - 2.54 (m, 1H), 2.45 - 2.30 (m, 1H), 2.05 - 1.94 (m, 1H), 1.82 - 1.72 (m, 2H), 1.67 (s, 2H), 1.58 - 1.42 (m, 1H), 1.35 - 1.18 (m, 4H).ESI-MS(M+H)⁺: 625.3.

The preparation methods of compounds A135 and A165 refer to A134. The characterization results are as follows:

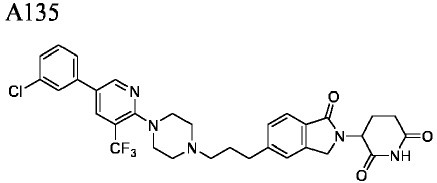

¹H NMR (400 MHz, DMSO) δ 10.98 (s, 1H), 8.85 (d, *J* = 2.2 Hz, 1H), 8.31 (d, *J* = 2.3 Hz, 1H), 7.87 (t, *J* = 1.7 Hz, 1H), 7.77 - 7.68 (m, 1H), 7.64 (d, *J* = 7.8 Hz, 1H), 7.55 - 7.42 (m, 3H), 7.38 (d, *J* = 7.9 Hz, 1H), 5.10 (dd, *J* = 13.3, 5.1 Hz, 1H), 4.36 (dd, *J* = 53.5, 17.2 Hz, 2H),3.34 - 3.26 (m, 8H), 2.96 - 2.85 (m, 1H), 2.74 (t, *J* = 7.5 Hz, 2H), 2.64 - 2.58 (m, 1H), 2.45 - 2.31 (m, 3H), 2.03 - 1.92 (m, 1H), 1.86 - 1.70 (m, 2H).ESI-MS(M+H)⁺: 626.3.

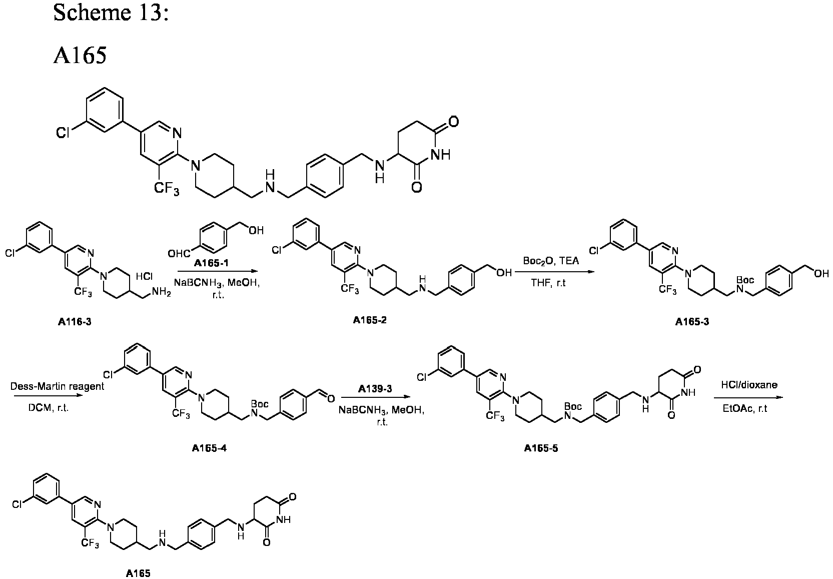

### Synthesis of compound A165-2

A116-3 (190 mg, 0.47 mmol) and compound A165-1 (64 mg, 0.47 mmol) were dissolved in 8 mL of methanol, and 1 drop of glacial acetic acid was added. The mixture was reacted at ambient temperature for 10 min, and then sodium cyanoborohydride (44 mg, 0.70 mmol) was added. The mixture was reacted overnight at ambient temperature, and then TLC showed that the reaction was completed. Saturated sodium bicarbonate solution (20 mL) was added to quench the reaction. The aqueous phase was extracted with dichloromethane (30 ml*3). The organic phases were combined and then dried by rotary evaporation. The crude product was separated and purified by column chromatography (petroleum ether : ethyl acetate = 2 : 1) to obtain compound A165-2 (150 mg, yield 65%).

### Synthesis of compound A165-3

A165-2 (150 mg, 0.31 mmol) and triethylamine (46 mg, 0.46 mmol) were dissolved in 8 mL of tetrahydrofuran, and (Boc)₂O (100 mg, 0.46 mmol) was added. The mixture was reacted at ambient temperature for 1 h, and then TLC showed that the reaction was completed. Water (20 mL) was added to quench the reaction. The aqueous phase was extracted with ethyl acetate (30 mL*3). The organic phases were combined, and dried by rotary evaporation to obtain compound A165-3 (190 mg, crude product).

### Synthesis of compound A165-4

A165-3 (190 mg, 0.31 mmol) was dissolved in 10 mL of dichloromethane, and Dess-Martin reagent (195 mg, 0.46 mmol) was added. The mixture was reacted at ambient temperature for 2 h, and then TLC showed that the reaction was completed. The reaction liquid was diluted by adding dichloromethane (30 mL). The organic phase was washed with saturated sodium bicarbonate solution (20 ml*3), and then dried by rotary evaporation. The crude product was separated by column chromatography (petroleum ether : ethyl acetate = 1 : 1) to obtain compound A165-4 (130 mg, yield 72%).

### Synthesis of compound A165-5

A165-4 (130 mg, 0.22 mmol) and compound A139-3 (73 mg, 0.44 mmol) were dissolved in 8 mL of methanol, and 1 drop of glacial acetic acid was added. The mixture was reacted at ambient temperature for 10 min, and then sodium cyanoborohydride (21 mg, 0.33 mmol) was added. The mixture was reacted overnight at ambient temperature, and then TLC showed that the reaction was completed. Saturated sodium bicarbonate solution (20 mL) was added to quench the reaction. The aqueous phase was extracted with dichloromethane (30 ml*3). The organic phases were combined and dried by rotary evaporation. The crude product was separated and purified by column chromatography (petroleum ether : ethyl acetate = 1 : 1) to obtain compound A165-5 (98 mg, yield 64%).

### Compound A165

A165-5 (70 mg, 0.1 mmol) was dissolved in 1 mL of dichloromethane, and HCl/dioxane (2 mL, 4 M) was added. The mixture was reacted at ambient temperature for 2 h, and then TLC showed that the reaction was completed. The reaction liquid was dried by rotary evaporation. The crude product was separated and purified by column chromatography (dichloromethane : methanol = 15 : 1) to obtain compound A165 (65 mg, yield 97%).

¹H NMR (400 MHz, DMSO) δ 11.37 (s, 1H), 8.85 (d, *J* = 2.3 Hz, 1H), 8.32 (d, *J* = 2.3 Hz, 1H), 7.87 (t, *J* = 1.7 Hz, 1H), 7.78 - 7.69 (m, 1H), 7.67 - 7.61 (m, 4H), 7.53 - 7.44 (m, 2H), 4.42 - 4.22 (m, 3H), 4.18 (s, 2H), 3.68 - 3.56 (m, 2H), 2.93 (t, *J* = 11.6 Hz, 2H), 2.88 - 2.78 (m, 2H), 2.73 - 2.61 (m, 2H), 2.46 - 2.36 (m, 1H), 2.25 - 2.10 (m, 1H), 2.06 - 1.96 (m, 1H), 1.93 - 1.85 (m, 2H), 1.39 - 1.26 (m, 2H).ESI-MS(M+H)⁺: 600.3.

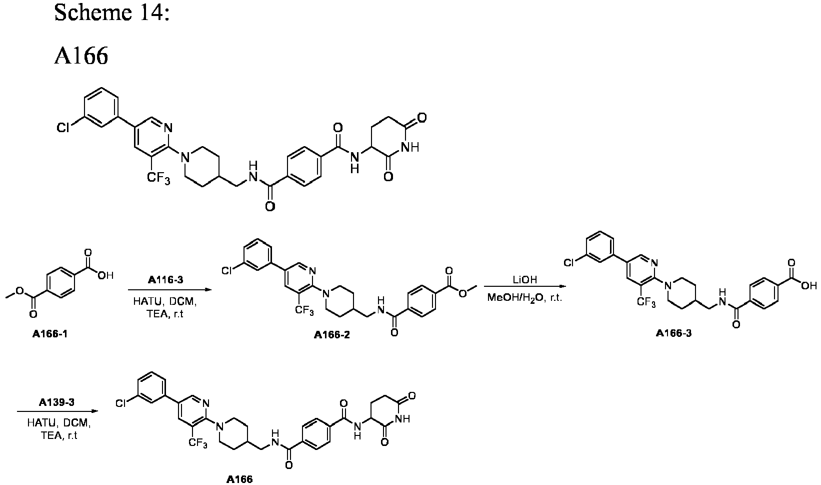

### Synthesis of compound A166-2

A166-1 (461 mg, 2.56 mmol), HATU (1.07 g, 2.81 mmol) and triethylamine (569 mg, 5.63 mmol) were dissolved in 15 mL of dichloromethane, and A116-3 (1.04 g, 2.56 mmol) was added. The mixture was reacted at ambient temperature for 2 h, and then TLC showed that the reaction was completed. The reaction liquid was quenched with water (20 mL). The aqueous phase was extracted with ethyl acetate (30 mL*3). The organic phase were combined and washed with saturated saline solution (10 ml*3) and then dried by rotary evaporation. The crude product was separated and purified by column chromatography (petroleum ether : ethyl acetate = 3 : 1) to obtain compound A166-2 (1.36 g, yield 100%).

### Synthesis of compound A166-3

A166-2 (1.61 g, 2.56 mmol) was dissolved in methanol/water (10 mL/3 mL), and lithium hydroxide (255 mg, 6.06 mmol) was added. The mixture was reacted overnight at 50 °C, and then TLC showed that the reaction was completed. Water (50 mL) was added to the reaction liquid to quench the reaction. 1 M hydrochloric acid was used to adjust the pH of the solution to 3-4. The precipitated solid was filtered. The filter cake was rinsed with water and dried to obtain compound A166-3 (1.29 g, yield 97%).

### Synthesis of compound A166

A166-3 (192 mg, 0.37 mmol), HATU (115 mg, 0.41 mmol) and triethylamine (83 mg, 0.82 mmol) were dissolved in 10 mL of dichloromethane, and A139-3 (67 mg, 0.41 mmol) was added. The mixture was reacted at ambient temperature for 2 h, and then TLC showed that the reaction was completed. The reaction liquid was quenched with water (20 mL). The aqueous phase was extracted with dichloromethane (30 mL*3). The organic phase was washed with saturated saline solution (10 ml*3) and then dried by rotary evaporation. The crude product was separated and purified by column chromatography (dichloromethane : methanol = 15 : 1) to obtain compound A166 (90 mg, yield 39%).

¹H NMR (400 MHz, DMSO) δ 10.89 (s, 1H), 8.90 (d, *J* = 8.3 Hz, 1H), 8.83 (d, *J* = 2.1 Hz, 1H), 8.69 (t, *J* = 5.7 Hz, 1H), 8.30 (d, *J* = 2.3 Hz, 1H), 7.96 (s, 4H), 7.86 (s, 1H), 7.72 (d, *J* = 7.7 Hz, 1H), 7.55 - 7.41 (m, 2H), 4.87 - 4.71 (m, 1H), 3.71 - 3.60 (m, 2H), 3.24 (t, *J* = 5.7 Hz, 2H), 2.92 (t, *J* = 12.0 Hz, 2H), 2.87 - 2.71 (m, 1H), 2.60 - 2.53 (m, 1H), 2.20 - 2.07 (m, 1H), 2.05 - 1.95 (m, 1H), 1.88 - 1.73 (m, 3H), 1.41 - 1.25 (m, 2H).ESI-MS(M+H)⁺: 628.2.

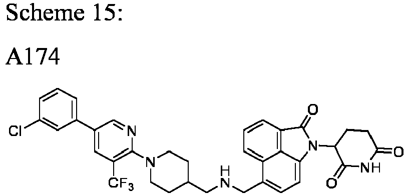

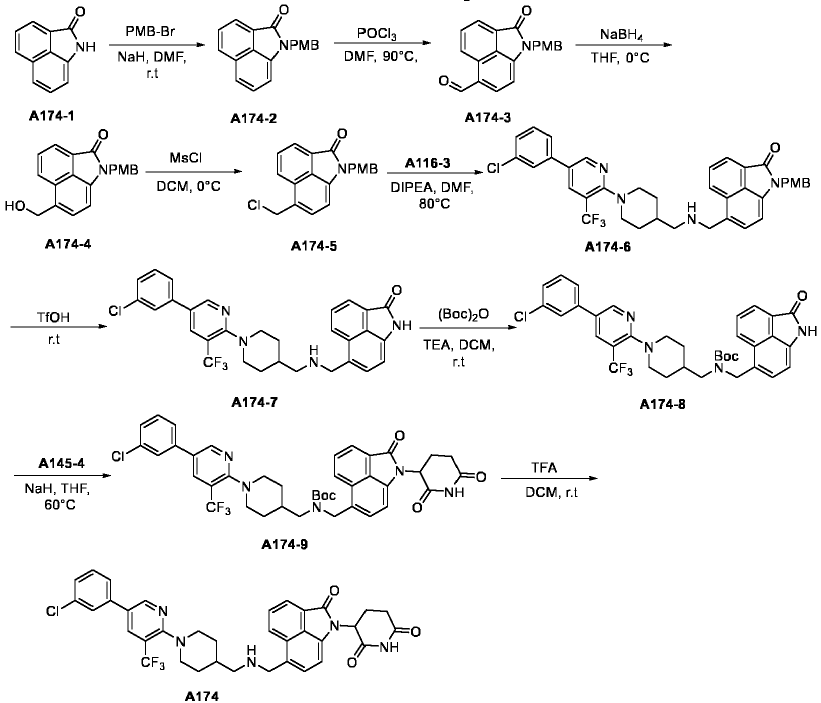

### Synthesis of compound A174-2

A174-1 (1.69 g, 10.0 mmol) was dissolved in 20 mL of DMF, and sodium hydride (600 mg, 60%, 15 mmol) was added in an ice bath. The mixture was reacted at 0 °C for 30 min, and then p-methoxybenzyl bromide (2.01 g, 10.0 mmol) was added. The mixture was reacted continuously overnight at ambient temperature, and then TLC showed that the reaction was completed. The reaction liquid was quenched with water (50 mL). The aqueous phase was extracted with ethyl acetate (30 mL*3). The organic phases were combined, washed with saturated saline solution (10 mL*3) and then dried by rotary evaporation. The crude product was separated by column chromatography (petroleum ether : ethyl acetate = 2 : 1) to obtain compound A174-2 (2.15 g, yield 74%).

### Synthesis of compound A174-3

In an ice bath, phosphorus oxychloride (15 mL) was added to 15 mL of DMF. The mixture was reacted at 0 °C for 30 min, and then A174-2 (2.15 g, 7.44 mmol) was added. The mixture was warmed to 90 °C and reacted overnight, and then TLC showed that the reaction was completed. The reaction liquid was poured into ice water (50 mL). The aqueous phase was extracted with ethyl acetate (30 mL*3). The organic phases were combined, washed with saturated saline solution (10 mL*3) and then dried by rotary evaporation. The crude product was separated by column chromatography (petroleum ether : ethyl acetate = 2 : 1) to obtain compound A174-3 (1.25 g, yield 53%).

### Synthesis of compound A174-4

A174-3 (951 mg, 3.0 mmol) was dissolved in 30 mL of tetrahydrofuran. In an ice bath, sodium borohydride (114 mg, 3.0 mmol) was added. The mixture was reacted at 0 °C for 1 h, and then TLC showed that the reaction was completed. The reaction liquid was quenched with water (50 mL). The aqueous phase was extracted with ethyl acetate (50 mL*3). The organic phases were combined and washed with saturated saline solution (20 mL*3), and then dried by rotary evaporation. The crude product was separated by column chromatography (petroleum ether : ethyl acetate = 1 : 1) to obtain compound A174-4 (900 mg, yield 94%).

### Synthesis of compound A174-5

A174-4 (900 mg, 2.82 mmol) and triethylamine (854 mg, 8.46 mmol) were dissolved in 20 mL of dichloromethane. Methylsufonyl chloride (484 mg, 4.23 mmol) was added in an ice bath. The mixture was reacted at 0 °C for 30 min, and then TLC showed that the reaction was completed. The reaction liquid was quenched with water (10 mL). The aqueous phase was extracted with dichloromethane (20 mL*3). The organic phases were combined and washed with saturated saline solution (10 mL), and then dried by rotary evaporation. The crude product was separated by column chromatography (petroleum ether : ethyl acetate = 2 : 1) to obtain compound A174-5 (850 mg, yield 89%).

### Synthesis of compound A174-6

A174-5 (674 mg, 2.0 mmol) and DIPEA (774 mg, 6.0 mmol) were dissolved in 20 mL of DMF, and A116-3 (740 mg, 2.0 mmol) was added. The mixture was reacted overnight at 80 °C, and then TLC showed that the reaction was completed. The reaction liquid was quenched with water (50 mL). The aqueous phase was extracted with ethyl acetate (30 mL*3). The organic phases were combined and washed with saturated saline solution (20 mL*3), and then dried by rotary evaporation. The crude product was separated by column chromatography (dichloromethane : methanol = 20 : 1) to obtain compound A174-6 (550 mg, yield 41%).

### Synthesis of compound A174-7

A174-6 (550 mg, 0.82 mmol) was dissolved in 5 mL of trifluoromethanesulfonic acid. The mixture was reacted overnight at ambient temperature, and then TLC showed that the reaction was completed. The solvent was dried by rotary evaporation to obtain compound A174-7 (600 mg, crude product), which was directly used for next step.

### Synthesis of compound A174-8

A174-7 (600 mg, 0.82 mmol) and triethylamine (249 mg, 2.46 mmol) were dissolved in 10 mL of dichloromethane, and (Boc)₂O (266 mg, 1.23 mmol) was added. The mixture was reacted at ambient temperature for 5 h, and then TLC showed that the reaction was completed. Water (20 mL) was added to quench the reaction. The aqueous phase was extracted with ethyl acetate (30 mL*3). The organic phases were combined and then dried by rotary evaporation. The crude product was separated by column chromatography (dichloromethane : methanol = 20 : 1) to obtain compound A174-8 (350 mg, yield 65%).

### Synthesis of compound A174-9

A174-8 (261 mg, 0.4 mmol) was dissolved in 20 mL of anhydrous tetrahydrofuran, and sodium hydride (160 mg, 60%, 4 mmol) was added in an ice bath. The mixture was reacted at 0 °C for 30 min, and then A145-4 (2.01 g, 10.0 mmol) was added. The mixture was warmed to 60 °C and reacted for 5 h, and then TLC showed that the reaction was completed. The reaction liquid was quenched with water (50 mL). The aqueous phase was extracted with ethyl acetate (30 mL*3). The organic phases were combined, washed with saturated saline solution (10 mL*3) and then dried by rotary evaporation. The crude product was separated by column chromatography (dichloromethane : methanol = 20 : 1) to obtain compound A174-9 (150 mg, yield 49%).

### Synthesis of compound A174

A174-9 (150 mg, 0.2 mmol) was dissolved in 10 mL of dichloromethane, and 2 mL of trifluoroacetic acid was added. The mixture was reacted at ambient temperature for 1 h, and then TLC showed that the reaction was completed. The solvent was dried by rotary evaporation. The crude product was separated by column chromatography (dichloromethane : methanol = 10 : 1) to obtain compound A174 (15 mg, yield 12%).

¹H NMR (400 MHz, DMSO) δ 8.83 (d, *J* = 2.2 Hz, 1H), 8.28 (d, *J* = 2.3 Hz, 1H), 7.69 - 7.47 (m, 4H), 7.55 - 7.46 (m, 2H), 7.25 - 7.18 (m, 1H), 5.22 (dd, *J* = 9.1, 6.3 Hz, 1H), 4.61 (d, *J* = 17.3 Hz, 1H), 4.35 (d, *J* = 17.3 Hz, 1H), 3.85 (s, 2H), 3.67 - 3.59 (m, 2H), 3.03 - 2.86 (m, 4H), 2.45 (d, *J* = 6.6 Hz, 2H), 1.88 - 1.79 (m, 2H), 1.74 - 1.62 (m, 1H), 1.34 - 1.24 (m, 2H).ESI-MS(M+H)⁺: 662.3.

The preparation methods of compound A175 refer to A174. The characterization results are as follows:

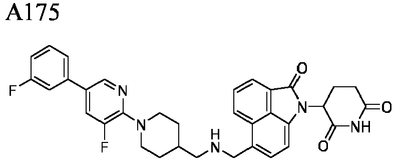

¹H NMR (400 MHz, DMSO) δ 10.55 (s, 1H), 8.82 (d, *J* = 2.1 Hz, 1H), 8.29 (d, *J* = 2.3 Hz, 1H), 7.86 (s, 1H), 7.72 (d, *J* = 7.6 Hz, 1H), 7.56 - 7.54 (m, 2H), 7.51 - 7.47 (m, 1H), 7.45 - 7.43 (m, 2H), 3.98 (s, 3H), 3.91 (t, *J* = 6.6 Hz, 2H), 3.78 (s, 2H), 3.68 - 3.58 (m, 2H), 2.91 (t, *J* = 12.1 Hz, 2H), 2.76 (t, *J* = 6.7 Hz, 2H), 2.45 - 2.39 (m, 2H), 1.86 - 1.77 (m, 2H), 1.70 - 1.57 (m, 1H), 1.33 - 1.17 (m, 2H).ESI-MS(M+H)⁺: 596.3.

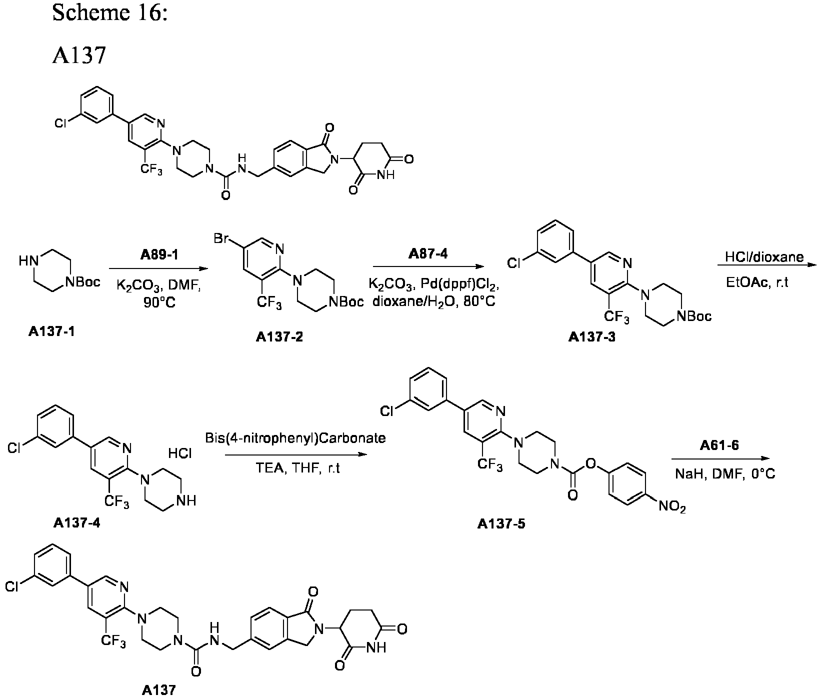

### Synthesis of compound A137-2

A89-1 (2 g, 7.7 mmol), 1-tert-butoxycarbonylpiperazine (1.7 g, 9.2 mmol) and potassium carbonate (2.1 g, 16.4 mmol) were dissolved in 30 mL of DMF. The mixture was reacted at 90 °C for 2 h, and then TLC showed that the reaction was completed. The reaction liquid was diluted by adding water (100 ml). The aqueous phase was extracted with ethyl acetate (60 mL*3). The organic phases were combined, washed with saturated saline solution (20 ml*3) and then dried by rotary evaporation. The crude product was separated and purified by column chromatography (petroleum ether : ethyl acetate = 5 : 1) to obtain compound A137-2 (2.5 g, yield 79%).

### Synthesis of compound A137-3

A137-3 (2.5 g, 6.1 mmol), A87-4 (1.0 g, 6.4 mmol) and potassium carbonate (1.7 g, 12.2 mmol) were dissolved in dioxane/water (16 mL/4 mL) and tetrakis triphenylphosphine palladium (700 mg, 0.6 mmol) was added. The mixture was replaced with nitrogen three times and then reacted at 80 °C overnight, and then TLC showed that the reaction was completed. The reaction liquid was filtered. The filtrate was diluted with ethyl acetate (60 mL). The organic phase was washed with saturated saline solution (20 ml*3), and the solution was dried by rotary evaporation. The crude product was separated and purified by column chromatography (petroleum ether : ethyl acetate = 1 : 1) to obtain compound A137-3 (2.4 g, yield 89%).

### Synthesis of compound A137-4

A137-3 (200 mg, 0.4 mmol) was dissolved in 5 mL of ethyl acetate, and HCl/dioxane (5 mL, 4 M) was added. The mixture was reacted at ambient temperature for 2 h, and then TLC showed that the reaction was completed. The reaction liquid was dried by rotary evaporation to obtain compound A137-4 (200 mg, crude product), which was directly used for next step.

### Synthesis of compound A137-5

A137-4 (200 mg, 0.4 mmol) and triethylamine (200 mg, 1.8 mmol) were dissolved in 10 mL of tetrahydrofuran, and bis(p-nitrobenzene)carbonate (210 mg, 0.7 mmol) was added. The mixture was reacted at ambient temperature for 2 h, and then TLC showed that the reaction was completed. The reaction liquid was diluted with water (20 mL). The aqueous phase was then extracted with ethyl acetate (20 mL*3). The organic phases were combined, washed with saturated saline solution (10 mL) and then dried by rotary evaporation. The crude product was separated and separated and purified by column chromatography (petroleum ether : ethyl acetate = 5 : 1) to obtain compound A137-5 (380 mg, yield 100%).

### Synthesis of compound A137

A137-5 (190 mg, 0.22 mmol) and A61-6 (30 mg, 0.1 mmol) were dissolved in 3 mL of DMF. Sodium hydride (20 mg, 60%, 0.44 mmol) was added in an ice bath. The mixture was reacted at ambient temperature for 2 h, and then TLC showed that the reaction was completed. 30 mL of saturated ammonium chloride solution was added to quench the reaction. The aqueous phase was extracted with ethyl acetate (20 mL*3). The organic phases were combined, washed with saturated saline solution (10 mL) and then dried by rotary evaporation. The crude product was separated and purified by column chromatography (dichloromethane : methanol = 15 : 1) to obtain compound A137 (35 mg, yield 21%).

¹H NMR (400 MHz, DMSO) δ 10.98 (s, 1H), 8.87 (s, 1H), 8.35 (d, *J* = 1.9 Hz, 1H), 7.87 (s, 1H), 7.70 (dd, *J* = 23.2, 7.7 Hz, 2H), 7.55 - 7.39 (m, 4H), 7.30 (t, *J* = 5.6 Hz, 1H), 5.10 (dd, *J* = 13.3, 5.0 Hz, 1H), 4.48 - 4.25 (m, 4H), 3.56 - 3.51 (m, 4H), 3.31 - 3.21 (m, 4H), 3.00 - 2.84 (m, 1H), 2.65 - 2.51 (m, 1H), 2.45 - 2.33 (m, 1H), 2.05 - 1.95 (m, 1H).ESI-MS(M+H)⁺: 641.4.

The preparation methods of compound A136 refer to A137. The characterization results are as follows:

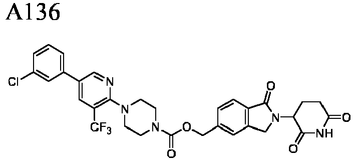

¹H NMR (400 MHz, DMSO) δ 10.99 (s, 1H), 8.88 (d, *J* = 2.1 Hz, 1H), 8.36 (d, *J* = 2.3 Hz, 1H), 7.88 (d, *J* = 1.6 Hz, 1H), 7.74 (d, *J* = 7.7 Hz, 2H), 7.63 (s, 1H), 7.56 - 7.44 (m, 3H), 5.24 (s, 2H), 5.12 (dd, *J* = 13.3, 5.1 Hz, 1H), 4.41 (dd, *J* = 54.4, 17.4 Hz, 2H), 3.68 - 3.51 (m, 4H), 3.29 - 3.22 (m, 4H), 2.99 - 2.83 (m, 1H), 2.65 - 2.54 (m, 1H), 2.46 - 2.28 (m, 1H), 2.05 - 1.93 (m, 1H).ESI-MS(M+H)⁺: 642.2.

The corresponding intermediates were prepared according to the following conditions:

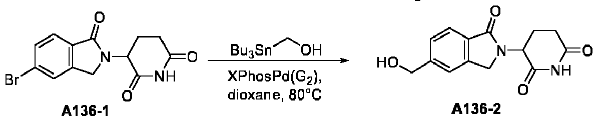

### Synthesis of compound A136-2

A136-1 (300 mg, 0.9 mmol) and (tributyltin)methanol (165 mg, 1.3 mmol) were dissolved in 20 mL of dioxane, and XphosPd(G2) (75 mg, 0.1 mmol) was added. The mixture was reacted overnight at 80 °C under nitrogen protection, and then TLC showed that the reaction was completed. The reaction liquid was filtered, and the filtrate was diluted with water (20 mL), and then extracted with ethyl acetate (20 mL*3). The organic phases were combined and washed with saturated saline solution (10 mL), and then dried by rotary evaporation. The crude product was separated and purified by column chromatography (dichloromethane : methanol = 10 : 1) to obtain compound A136-2 (190 mg, yield 74%).

### 2. Biological activity determination:

Biological example 1: Test of the anti-proliferation ability of the compound of the present invention on HL60 cells:

### 1. Cell plating

HL60 cells (Cell Bank of Chinese Academy of Sciences) in the logarithmic growth phase were counted and plated evenly in a 96-well transparent bottom white plate at 20000 cells per well and 100 µL per well.

### 2 Drug addition to cells

A certain amount of the compound of the present invention was weighed and diluted with DMSO to a 10 mM stock solution. The culture medium RPMI-1640 (meilunbio, MA0215) corresponding to the cells were taken, and the compound was diluted to the concentration required by the test, shaken gently and mixed evenly. 50 µL of the gradient dilution of the diluted compound was added to each well of the plated cells, and 50 µL of 0.1% DMSO was added to the control group. The plate was shaken gently, mixed evenly and incubated in a cell culture incubator for 3 days.

### 3 CTG detection

3.1 The CellTiter-Glo^{™} (CTG) (purchased from Promega, Cat. No. G9242) reagent was taken in advance from the -20 °C refrigerator and equilibrated to room temperature in the dark. Cell Titer-Glo buffer and Cell Titer-Glo substrate were mixed at a ratio of 1:1 to prepare a CTG detection reagent.

3.2 The 96-well plate was taken out and inspected under a microscope for growth.

3.3 The bottom of the 96-well plate was tagged with white paper, and then the plate was wrapped with tin foil paper, shaken at 37 °C at 300 r for 10 min and mixed evenly.

3.4. 50µL of CTG detection reagent was added to each well, Luminescence signal was detected using a microplate reader Biotek Synergy H1, and the inhibition rate was calculated. IC₅₀ values of the compounds were calculated by XLfit 5.5.0 software to evaluate the proliferation inhibitory activity on HL60 cells. The experimental results are shown in Table 1.

**Table 1 Anti-proliferation activity of compounds against HL60 cells**

| Compound | HL60 cells Proliferation inhibitory activity (nM) | Compound | HL60 cells Proliferation inhibitory activity (nM) | Compound | HL60 cells Proliferation inhibitory activity (nM) |
|---|---|---|---|---|---|
| A1 | 190 | A60 | 60 | A119 | 1.09 |
| A2 | 58 | A61 | 0.3 | A120 | 2.47 |
| A3 | 7.9 | A62 | 2 | A121 | 4.69 |
| A4 | 614 | A63 | 0.2 | A122 | 1.76 |
| A5 | 10 | A64 | 10 | A123 | <3.04 |
| A6 | 192 | A65 | <0.2 | A124 | <3.04 |
| A7 | 94 | A66 | <0.2 | A125 | 2.48 |
| A8 | 14 | A67 | 2 | A126 | 6.7 |
| A9 | 6 | A68 | <0.2 | A127 | 6.75 |
| A10 | 7.4 | A69 | 22 | A128 | 9.04 |
| A11 | 39 | A70 | <0.2 | A129 | 120 |
| A12 | 4.5 | A71 | <0.2 | A130 | 0.54 |
| A13 | 6.4 | A72 | 63 | A131 | 35.8 |
| A14 | 0.9 | A73 | <0.2 | A132 | 8.37 |
| A15 | 1 | A74 | 1.1 | A133 | 175 |
| A16 | 1.5 | A75 | <0.2 | A134 | 223 |
| A17 | 1.2 | A76 | <0.2 | A135 | 25.7 |
| A18 | 5.5 | A77 | <0.2 | A138 | 1105 |
| A19 | 29 | A78 | 3 | A139 | 17.7 |
| A20 | 21 | A79 | <0.2 | A140 | 17.1 |
| A21 | 2.9 | A80 | <0.2 | A141 | 160 |
| A22 | 4 | A81 | <0.2 | A142 | 397 |
| A23 | 0.2 | A82 | 1 | A143 | 3038 |
| A24 | 0.5 | A83 | 1.2 | A144 | 752 |
| A25 | 0.3 | A84 | 2.6 | A145 | 387 |
| A26 | <0.2 | A85 | 1 | A146 | 139 |
| A27 | 3.7 | A86 | 6.5 | A147 | 12320 |
| A28 | 0.9 | A87 | 0.6 | A148 | 8525 |
| A29 | 0.2 | A88 | 0.8 | A149 | 1743 |
| A30 | <0.2 | A89 | 9.2 | A150 | 3361 |
| A31 | <0.2 | A90 | 45.6 | A151 | 687 |
| A32 | 12 | A91 | 36.1 | A152 | 7316 |
| A33 | 0.3 | A92 | <0.2 | A153 | 11600 |
| A34 | <0.2 | A93 | 1.6 | A154 | 116 |
| A35 | <0.2 | A94 | 0.1 | A155 | 825 |
| A36 | 0.3 | A95 | <0.2 | A156 | 268 |
| A37 | 0.2 | A96 | 1.8 | A157 | 11700 |
| A38 | 0.9 | A97 | 0.4 | A158 | 6660 |
| A39 | 2.6 | A98 | 1 | A159 | 6700 |
| A40 | 4.3 | A99 | 1.2 | A160 | 1097 |
| A41 | 0.2 | A100 | <0.2 | A161 | 11200 |
| A42 | 0.2 | A101 | 10.9 | A162 | 231 |
| A43 | <0.2 | A102 | 0.5 | A163 | 8224 |
| A44 | 5 | A103 | 1.5 | A164 | 7231 |
| A45 | <0.2 | A104 | 20.5 | A165 | 9557 |
| A46 | <0.2 | A105 | 55 | A166 | 2666 |
| A47 | <0.2 | A106 | 878 | A167 | 9575 |
| A48 | 4.1 | A107 | 9.1 | A168 | 109 |
| A49 | 1.5 | A108 | <0.2 | A169 | 46.3 |
| A50 | 0.6 | A109 | 6.2 | A170 | 9856 |
| A51 | <0.2 | A110 | <0.2 | A171 | 1566 |
| A52 | 1.4 | A111 | 1.3 | A172 | 9994 |
| A53 | <0.2 | A112 | <0.2 | A173 | 9372 |
| A54 | <0.2 | A113 | 3 | A174 | 3924 |
| A55 | <0.2 | A114 | 11 | A175 | 11520 |
| A56 | <0.2 | A115 | 1.5 | A176 | 5211 |
| A57 | <0.2 | A116 | 106 | A177 | 4353 |
| A58 | <0.2 | A117 | 12 | | |
| A59 | 0.7 | A118 | 3.97 | | |

Biological example 2: Test of the ability of compounds to degrade c-Myc and GSPT1 proteins in HL60 cells:

### 1 Cell drug treatment and protein extraction

### 1.1 Cell treatment

HL60 cells in the exponential growth phase were digested and plated into a 6-well plate at 1 ×10⁶ cells per well. The cell culture medium was RPMI-1640 (meilunbio, MA0215). After a day of adherent growth of the cells, the compound of the present invention was added as a test drug for culture, and proteins were extracted 24 h later (Ctrl meant adding only DMSO at the same volume).

### 1.2 Cell protein extraction

In the 6-well plate, the culture medium RPMI-1640 (meilunbio, MA0215) was removed, and the cells were washed once with PBS, digested with trypsin, and centrifuged and collected separately into 1.5 mL centrifuge tubes. 100 µL of RIPA lysis buffer (containing 100 µM PMSF) was added to each tube, mixed thoroughly, allowed to stand on ice for 30 min and then centrifuged at 12000 rpm at 4 °C using a centrifuge for 20 min. The supernatant was used for Western blotting experiments. The sample can be stored at -80 °C.

### 1.3 Determination of protein concentration

By using the BCA protein concentration determination kit (from Thermo Fisher, Cat. No. 23225), the BSA standard determination solution and the samples to be tested (the samples to be tested can be diluted and then tested) were prepared according to the table A below. Samples were added to the 96-well plate. Each well was supplemented with PBS to 20 µL, and then 200 µL of BCA working solution (prepared according to the kit) was added respectively and mixed evenly. The plate was placed at 60 °C for 10 min, and then the absorbance at 562 nm was detected. After the reading was recorded, a standard curve was plotted using concentration gradients of the standards, and the absorbance of the samples was substituted to calculate the protein concentration of the samples.

**Table A Preparation of protein quantitative standards**

| Number | 0 | 1 | 2 | 3 | 4 | 5 | 6 | 7 | 8 |
|---|---|---|---|---|---|---|---|---|---|
| | 1 mg/ml BSA standard solution (µl) | | | | | | | 5 mg/ml BSA standard solution (µl) | |
| BSA standard solution µL | 0 | 0.5 | 2.5 | 5.0 | 10 | 15 | 20 | 6 | 8 |
| PBS solution µL | 20 | 19.5 | 17.5 | 15 | 10 | 5 | 0 | 14 | 12 |
| BSA final concentration µg/mL | 0 | 25 | 125 | 250 | 500 | 750 | 1000 | 1500 | 2000 |
| Total volume µL | 20 µL | | | | | | | | |

### 2 Standard procedure for Western blotting experiment

2.1 Protein denaturation: RIPA protein buffer (purchased from Solarbio, Cat. No. R0010) and 5 × Loading Buffer (SDS, glycerin, bromophenol blue, TRIS) (purchased from Solarbio, Cat. No. P1040) were added, and the protein samples were denatured at 100 °C for 5 min.

2.2 Sample loading and electrophoresis: 10% precast gel and the corresponding electrophoresis buffer were used. The same mass of protein sample and protein ladder (purchased from thermo, Cat. No. 26617) were loaded into each well, and electrophoresis at 200 V was performed for 30 min.

2.3 Blocking: The gel was excised to remove the excess part. Transferring to PVDF membrane was performed by the wet transfer method (before use, it was necessary to activate the PVDF membrane with methanol for 1 min) at 300 mA for 2 h, during which a lot of heat was generated, and thus cooling with an ice box was required.

2.4 Blocking: After the transferring, the PVDF membrane was placed in 5% skim milk and blocked with shaking at room temperature for 1 h.

2.5 Incubating with primary antibody: The PVDF membrane was cut according to the molecular weight indicated on the marker and placed in c-myc (purchased from abcam, Cat. No. ab32072), or GSPT1 (purchased from abeam, Cat. No. ab234433), or CK1α (purchased from abcam, Cat. No. ab206652), or IKZF2 (purchased from proteintech, Cat. No. 13554-1-AP), and GAPDH (purchased from CST, Cat. No. #97166) primary antibodies. The antibodies were diluted with TBST buffer (a solution prepared from TRIS, KCL and NaCl and adjusted to pH 7.4 with hydrochloric acid, and Tween 20 was added) at a ratio of 1:1000, and blocked overnight on a shaker at 4 °C.

2.6 Incubating with secondary antibody: The PVDF membrane incubated with the primary antibody was washed with TBST for 3 times, each time for 10 min. After washing, the membrane was placed in the secondary antibody of the corresponding species, and incubated at room temperature in a shaker for 2 h.

2.7 Washing membrane and exposure: After incubated with the secondary antibodies, the membrane was washed 3 times with TBST on a shaker, 10 minutes each time. After washed, fluorescence on the membrane was excited by ECL method.

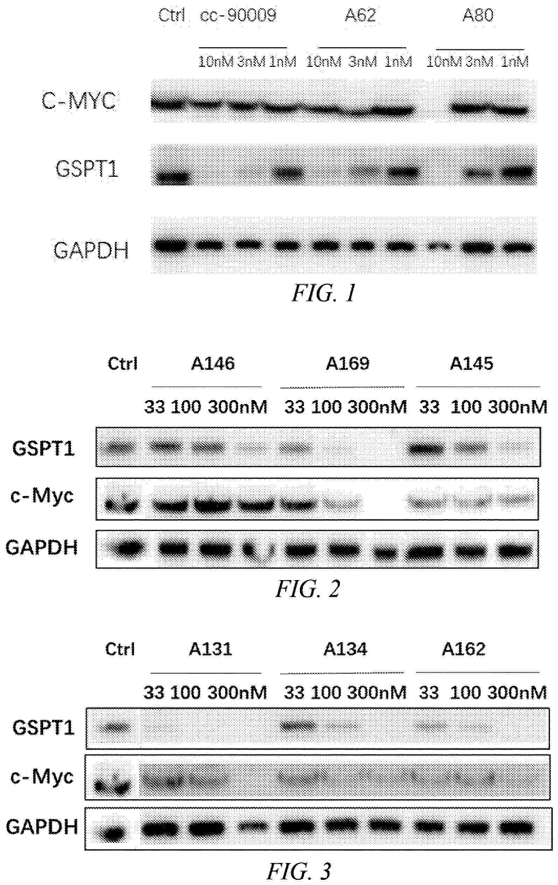

2.8 Remaining amount of protein: Image J (1.8.0) software was used to measure the gray value of the Western blot, which corresponded to the remaining amount of protein. The experimental results are shown in Table 2-Table 4, and Fig. 1-Fig. 3.

Fig. 1 shows that compounds A62 and A80 can significantly degrade GSPT1 protein in HL60 cells, wherein A80 can simultaneously completely degrade both GSPT1 and c-Myc in cells at 10 nM, while GSPT1 degradation agent CC-90009 has no ability to degrade c-Myc protein at the same concentration; Fig. 2 shows that compounds A146, A169 and A145 can significantly degrade GSPT1 protein in HL60 cells at 300 nM, wherein A169 can simultaneously degrade both GSPT1 and c-Myc proteins at this concentration; Fig. 3 shows that compounds A131, A134 and A162 can significantly degrade GSPT1 protein in HL60 cells at 100 nM, and can simultaneously degrade both GSPT1 and c-Myc proteins at 300 nM.

**Table 2 Activity of compounds in c-Myc protein degradation**

| Compound | Remaining amount of c-Myc protein (@10 nM/Ctrl) | Compound | Remaining amount of c-Myc protein (@10 nM/Ctrl) |
|---|---|---|---|
| A17 | 23% | A75 | 0% |
| A24 | 0% | A77 | 50% |
| A28 | 43% | A80 | 0% |
| A29 | 13% | A81 | 0% |
| A30 | 14% | A82 | 7% |
| A37 | 44% | A83 | 4% |
| A38 | 52% | A85 | 8% |
| A43 | 23% | A87 | 11% |
| A47 | 19% | A92 | 6% |
| A53 | 10% | A93 | 12% |
| A61 | 0% | A94 | 0% |
| A62 | 17% | A95 | 0% |
| A63 | 19% | A98 | 157% |
| A64 | 0% | A100 | 0% |
| A65 | 0% | A102 | 0% |
| A66 | 9% | A103 | 6% |
| A67 | 19% | A105 | 333% |
| A68 | 0% | A107 | 46% |
| A69 | 30% | A108 | 2% |
| A70 | 216% | A109 | 3% |
| A71 | 26% | A110 | 3% |
| A72 | 16% | A111 | 6% |
| A73 | 0% | A112 | 0% |
| A74 | 104% | A140 | 95% |
| A169 | 105% | | |

| | | | |
|---|---|---|---|
| Notes: @10 nM/Ctrl refers to the percentage of the remaining amount of c-Myc protein of the compound at 10 nM to the remaining amount of c-Myc protein in the Ctrl group to evaluate the c-Myc protein degradation activity of the compound. | | | |

**Table 3 GSPT1 protein degradation activity of compounds**

| Compound | Remaining amount of GSPT1 protein (@10 nM/Ctrl) | Compound | Remaining amount of GSPT1 protein (@10 nM/Ctrl) |
|---|---|---|---|
| A61 | 4% | A83 | 0% |
| A62 | 16% | A85 | 3% |
| A63 | 5% | A87 | 0% |
| A64 | 5% | A92 | 0% |
| A65 | 14% | A93 | 0% |
| A67 | 25% | A94 | 0% |
| A68 | 0% | A95 | 0% |
| A69 | 53% | A98 | 3% |
| A70 | 23% | A100 | 0% |
| A71 | 0% | A102 | 0% |
| A72 | 0% | A103 | 0% |
| A73 | 0% | A105 | 7% |
| A74 | 10% | A107 | 1% |
| A75 | 0% | A108 | 29% |
| A77 | 2% | A109 | 6% |
| A80 | 3% | A110 | 0% |
| A81 | 0% | A111 | 2% |
| A82 | 0% | A112 | 0% |
| A140 | 47% | A169 | 65% |

| | | | |
|---|---|---|---|
| Notes: @10 nM/Ctrl refers to the percentage of the remaining amount of GSPT1 protein of the compound at 10 nM to the remaining amount of GSPT1 protein in the Ctrl group to evaluate the GSPT1 protein degradation activity of the compound. | | | |

**Table 4 CK1α and IKZF2 protein degradation activity of compounds**

| Compound | Remaining amount of CK1α protein (@10 nM/ Ctrl) | Remaining amount of IKZF2 protein (@10 nM/ Ctrl) |
|---|---|---|
| CC-90009 | 78% | 85% |
| A62 | 27% | 45% |
| A80 | 5% | 17% |

| | | |
|---|---|---|
| Notes: The CAS number of CC-90009 is 1860875-51-9. @ 10 nM/Ctrl refers to the percentage of the remaining amount of CK1α protein of the compound at 10 nM to the remaining amount of CK1α protein in the Ctrl group to evaluate the CK1α protein degradation activity of the compound. The same is true for IKZF2. | | |

The preferred embodiments of the present invention have been described above, but they are not intended to limit the present invention. Those skilled in the art may make modifications and changes to the embodiments disclosed herein without departing from the scope and spirit of the present invention.

## Claims

1. A compound of formula (I₀) or a pharmaceutically acceptable salt, solvate, hydrate, stereoisomer, tautomer, or prodrug thereof: 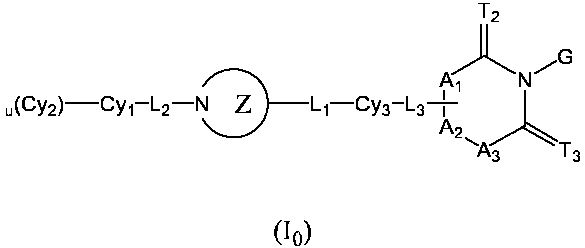 **characterized in that**,
G is selected from: H or C₁₋₆ alkyl, wherein the C₁₋₆ alkyl is optionally substituted with a group selected from the following: hydroxyl, -OPO(OR^{G})₂, - OCOR^{G}, carboxyl, -OS(O)₁₋₂R^{G}, and sulfo, wherein each R^{G} is independently selected from: H or C₁₋₄ alkyl;
T₂-T₃ are independently selected from: O or S;
A₁-A₂ are independently selected from: C(R^{A})₂ or NR^{A'}, wherein each R^{A} is independently selected from: H, halogen, C₁₋₁₅ alkyl optionally substituted with halogen, C₃₋₁₅ cycloalkyl, -CONR'R", -COOR', -OR ', or -NR'R", and R^{A'} is selected from: H, C₁₋₁₅ alkyl optionally substituted with halogen, C₃₋₁₅ cycloalkyl, or -COR';
A₃ is selected from: a bond, or C(R^{A})₂, wherein each R^{A} is independently selected from: H, halogen, C₁₋₁₅ alkyl optionally substituted with halogen, C₃₋₁₅ cycloalkyl, -CONR'R", -COOR', -OR', or -NR'R";
R' and R" are independently selected from: H, C₁₋₁₅ alkyl optionally substituted with halogen, or C₃₋₆ cycloalkyl;
L₃ is selected from: a bond, or a group represented by the structure of the following general formula:
-(CHR¹)ₖ-;
-(CHR¹)ₘO-(CHR¹)ₙ-;
-(CHR¹)ₘN(R¹)-(CHR¹)ₙ-;
-(CHR¹)ₘ-CO-(CHR¹)ₙ-;
-(CHR¹ₘ-CO-N(R¹)-(CHR¹)ₙ-;
-(CHR¹)ₘ-N(R¹)-CO-(CHR¹)ₙ-;
-(CHR¹)ₘ-CO-O-(CHR¹)ₙ-;
-(CHR¹)ₘ-O-CO-(CHR¹)ₙ-;
-(CHR¹)ₘ-SO₂-N(R¹)-(CHR¹)ₙ-;
-(CHR¹)ₘ-N(R¹)-SO₂-(CHR¹)ₙ-;
or substituted or unsubstituted triazolylidene;
wherein k is selected from 0, 1, 2, 3, 4, 5, 6, 7, 8, 9, 10, 11, and 12, m and n are independently selected from: 0, 1, 2, 3, 4, 5, and 6, each R¹ is independently selected from: H, or C₁₋₆ alkyl optionally substituted with halogen;
Cy₃ is selected from optionally substituted aryl, optionally substituted heteroaryl, optionally substituted heterocyclyl, optionally substituted biaryl or optionally substituted biheteroaryl;
L₁ represents a linking group;
ring Z represents an optionally substituted heterocycloalkyl containing at least one N atom as a heteroatom, and is linked to L₂ through the N;
L₂ is selected from: a bond, or a group represented by the structure of the following general formula:
-(CHR¹)ₖ-;
-(CHR¹)ₘ-O-(CHR¹)ₙ-;
-(CHR¹)ₘ-N(R¹)-(CHR¹)ₙ-;
-(CHR¹)ₘ-CO-(CHR¹)ₙ-;
-(CHR¹)ₘ-CO-N(R¹)-(CHR¹)ₙ-;
-(CHR¹)ₘ-N(R¹)-CO-(CHR¹)ₙ-;
wherein k is selected from 0, 1, 2, 3, 4, 5, 6, 7, 8, 9, 10, 11, and 12, m and n are independently selected from: 0, 1, 2, 3, 4, 5, and 6, each R¹ is independently selected from: H, or C₁₋₆ alkyl optionally substituted with halogen;
Cy₁ is selected from: optionally substituted aryl, optionally substituted heteroaryl, optionally substituted heterocyclyl, optionally substituted biaryl or optionally substituted biheteroaryl;
Cy₂ is selected from H, optionally substituted aryl, optionally substituted heteroaryl, optionally substituted heterocyclyl, optionally substituted biaryl or optionally substituted biheteroaryl;
and u is selected from 0, 1, 2, 3, 4, and 5.

2. The compound or the pharmaceutically acceptable salt, solvate, hydrate, stereoisomer, tautomer, or prodrug thereof of claim 1, **characterized in that**, Cy₃ is selected from optionally substituted monocyclic aryl, optionally substituted monocyclic heteroaryl, optionally substituted monocyclic heterocyclyl, optionally substituted bicyclic aryl, optionally substituted bicyclic heteroaryl, optionally substituted bicyclic heterocyclyl, optionally substituted tricyclic aryl, optionally substituted tricyclic heteroaryl, and optionally substituted tricyclic heterocyclyl;
preferably, Cy₃ is selected from one of the following structures: 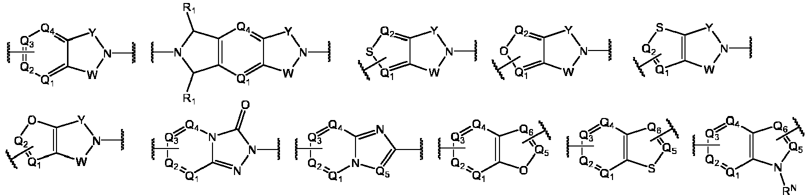 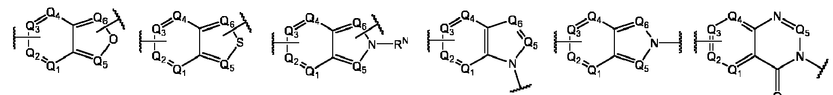 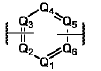
Y is selected from: C(R^{Y})₂, or C=T₁;
T₁ is selected from: O or S;
W is selected from: C(R^{W})₂, C=O, C=S, NH or -N-C₁₋₆ alkyl;
R^{Y} and R^{W} are independently selected from: H, halogen, cyano, nitro, C₁₋₁₅ alkyl optionally substituted with halogen, C₃₋₁₅ cycloalkyl, -CONR'R", -COOR', - OR', or -NR'R";
R^{N} is selected from: H, C₁₋₁₅ alkyl optionally substituted with halogen, or C₃₋₁₅ cycloalkyl;
Q₁-Q₆ are independently selected from: CR^{Q} or N;
R^{Q} is selected from: H, halogen, cyano, nitro, C₁₋₁₅ alkyl optionally substituted with halogen, C₃₋₁₅ cycloalkyl, -CONR'R", -COOR', -OR', or -NR'R"; or R^{Q} and R^{W} together form an aromatic ring optionally substituted with halogen;
R' and R" are independently selected from: H, C₁₋₁₅ alkyl optionally substituted with halogen, or C₃₋₆ cycloalkyl;
R¹ is independently selected from: H, or C₁₋₆ alkyl optionally substituted with halogen.

3. The compound or the pharmaceutically acceptable salt, solvate, hydrate, stereoisomer, tautomer, or prodrug thereof of claim 1, **characterized in that**, the compound of formula (I₀) is selected from the following compound of formula (I): 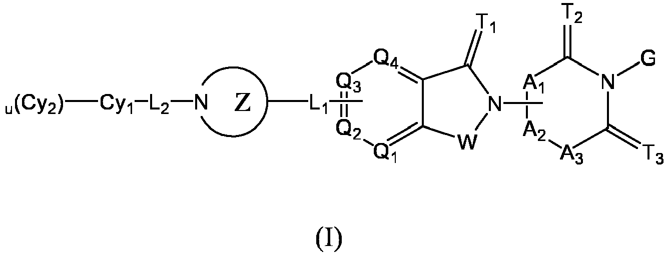 wherein,
W is selected from: C(R^{W})₂, C=O, C=S, NH or -N-C₁₋₆ alkyl;
R^{W} is selected from: H, halogen, cyano, nitro, C₁₋₁₅ alkyl optionally substituted with halogen, C₃₋₁₅ cycloalkyl, -CONR'R", -COOR', -OR', or -NR'R";
G is selected from: H or C₁₋₆ alkyl, wherein the C₁₋₆ alkyl is optionally substituted with a group selected from the following: hydroxyl, -OPO(OR^{G})₂, - OCOR^{G}, carboxyl, -OS(O)₁₋₂R^{G}, and sulfo, wherein each R^{G} is independently selected from: H or C₁₋₄ alkyl;
T₁-T₃ are independently selected from: O or S;
A₁-A₃ are independently selected from: C(R^{A})₂, wherein each R^{A} is independently selected from: H, halogen, C₁₋₁₅ alkyl optionally substituted with halogen, C₃₋₁₅ cycloalkyl, -CONR'R", -COOR', -OR', or -NR'R";
Q₁-Q₄ are independently selected from: CR^{Q} or N;
R^{Q} is selected from: H, halogen, cyano, nitro, C₁₋₁₅ alkyl optionally substituted with halogen, C₃₋₁₅ cycloalkyl, -CONR'R", -COOR', -OR', or -NR'R";
R' and R" are independently selected from: H, C₁₋₁₅ alkyl optionally substituted with halogen, or C₃₋₆ cycloalkyl;
L₁ represents a linking group;
ring Z represents an optionally substituted heterocycloalkyl containing at least one N atom as a heteroatom, and is linked to L₂ through the N;
L₂ is selected from: a bond, or a group represented by the structure of the following general formula:
-(CHR¹)ₖ-;
-(CHR¹)ₘ-O-(CHR¹)ₙ-;
-(CHR¹)ₘ-N(R¹)-(CHR¹)ₙ-;
-(CHR¹)ₘ-CO-(CHR¹)ₙ-;
-(CHR¹)ₘ-CO-N(R¹)-(CHR¹)ₙ-;
-(CHR¹)ₘ-N(R¹)-CO-(CHR¹)ₙ-;
wherein k is selected from 0, 1, 2, 3, 4, 5, 6, 7, 8, 9, 10, 11, and 12, m and n are independently selected from: 0, 1, 2, 3, 4, 5, and 6, each R¹ is independently selected from: H, or C₁₋₆ alkyl optionally substituted with halogen;
Cy₁ is selected from: optionally substituted aryl, optionally substituted heteroaryl, optionally substituted heterocyclyl, optionally substituted biaryl or optionally substituted biheteroaryl;
Cy₂ is selected from H, optionally substituted aryl, optionally substituted heteroaryl, optionally substituted heterocyclyl, optionally substituted biaryl or optionally substituted biheteroaryl;
and u is selected from 0, 1, 2, 3, 4, and 5.

4. The compound or the pharmaceutically acceptable salt, solvate, hydrate, stereoisomer, tautomer, or prodrug thereof of claim 3, **characterized in that**, W is selected from: C(R^{W})₂ or C=O, wherein R^{W} is selected from H, halogen, cyano, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, or cyclohexyl; preferably, W is selected from: CH₂ or C=O.

5. The compound or the pharmaceutically acceptable salt, solvate, hydrate, stereoisomer, tautomer, or prodrug thereof of claim 3 or 4, **characterized in that**, T₁-T₃ are selected from: O.

6. The compound or the pharmaceutically acceptable salt, solvate, hydrate, stereoisomer, tautomer, or prodrug thereof of any one of claims 3 to 5, **characterized in that**, each R^{A} is independently selected from: H, halogen, cyano, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, or cyclohexyl.

7. The compound of or the pharmaceutically acceptable salt, solvate, hydrate, stereoisomer, tautomer, or prodrug thereof any one of claims 3 to 6, **characterized in that**, 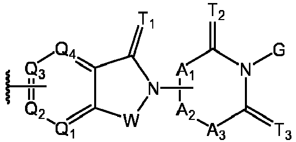 is selected from one of the following structures: 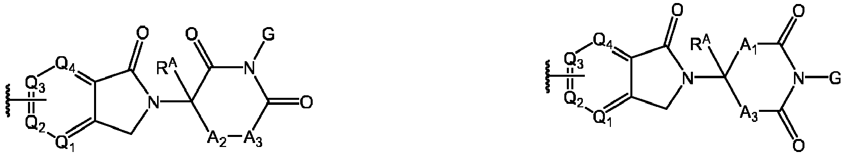 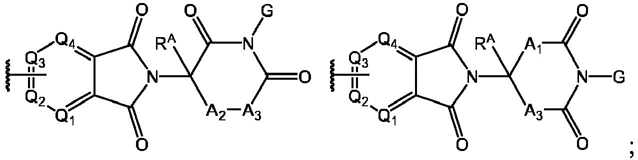
preferably, 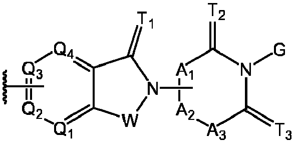 is selected from one of the following structures: 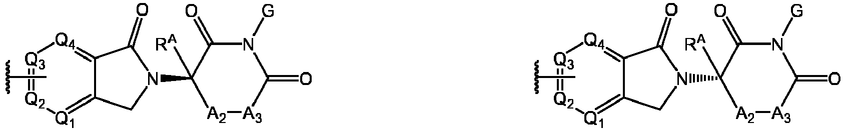 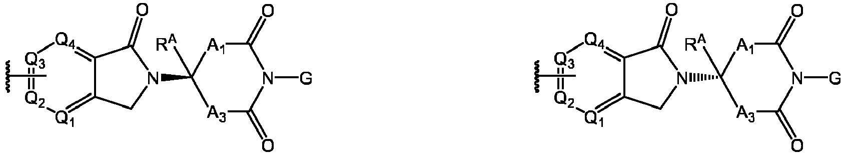 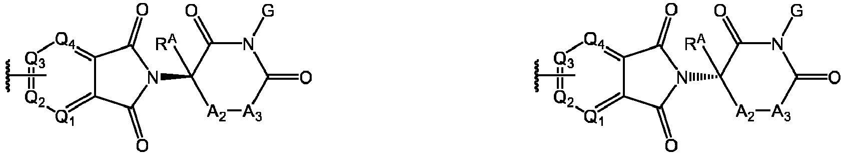 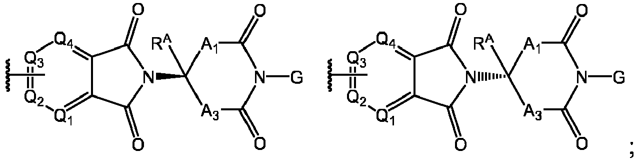
preferably, 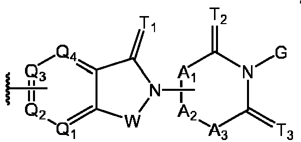 is selected from one of the following structures: 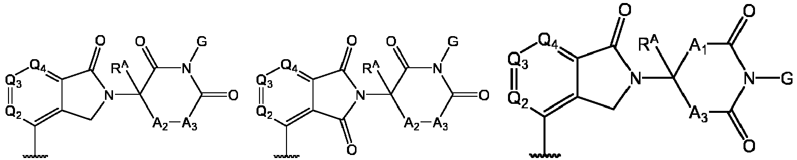 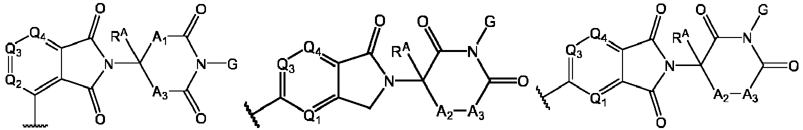 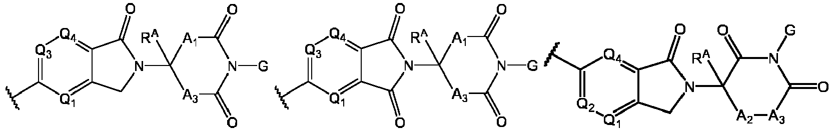 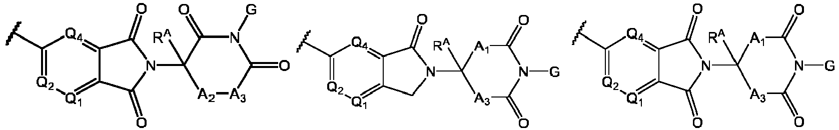 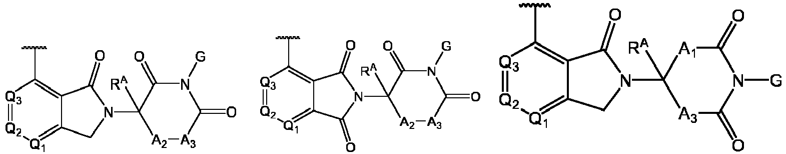 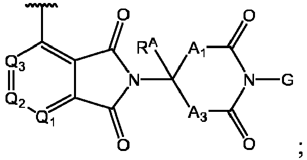
more preferably, 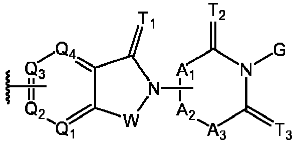 is selected from one of the following structures:

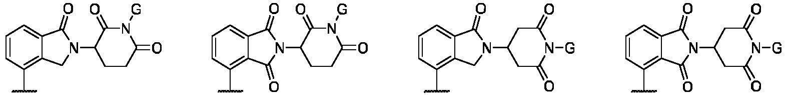

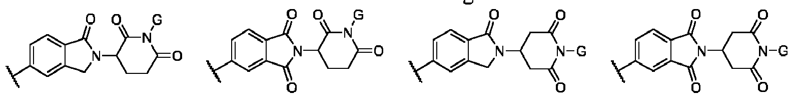

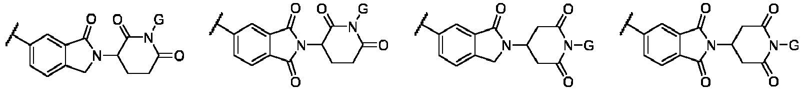

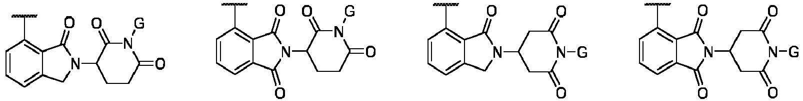

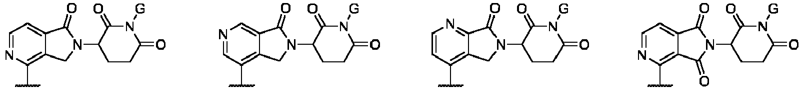

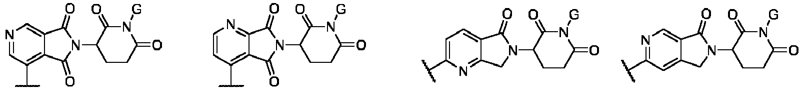

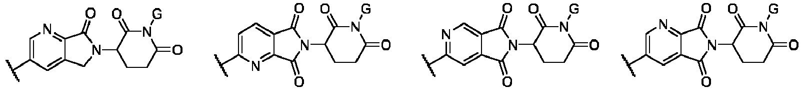

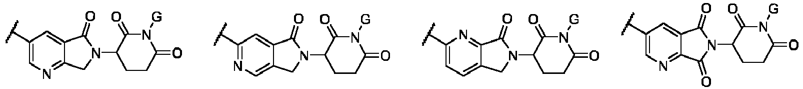

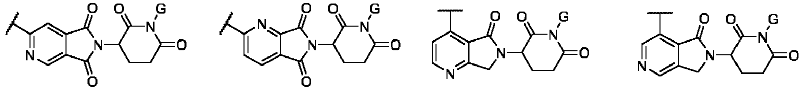

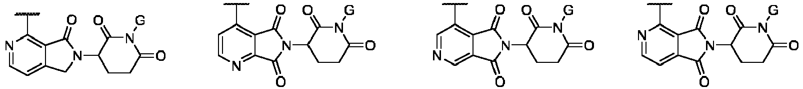

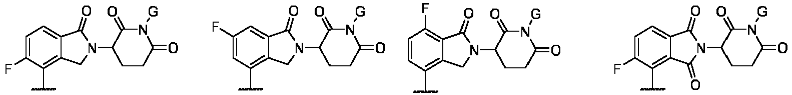

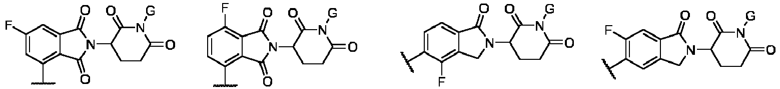

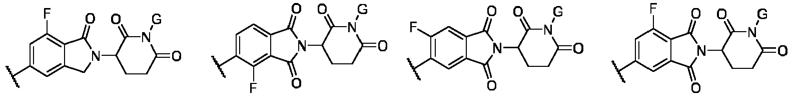

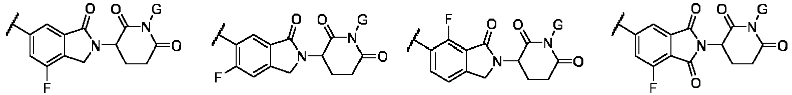

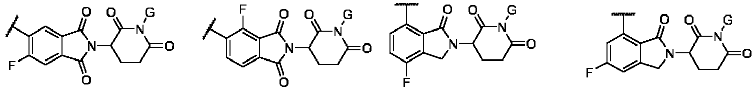

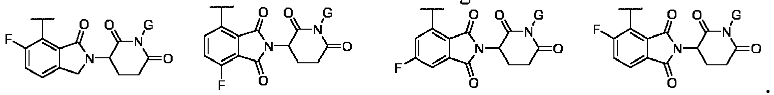

8. The compound or the pharmaceutically acceptable salt, solvate, hydrate, stereoisomer, tautomer, or prodrug thereof of claim 1 or 2, **characterized in that**, Cy₃ is selected from one of the following structures: 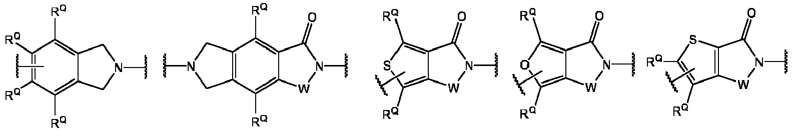 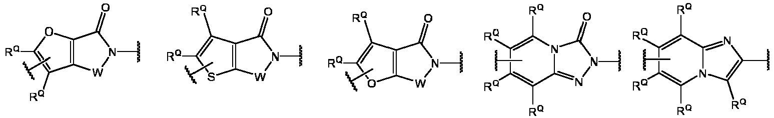 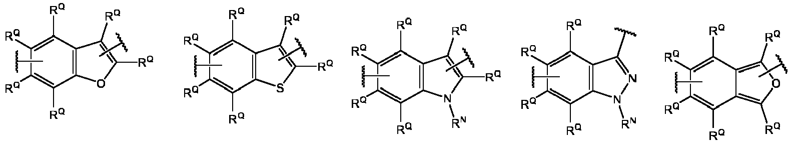 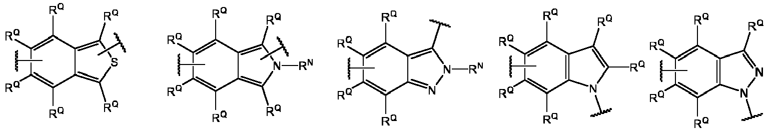 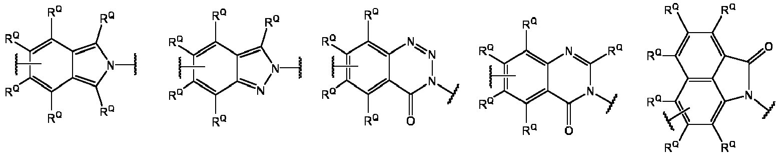 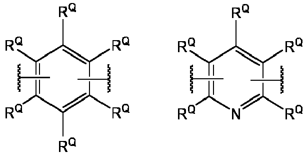
R^{Q} is selected from: H, halogen, cyano, nitro, hydroxyl, C₁₋₆ alkyl optionally substituted with halogen, C₁₋₆ alkoxy optionally substituted with halogen, C₃₋₆ cycloalkyl, or -NR'R"; R^{N} is selected from H, C₁₋₆ alkyl optionally substituted with halogen, or C₃₋₆ cycloalkyl;
preferably, R^{Q} is selected from: H, halogen, cyano, nitro, hydroxyl, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, cyclohexyl, amino, methylamino, dimethylamino, ethylamino, or diethylamino; R^{N} is selected from: H, methyl, ethyl, n-propyl, isopropyl, n-butyl, isobutyl, sec-butyl, tert-butyl, pentyl, hexyl, CH₂F, CHF₂, CF₃, cyclopropyl, cyclobutyl, cyclopentyl, or cyclohexyl;
more preferably, R^{Q} is selected from: H, halogen, cyano, nitro, methyl, CH₂F, CHF₂, CF₃, methoxy, ethoxy, CF₃O-, cyclopropyl, cyclobutyl, cyclopentyl, or cyclohexyl; R^{N} is selected from: methyl, ethyl, isopropyl, tert-butyl, CH₂F, CHF₂, CF₃, cyclopropyl, cyclobutyl, cyclopentyl, or cyclohexyl.

9. The compound or the pharmaceutically acceptable salt, solvate, hydrate, stereoisomer, tautomer, or prodrug thereof of claim 8, **characterized in that**, Cy₃ is selected from one of the following structures: 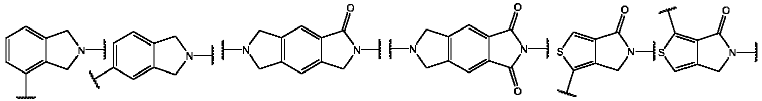 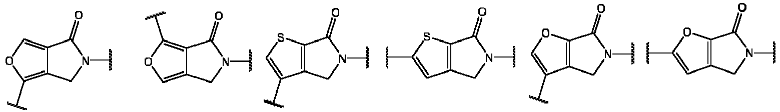 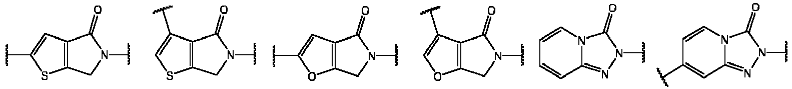 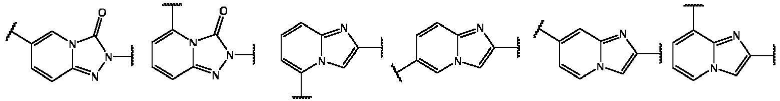 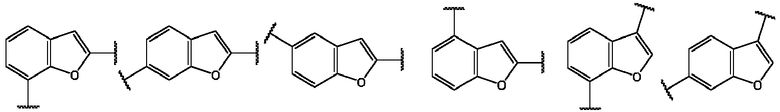 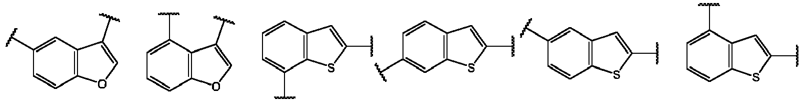 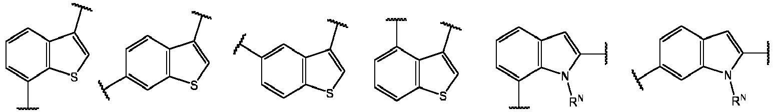 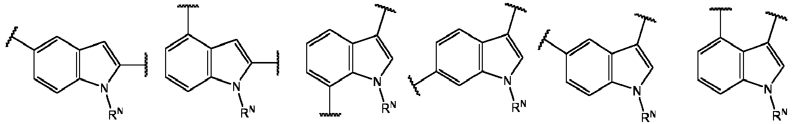 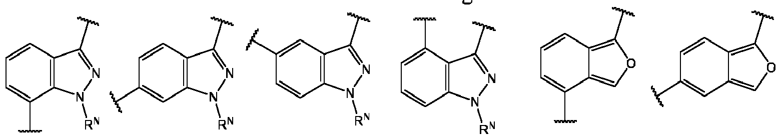 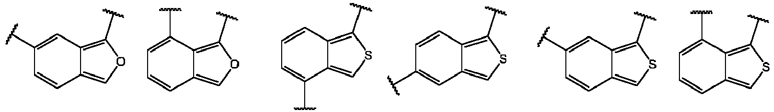 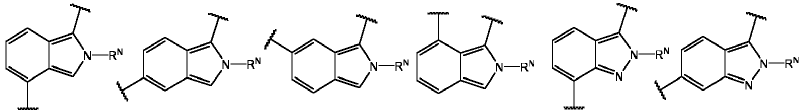 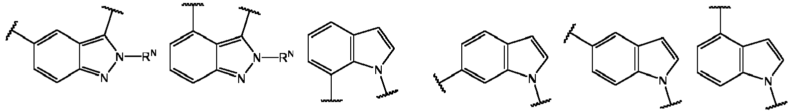 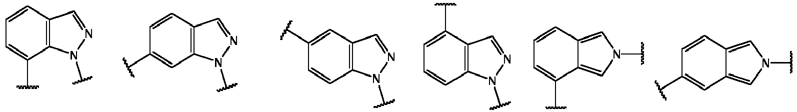 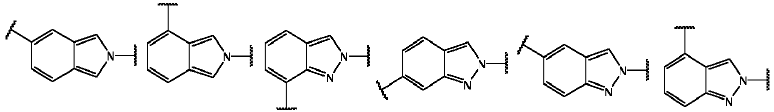 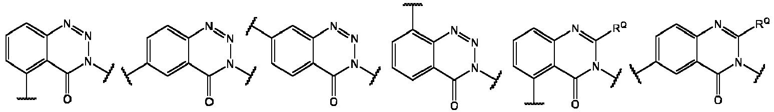 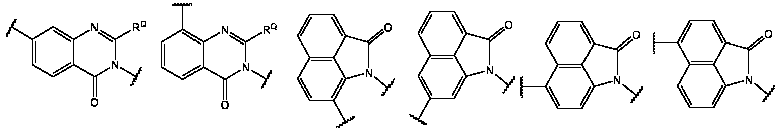 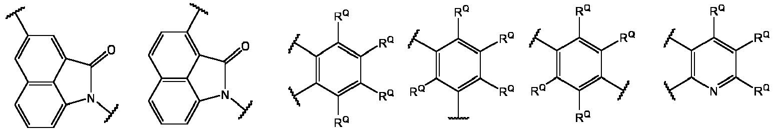 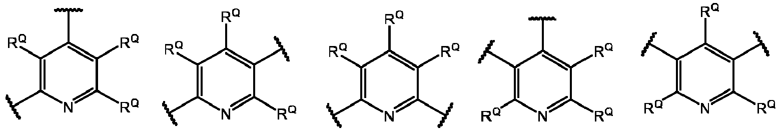 R^{Q} is selected from: H, halogen, nitro, methyl, CH₂F, CHF₂, CF₃, methoxy, ethoxy, CF₃O-, cyclopropyl, cyclobutyl, cyclopentyl, or cyclohexyl; R^{N} is selected from: methyl, ethyl, isopropyl, tert-butyl, CH₂F, CHF₂, CF₃, cyclopropyl, cyclobutyl, cyclopentyl, or cyclohexyl.

10. The compound or the pharmaceutically acceptable salt, solvate, hydrate, stereoisomer, tautomer, or prodrug thereof of any one of claims 1-2 and 8-9, **characterized in that**, A₁-A₃ are selected from CH₂ and L₃ is linked to A₁; or A₁ is selected from NH and L₃ is linked to A₁; or A₂ is selected from NH and L₃ is linked to A₂; or A₃ is selected from NH and L₃ is linked to A₃; or A₃ is selected from a bond.

11. The compound or the pharmaceutically acceptable salt, solvate, hydrate, stereoisomer, tautomer, or prodrug thereof of any one of claims 1-2 and 8-10, **characterized in that**, 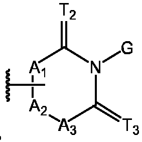 is selected from: 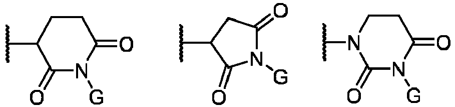
G is selected from: H or C₁₋₆ alkyl, wherein the C₁₋₆ alkyl is optionally substituted with a group selected from the following: hydroxyl, -OPO(OR^{G})₂, - OCOR^{G}, or -OS(O)₁₋₂R^{G}, wherein each R^{G} is independently selected from: H or
C₁₋₄ alkyl;
preferably, 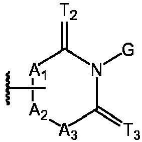 is selected from:

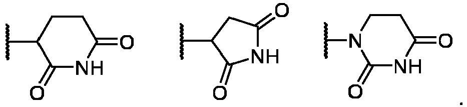

12. The compound or the pharmaceutically acceptable salt, solvate, hydrate, stereoisomer, tautomer, or prodrug thereof of any one of claims 1-2 and 8-11, **characterized in that**, L₃ is selected from: a bond, or a group represented by the structure of the following general formula:
-(CHR¹)ₖ-;
-O-; -(CHR¹)ₘ-O-; -O-(CHR¹)ₙ-; -(CHR¹)ₘ-O-(CHR¹)ₙ-;
-N(R¹)-; -(CHR¹)ₘ-N(R¹)-; -N(R¹)-(CHR¹)ₙ-; -(CHR¹)ₘ-N(R¹)-(CHR¹)ₙ-;
-CO-; -(CHR¹)ₘ-CO-; -CO-(CHR¹)ₙ-; -(CHR¹)ₘ-CO-(CHR¹)ₙ-;
-CO-N(R¹)-; -(CHR¹)ₘ-CO-N(R¹)-; -CO-N(R¹)-(CHR¹)ₙ-; -(CHR¹)ₘ-CO-N(R¹)-(CHR¹)ₙ-;
-N(R¹)-CO-; -(CHR¹)ₘ-N(R¹)-CO-; -N(R¹)-CO-(CHR¹)ₙ-; -(CHR¹)ₘ-N(R¹)-CO-(CHR¹)ₙ-;
-CO-O-; -(CHR¹)ₘ-CO-O; -CO-O-(CHR¹)ₙ-; -(CHR¹)ₘ-CO-O-(CHR¹)ₙ-;
-O-CO-; -(CHR¹)ₘ-O-CO-; -O-CO-(CHR¹)ₙ-; -(CHR¹)ₘ-O-CO-(CHR¹)ₙ-;
-SO₂-N(R¹)-; -(CHR¹)ₘ-SO₂-N(R¹)-; -SO₂-N(R¹)-(CHR¹)ₙ-; -(CHR¹)ₘ-SO₂-N(R¹)-(CHR¹)ₙ-;
-N(R¹)-SO₂-; -(CHR¹)ₘ-N(R¹)-SO₂-; -N(R¹)-SO₂-(CHR¹)ₙ-; -(CHR¹)ₘ-N(R¹)-SO₂-(CHR¹)ₙ-;
-triazolyl-
wherein k, m and n are independently selected from: 1, 2, 3, 4, 5, and 6, each R¹ is independently selected from: H, methyl, or ethyl;
preferably, L₃ is selected from: a bond, or one of the following structures:

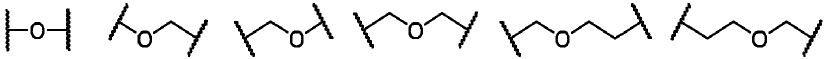

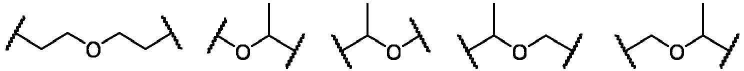

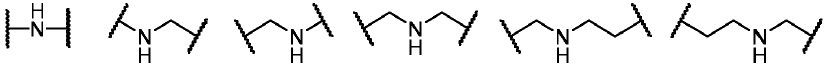

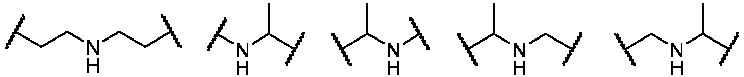

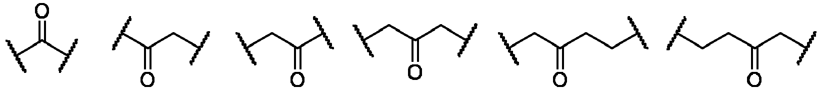

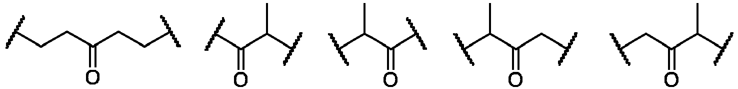

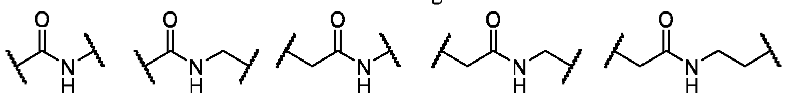

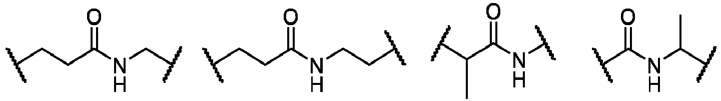

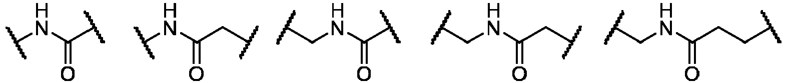

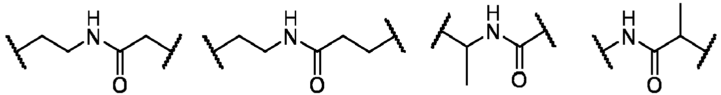

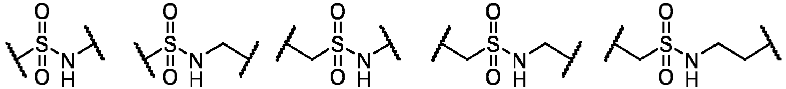

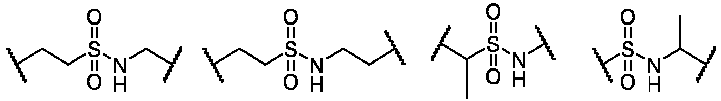

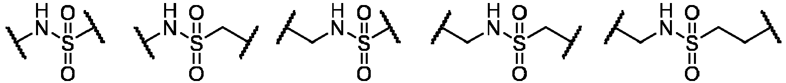

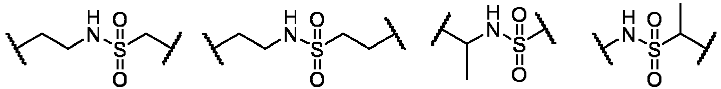

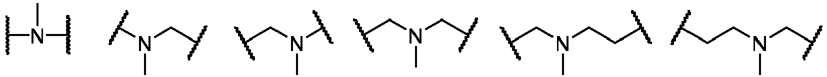

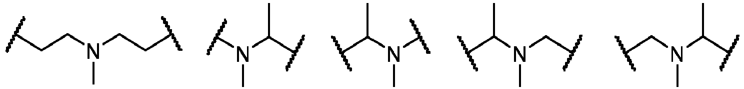

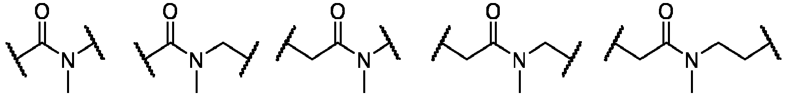

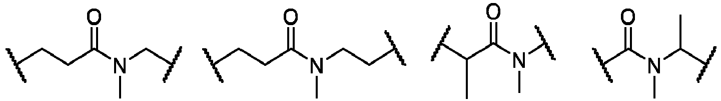

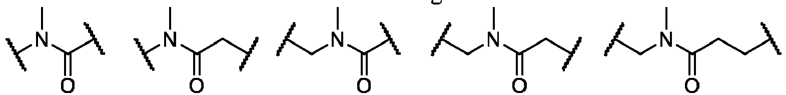

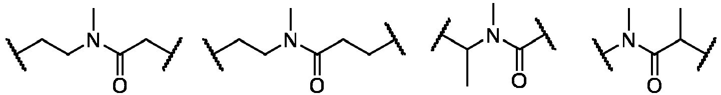

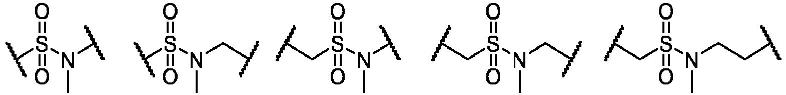

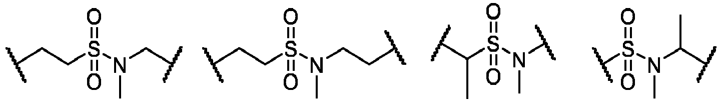

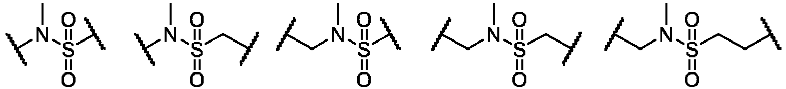

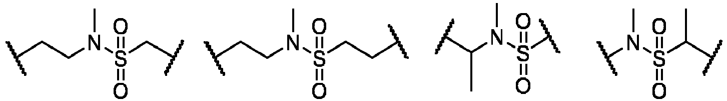

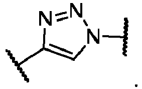

13. The compound or the pharmaceutically acceptable salt, solvate, hydrate, stereoisomer, tautomer, or prodrug thereof of any one of claims 1 to 12, **characterized in that**, L₁ is selected from: -O-, -S-, -N(R¹)-, -CO-, -CO-O-, -O-CO-, -O-CO-O-, -CO-N (R¹)-, -N(R¹)-CO-, -N(R¹)-CO-N(R¹)-, -CO-N(R¹)-CO and optionally substituted C₁₋₁₅ alkylene-, where the head, tail or middle of the alkylene is optionally inserted by 1, 2, 3, 4, 5 or more groups selected from: -O-, -S-, - N(R¹)-, -CO-, -CO-O-, -O-CO-, -O-CO-O-, -CO-N (R¹)-, -N(R¹)-CO-, -N(R¹)-CON(R¹)-, -CO-N(R¹)-CO, C₂ alkenylene, C₂ alkynylene, C₃₋₆ cycloalkylene, C₆₋₁₀ arylene, 5- to 6-membered heteroarylene, 5- to 7-membered heterocyclylene or any combination thereof; wherein each R¹ is independently selected from: H, or C₁₋₆ alkyl optionally substituted with halogen;
preferably, L₁ is selected from: -O-, -S-, -N(R¹)-, -CO-, -CO-O-, -O-CO-, -O-CO-O-, -CO-N (R¹)-, -N(R¹)-CO-, -N(R¹)-CO-N(R¹)-, -CO-N(R¹)-CO and optionally substituted C₁₋₁₅ alkylene-, where the head, tail or middle of the alkylene is optionally inserted by 1, 2, 3, 4, 5 or more groups selected from: -O-, -S-, - N(R¹)-, -CO-, -CO-O-, -O-CO-, -O-CO-O-, -CO-N(R¹)-, -N(R¹)-CO-, -N(R¹)-CON(R¹)-, -CO-N(R¹)-CO or any combination thereof; wherein each R¹ is independently selected from: H, or C₁₋₆ alkyl optionally substituted with halogen; the "optionally substituted" refers to being unsubstituted or mono- or poly-substituted with a group selected from the following: halogen, hydroxyl, cyano, nitro, amino, methyl, ethyl, n-propyl, isopropyl, CH₂F, CHF₂, CF₃, methoxy, ethoxy, CF₃O-, methylamino, dimethylamino, ethylamino, or diethylamino;
more preferably, L₁ is selected from a group represented by the structure of the following general formula:
-(CHR¹)ₖ-;
-(CHR¹)ₘ-O-(CHR¹)ₙ-;
-(CHR¹)ₘ-S-(CHR¹)ₙ-;
-(CHR¹)ₘ-N(R¹)-(CHR¹)ₙ-;
-(CHR¹)ₘ-CO-(CHR¹)ₙ-;
-(CHR¹)ₘ-CO-O-(CHR¹)ₙ-;
-(CHR¹)ₘ-O-CO-(CHR¹)ₙ-;
-(CHR¹)ₘ-O-CO-O-(CHR¹)ₙ-;
-(CHR¹)ₘ-CO-N(R¹)-(CHR¹)ₙ-;
-(CHR¹)ₘ-N(R¹)-CO-(CHR¹)ₙ-;
-(CHR¹)ₘ-CO-N(R¹)-(CHR¹)ₙ-;
-(CHR¹)ₘ-N(R¹)-CO-N(R¹)-(CHR¹)ₙ-;
-(CHR¹)ₘ-CO-N(R¹)-CO-(CHR¹)ₙ-;
-(CHR¹)ₘ-O-(CHR¹)ₗ-O-(CHR¹)ₙ-;
-(CHR¹)ₘ-O-(CHR¹)ₗ-N(R¹)-(CHR¹)ₙ-;
-(CHR¹)ₘ-N(R¹)-(CHR¹)ₗ-O-(CHR¹)ₙ-;
-(CHR¹)ₘ-O-(CHR¹)ₗ-CO-(CHR¹)ₙ-;
-(CHR¹)ₘ-CO-(CHR¹)ₗ-O-(CHR¹)ₙ-;
-(CHR¹)ₘ-O-(CHR¹)ₗ-CO-N(R¹)-(CHR¹)ₙ-;
-(CHR¹)ₘ-CO-N(R¹)-(CHR¹)ₗ-O-(CHR¹)ₙ-;
-(CHR¹)ₘ-O-(CHR¹)ₗ-N(R¹)-CO-(CHR¹)ₙ-;
-(CHR¹)ₘ-N(R¹)-CO-(CHR¹)ₗ-O-(CHR¹)ₙ-;
-(CHR¹)ₘ-N(R¹)-(CHR¹)ₗ-N(R¹)-(CHR¹)ₙ-;
-(CHR¹)ₘ-N(R¹)-(CHR¹)ₗ-CO-(CHR¹)ₙ-;
-(CHR¹)ₘ-CO-(CHR¹)ₗ-N(R¹)-(CHR¹)ₙ-;
-(CHR¹)ₘ-N(R¹)-(CHR¹)ₗ-CO-N(R¹)-(CHR¹)ₙ-;
-(CHR¹)ₘ-CO-N(R¹)-(CHR¹)ₗ-N(R¹)-(CHR¹)ₙ-;
-(CHR¹)ₘ-N(R¹)-(CHR¹)ₗ-N(R¹)-CO-(CHR¹)ₙ-;
-(CHR¹)ₘ-N(R¹)-CO-(CHR¹)ₗ-N(R¹)-(CHR¹)ₙ-;
-(CHR¹)ₘ-CO-(CHR¹)ₗ-CO-N(R¹)-(CHR¹)ₙ-;
-(CHR¹)ₘ-CO-N(R¹)-(CHR¹)ₗ-CO-(CHR¹)ₙ-;
-(CHR¹)ₘ-CO-(CHR¹)ₗ-N(R¹)-CO-(CHR¹)ₙ-;
-(CHR¹)ₘ-N(R¹)-CO-(CHR¹)ₗ-CO-(CHR¹)ₙ-;
-(CHR¹)ₘ-N(R¹)-CO-(CHR¹)ₗ-CO-N(R¹)-(CHR¹)ₙ-;
-(CHR¹)ₘ-N(R¹)-CO-(CHR¹)ₗ-N(R¹)-CO-(CHR¹)ₙ-;
-(CHR¹)ₘ-CO-N(R¹)-(CHR¹)ₗ-CO-N(R¹)-(CHR¹)ₙ-;
-(CHR¹)ₘ-CO-N(R¹)-(CHR¹)ₗ-N(R¹)-CO-(CHR¹)ₙ-;
-(CHR¹)ₘ-C₆₋₁₀arylene-CO-O-(CHR¹)ₙ-;
-(CHR¹)ₘ-C₆₋₁₀arylene-O-CO-(CHR¹)ₙ-;
-(CHR¹)ₘ-CO-O-C₆₋₁₀arylene-(CHR¹)ₙ-;
-(CHR¹)ₘ-O-CO-C₆₋₁₀arylene-(CHR¹)ₙ-;
-(CHR¹)ₘ-C₆₋₁₀arylene-N(R¹)-CO-(CHR¹)ₙ-;
-(CHR¹)ₘ-C₆₋₁₀arylene-CO-N(R¹)-(CHR¹)ₙ-;
-(CHR¹)ₘ-N(R¹)-CO-C₆₋₁₀arylene-(CHR¹)ₙ-;
-(CHR¹)ₘ-CO-N(R¹)-C₆₋₁₀arylene-(CHR¹)ₙ-;
wherein k is selected from: 0, 1, 2, 3, 4, 5, 6, 7, 8, 9, 10, 11, and 12, m and n are independently selected from: 0, 1, 2, 3, 4, 5, and 6, 1 is selected from 1, 2, 3, 4, 5, and 6, and each R¹ is independently selected from: H, or C₁₋₆ alkyl.

14. The compound of formula (I) or the pharmaceutically acceptable salt, solvate, hydrate, stereoisomer, tautomer, or prodrug thereof of any one of claims 1 to 13, **characterized in that**, L₁ is selected from a group represented by the structure of the following general formula:
-(CHR¹)ₖ-;
-O-; -(CHR¹)ₘ-O-; -O-(CHR¹)ₙ-; -(CHR¹)ₘ-O-(CHR¹)ₙ-;
-N(R¹)-; -(CHR¹)ₘ-N(R¹)-; -N(R¹)-(CHR¹)ₙ-; -(CHR¹)ₘ-N(R¹)-(CHR¹)ₙ-;
-CO-; -(CHR¹)ₘ-CO-; -CO-(CHR¹)ₙ-; -(CHR¹)ₘ-CO-(CHR¹)ₙ-;
-CO-N(R¹)-; -(CHR¹)ₘ-CO-N(R¹)-; -CO-N(R¹)-(CHR¹)ₙ-; -(CHR¹)ₘ-CO-N(R¹)-(CHR¹)ₙ-;
-N(R¹)-CO-; -(CHR¹)ₘ-N(R¹)-CO-; -N(R¹)-CO-(CHR¹)ₙ-; -(CHR¹)ₘ-N(R¹)-CO-(CHR¹)ₙ-;
-CO-O-; -(CHR¹)ₘ-CO-O; -CO-O-(CHR¹)ₙ-; -(CHR¹)ₘ-CO-O-(CHR¹)ₙ-;
-O-CO-; -(CHR¹)ₘ-O-CO-; -O-CO-(CHR¹)ₙ-; -(CHR¹)ₘ-O-CO-(CHR¹)ₙ-;
-(CHR¹)ₘ-C₆arylene-N(R¹)-CO-(CHR¹)ₙ-;
-(CHR¹)ₘ-C₆arylene-CO-N(R¹)-(CHR¹)ₙ-;
wherein k, m and n are independently selected from: 1, 2, 3, 4, 5, and 6, each R¹ is independently selected from: H, methyl, or ethyl;
preferably, L₁ is selected from one of the following structures:

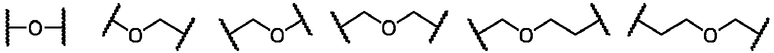

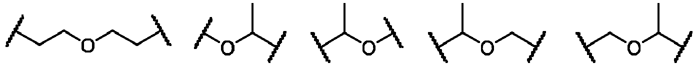

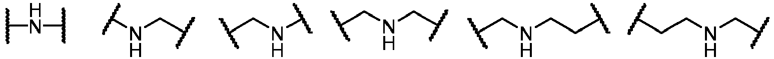

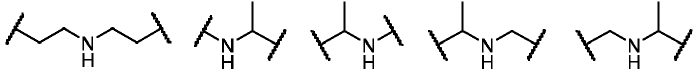

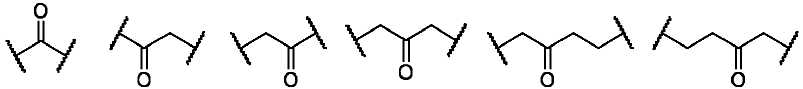

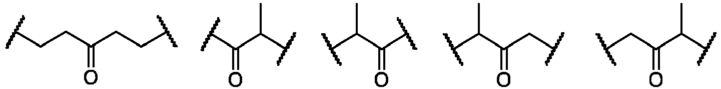

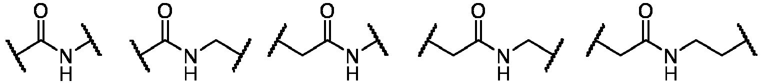

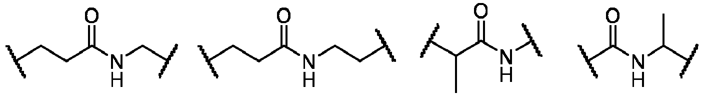

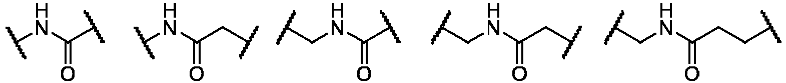

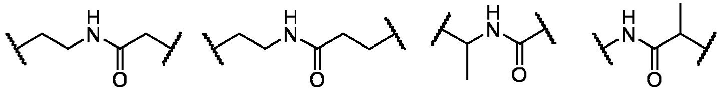

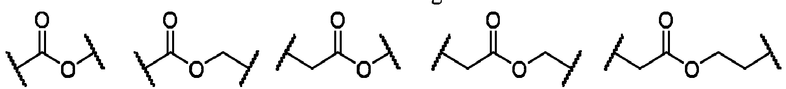

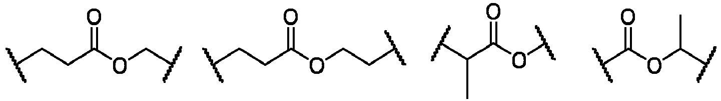

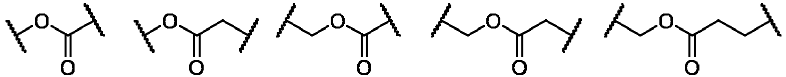

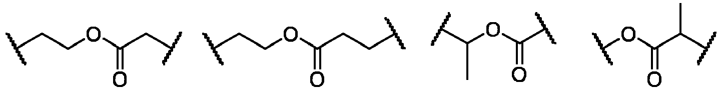

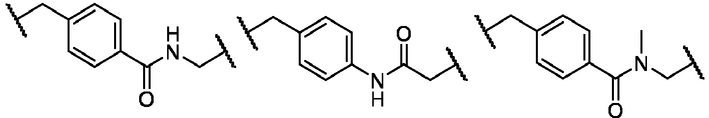

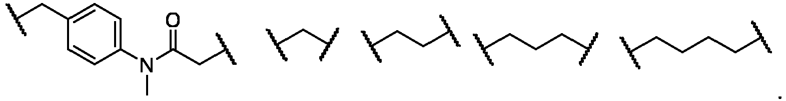

15. The compound of or the pharmaceutically acceptable salt, solvate, hydrate, stereoisomer, tautomer, or prodrug thereof any one of claims 1 to 14, **characterized in that**, 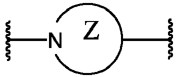 is selected from one of the following structures: 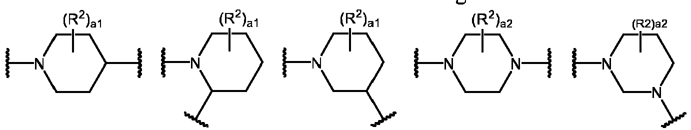 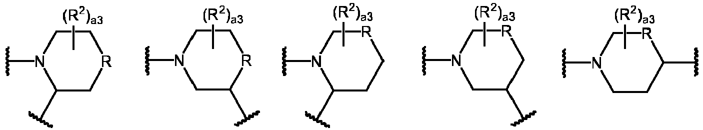 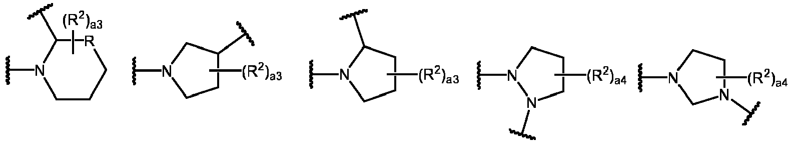 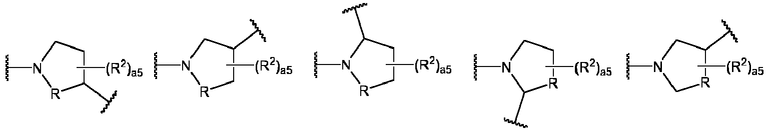 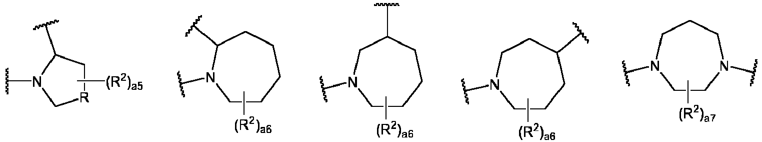 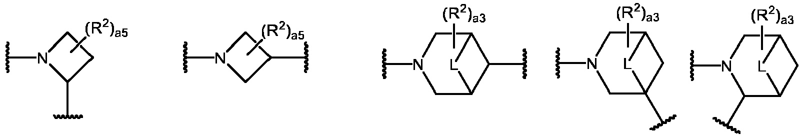 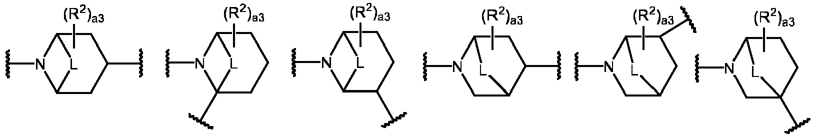 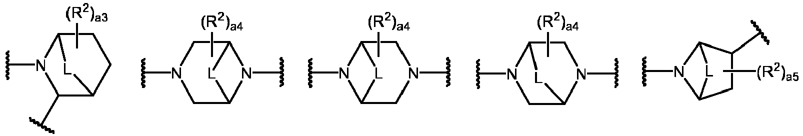 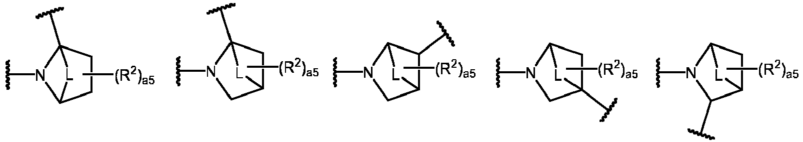 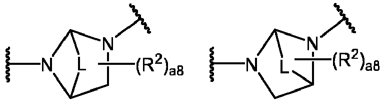
wherein each R² is independently selected from: H, halogen, cyano, nitro, C₁₋₁₅ alkyl optionally substituted with halogen, C₃₋₁₅ cycloalkyl, -COOR', -CONR' R", -OR', or -NR'R";
R' and R" are independently selected from: H, C₁₋₆ alkyl optionally substituted with halogen, or C₃₋₆ cycloalkyl;
each a1 is independently selected from: 0, 1, 2, 3, 4, 5, 6, 7, 8, and 9, each a2 is independently selected from: 0, 1, 2, 3, 4, 5, 6, 7, and 8, each a3 is independently selected from: 0, 1, 2, 3, 4, 5, 6, and 7, each a4 is independently selected from: 0, 1, 2, 3, 4, 5, and 6, each a5 is independently selected from: 0, 1, 2, 3, 4, and 5, each a6 is independently selected from: 0, 1, 2, 3, 4, 5, 6, 7, 8, 9, 10, and 11, each a7 is independently selected from: 0, 1, 2, 3, 4, 5, 6, 7, 8, 9, and 10, and each a8 is independently selected from 0, 1, 2, 3, and 4;
each R is independently selected from: O, S, or NR³, wherein R³ is selected from: H, C₁₋₆ alkyl, or -COC₁₋₆ alkyl;
each L is independently selected from: -CH₂-, -CH₂CH₂-, -C(CH₃)₂-, or - C(CH₃)₂CH₂-;
preferably, 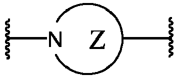 is selected from one of the following structures:

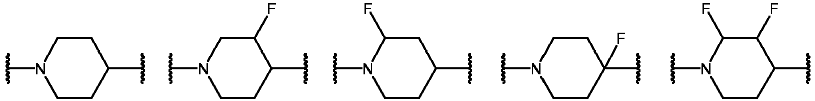

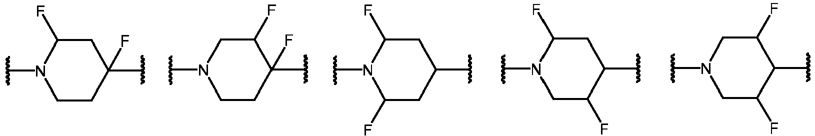

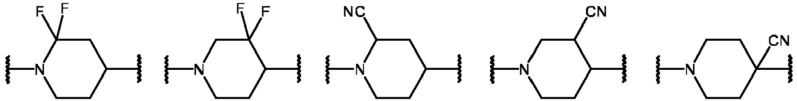

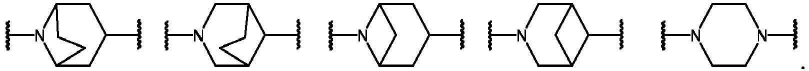

16. The compound or the pharmaceutically acceptable salt, solvate, hydrate, stereoisomer, tautomer, or prodrug thereof of any one of claims 1 to 15, **characterized in that**, L₂ is selected from: a bond, or a group represented by the structure of the following general formula:
-(CHR¹)ₖ-;
-O-; -(CHR¹)ₘ-O-; -O-(CHR¹)ₙ-; -(CHR¹)ₘ-O-(CHR¹)ₙ-;
-N(R¹)-; -(CHR¹)ₘ-N(R¹)-; -N(R¹)-(CHR¹)ₙ-; -(CHR¹)ₘ-N(R¹)-(CHR¹)ₙ-;
-CO-; -(CHR¹)ₘ-CO-; -CO-(CHR¹)ₙ-; -(CHR¹)ₘ-CO-(CHR¹)ₙ-;
-CO-N(R¹)-; -(CHR¹)ₘ-CO-N(R¹)-; -CO-N(R¹)-(CHR¹)ₙ-; -(CHR¹)ₘ-CO-N(R¹)-(CHR¹)ₙ-;
-N(R¹)-CO-; -(CHR¹)ₘ-N(R¹)-CO-; -N(R¹)-CO-(CHR¹)ₙ-; -(CHR¹)ₘ-N(R¹)-CO-(CHR¹)ₙ-;
wherein k, m and n are independently selected from: 1, 2, 3, 4, 5, and 6, each R¹ is independently selected from: H, methyl, or ethyl; preferably, L² is selected from: a bond, or one of the following structures:

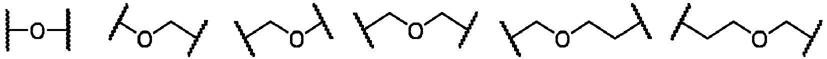

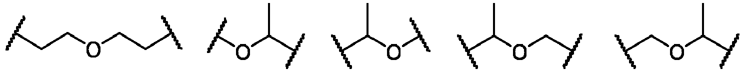

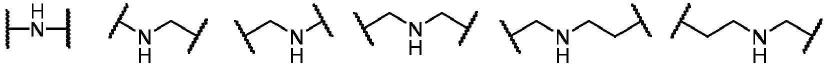

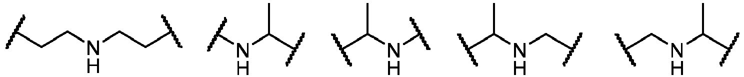

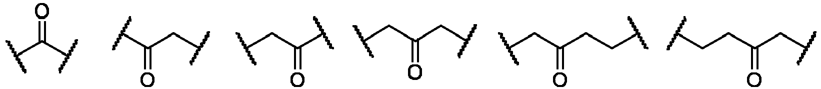

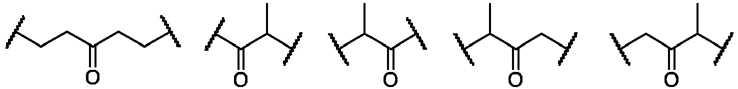

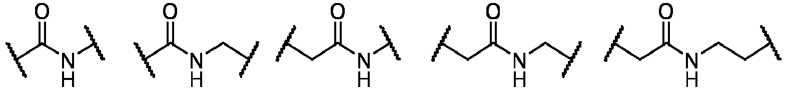

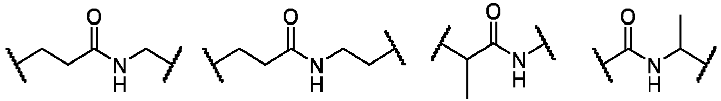

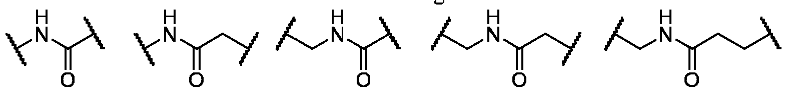

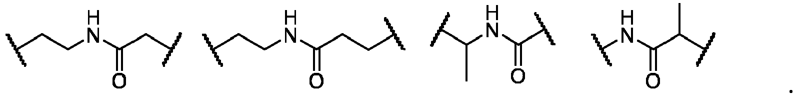

17. The compound or the pharmaceutically acceptable salt, solvate, hydrate, stereoisomer, tautomer, or prodrug thereof of any one of claims 1 to 16, **characterized in that**, Cy₁ is selected from: optionally substituted C₆₋₁₈ aryl, optionally substituted 5- to 18-membered heteroaryl, optionally substituted 5- to 18-membered heterocyclyl, optionally substituted bi-C₆₋₁₈ aryl or optionally substituted 5- to 18-membered heteroaryl; Cy₂ is independently selected from: H, optionally substituted C₆₋₁₈ aryl, optionally substituted 5- to 18-membered heteroaryl, optionally substituted 5- to 18-membered heterocyclyl, optionally substituted bi-C₆₋₁₈ aryl or optionally substituted 5- to 18-membered heteroaryl; preferably, Cy₁ is selected from: optionally substituted C₆₋₁₀ aryl, and optionally substituted 5- to 6-membered heteroaryl; Cy₂ is independently selected from: H, optionally substituted C₆₋₁₀ aryl, or optionally substituted 5- to 6-membered hetero aryl.

18. The compound or the pharmaceutically acceptable salt, solvate, hydrate, stereoisomer, tautomer, or prodrug thereof of any one of claims 1 to 17, **characterized in that**, Cy₁ is selected from optionally substituted: phenyl, naphthyl, anthracyl, phenanthrenyl, triphenylene, fluorenyl, biphenyl, furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyrimidinyl, pyridazinyl, pyrazinyl, triazinyl, indolyl, isoindolyl, benzoimidazolyl, benzopyrazolyl, benzofuranyl, benzothienyl, benzoxazolyl, benzothiazolyl, carbazolyl, azacarbazolyl, quinolinyl, isoquinolinyl, indolizinyl, azaindolizinyl, dibenzofuranyl, dibenzothienyl, naphthyridinyl, azetidinyl, oxacyclobutyl, pyrrolidinyl, pyrrolinyl, pyrazolidinyl, pyrazolinyl, imidazolidinyl, imidazolinyl, oxazolidinyl, oxazolinyl, isoxazolidinyl, isoxazolinyl, thiazolidinyl, thiazolinyl, isothiazolidinyl, isothiazolinyl, dihydrofuranyl, dihydrothienyl, tetrahydrofuranyl, tetrahydrothienyl, dioxacyclopentyl, dithiacyclopentyl, oxathiacyclopentyl, oxanyl, thianyl, dihydropyranyl, piperidinyl, morpholinyl, thiomorpholinyl, piperazinyl, N-methylpiperazinyl, dithianyl, hexahydropyridazinyl, hexahydropyrimidinyl, homopiperidinyl, homopiperazinyl, tetrahydroquinolinyl, indolinyl, pyridonyl, 2-pyrrolidonyl, 1,4-benzodioxanyl, 1,3-benzodioxolyl, ethyleneureido, phthalimidyl, or succinimidyl; Cy₂ is independently selected from: H, or optionally substituted: phenyl, naphthyl, anthracyl, phenanthrenyl, triphenylene, fluorenyl, biphenyl, furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyrimidinyl, pyridazinyl, pyrazinyl, triazinyl, indolyl, isoindolyl, benzoimidazolyl, benzopyrazolyl, benzofuranyl, benzothienyl, benzoxazolyl, benzothiazolyl, carbazolyl, azacarbazolyl, quinolinyl, isoquinolinyl, indolizinyl, azaindolizinyl, dibenzofuranyl, dibenzothienyl, naphthyridinyl, azetidinyl, oxacyclobutyl, pyrrolidinyl, pyrrolinyl, pyrazolidinyl, pyrazolinyl, imidazolidinyl, imidazolinyl, oxazolidinyl, oxazolinyl, isoxazolidinyl, isoxazolinyl, thiazolidinyl, thiazolinyl, isothiazolidinyl, isothiazolinyl, dihydrofuranyl, dihydrothienyl, tetrahydrofuranyl, tetrahydrothienyl, dioxacyclopentyl, dithiacyclopentyl, oxathiacyclopentyl, oxanyl, thianyl, dihydropyranyl, piperidinyl, morpholinyl, thiomorpholinyl, piperazinyl, N-methylpiperazinyl, dithianyl, hexahydropyridazinyl, hexahydropyrimidinyl, homopiperidinyl, homopiperazinyl, tetrahydroquinolinyl, indolinyl, pyridonyl, 2-pyrrolidonyl, 1,4-benzodioxanyl, 1,3-benzodioxolyl, ethyleneureido, phthalimidyl, or succinimidyl;
preferably, Cy₁ is selected from optionally substituted phenyl, naphthyl, anthracyl, phenanthrenyl, furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyrimidinyl, pyridazinyl, pyrazinyl, triazinyl, carbazolyl, azacarbazolyl, or tetrahydroquinolinyl; Cy₂ is selected from: H, or optionally substituted phenyl, naphthyl, anthracyl, phenanthrenyl, triphenylene, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyrimidinyl, pyridazinyl, pyrazinyl, or triazinyl.

19. The compound or the pharmaceutically acceptable salt, solvate, hydrate, stereoisomer, tautomer, or prodrug thereof of any one of claims 1 to 18, **characterized in that**, the "optionally substituted" refers to being unsubstituted or mono- or poly-substituted with a group selected from the following: halogen, hydroxyl, thiol, carboxyl, cyano, nitro, amino, C₁₋₆ alkyl optionally substituted with halogen, C₁₋₆ alkoxy optionally substituted with halogen, C₁₋₆ alkylthio optionally substituted with halogen, C₃₋₆ cycloalkyl, C₆₋₁₄ aryl, C₆₋₁₄ arylC₁₋₂ alkyl-, 5- to 7-membered heteroaryl, 5- to 7-membered heterocyclyl, C₁₋₆ ester group, C₁₋₆ acyl, C₁₋₆ alkylamino, di(C₁₋₆ alkyl)amino, or amido substituted with 0, 1 or 2 C₁₋₆ alkyl, wherein the aryl, heteroaryl, and heterocyclyl, alone or as part of a group, can be optionally substituted with a group selected from halogen, hydroxyl, thiol, carboxyl, cyano, nitro, C₁₋₆ alkyl optionally substituted with halogen, C₁₋₆ alkoxy optionally substituted with halogen, or C₃₋₆ cycloalkyl; preferably, the "optionally substituted" refers to being unsubstituted or mono- or poly-substituted with a group selected from the following: halogen, hydroxyl, cyano, nitro, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, cyclohexyl, phenyl, naphthyl, pyridyl, pyrimidinyl, -COOMe, -COOEt, -COMe, - COEt, amino, methylamino, dimethylamino, ethylamino, diethylamino, -CONH₂, - CONHMe, -CONHEt, -CON(Me)₂, or -CON(Et)₂.

20. The compound of or the pharmaceutically acceptable salt, solvate, hydrate, stereoisomer, tautomer, or prodrug thereof any one of claims 1 to 19, **characterized in that**, 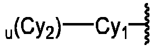 is selected from one of the following structures: 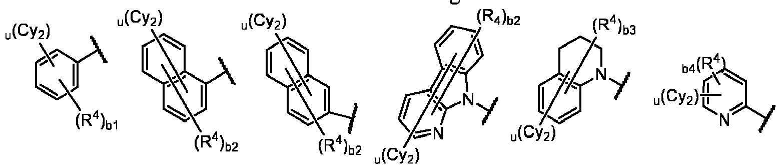 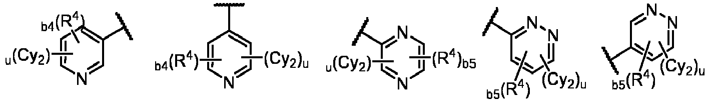 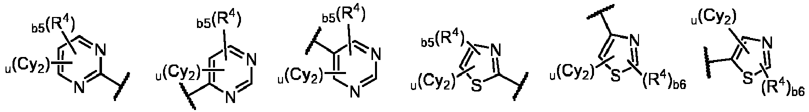 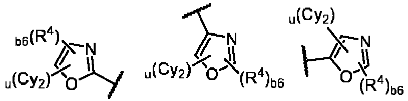
each R⁴ is independently selected from: halogen, hydroxyl, thiol, carboxyl, cyano, nitro, amino, C₁₋₆ alkyl optionally substituted with halogen, C₁₋₆ alkoxy optionally substituted with halogen, C₁₋₆ alkylthio optionally substituted with halogen, C₃₋₆ cycloalkyl, C₆₋₁₄ aryl, C₆₋₁₄ arylC₁₋₂ alkyl-, 5- to 7-membered heteroaryl, 5- to 7-membered heterocyclyl, C₁₋₆ ester group, C₁₋₆ acyl, C₁₋₆ alkylamino, di(C₁₋₆ alkyl)amino, or amido substituted with 0, 1 or 2 C₁₋₆ alkyl, wherein the aryl, heteroaryl, and heterocyclyl, alone or as part of a group, can be optionally substituted with a group selected from halogen, hydroxyl, thiol, carboxyl, cyano, nitro, C₁₋₆ alkyl optionally substituted with halogen, C₁₋₆ alkoxy optionally substituted with halogen, or C₃₋₆ cycloalkyl;
each b1 is independently selected from 0, 1, 2, 3, 4, and 5, each b2 is independently selected from 0, 1, 2, 3, 4, 5, 6, and 7, each b3 is independently selected from 0, 1, 2, 3, 4, 5, 6, 7, 8, 9, and 10, each b4 is independently selected from 0, 1, 2, 3, and 4, each b5 is independently selected from 0, 1, 2, and 3, and each b6 is independently selected from 0, 1, and 2.

21. The compound or the pharmaceutically acceptable salt, solvate, hydrate, stereoisomer, tautomer, or prodrug thereof of claim 20, **characterized in that**, the R⁴ is independently selected from: halogen, hydroxyl, cyano, nitro, C₁₋₆ alkyl optionally substituted with halogen, C₁₋₆ alkoxy optionally substituted with halogen, C₃₋₆ cycloalkyl, C₆₋₁₀ aryl, C₆₋₁₀ arylC₁₋₂ alkyl-, 5- to 6-membered heteroaryl, 5- to 7-membered heterocyclyl, C₁₋₆ ester group, C₁₋₆ acyl, C₁₋₆ alkylamino, di(C₁₋₆ alkyl)amino, or amido substituted with 0, 1 or 2 C₁₋₆ alkyl, wherein the aryl, heteroaryl, and heterocyclyl, alone or as part of a group, can be optionally substituted with a group selected from cyano, nitro, halogen, C₁₋₄ alkyl optionally substituted with halogen, or C₁₋₄ alkoxy optionally substituted with halogen; preferably, the R⁴ is independently selected from: halogen, hydroxyl, cyano, nitro, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, cyclohexyl, phenyl, naphthyl, pyridyl, pyrimidinyl, - COOMe, -COOEt, -COMe, -COEt, amino, methylamino, dimethylamino, ethylamino, diethylamino, -CONH₂, -CONHMe, -CONHEt, -CON(Me)₂, or - CON(Et)₂.

22. The compound or the pharmaceutically acceptable salt, solvate, hydrate, stereoisomer, tautomer, or prodrug thereof of any one of claims 1 to 21, **characterized in that**, u is selected from 1 and Cy₁ is selected from one of the following structures: 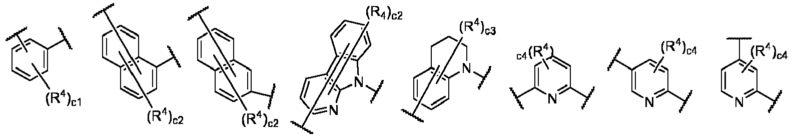 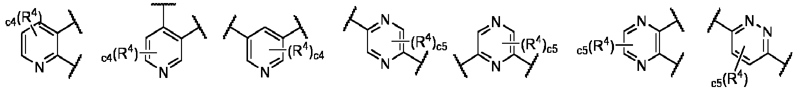 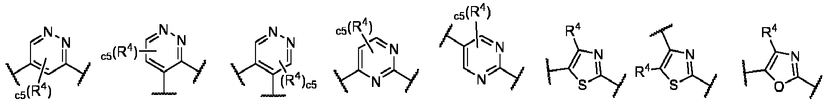 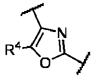
wherein R₄ is as defined in the preceding claims;
each c1 is independently selected from 0, 1, 2, 3, and 4, each c2 is independently selected from 0, 1, 2, 3, 4, 5, and 6, each c3 is independently selected from 0, 1, 2, 3, 4, 5, 6, 7, 8, and 9, each c4 is independently selected from 0, 1, 2, and 3, and each c5 is independently selected from 0, 1, and 2.

23. The compound or the pharmaceutically acceptable salt, solvate, hydrate, stereoisomer, tautomer, or prodrug thereof of any one of claims 1 to 22, **characterized in that**, Cy₂ is selected from: H, or one of the following structures: 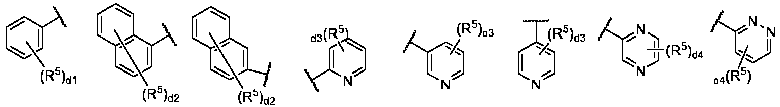 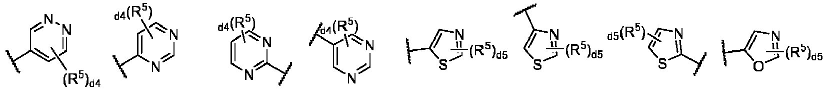 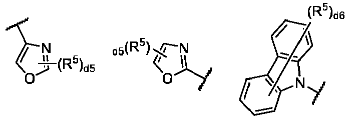
each R⁵ is independently selected from: halogen, hydroxyl, thiol, carboxyl, cyano, nitro, amino, C₁₋₆ alkyl optionally substituted with halogen, C₁₋₆ alkoxy optionally substituted with halogen, C₁₋₆ alkylthio optionally substituted with halogen, C₃₋₆ cycloalkyl, C₆₋₁₄ aryl, C₆₋₁₄ arylC₁₋₂ alkyl-, 5- to 7-membered heteroaryl, 5- to 7-membered heterocyclyl, C₁₋₆ ester group, C₁₋₆ acyl, C₁₋₆ alkylamino, di(C₁₋₆ alkyl)amino, or amido substituted with 0, 1 or 2 C₁₋₆ alkyl, wherein the aryl, heteroaryl, and heterocyclyl, alone or as part of a group, can be optionally substituted with a group selected from halogen, hydroxyl, thiol, carboxyl, cyano, nitro, C₁₋₆ alkyl optionally substituted with halogen, C₁₋₆ alkoxy optionally substituted with halogen, or C₃₋₆ cycloalkyl;
each d1 is independently selected from 0, 1, 2, 3, 4, and 5, each d2 is independently selected from 0, 1, 2, 3, 4, 5, 6, and 7, each d3 is independently selected from 0, 1, 2, 3, and 4, each d4 is independently selected from 0, 1, 2, and 3, each d5 is independently selected from 0, 1, and 2, each d6 is independently selected from 0, 1, 2, 3, 4, 5, 6, 7, and 8.

24. The compound or the pharmaceutically acceptable salt, solvate, hydrate, stereoisomer, tautomer, or prodrug thereof of claim 23, **characterized in that**, each R⁵ is independently selected from: halogen, hydroxyl, cyano, nitro, C₁₋₆ alkyl optionally substituted with halogen, C₁₋₆ alkoxy optionally substituted with halogen, C₃₋₆ cycloalkyl, C₆₋₁₀ aryl, C₆₋₁₀ arylC₁₋₂ alkyl-, 5- to 6-membered heteroaryl, 5- to 7-membered heterocyclyl, C₁₋₆ ester group, C₁₋₆ acyl, C₁₋₆ alkylamino, di(C₁₋₆ alkyl)amino, or amido substituted with 0, 1 or 2 C₁₋₆ alkyl, wherein the aryl, heteroaryl, and heterocyclyl, alone or as part of a group, can be optionally substituted with a group selected from cyano, nitro, halogen, C₁₋₄ alkyl optionally substituted with halogen, or C₁₋₄ alkoxy optionally substituted with halogen; preferably, each R⁵ is independently selected from: halogen, hydroxyl, cyano, nitro, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, cyclohexyl, phenyl, naphthyl, pyridyl, pyrimidinyl, - COOMe, -COOEt, -COMe, -COEt, amino, methylamino, dimethylamino, ethylamino, diethylamino, -CONH₂, -CONHMe, -CONHEt, -CON(Me)₂, or - CON(Et)₂.

25. The compound of or the pharmaceutically acceptable salt, solvate, hydrate, stereoisomer, tautomer, or prodrug thereof any one of claims 1 to 24, **characterized in that**, 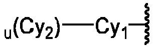 is selected from one of the following structures:

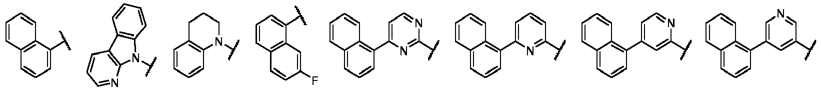

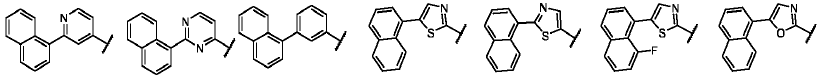

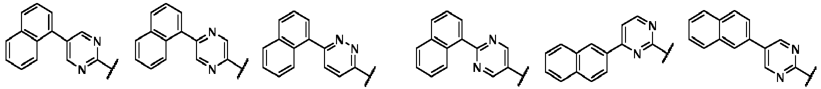

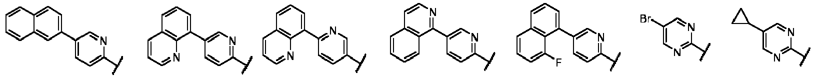

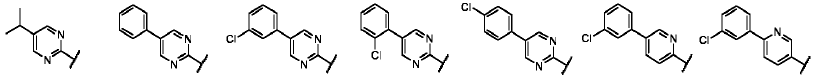

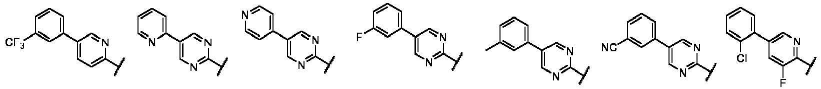

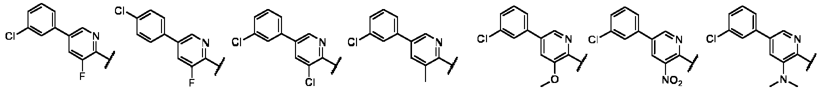

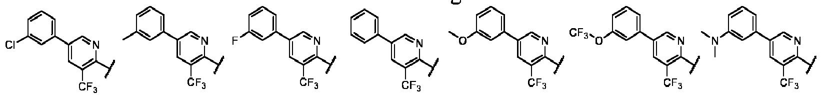

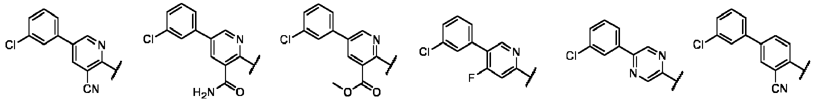

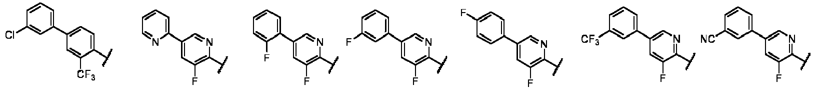

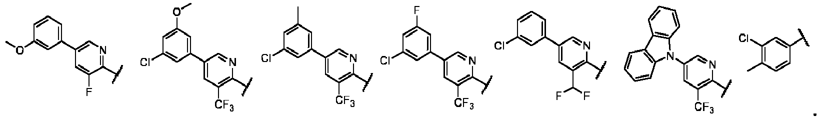

26. A compound of formula (I-A) or a pharmaceutically acceptable salt, solvate, hydrate, stereoisomer, tautomer, or prodrug thereof: 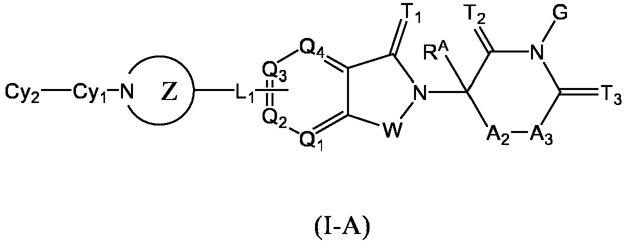
**characterized in that** W is selected from: C(R^{W})₂, C=O, or C=S;
R^{W} is selected from: H, halogen, C₁₋₆ alkyl optionally substituted with halogen, C₁₋₆ alkoxy optionally substituted with halogen, or C₃₋₆ cycloalkyl;
G is selected from: H or C₁₋₆ alkyl, wherein the C₁₋₆ alkyl is optionally substituted with a group selected from the following: hydroxyl, -OPO(OR^{G})₂, - OCOR^{G}, or -OS(O)₁₋₂R^{G}, wherein each R^{G} is independently selected from: H or C₁₋₄ alkyl;
T₁-T₃ are independently selected from: O or S;
A₂-A₃ are independently selected from: C(R^{A})₂;
each R^{A} is independently selected from: H, halogen, C₁₋₆ alkyl optionally substituted with halogen, C₁₋₆ alkoxy optionally substituted with halogen, or C₃₋₆ cycloalkyl;
Q₁-Q₄ are independently selected from: CR^{Q} or N;
R^{Q} is selected from: H, halogen, cyano, nitro, hydroxyl, C₁₋₆ alkyl optionally substituted with halogen, C₁₋₆ alkoxy optionally substituted with halogen, or C₃₋₆ cycloalkyl;
L₁ represents a linking group;
ring Z represents an optionally substituted heterocycloalkyl containing at least one N atom as a heteroatom, and is linked to Cy₁ through the N;
Cy¹ is selected from: optionally substituted aryl, optionally substituted heteroaryl, optionally substituted heterocyclyl, optionally substituted biaryl or optionally substituted biheteroaryl;
Cy² is selected from: H, optionally substituted aryl, optionally substituted heteroaryl, optionally substituted heterocyclyl, optionally substituted biaryl or optionally substituted biheteroaryl.

27. The compound or the pharmaceutically acceptable salt, solvate, hydrate, stereoisomer, tautomer, or prodrug thereof of claim 26, **characterized in that**, the compound of formula (I-A) is selected from the following compound of formula (I-Aa) or formula (I-Ab):

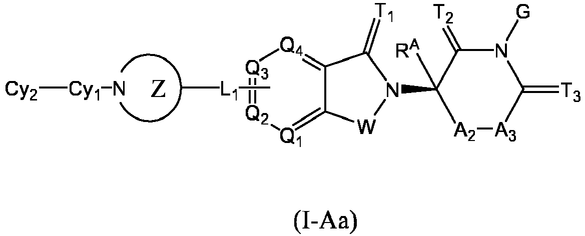

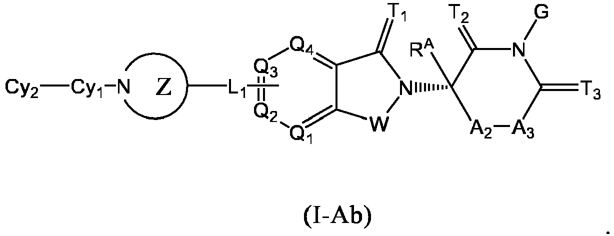

28. The compound or the pharmaceutically acceptable salt, solvate, hydrate, stereoisomer, tautomer, or prodrug thereof of any one of claims 3 and 26-27, **characterized in that**,
W is selected from: C(R^{W})₂, or C=O;
R^{W} is selected from: H, halogen, C₁₋₆ alkyl optionally substituted with halogen, C₁₋₆ alkoxy optionally substituted with halogen, or C₃₋₆ cycloalkyl;
G is selected from: H, or C₁₋₆ alkyl, wherein the C₁₋₆ alkyl is substituted with a group selected from the following: -OPO(OR^{G})₂, wherein each R^{G} is independently selected from: H or C₁₋₄ alkyl;
T₁-T₃ are independently selected from: O;
A₂-A₃ are independently selected from: C(R^{A})₂;
each R^{A} is independently selected from: H, halogen, cyano, C₁₋₆ alkyl optionally substituted with halogen, or C₁₋₆ alkoxy optionally substituted with halogen;
Q₁-Q₄ are independently selected from: CR^{Q} or N;
R^{Q} is selected from: H, halogen, cyano, C₁₋₆ alkyl optionally substituted with halogen, or C₁₋₆ alkoxy optionally substituted with halogen;
L₁ is selected from: -O-, -S-, -N(R¹)-, -CO-, -CO-O-, -O-CO-, -O-CO-O-, - CO-N (R¹)-, -N(R')-CO-, -N(R¹)-CO-N(R¹)-, -CO-N(R¹)-CO and optionally substituted C₁₋₁₅ alkylene-, where the head, tail or middle of the alkylene is optionally inserted by 1, 2, 3, 4, 5 or more groups selected from: -O-, -S-, -N(R')-, -CO-, -CO-O-, -O-CO-, -O-CO-O-, -CO-N (R¹)-, -N(R¹)-CO-, -N(R¹)-CO-N(R¹)-, -CO-N(R¹)-CO, C₂ alkenylene, C₂ alkynylene, C₃₋₆ cycloalkylene, C₆₋₁₀ arylene, 5-to 6-membered heteroarylene, 5- to 7-membered heterocyclylene or any combination thereof; wherein each R¹ is independently selected from: H, or C₁₋₆ alkyl optionally substituted with halogen; 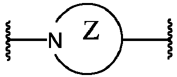 is selected from one of the following structures: 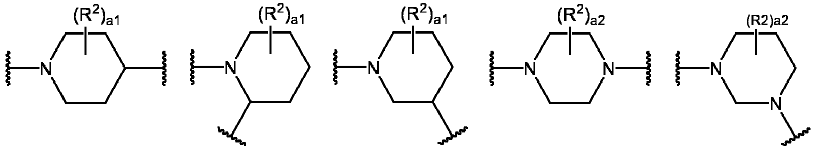 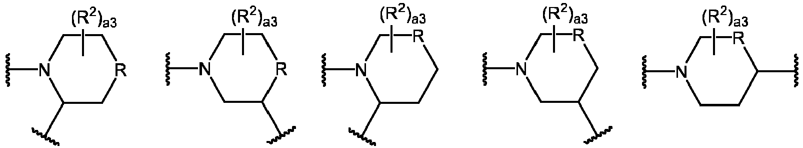 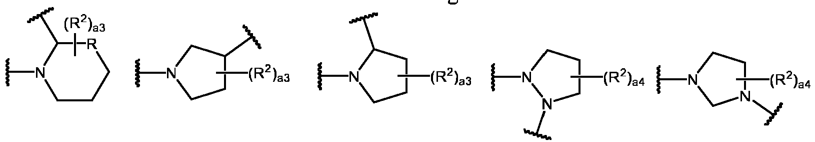 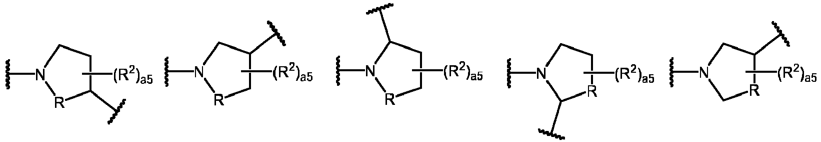 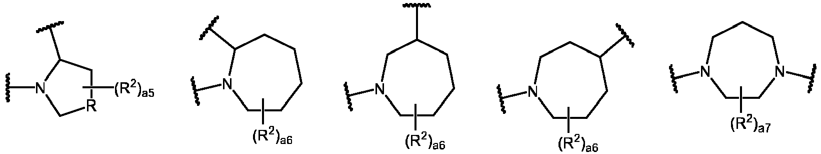 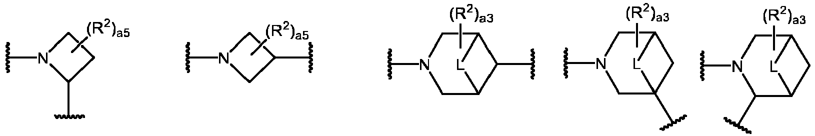 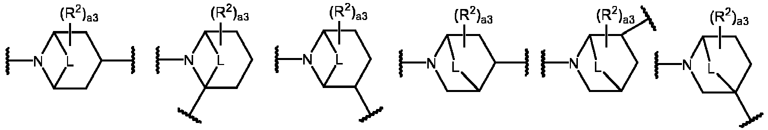 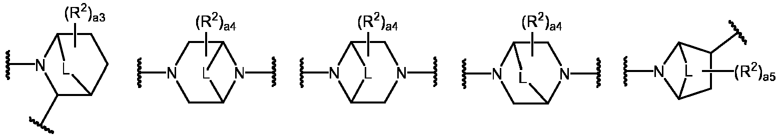 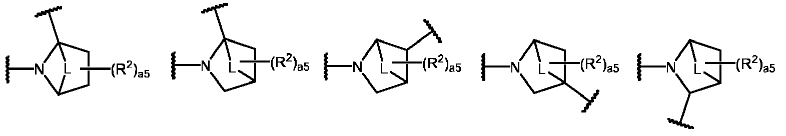 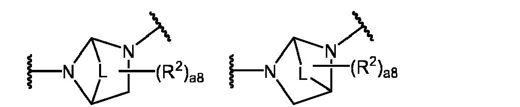
wherein each R² is independently selected from: H, halogen, cyano, nitro, hydroxyl, amino, C₁₋₁₅ alkyl optionally substituted with halogen, C₃₋₁₅ cycloalkyl, - COOR', -CONR' R", -OR', or -NR'R";
R' and R" are independently selected from: H, C₁₋₆ alkyl optionally substituted with halogen, or C₃₋₆ cycloalkyl;
each a1 is independently selected from: 0, 1, 2, 3, 4, 5, 6, 7, 8, and 9, each a2 is independently selected from: 0, 1, 2, 3, 4, 5, 6, 7, and 8, each a3 is independently selected from: 0, 1, 2, 3, 4, 5, 6, and 7, each a4 is independently selected from: 0, 1, 2, 3, 4, 5, and 6, each a5 is independently selected from: 0, 1, 2, 3, 4, and 5, each a6 is independently selected from: 0, 1, 2, 3, 4, 5, 6, 7, 8, 9, 10, and 11, each a7 is independently selected from: 0, 1, 2, 3, 4, 5, 6, 7, 8, 9, and 10, and each a8 is independently selected from 0, 1, 2, 3, and 4;
each R is independently selected from: O, S, or NR³, wherein R³ is selected from: H, C₁₋₆ alkyl, or -COC₁₋₆ alkyl;
each L is independently selected from: -CH₂-, -CH₂CH₂-, -C(CH₃)₂-, or - C(CH₃)₂CH₂-;
Cy¹ is selected from optionally substituted phenyl, naphthyl, anthracyl, phenanthrenyl, furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyrimidinyl, pyridazinyl, pyrazinyl, triazinyl, indolyl, isoindolyl, benzoimidazolyl, benzopyrazolyl, benzofuranyl, benzothienyl, benzoxazolyl, benzothiazolyl, quinolinyl, isoquinolinyl, indolizinyl, or azaindolizinyl;
Cy² is selected from H, and optionally substituted phenyl, naphthyl, anthracyl, phenanthrenyl, furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyrimidinyl, pyridazinyl, pyrazinyl, triazinyl, indolyl, isoindolyl, benzoimidazolyl, benzopyrazolyl, benzofuranyl, benzothienyl, benzoxazolyl, benzothiazolyl, quinolinyl, isoquinolinyl, indolizinyl, azaindolizinyl, or carbazolyl;
the "optionally substituted" refers to being unsubstituted or mono- or poly-substituted with a group selected from the following: halogen, hydroxyl, thiol, carboxyl, cyano, nitro, amino, C₁₋₆ alkyl optionally substituted with halogen, C₁₋₆ alkoxy optionally substituted with halogen, C₁₋₆ alkylthio optionally substituted with halogen, C₃₋₆ cycloalkyl, C₆₋₁₄ aryl, C₆₋₁₄ arylC₁₋₂ alkyl-, 5- to 7-membered heteroaryl, 5- to 7-membered heterocyclyl, C₁₋₆ ester group, C₁₋₆ acyl, C₁₋₆ alkylamino, di(C₁₋₆ alkyl)amino, or amido substituted with 0, 1 or 2 C₁₋₆ alkyl, wherein the aryl, heteroaryl, and heterocyclyl, alone or as part of a group, can be optionally substituted with a group selected from halogen, hydroxyl, thiol, carboxyl, cyano, nitro, C₁₋₆ alkyl optionally substituted with halogen, C₁₋₆ alkoxy optionally substituted with halogen, or C₃₋₆ cycloalkyl.

29. The compound or the pharmaceutically acceptable salt, solvate, hydrate, stereoisomer, tautomer, or prodrug thereof of any one of claims 3 and 26-28, **characterized in that**,
W is selected from: CH₂, or C=O;
G is selected from: H, or C₁₋₆ alkyl, wherein the C₁₋₆ alkyl is substituted with a group selected from the following: -OPO(OR^{G})₂, wherein each R^{G} is independently selected from: H;
T₁-T₃ are independently selected from: O;
A₂-A₃ are independently selected from: CH₂;
R^{A} is selected from: H;
Q₁-Q₄ are independently selected from: CR^{Q} or N;
R^{Q} is selected from: H, or halogen;
L₁ is selected from: -O-, -S-, -N(R¹)-, -CO-, -CO-O-, -O-CO-, -O-CO-O-, - CO-N (R¹)-, -N(R¹)-CO-, -N(R¹)-CO-N(R¹)-, -CO-N(R¹)-CO and optionally substituted C₁₋₁₅ alkylene-, where the head, tail or middle of the alkylene is optionally inserted by 1, 2, 3, 4, 5 or more groups selected from: -O-, -S-, -N(R¹)-, -CO-, -CO-O-, -O-CO-, -O-CO-O-, -CO-N (R¹)-, -N(R¹)-CO-, -N(R¹)-CO-N(R¹)-, -CO-N(R¹)-CO or any combination thereof; wherein each R¹ is independently selected from: H, or C₁₋₆ alkyl optionally substituted with halogen; 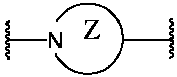 is selected from one of the following structures: 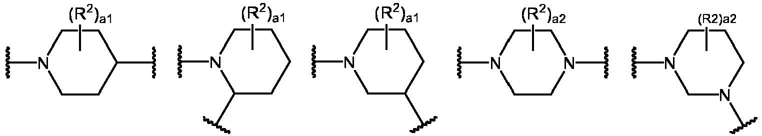 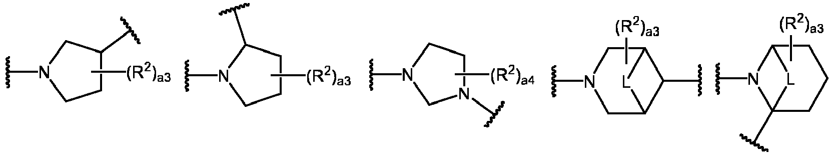
wherein each R² is independently selected from: H, halogen, cyano, nitro, hydroxyl, C₁₋₆ alkyl optionally substituted with halogen, or C₁₋₆ alkoxy optionally substituted with halogen;
each a1 is independently selected from: 0, 1, 2, 3, 4, 5, 6, 7, 8, and 9, each a2 is independently selected from: 0, 1, 2, 3, 4, 5, 6, 7, and 8, each a3 is independently selected from: 0, 1, 2, 3, 4, 5, 6, and 7, each a4 is independently selected from: 0, 1, 2, 3, 4, 5, and 6;
each L is independently selected from: -CH₂-, or -CH₂CH₂-;
Cy¹ is selected from optionally substituted phenyl, naphthyl, anthracyl, phenanthrenyl, furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyrimidinyl, pyridazinyl, pyrazinyl, or triazinyl;
Cy² is selected from H, and optionally substituted phenyl, naphthyl, anthracyl, phenanthrenyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyrimidinyl, pyridazinyl, pyrazinyl, triazinyl, or carbazol-9-yl;
the "optionally substituted" refers to being unsubstituted or mono- or poly-substituted with a group selected from the following: halogen, hydroxyl, cyano, nitro, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, cyclohexyl, phenyl, naphthyl, pyridyl, pyrimidinyl, -COOMe, - COOEt, -COMe, -COEt, amino, methylamino, dimethylamino, ethylamino, diethylamino, -CONH₂, -CONHMe, -CONHEt, -CON(Me)₂, or -CON(Et)₂.

30. The compound or the pharmaceutically acceptable salt, solvate, hydrate, stereoisomer, tautomer, or prodrug thereof of any one of claims 3 and 26-29, **characterized in that**,
W is selected from: CH₂, or C=O;
G is selected from: H;
T₁-T₃ are independently selected from: O;
A₂-A₃ are independently selected from: CH₂;
R^{A} is selected from: H;
Q₁-Q₄ are independently selected from: CR^{Q};
R^{Q} is selected from: H, or halogen;
L₁ is selected from a group represented by the structure of the following general formula:
-(CHR¹)ₖ-;
-(CHR¹)ₘ-O-(CHR¹)ₙ-;
-(CHR¹)ₘ-S-(CHR¹)ₙ-;
-(CHR¹)ₘ-N(R¹)-(CHR¹)ₙ-;
-(CHR¹)ₘ-CO-(CHR¹)ₙ-;
-(CHR¹)ₘ-CO-O-(CHR¹)ₙ-;
-(CHR¹)ₘ-O-CO-(CHR¹)ₙ-;
-(CHR¹)ₘ-O-CO-O-(CHR¹)ₙ-;
-(CHR¹)ₘ-CO-N(R¹)-(CHR¹)ₙ-;
-(CHR¹)ₘ-N(R¹)-CO-(CHR¹)ₙ-;
-(CHR¹)ₘ-CO-N(R¹)-(CHR¹)ₙ-;
-(CHR¹)ₘ-N(R¹)-CO-N(R¹)-(CHR¹)ₙ-;
-(CHR¹)ₘ-CO-N(R¹)-CO-(CHR¹)ₙ-;
-(CHR¹)ₘ-O-(CHR¹)ₗ-O-(CHR¹)ₙ-;
-(CHR¹)ₘ-O-(CHR¹)ₗ-N(R¹)-(CHR¹)ₙ-;
-(CHR¹)ₘ-N(R¹)-(CHR¹)ₗ-O-(CHR¹)ₙ-;
-(CHR¹)ₘ-O-(CHR¹)ₗ-CO-(CHR¹)ₙ-;
-(CHR¹)ₘ-CO-(CHR¹)ₗ-O-(CHR¹)ₙ-;
-(CHR¹)ₘ-O-(CHR¹)ₗ-CO-N(R¹)-(CHR¹)ₙ-;
-(CHR¹)ₘ-CO-N(R¹)-(CHR¹)ₗ-O-(CHR¹)ₙ-;
-(CHR¹)ₘ-O-(CHR¹)ₗ-N(R¹)-CO-(CHR¹)ₙ-;
-(CHR¹)ₘ₋N(R¹)-CO-(CHR¹)ₗ-O-(CHR¹)ₙ-;
-(CHR¹)ₘ-N(R¹)-(CHR¹)ₗ-N(R¹)-(CHR¹)ₙ-;
-(CHR¹)ₘ-N(R¹)-(CHR¹)ₗ-CO-(CHR¹)ₙ-;
-(CHR¹)ₘ-CO-(CHR¹)ₗ-N(R¹)-(CHR¹)ₙ-;
-(CHR¹)ₘ-N(R¹)-(CHR¹)ₗ-CO-N(R¹)-(CHR¹)ₙ-;
-(CHR¹)ₘ-CO-N(R¹)-(CHR¹)ₗ-N(R¹)-(CHR¹)ₙ-;
-(CHR¹)ₘ-N(R¹)-(CHR¹)ₗ-N(R¹)-CO-(CHR¹)ₙ-;
-(CHR¹)ₘ-N(R¹)-CO-(CHR¹)ₗ-N(R¹)-(CHR¹)ₙ-;
-(CHR¹)ₘ-CO-(CHR¹)ₗ-CO-N(R¹)-(CHR¹)ₙ-;
-(CHR¹)ₘ-CO-N(R¹)-(CHR¹)ₗ-CO-(CHR¹)ₙ-;
-(CHR¹)ₘ-CO-(CHR¹)ₗ-N(R¹)-CO-(CHR¹)ₙ-;
-(CHR¹)ₘ-N(R¹)-CO-(CHR¹)ₗ-CO-(CHR¹)ₙ-;
-(CHR¹)ₘ-N(R¹)-CO-(CHR¹)ₗ-CO-N(R¹)-(CHR¹)ₙ-;
-(CHR¹)ₘ-N(R¹)-CO-(CHR¹)ₗ-N(R¹)-CO-(CHR¹)ₙ-;
-(CHR¹)ₘ-CO-N(R¹)-(CHR¹)ₗ-CO-N(R¹)-(CHR¹)ₙ-;
-(CHR¹)ₘ-CO-N(R¹)-(CHR¹)ₗ-N(R¹)-CO-(CHR¹)ₙ-;
-(CHR¹)ₘ-C₆₋₁₀arylene-CO-O-(CHR¹)ₙ-;
-(CHR¹)ₘ-C₆₋₁₀arylene-O-CO-(CHR¹)ₙ-;
-(CHR¹)ₘ-CO-O-C₆₋₁₀arylene-(CHR¹)ₙ-;
-(CHR¹)ₘ-O-CO-C₆₋₁₀arylene-(CHR¹)ₙ-;
-(CHR¹)ₘ-C₆₋₁₀arylene-N(R¹)-CO-(CHR¹)ₙ-;
-(CHR¹)ₘ-C₆₋₁₀arylene-CO-N(R¹)-(CHR¹)ₙ-;
-(CHR¹)ₘ-N(R¹)-CO-C₆₋₁₀arylene-(CHR¹)ₙ-;
-(CHR¹)ₘ-CO-N(R¹)-C₆₋₁₀arylene-(CHR¹)ₙ-;
wherein k is selected from: 0, 1, 2, 3, 4, 5, 6, 7, 8, 9, 10, 11, and 12, m and n are independently selected from: 0, 1, 2, 3, 4, 5, and 6, 1 is selected from 1, 2, 3, 4, 5, and 6, and each R¹ is independently selected from: H, or C₁₋₆ alkyl; 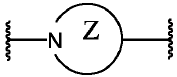 is selected from one of the following structures: 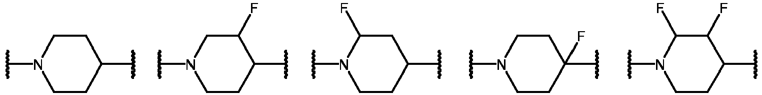 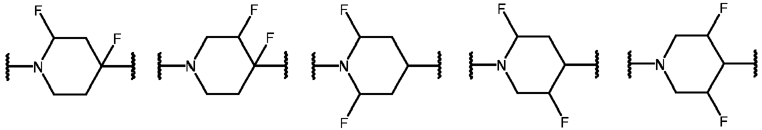 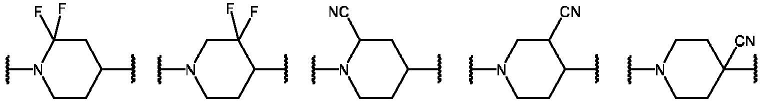 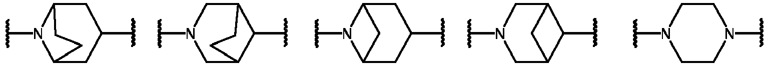
Cy¹ is selected from one of the following structures: 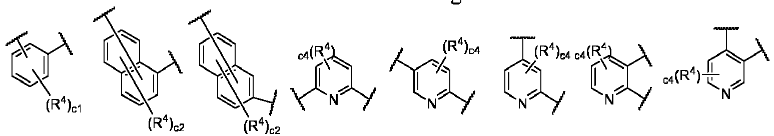 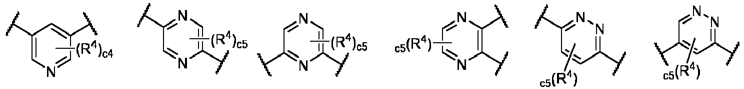 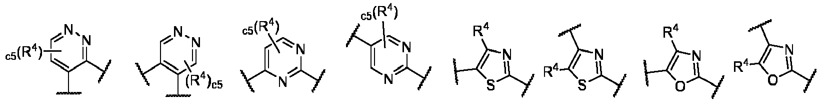
each R⁴ is independently selected from: halogen, hydroxyl, cyano, nitro, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, cyclohexyl, phenyl, naphthyl, pyridyl, pyrimidinyl, -COOMe, - COOEt, -COMe, -COEt, amino, methylamino, dimethylamino, ethylamino, diethylamino, -CONH₂, -CONHMe, -CONHEt, -CON(Me)₂, or -CON(Et)₂;
each c1 is independently selected from 0, 1, 2, 3, and 4, each c2 is independently selected from 0, 1, 2, 3, 4, 5, and 6, each c3 is independently selected from 0, 1, 2, 3, 4, 5, 6, 7, 8, and 9, each c4 is independently selected from 0, 1, 2, and 3, and each c5 is independently selected from 0, 1, and 2;
Cy² is selected from H and one of the following structures: 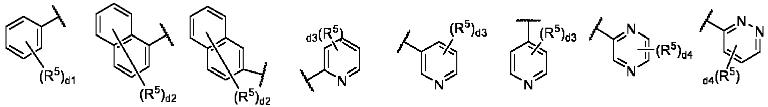 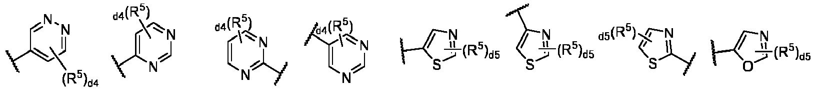 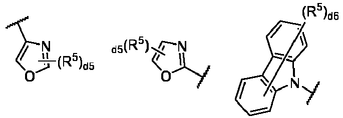
each R⁵ is independently selected from: halogen, hydroxyl, cyano, nitro, methyl, ethyl, n-propyl, isopropyl, n-butyl, isobutyl, sec-butyl, tert-butyl, pentyl, hexyl, CH₂F, CHF₂, CF₃, methoxy, ethoxy, CF₃O-, cyclopropyl, cyclobutyl, cyclopentyl, cyclohexyl, phenyl, naphthyl, pyridyl, pyrimidinyl, -COOMe, - COOEt, -COMe, -COEt, amino, methylamino, dimethylamino, ethylamino, diethylamino, -CONH₂, -CONHMe, -CONHEt, -CON(Me)₂, or -CON(Et)₂;
each d1 is independently selected from 0, 1, 2, 3, 4, and 5, each d2 is independently selected from 0, 1, 2, 3, 4, 5, 6, and 7, each d3 is independently selected from 0, 1, 2, 3, and 4, each d4 is independently selected from 0, 1, 2, and 3, each d5 is independently selected from 0, 1, and 2, each d6 is independently selected from 0, 1, 2, 3, 4, 5, 6, 7, and 8.

31. The compound of formula (Ia) or the pharmaceutically acceptable salt, solvate, hydrate, stereoisomer, tautomer, or prodrug thereof of any one of claims 3 and 26-30, **characterized in that**,
W is selected from: CH₂, or C=O;
G is selected from: H;
T₁-T₃ are independently selected from: O;
A₂-A₃ are independently selected from: CH₂;
R^{A} is selected from: H;
Q₁-Q₄ are independently selected from: CH;
L₁ is selected from a group represented by the structure of the following general formula:
-(CHR¹)ₖ-;
-O-; -(CHR¹)ₘ-O-; -O-(CHR¹)ₙ-; -(CHR¹)ₘ-O-(CHR¹)ₙ-;
-N(R¹)-; -(CHR¹)ₘ-N(R¹)-; -N(R¹)-(CHR¹)ₙ-; -(CHR¹)ₘ-N(R¹)-(CHR¹)ₙ-;
-CO-; -(CHR¹)ₘ-CO-; -CO-(CHR¹)ₙ-; -(CHR¹)ₘ-CO-(CHR¹)ₙ-;
-CO-N(R')-; -(CHR¹)ₘ-CO-N(R¹)-; -CO-N(R¹)-(CHR¹)ₙ-; -(CHR¹)ₘ-CO-N(R¹)-(CHR¹)ₙ-;
-N(R¹)-CO-; -(CHR¹)ₘ-N(R¹)-CO-; -N(R¹)-CO-(CHR¹)ₙ-; -(CHR¹)ₘ-N(R¹)-CO-(CHR¹)ₙ-;
-CO-O-; -(CHR¹)ₘ-CO-O; -CO-O-(CHR¹)ₙ-; -(CHR¹)ₘ-CO-O-(CHR¹)ₙ-;
-O-CO-; -(CHR¹)ₘ-O-CO-; -O-CO-(CHR¹)ₙ-; -(CHR¹)ₘ-O-CO-(CHR¹)ₙ-;
-(CHR¹)ₘ-C₆arylene-N(R¹)-CO-(CHR¹)ₙ-;
-(CHR¹)ₘ-C₆arylene-CO-N(R¹)-(CHR¹)ₙ-;
wherein k, m and n are independently selected from: 1, 2, 3, 4, 5, and 6, each R¹ is independently selected from: H, methyl, or ethyl; 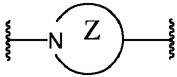 is selected from one of the following structures: 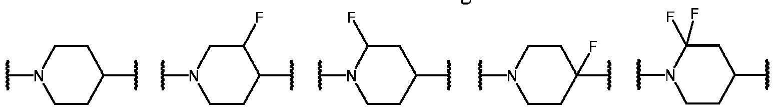 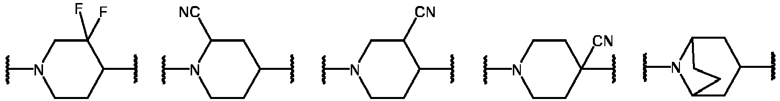 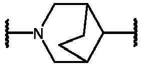 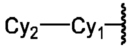
is selected from one of the following structures:

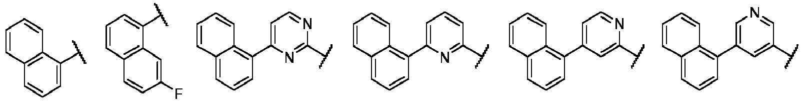

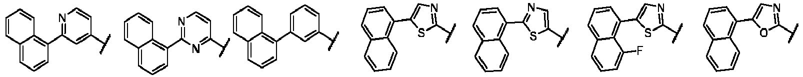

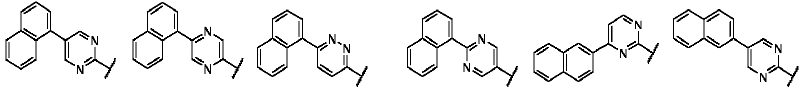

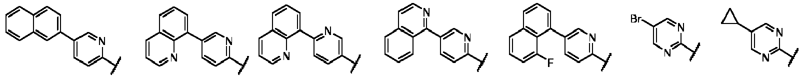

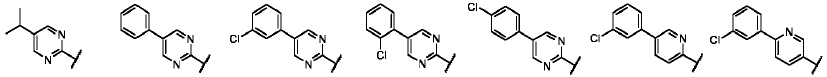

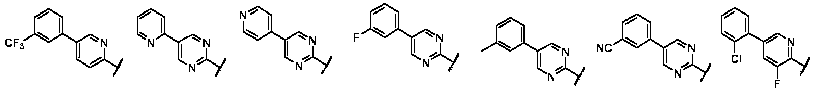

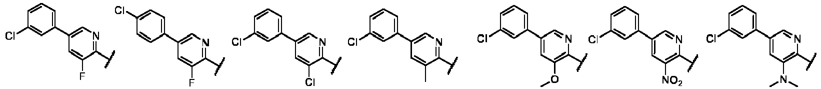

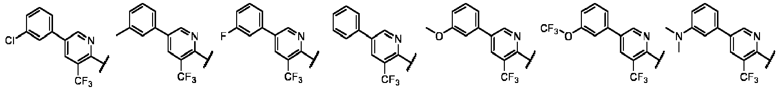

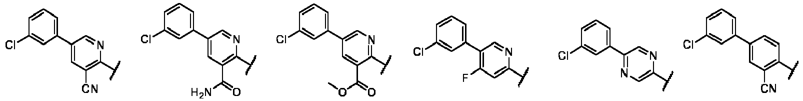

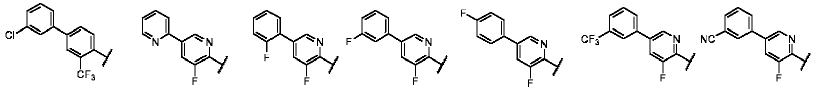

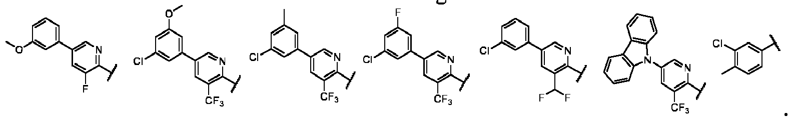

32. The compound or the pharmaceutically acceptable salt, solvate, hydrate, stereoisomer, tautomer, or prodrug thereof of any one of claims 1 to 31, **characterized in that**, the compound is selected from:
| | | | |
|---|---|---|---|
| A1 | 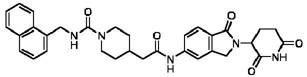 | A90 | 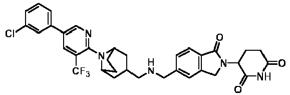 |
| A2 | 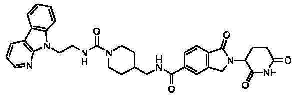 | A91 | 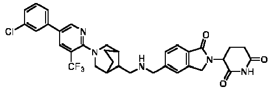 |
| A3 | 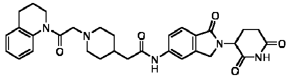 | A92 | 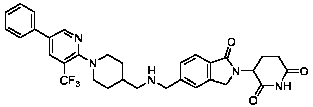 |
| A4 | 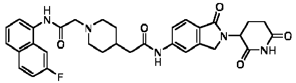 | A93 | 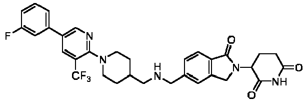 |
| A5 | 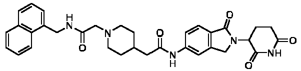 | A94 | 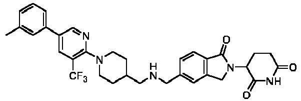 |
| A6 | 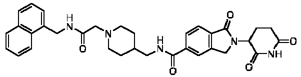 | A95 | 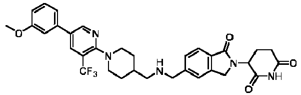 |
| A7 | 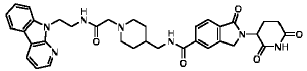 | A96 | 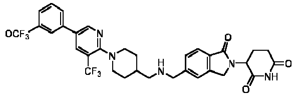 |
| A8 | 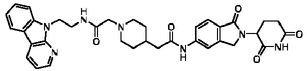 | A97 | 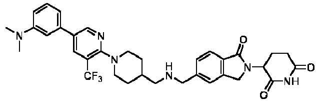 |
| A9 | 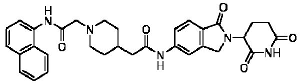 | A98 | 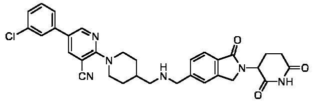 |
| A10 | 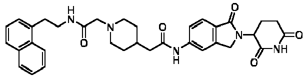 | A99 | 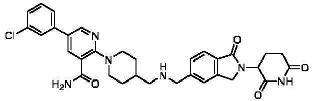 |
| A11 | 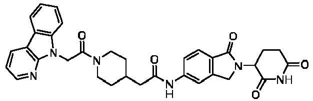 | A100 | 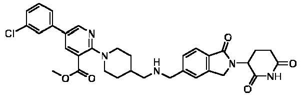 |
| A12 | 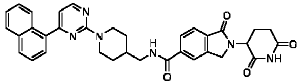 | A101 | 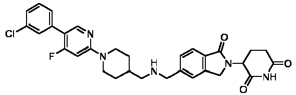 |
| A13 | 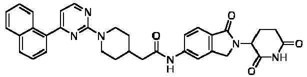 | A102 | 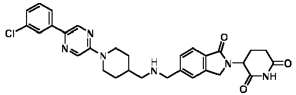 |
| A14 | 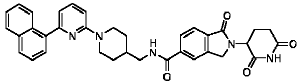 | A103 | 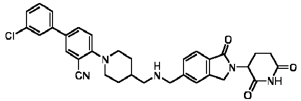 |
| A15 | 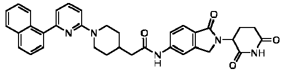 | A104 | 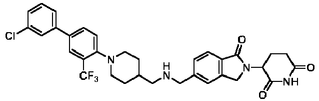 |
| A16 | 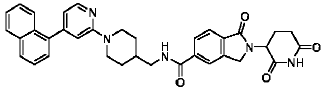 | A105 | 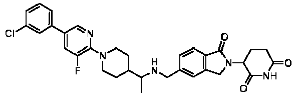 |
| A17 | 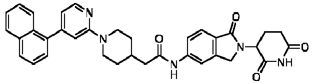 | A106 | 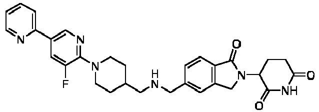 |
| A18 | 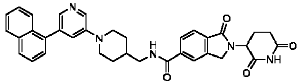 | A107 | 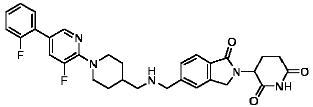 |
| A19 | 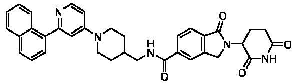 | A108 | 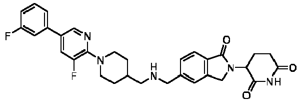 |
| A20 | 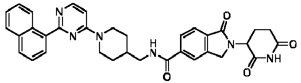 | A109 | 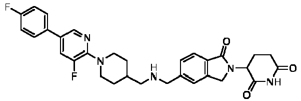 |
| A21 | 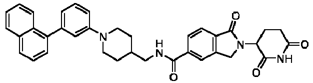 | A110 | 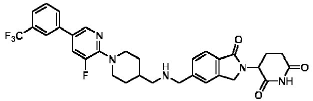 |
| A22 | 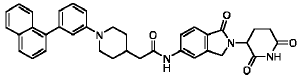 | A111 | 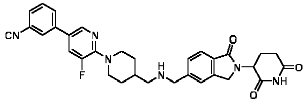 |
| A23 | 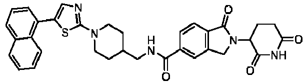 | A112 | 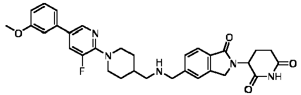 |
| A24 | 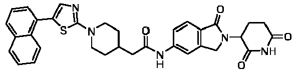 | A113 | 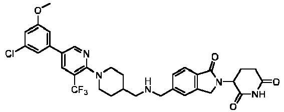 |
| A25 | 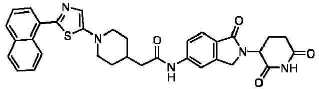 | A114 | 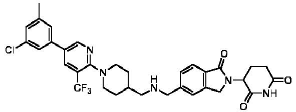 |
| A26 | 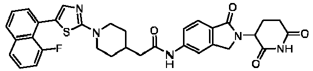 | A115 | 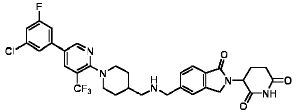 |
| A27 | 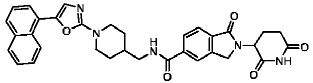 | A116 | 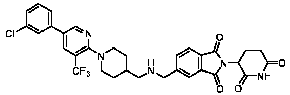 |
| A28 | 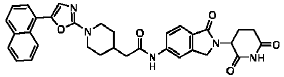 | A117 | 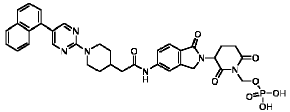 |
| A29 | 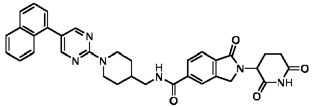 | A118 | 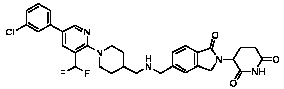 |
| A30 | 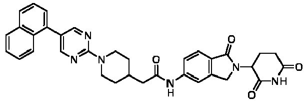 | A119 | 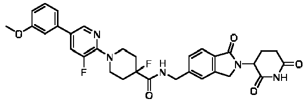 |
| A31 | 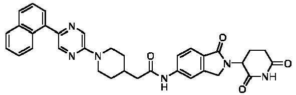 | A120 | 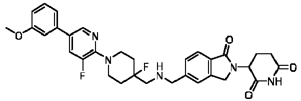 |
| A32 | 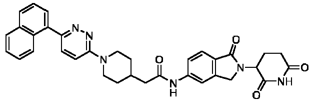 | A121 | 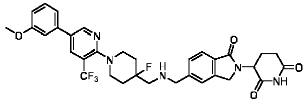 |
| A33 | 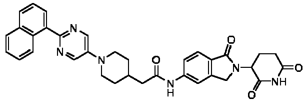 | A122 | 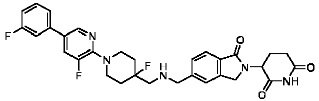 |
| A34 | 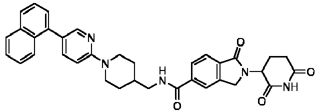 | A123 | 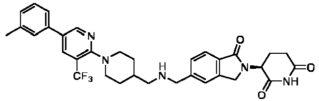 |
| A35 | 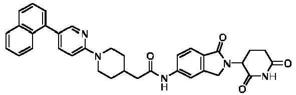 | A124 | 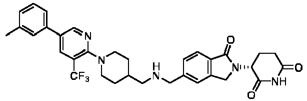 |
| A36 | 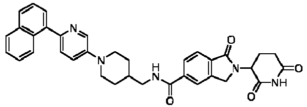 | A125 | 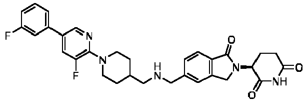 |
| A37 | 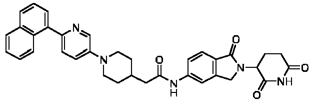 | A126 | 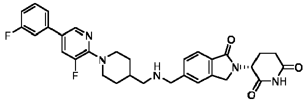 |
| A38 | 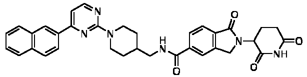 | A127 | 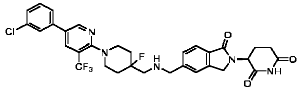 |
| A39 | 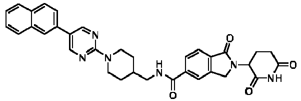 | A128 | 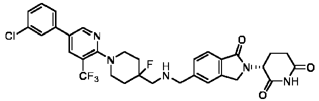 |
| A40 | 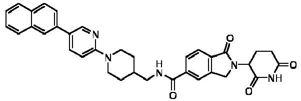 | A129 | 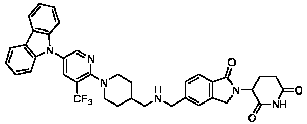 |
| A41 | 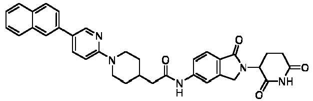 | A130 | 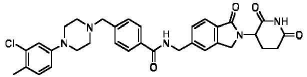 |
| A42 | 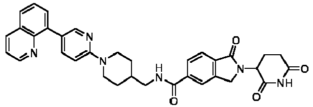 | A131 | 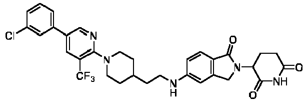 |
| A43 | 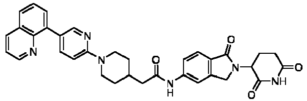 | A132 | 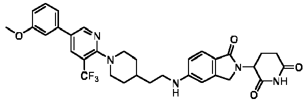 |
| A44 | 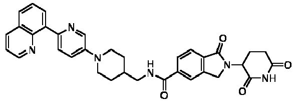 | A133 | 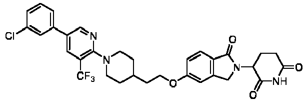 |
| A45 | 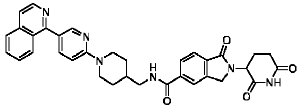 | A134 | 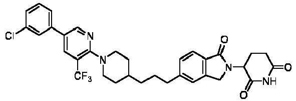 |
| A46 | 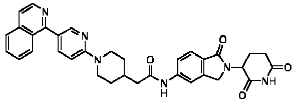 | A135 | 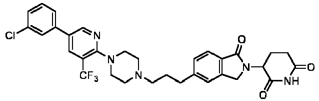 |
| A47 | 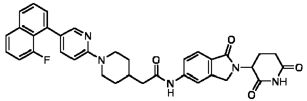 | A136 | 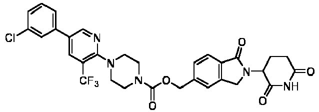 |
| A48 | 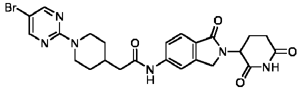 | A137 | 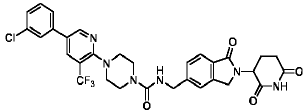 |
| A49 | 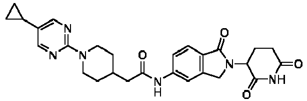 | A138 | 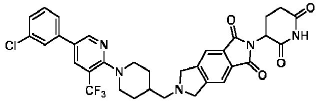 |
| A50 | 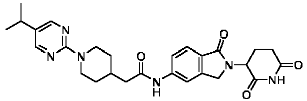 | A139 | 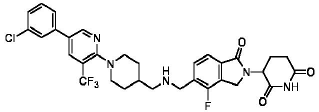 |
| A51 | 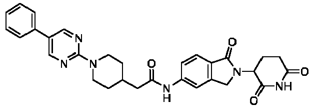 | A140 | 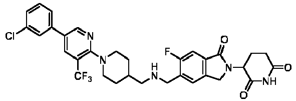 |
| A52 | 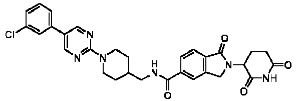 | A141 | 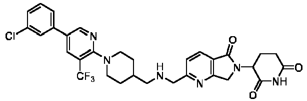 |
| A53 | 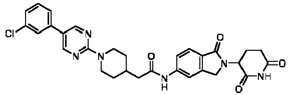 | A142 | 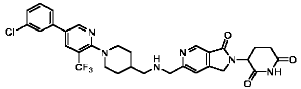 |
| A54 | 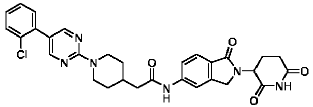 | A143 | 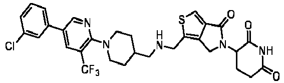 |
| A55 | 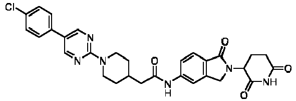 | A144 | 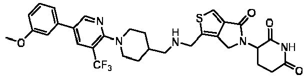 |
| A56 | 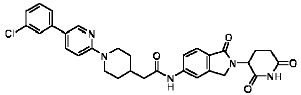 | A145 | 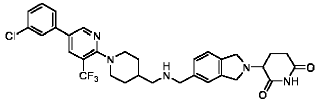 |
| A57 | 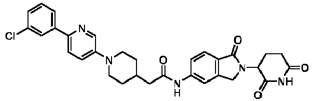 | A146 | 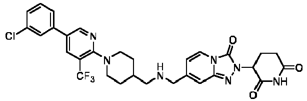 |
| A58 | 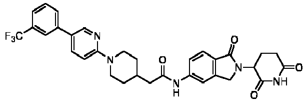 | A147 | 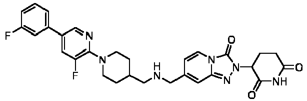 |
| A59 | 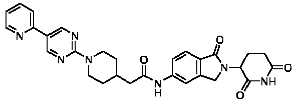 | A148 | 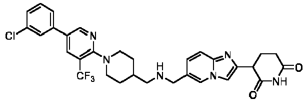 |
| A60 | 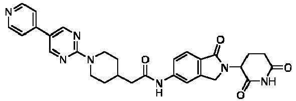 | A149 | 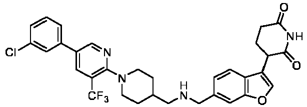 |
| A61 | 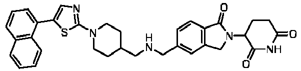 | A150 | 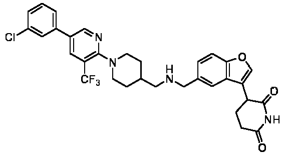 |
| A62 | 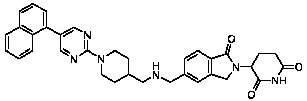 | A151 | 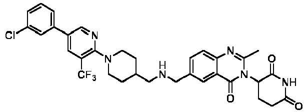 |
| A63 | 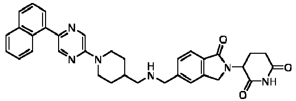 | A152 | 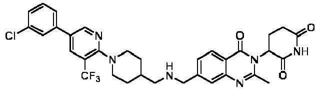 |
| A64 | 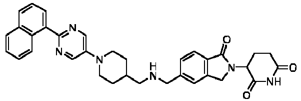 | A153 | 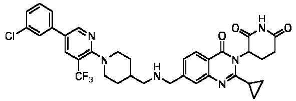 |
| A65 | 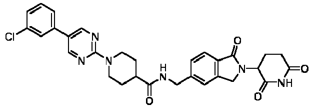 | A154 | 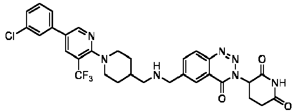 |
| A66 | 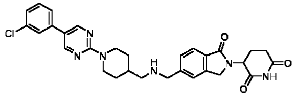 | A155 | 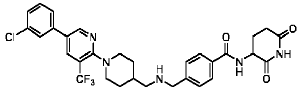 |
| A67 | 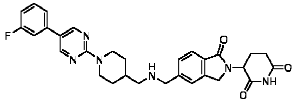 | A156 | 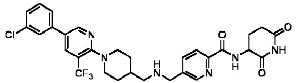 |
| A68 | 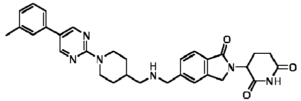 | A157 | 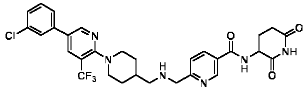 |
| A69 | 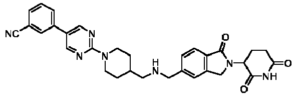 | A158 | 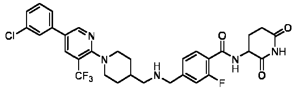 |
| A70 | 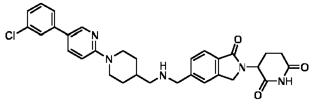 | A159 | 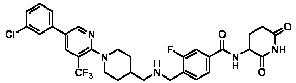 |
| A71 | 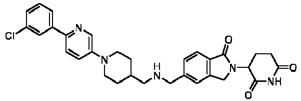 | A160 | 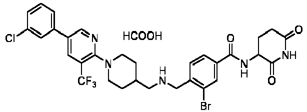 |
| A72 | 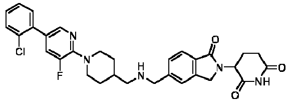 | A161 | 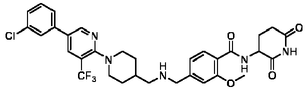 |
| A73 | 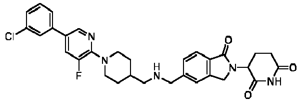 | A162 | 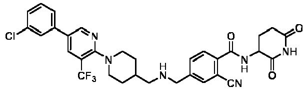 |
| A74 | 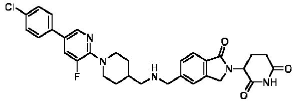 | A163 | 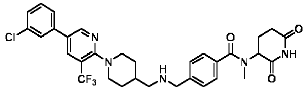 |
| A75 | 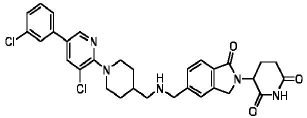 | A164 | 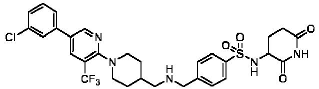 |
| A76 | 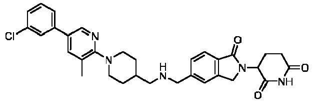 | A165 | 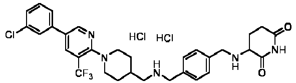 |
| A77 | 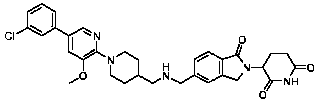 | A166 | 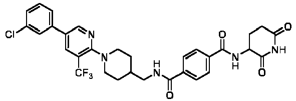 |
| A78 | 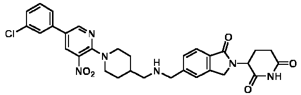 | A167 | 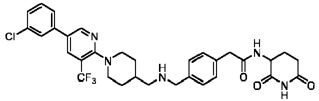 |
| A79 | 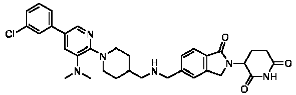 | A168 | 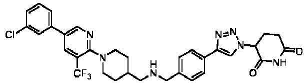 |
| A80 | 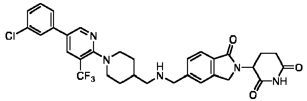 | A169 | 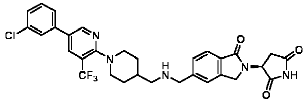 |
| A81 | 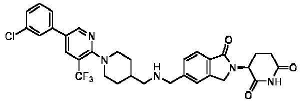 | A170 | 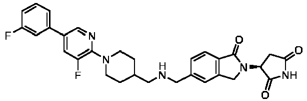 |
| A82 | 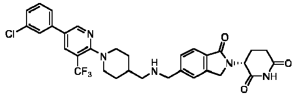 | A171 | 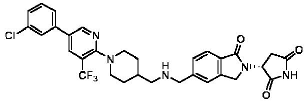 |
| A83 | 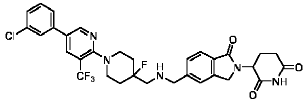 | A172 | 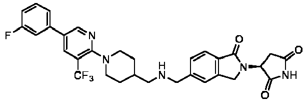 |
| A84 | 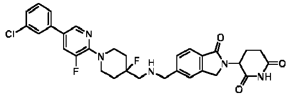 | A173 | 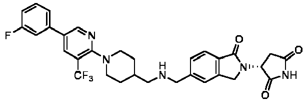 |
| A85 | 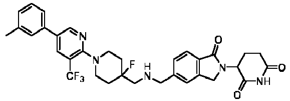 | A174 | 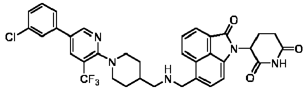 |
| A86 | 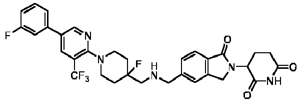 | A175 | 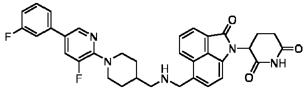 |
| A87 | 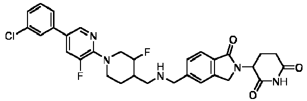 | A176 | 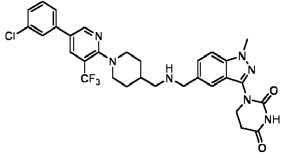 |
| A88 | 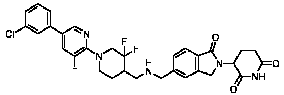 | A177 | 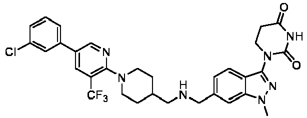 |
| A89 | 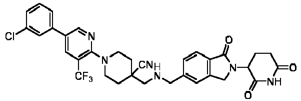 | | |

33. A pharmaceutical composition, **characterized in that** the pharmaceutical composition comprises an effective amount of the compound or the pharmaceutically acceptable salt, solvate, hydrate, stereoisomer, tautomer, or prodrug thereof of any one of claims 1 to 32 and a pharmaceutically acceptable carrier and/or an excipient.

34. The pharmaceutical composition of claim 33, **characterized in that** the pharmaceutical composition further comprises at least one additional bioactive agent.

35. The compound or the pharmaceutically acceptable salt, solvate, hydrate, stereoisomer, tautomer, or prodrug thereof of any one of claims 1 to 32 for use in the preparation of a protein degradation agent.

36. The compound or the pharmaceutically acceptable salt, solvate, hydrate, stereoisomer, tautomer, or prodrug thereof of any one of claims 1 to 32 for use in the preparation of a drug, **characterized in that** the drug is used to prevent or treat a disease or a disorder associated with protein degradation.

37. A method of treating a disease or a disorder in a subject, **characterized in that** the method comprises the steps of administering the compound or the pharmaceutically acceptable salt, solvate, hydrate, stereoisomer, tautomer, or prodrug thereof of any one of claims 1 to 32 to the subject in need thereof; preferably, the disease or the disorder is associated with protein degradation.

38. The use or method of any one of claims 35 to 37, **characterized in that** the protein in the protein degradation is any one or more of c-Myc, GSPT1, CK1α and IKZF (1/2/3) proteins.

39. The use or method of any one of claims 35 to 38, **characterized in that** the disease or the disorder that can be treated by the protein degradation agent, or the disease or the disorder that is associated with protein degradation, or the disease or the disorder of the subject is selected from: a cancer, a cardiovascular and cerebrovascular disease, a viral infection-related disease, or an immune disease;
the cancer preferably comprises: leukemia, lymphoma, malignant glioma, medulloblastoma, melanoma, multiple myeloma, malignant glioma, osteosarcoma, liver cancer, lung cancer, kidney cancer, pancreatic cancer, oral cancer, gastric cancer, esophageal cancer, laryngeal cancer, nasopharyngeal cancer, skin cancer, breast cancer, colon cancer, rectal cancer, cervical cancer, bladder cancer, ovarian cancer, prostate cancer, rhabdomyosarcoma, osteoblastic sarcoma, chondrosarcoma and small cell lung cancer;
the viral infection-related disease preferably comprises: HIV, hepatitis B virus (HBV), hepatitis B, hepatitis C, hepatitis A, influenza, epidemic encephalitis B and herpes;
the immune disease preferably comprises: systemic lupus erythematosus, rheumatoid arthritis, scleroderma, hyperthyroidism, juvenile diabetes, idiopathic thrombocytopenic purpura, autoimmune hemolytic anemia, ulcerative colitis, and chronic liver disease.

40. The compound or the pharmaceutically acceptable salt, solvate, hydrate, stereoisomer, tautomer, or prodrug thereof of any one of claims 1 to 32 for use in the preparation of a proteolysis-targeting chimera (Protac) or an antibody-drug conjugate (ADC).
